# EUROPEAN PATENT SPECIFICATION

(11) **EP 2 977 378 B1**
(45) Date of publication and mention of the grant of the patent: **10.06.2020**
(21) Application number: 15177484.1
(22) Date of filing: 20.07.2015
(51) Int. Cl.: C07F 7/08, C09K 11/00, H01L 51/50, H05B 33/14, C09K 11/06, H01L 51/00

(54) **CONDENSED CYCLIC COMPOUND AND ORGANIC LIGHT-EMITTING DEVICE INCLUDING THE SAME**
KONDENSIERTE CYCLISCHE VERBINDUNG UND ORGANISCHE LICHTEMITTIERENDE VORRICHTUNG DAMIT
COMPOSÉ CYCLIQUE CONDENSÉ ET DISPOSITIF ÉLECTROLUMINESCENT ORGANIQUE COMPRENANT CELUI-CI

(30) Priority: 23.07.2014 KR 20140093540
(43) Date of publication of application: 27.01.2016
(73) Proprietor: Samsung Electronics Co., Ltd., Gyeonggi-do 443-742 (KR); Samsung SDI Co., Ltd., Yongin-si, Gyeonggi-do (KR)
(72) Inventor: JUNG, Yongsik, 443-803 Gyeonggi-do (KR); SON, Jhunmo, 443-803 Gyeonggi-do (KR); KIM, Sangmo, 443-803 Gyeonggi-do (KR); KIM, Hyunjung, 443-803 Gyeonggi-do (KR); JEON, Soonok, 443-803 Gyeonggi-do (KR); CHUNG, Yeonsook, 443-803 Gyeonggi-do (KR); HUH, Dalho, 443-803 Gyeonggi-do (KR)
(74) Representative: Elkington and Fife LLP

(56) References cited:
- EP-A1- 2 475 020
- EP-A1- 2 757 608
- WO-A1-2010/079051
- CN-A- 102 417 519
- JP-A- 2009 224 762
- JP-A- 2012 146 807
- US-A1- 2011 278 552
- US-A1- 2013 175 507
- DATABASE CA [Online] CHEMICAL ABSTRACTS SERVICE, COLUMBUS, OHIO, US; April 2014 (2014-04), OGAWA, JUNYA ET AL: "Organic electroluminescent element material comprising indolocarbazole compound and organic electroluminescent element using same", XP002751268, retrieved from STN Database accession no. 2014:531931 -& WO 2014/050588 A1 (NIPPON STEEL & SUMIKIN CHEM CO [JP]) 3 April 2014 (2014-04-03)
- DATABASE CA [Online] CHEMICAL ABSTRACTS SERVICE, COLUMBUS, OHIO, US; 2010, FUKUZAKI, EIJI: "Organic electroluminescence devices provided with electroluminescent pyridoindolyl biphenyl derivatives", XP002751269, retrieved from STN Database accession no. 2010:1301642 -& JP 4 564585 B1 (FUJIFILM CORP) 20 October 2010 (2010-10-20)
- DATABASE CA [Online] CHEMICAL ABSTRACTS SERVICE, COLUMBUS, OHIO, US; 2009, KOGUCHI, RYOHEI ET AL: "Appropriately Conjugated Poly[oligo(N-phenyl-2,7-carbazolylene)-alt - diphenylsilylene]s", XP002751270, retrieved from STN Database accession no. 2009:805635

## Description

### FIELD OF THE INVENTION

The present disclosure relates to a condensed cyclic compound and an organic light-emitting device including the same.

### BACKGROUND OF THE INVENTION

Organic light-emitting devices (OLEDs) are self-emission devices that have wide viewing angles, high contrast ratios, short response times. OLEDs also have excellent brightness, driving voltage, and response speed characteristics, and produce full-color images.

A typical organic light-emitting device includes an anode, a cathode, and an organic layer that is disposed between the anode and the cathode and includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. Carriers, such as holes and electrons, are recombined in the emission layer to produce excitons. These excitons change from an excited state to a ground state, thereby generating light.

Various types of organic light emitting devices are known. However, there still remains a need in OLEDs having low driving voltage, high efficiency, high brightness, and long lifespan.

JP 2009 224 762 aims to provide an organic electroluminescent element with reduced variations of luminescent chromaticity, favourable luminance efficiency, and low driving voltage.

EP 2 475 020 discloses an organic electroluminescence device including at least one organic layer including a light emitting layer containing a light emitting material between a pair of electrodes, in which the organic electroluminescence device contains a compound having at least one specific group and a phosphorescent metal complex having a specific structure.

CN 102 417 519 relates to the technical field of organic luminescent materials and preparation methods for the organic luminescent materials, particularly relates to functionalized silanes in which a silicon atom is taken as the centre and which contain carbazole or other nitrogen heterocycles, and a preparation method for the functionalized silanes, and provides organosilicon luminescent materials which contain two kinds or more than two kinds of functional groups and have high thermal stability.

EP 2 757 608 discloses an organic electroluminescent device which includes an organic layer including a light-emitting layer between an anode and cathode laminated on a substrate, and at least one layer of the organic layer contains a carbazole compound.

US 2011/278552 discloses a material for an organic electroluminescence device having a specific structure in which a dibennzofuranyl group or a dibenzothiophenyl group is bonded at an N-position (9-position) of a carbazolyl group and an organic electroluminescence device which is provided with one or more organic thin film layers including a light emitting layer between a cathode and an anode and in which at least one layer of the organic thin film layers described above contains the material for an organic electroluminescence device.

US 2013/175507 discloses heterocyclic materials which contain a fused tetracyclic structure that can be used in OLED devices.

JP 2012 146807 aims to provide an organic electroluminescent element having high efficiency and long life in which dark spots are reduced, and to provide a display device and a luminaire using the same.

WO 2014/050588 discloses an organic EL element. The organic EL element has a light-emitting layer between a positive electrode and a negative electrode stacked on a substrate, and the light-emitting layer contains, as a host material, an organic EL element material comprising an indolocarbazole compound that has a phosphorescent dopant and a silyl group. The organic EL element material is an indolocarbazole compound having a structure in which the silyl group is replaced by a nitrogen atom of an indolocarbazole ring with a nitrogen-containing aromatic heterocyclic compound interposed therebetween.

JP 4 564585 aims to provide an organic electroluminescent element which has high durability, high efficiency and low driving voltage, and is suppressed in colour shift after deterioration of the element.

In R. Koguchi et al., Macromolecules, vol. 42, issue 16, pp. 5946-5952, a series of poly[oligo(2,7-carbazolylene)-alt-diphenylsilylene]s were synthesized by Suzuki couplings.

WO 2010/079051 relates to silyl and heteroatom substituted compounds, selected from certain carbazoles, dibenzofurans, dibenzothiophenes and disilylbenzophospholes, and to the application of such compounds in organic-electronic applications, preferably in organic light diodes.

### SUMMARY OF THE INVENTION

Provided are a novel condensed cyclic compound and an organic light-emitting device including the same.

Additional aspects will be set forth in part in the description which follows and, in part, will be apparent from the description, or may be learned by practice of the presented exemplary embodiments.

According to an aspect of an exemplary embodiment, a condensed cyclic compound is represented by one of Formulae 1A to 1D: 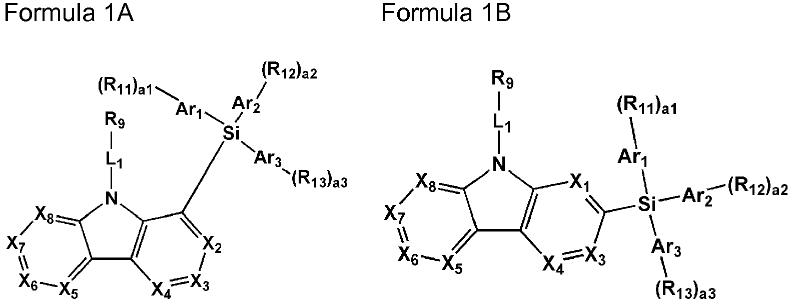 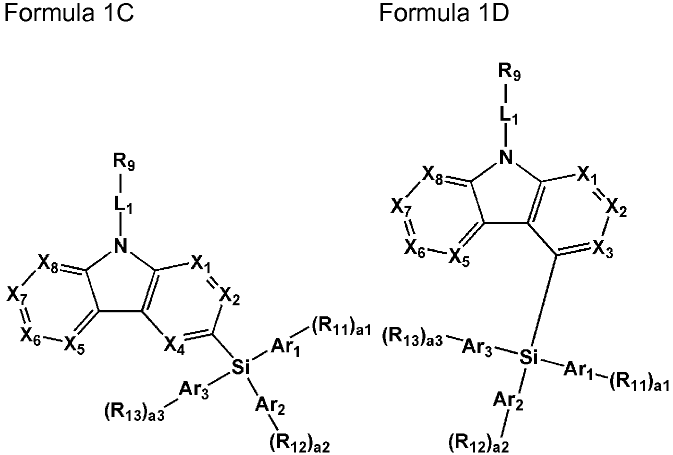 wherein in Formulae 1A to 1D,
X₁ is N or C(R₁),
X₂ is N or C(R₂),
X₃ is N or C(R₃),
X₄ is N or C(R₄),
X₅ is N or C(R₅),
X₆ is N or C(R₆),
X₇ is N or C(R₇), and
X₈ is N or C(R₈);
L₁ is each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
Ar₁ to Ar₃ are each independently a group derived from a C₅-C₉ non-condensed carbocyclic group or a C₁-C₇ non-condensed heterocyclic group,
R₁ to R₈ are each independently selected from a hydrogen, a deuterium, -F, -CI, -Br, -I, a cyano group, and a substituted or unsubstituted group selected from: a methyl group, an ethyl group, a propyl group, an isobutyl group, a sec-butyl group, a tert-butyl group, a pentyl group, an iso-amyl group, a hexyl group, a phenyl group, a naphthyl group, an anthracenyl group, a phenanthrenyl group, a pyrenyl group, and a chrysenyl group;
R₉ is selected from a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted a monovalent non-aromatic condensed polycyclic group and a substituted or unsubstituted monovalent non-aromatic condensed heteropolycyclic group;
R₁₁ to R₁₃ are each independently a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, -N(Q₁)(Q₂), wherein Q₁ and Q₂ are each independently selected from a C₁-C₁₀ alkyl group, and a group represented by Formulae 3-1 to 3-29 and 3-31 to 3-37: 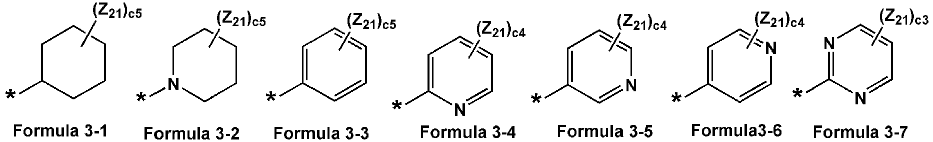 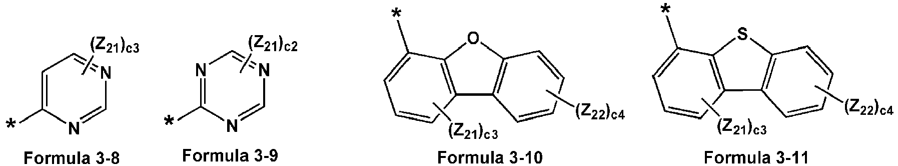 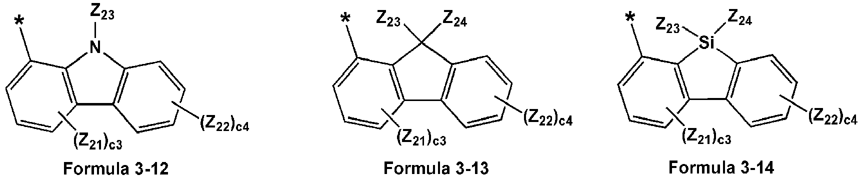 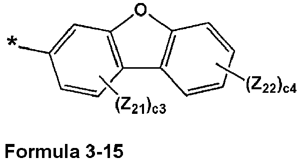 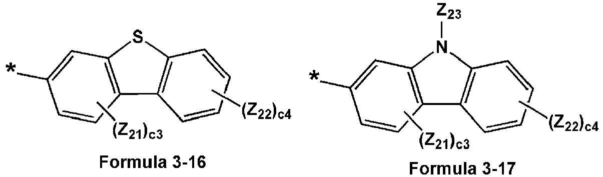 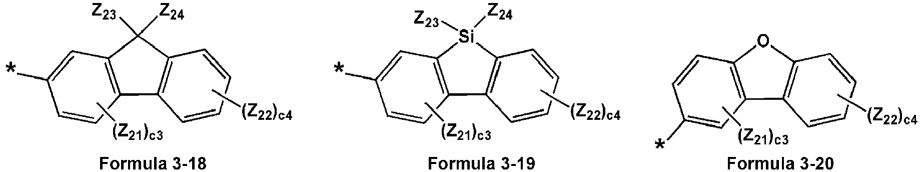 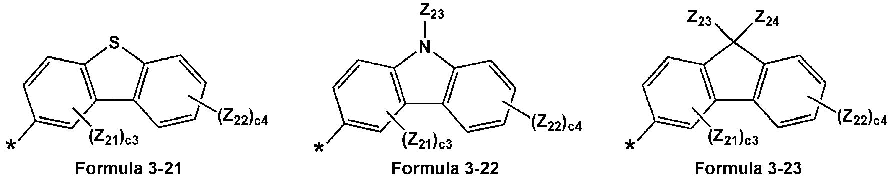 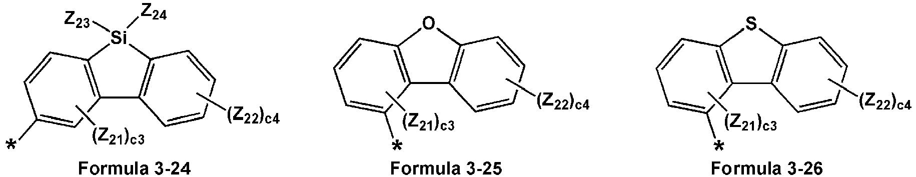 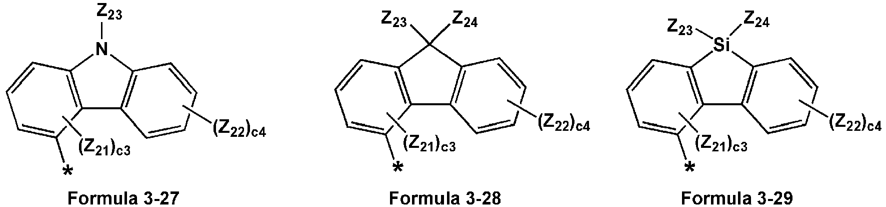 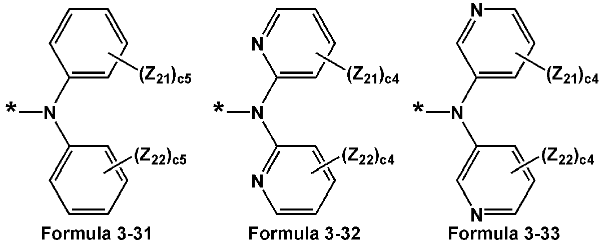 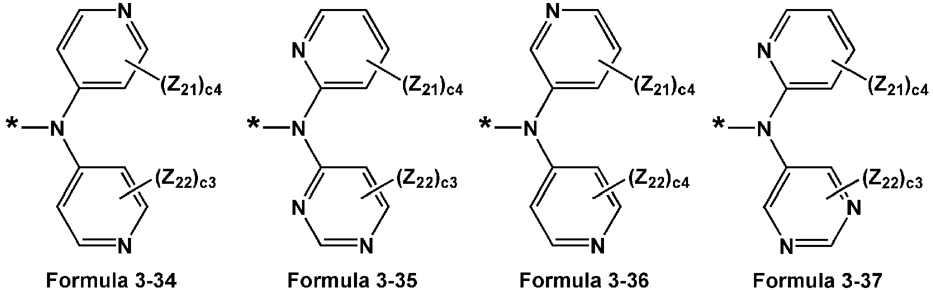 wherein in Formulae 3-1 to 3-29 and 3-31 to 3-37,
   Z₂₁ to Z₂₄ are each independently selected from a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a pyridinyl group, a pyrimidinyl group, and a triazinyl group,
   c5 is an integer selected from 1, 2, 3, 4, and 5,
   c4 is an integer selected from 1, 2, 3, and 4,
   c3 is an integer selected from 1, 2, and 3,
   c2 is an integer selected from 1 to 2, and
   * indicates a binding site to each of Ar₁ to Ar₃ in Formulae 1A to 1D,
   a1 to a3 are each independently an integer selected from 1, 2, 3, 4, 5, and 6, provided that when a1 is 2 or more, two or more groups R₁₁ are identical to or different from each, when a2 is 2 or more, two or more groups R₁₂ are identical to or different from each other, and when a3 is 2 or more, two or more groups R₁₃ are identical to or different from each other;
   wherein in each of Formulae 1A to 1D, groups *-Ar₁-(R₁₁)ₐ₁, *-Ar₂-(R₁₂)ₐ₂ and *-Ar₃-(R₁₃)ₐ₃ are not identical to each other;
   at least one substituent of the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from
   a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
   a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -N(Q₁₁)(Q₁₂);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₂-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -N(Q₂₁)(Q₂₂); and
   -N(Q31)(Q32);
wherein Q₁, Q₂, Q₁₁, Q₁₂, Q₂₁, Q₂₂, Q₃₁ and Q₃₂ are each independently selected from
a hydrogen, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group; and
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

Another aspect provides an organic light-emitting device including:
a first electrode;
a second electrode; and
an organic layer disposed between the first electrode and the second electrode, wherein the organic layer includes an emission layer, and further includes at least one of the above-mentioned condensed cyclic compounds.

### BRIEF DESCRIPTION OF THE DRAWINGS

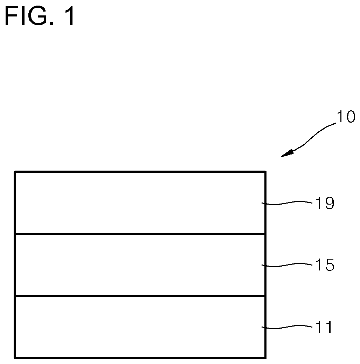

These and/or other aspects will become apparent and more readily appreciated from the following description of the exemplary embodiments, taken in conjunction with FIG. 1 which is a schematic view of an organic light-emitting device according to an embodiment.

### DETAILED DESCRIPTION OF THE EMBODIMENTS

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Spatially relative terms, such as "beneath," "below," "lower," "above," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the exemplary term "below" can encompass both an orientation of above and below. The device may be otherwise oriented (rotated 90 degrees or at other orientations) and the spatially relative descriptors used herein interpreted accordingly.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

An aspect provides a condensed cyclic compound represented by Formulae 1A to 1D below:

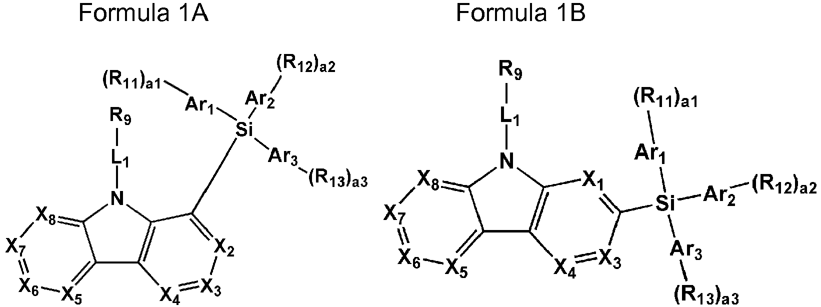

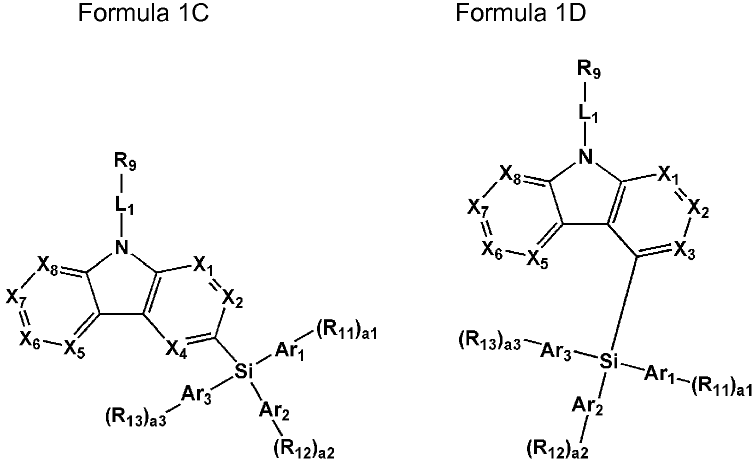

In Formulae 1A to 1D,
X₁ to X₈ are as described above.

In some embodiments, in Formulae 1A to 1D,
X₁ may be C(R₁),
X₂ may be C(R₂),
X₃ may be C(R₃),
X₄ may be C(R₄),
X₅ may be C(R₅),
X₆ may be C(R₆),
X₇ may be C(R₇), and
X₈ may be C(R₈).

In some embodiments,
one, two, or three elements selected from X₂ to X₈ in Formula 1A may be N;
one, two, or three elements selected from X₁ and X₃ to X₈ in Formula 1B may be N;
one, two, or three elements selected from X₁, X₂ and X₄ to X₈ in Formula 1C may be N; and
one, two, or three elements selected from X₁ to X₃ and X₅ to X₈ in Formula 1D may be N,
but they are not limited thereto.

For example, in Formulae 1A to 1D,
X₁ is N, X₂ is C(R₂), X₃ is C(R₃), X₄ is C(R₄), X₅ is C(R₅), X₆ is C(R₆), X₇ is C(R₇), and X₈ is C(R₈);
X₁ is C(R₁), X₂ is N, X₃ is C(R₃), X₄ is C(R₄), X₅ is C(R₅), X₆ is C(R₆), X₇ is C(R₇), and X₈ is C(R₈);
X₁ is C(R₁), X₂ is C(R₂), X₃ is N, X₄ is C(R₄), X₅ is C(R₅), X₆ is C(R₆), X₇ is C(R₇), and X₈ is C(R₈);
X₁ is C(R₁), X₂ is C(R₂), X₃ is C(R₃), X₄ is N, X₅ is C(R₅), X₆ is C(R₆), X₇ is C(R₇), and X₈ is C(R₈);
X₁ is C(R₁), X₂ is C(R₂), X₃ is C(R₃), X₄ is C(R₄), X₅ is N, X₆ is C(R₆), X₇ is C(R₇), and X₈ is C(R₈);
X₁ is C(R₁), X₂ is C(R₂), X₃ is C(R₃), X₄ is C(R₄), X₅ is C(R₅), X₆ is N, X₇ is C(R₇), and X₈ is C(R₈);
X₁ is C(R₁), X₂ is C(R₂), X₃ is C(R₃), X₄ is C(R₄), X₅ is C(R₅), X₆ is C(R₆), X₇ is N, and X₈ is C(R₈);
X₁ is C(R₁), X₂ is C(R₂), X₃ is C(R₃), X₄ is C(R₄), X₅ is C(R₅), X₆ is C(R₆), X₇ is C(R₇), and X₈ is N;
X₁ is C(R₁), X₂ is C(R₂), X₃ is C(R₃), X₄ is C(R₄), X₅ is C(R₅), X₆ is N, X₇ is C(R₇), and X₈ is N;
X₁ is C(R₁), X₂ is C(R₂), X₃ is C(R₃), X₄ is C(R₄), X₅ is N, X₆ is C(R₆), X₇ is N, and X₈ is C(R₈);
X₁ is C(R₁), X₂ is N, X₃ is C(R₃), X₄ is C(R₄), X₅ is C(R₅), X₆ is C(R₆), X₇ is N, and X₈ is C(R₈);
X₁ is C(R₁), X₂ is C(R₂), X₃ is C(R₃), X₄ is N, X₅ is N, X₆ is C(R₆), X₇ is C(R₇), and X₈ is C(R₈); or
X₁ is C(R₁), X₂ is C(R₂), X₃ is N, X₄ is C(R₄), X₅ is C(R₅), X₆ is N, X₇ is C(R₇), and X₈ is C(R₈), but they are not limited thereto.
L₁ in Formulae 1A and 1D are as described above.

For example, L₁ in Formulae 1A to 1D may be each independently selected from a substituted or unsubstituted C₆-C₂₀ arylene group, a substituted or unsubstituted C₁-C₂₀ heteroarylene group, a substituted or unsubstituted divalent non-aromatic condensed polycyclic group, and a substituted or unsubstituted divalent non-aromatic condensed heteropolycyclic group.

In some embodiments, L₁ in Formulae 1A to 1D may be selected from
a phenylene group, a naphthylene group, a fluorenylene group, a phenanthrenylene group, an anthracenylene group, a triphenylenylene group, a pyrenylene group, a chrysenylene group, a pyridinylene group, a pyrazinylene group, a pyrimidinylene group, a pyridazinylene group, and a triazinylene group; and
a phenylene group, a naphthylene group, a fluorenylene group, a phenanthrenylene group, an anthracenylene group, a triphenylenylene group, a pyrenylene group, a chrysenylene group, a pyridinylene group, a pyrazinylene group, a pyrimidinylene group, a pyridazinylene group, and a triazinylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a phenanthrenyl group, an anthracenyl group, a triphenylenyl group, a pyrenyl group, a chrysenyl group, a pyridinyl group, a pyrazinyl group, a pyrimidinyl group, a pyridazinyl group, and a triazinyl group,
but they are not limited thereto.

In some embodiments, L₁ in Formulae 1A to 1D may be selected from
a phenylene group, a naphthylene group, a pyridinylene group, a pyrimidinylene group, and a triazinylene group; and
a phenylene group, a naphthylene group, a pyridinylene group, a pyrimidinylene group, and a triazinylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, and a naphthyl group.

Ar₁ to Ar₃ in Formulae 1A to 1D are as described above.

In some embodiment, Ar₁ and Ar₃ in Formulae 1A to 1D may be each independently represented by one of Formulae 2-1 to 2-40 below: 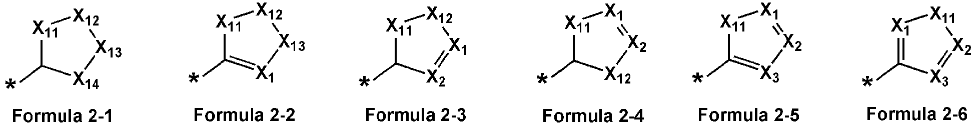 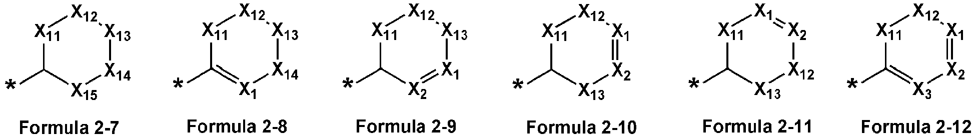 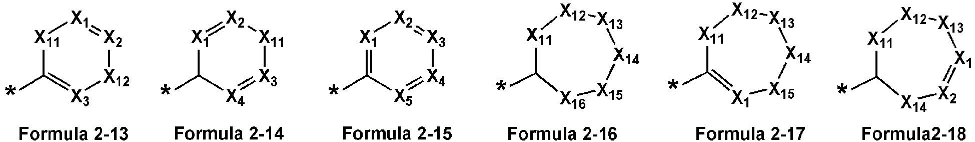 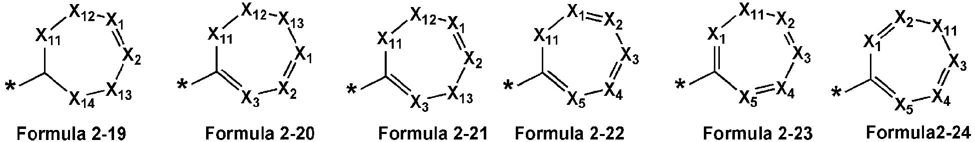 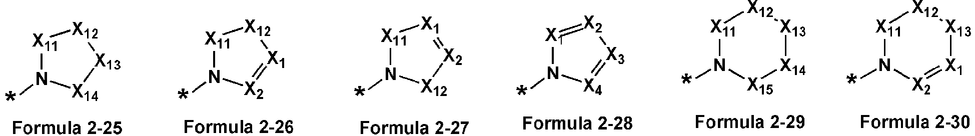 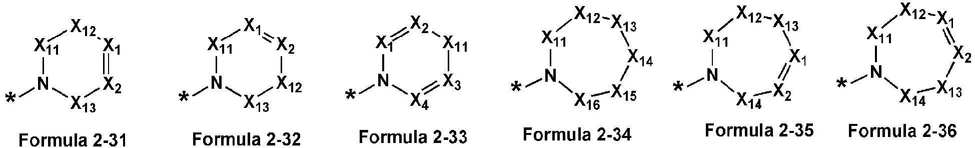 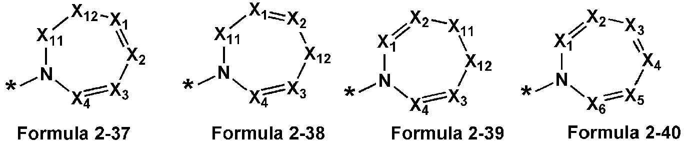 wherein in Formulae 2-1 to 2-40,
X₁₁ is O, S, N(Z₁₁) or C(Z₁₁ₐ)(Z_{11b}), X₁₂ is O, S, N(Z₁₂) or C(Z₁₂ₐ)(Z_{12b}), X₁₃ is O, S, N(Z₁₃) or C(Z₁₃ₐ)(Z_{13b}), X₁₄ is O, S, N(Z₁₄) or C(Z₁₄ₐ)(Z_{14b}), X₁₅ is O, S, N(Z₁₅) or C(Z₁₅ₐ)(Z_{15b}), X₁₆ is O, S, N(Z₁₆) or C(Z₁₆ₐ)(Z_{16b});
X₁ is N or C(Z₁), X₂ is N or C(Z₂), X₃ is N or C(Z₃), X₄ is N or C(Z₄), X₅ is N or C(Z₅), and X₆ is N or C(Z₆);
Z₁₁ to Z₁₆, Z₁₁ₐ to Z₁₆ₐ, Z_{11b} to Z_{16b}, and Z₁ to Z₆ are each independently a hydrogen, or a binding site to each of R₁₁, R₁₂, and R₁₃ in Formulae 1A to 1D; and
* is a binding site to Si in Formulae 1A to 1D.

For example, Ar₁ to Ar₃ in Formulae 1A to 1D may be each independently selected from groups represented by Formulae 2-5 to 2-7, 2-15, 2-22, 2-23, and 2-24, but they are not limited thereto.

In some embodiments, Ar₁ to Ar₃ in Formulae 1A to 1D may be each independently a group derived from a compound selected from a benzene, a pyridine, a pyrazine, a pyrimidine, a pyridazine, a triazine, a furan, a thiophene, a pyrrole, an imidazole, a triazole, a cyclohexane, a tetrahydro-2H-pyran, a piperidine, a tetrahydro-2H-thiopyran, a (2Z,4Z,6Z)-oxepine, a (2Z,4Z,6Z)-1H-azepine, and a (2Z,4Z,6Z)-thiepine.

R₁ to R₉ and R₁₁ to R₁₃ in Formulae 1A to 1D are as described above.

In some embodiments, R₁₁ to R₁₃ are each independently selected from a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, -N(Q₁)(Q₂) (wherein Q₁ and Q₂ are each independently selected from a C₁-C₅ alkyl group), and groups represented by Formulae 4-1 to 4-49 and 4-61 to 4-74 below: 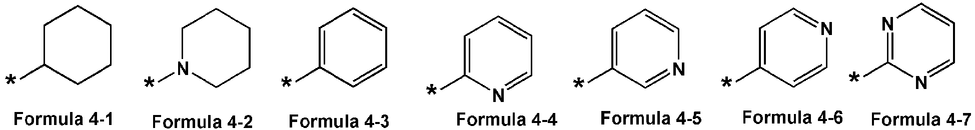 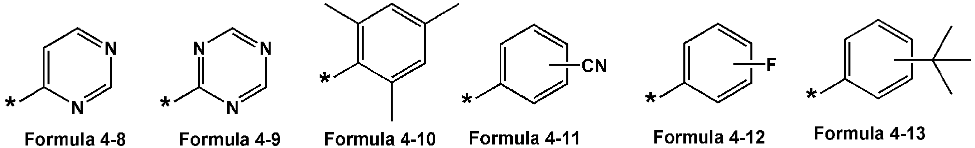 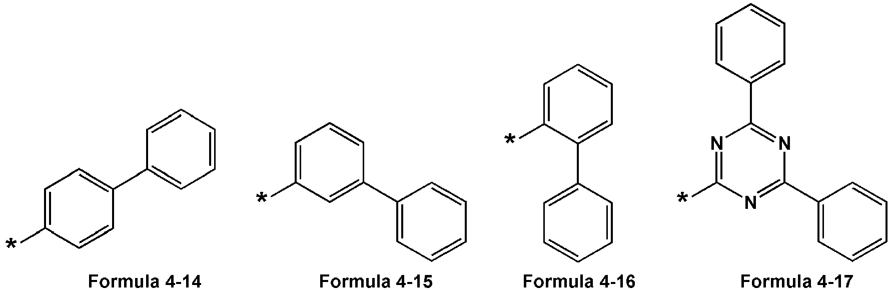 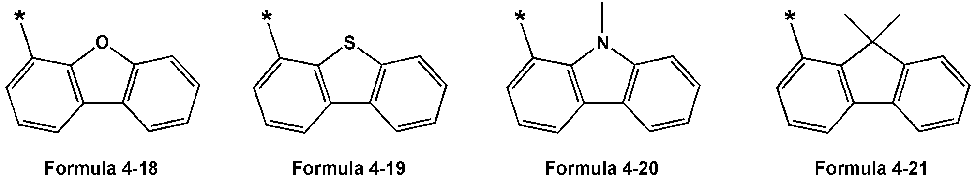 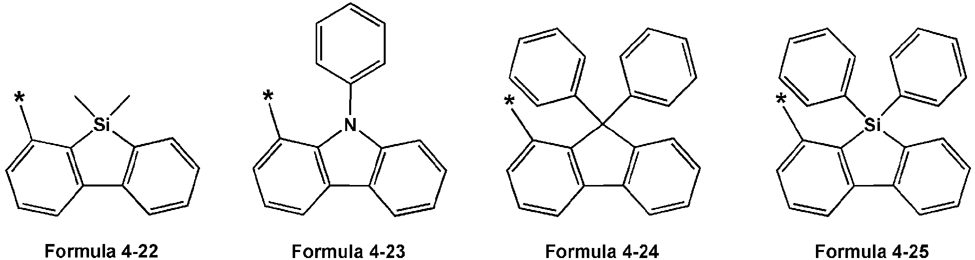 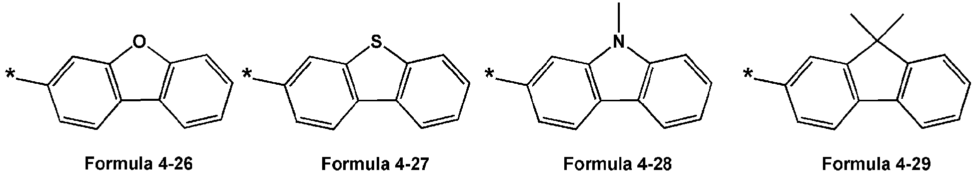 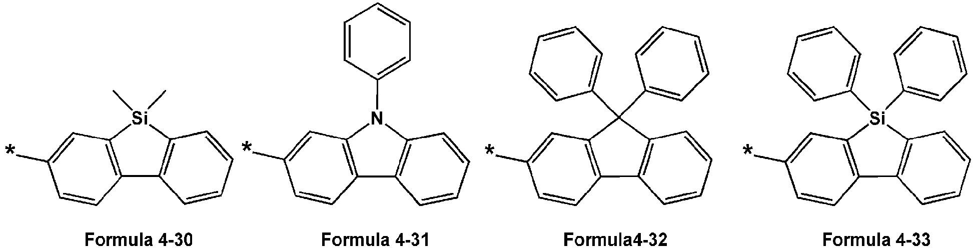 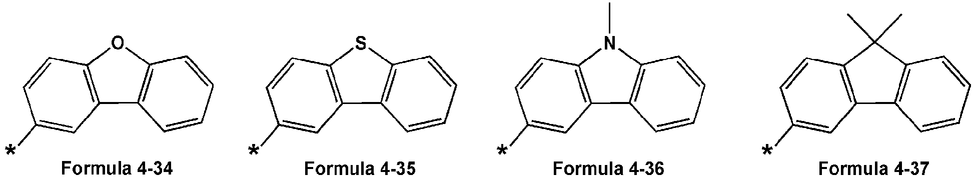 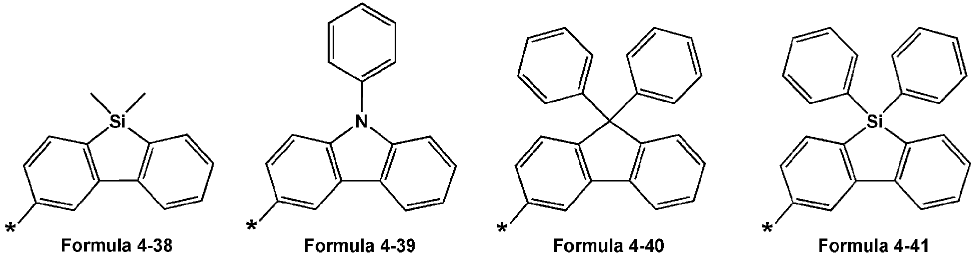 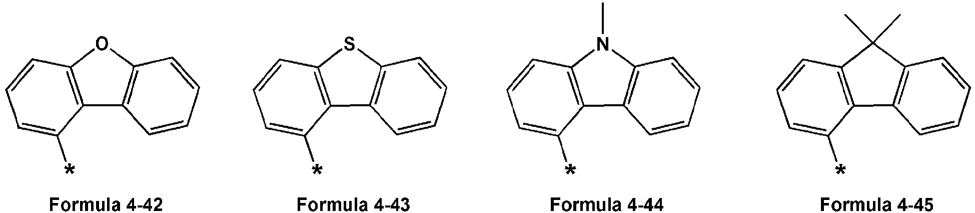 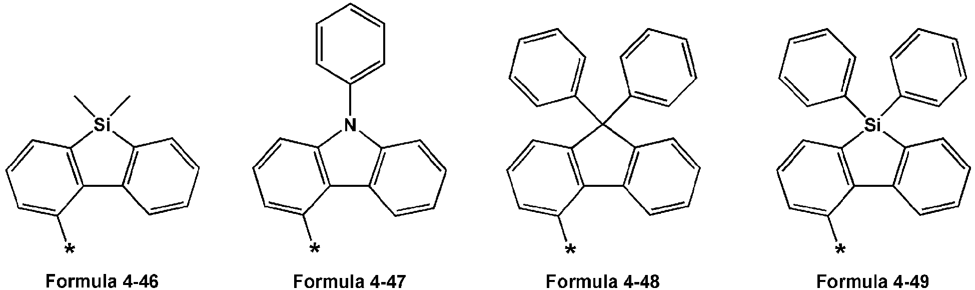 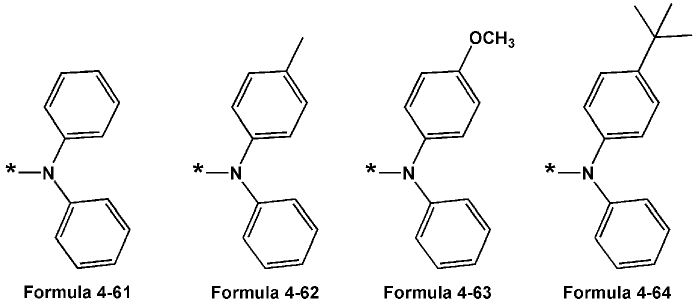 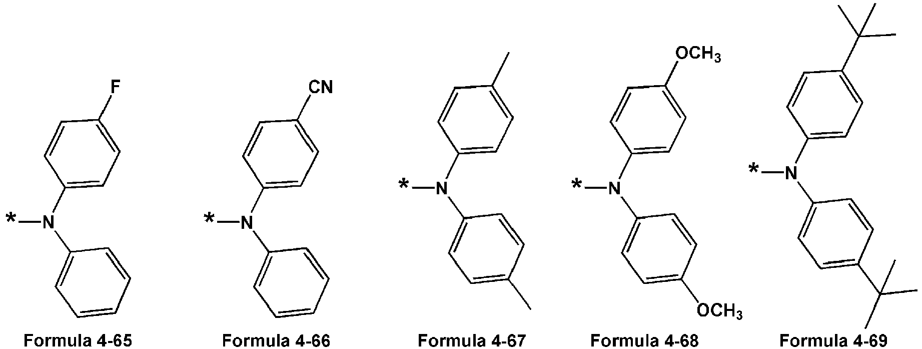 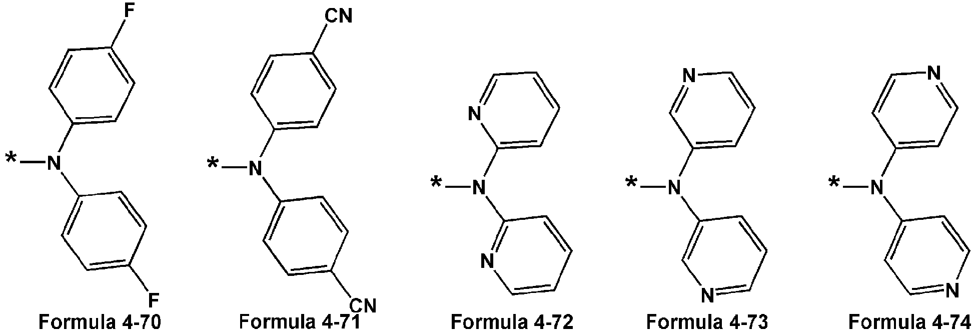 wherein in Formulae 4-1 to 4-49 and 4-61 to 4-74, * indicates a binding site to each of Ar₁ to Ar₃ in Formulae 1A to 1D.

In some embodiments,
*-Ar₁-(R₁₁)ₐ₁ in Formulae 1A to 1D is a group represented by any one selected from Formulae 11-1 to 11-47 below,
*-Ar₂-(R₁₂)ₐ₂ in Formulae 1A to 1D is a group represented by any one selected from Formulae 12-1 to 12-47 below, and
*-Ar₃-(R₁₃)ₐ₃ in Formulae 1A to 1D is a group represented by any one selected from Formulae 13-1 to 13-47: 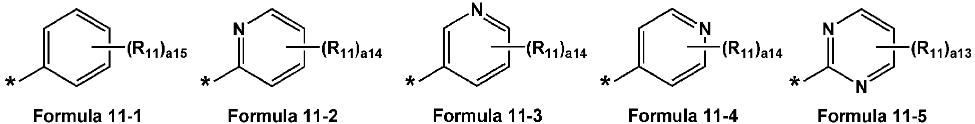 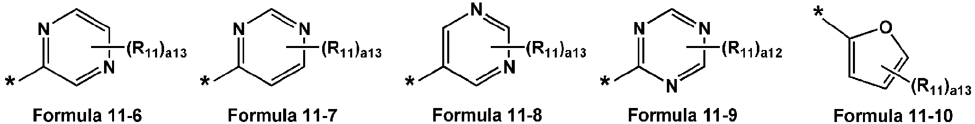 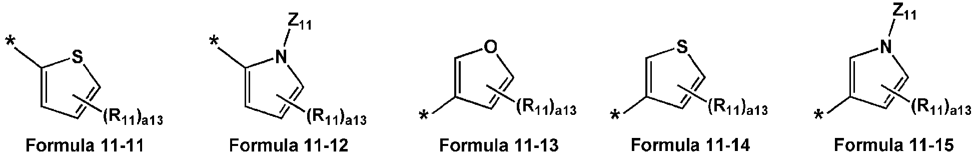 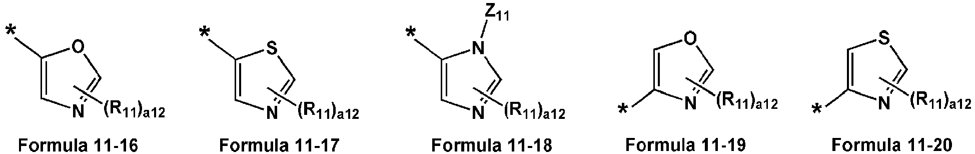 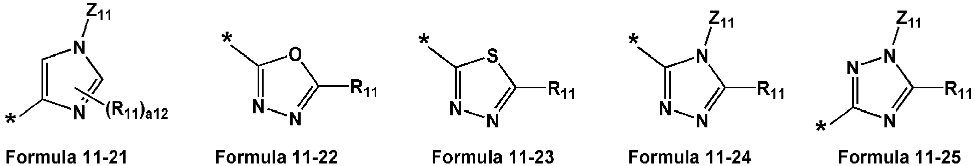 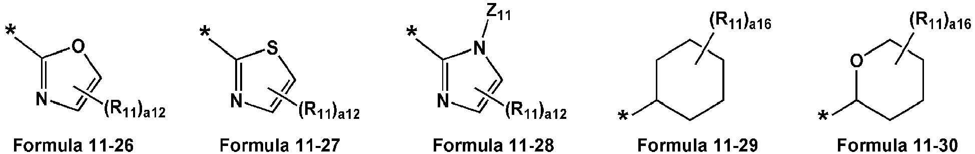 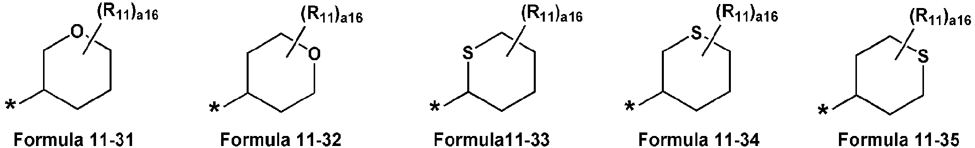 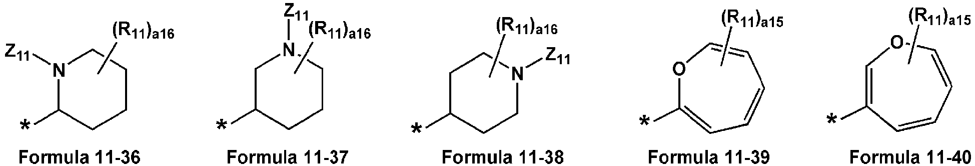 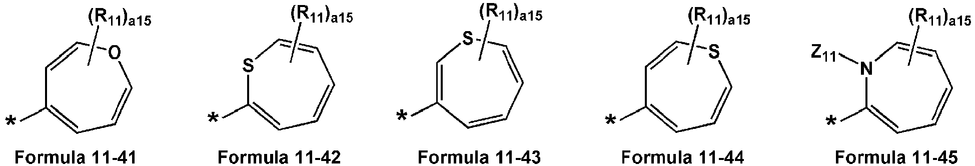 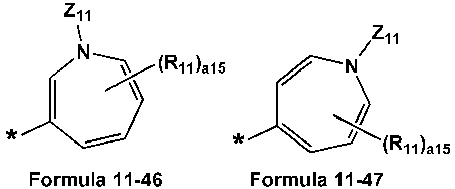 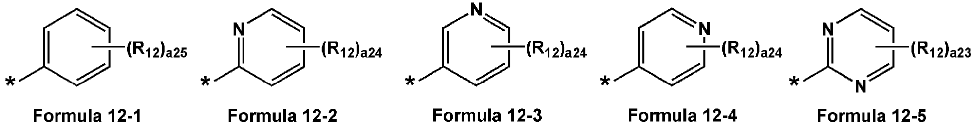 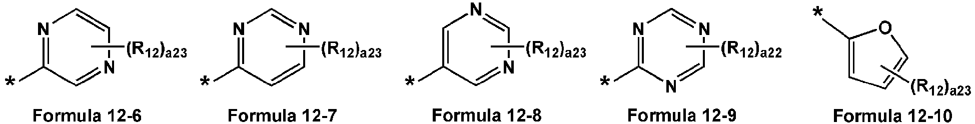 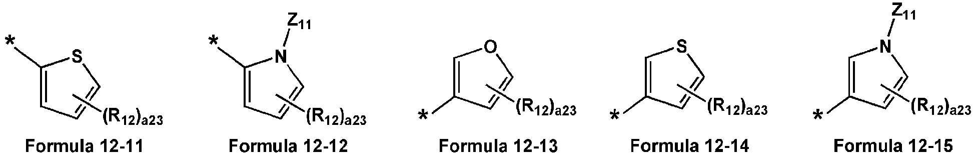 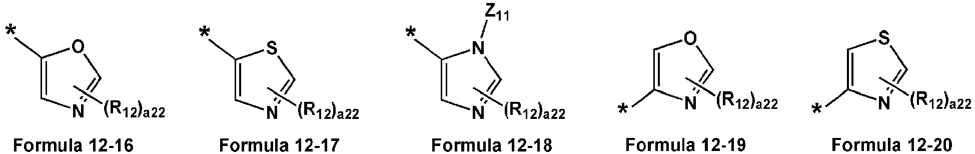 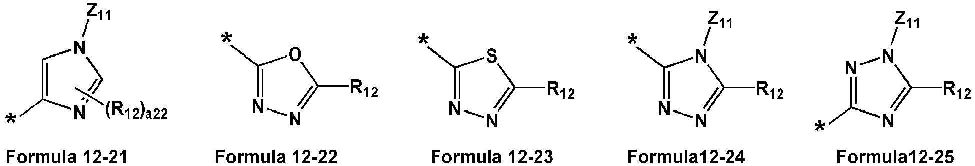 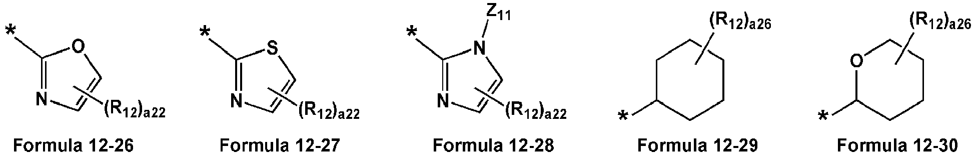 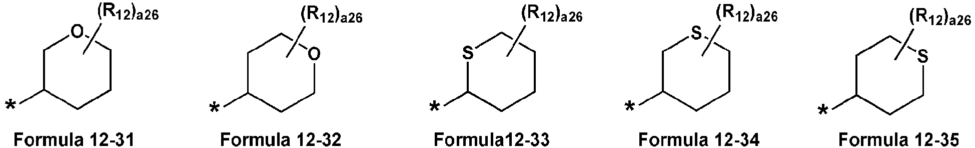 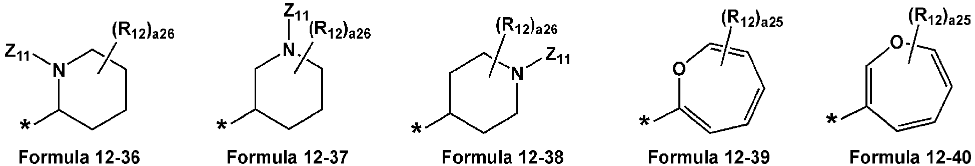 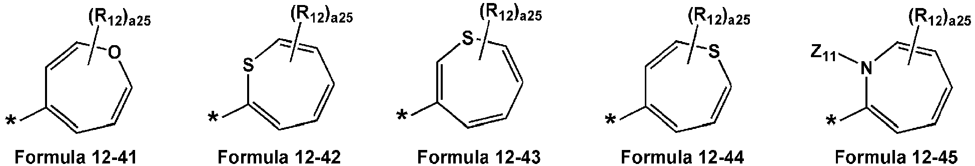 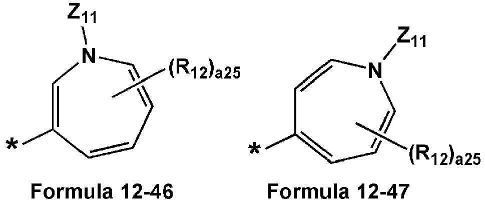 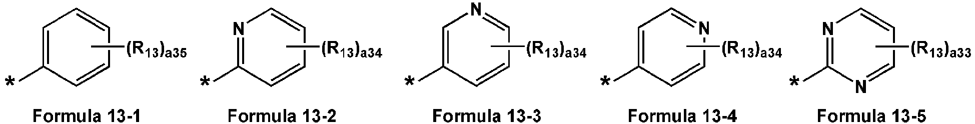 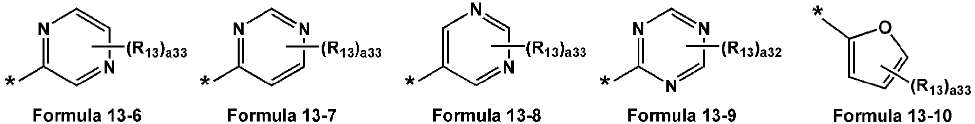 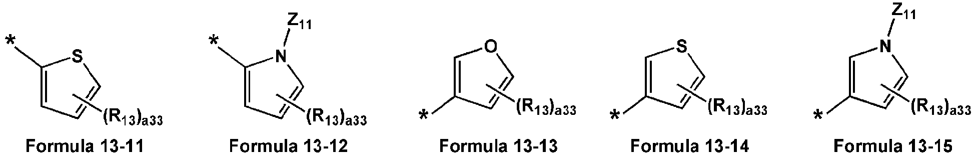 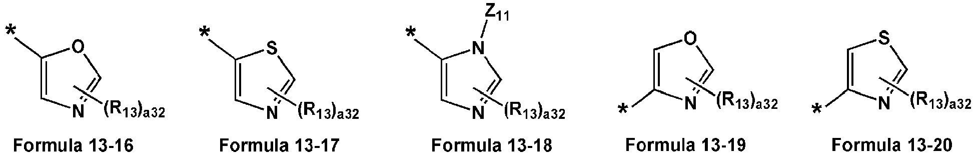 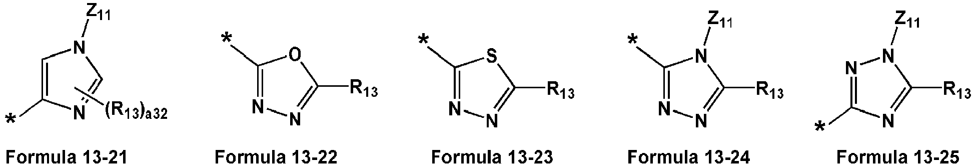 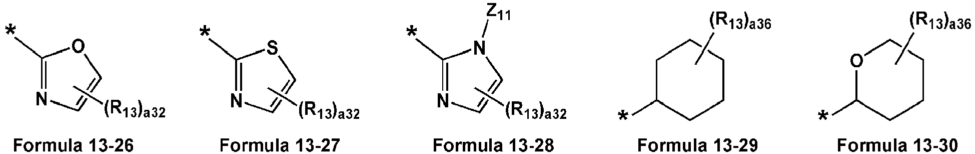 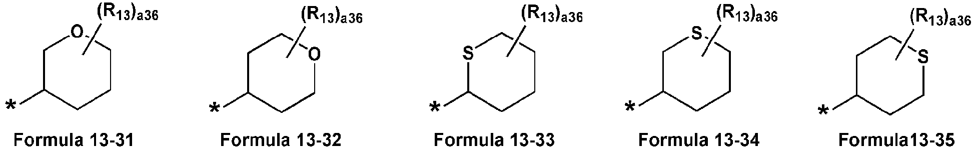 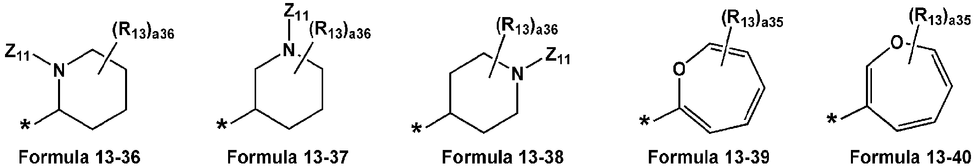 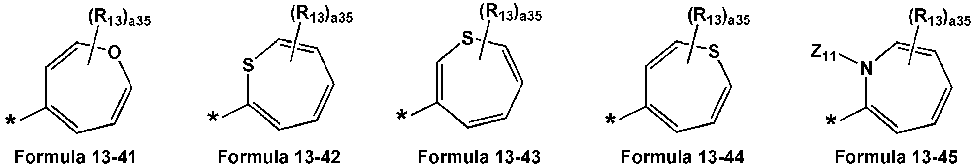 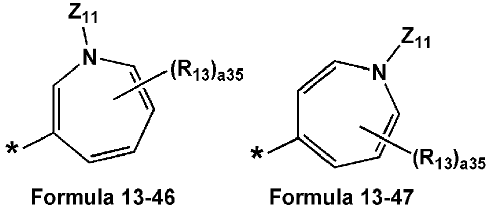
wherein in Formulae 11-1 to 11-47, 12-1 to 12-47, and 13-1 to 13-47,
R₁₁ to R₁₃ are the same as recited in the present specification,
a16, a26, and a36 are each independently an integer selected from 1, 2, 3, 4, 5, and 6,
a15, a25, and a35 are each independently an integer selected from 1, 2, 3, 4, and 5,
a14, a24, and a34 are each independently an integer selected from 1, 2, 3, and 4,
a13, a23, and a33 are each independently an integer selected from 1, 2, and 3,
a12, a22, and a32 are each independently an integer selected from 1 and 2, and
* indicates a binding site to Si in Formulae 1A to 1D.

In some embodiments, Ar₁ in Formulae 1A to 1D is a group represented by any one selected from Formulae 11-1 to 11-9 above, and Ar₃ is a group represented by any one selected from Formulae 13-1 to 13-9 above.

In some embodiments, in Formulae 1A to 1D, X₆ may be C(R₆) and R₆ may not be a hydrogen.
a1 to a3 in Formulae 1A to 1D may be each independently an integer of 1, 2, 3, 4, 5, and 6. For example, a1 to a3 may be each independently 1, 2, or 3.

When a1 is 2 or more, two or more groups R₁₁ are identical to or different from each other, when a2 is 2 or more, two or more groups R₁₂ are identical to or different from each other, and when a3 is 2 or more, two or more groups R₁₃ are identical to or different from each other;
*-Ar₁-(R₁₁)ₐ₁, *-Ar₂-(R₁₂)ₐ₂, and *-Ar₃-(R₁₃)ₐ₃ in Formula 1A are not identical, *-Ar₁-(R₁₁)ₐ₁, *-Ar₂-(R₁₂)ₐ₂ and *-Ar₃-(R₁₃)ₐ₃ in Formula 1B are not identical,*-Ar₁-(R₁₁)ₐ₁, *-Ar₂-(R₁₂)ₐ₂ and *-Ar₃-(R₁₃)ₐ₃ in Formula 1C are not identical, and *-Ar₁-(R₁₁)ₐ₁, *-Ar₂-(R₁₂)ₐ₂ and *-Ar₃-(R₁₃)ₐ₃ in Formula 1D are not identical.

In some embodiments, in Formulae 1A to 1D, Ar₁ to Ar₃ may be identical, a1, a2, and a3 are each independently selected from 1, 2, and 3, and R₁₁ and R₁₂ may be different from each other.

In some embodiments, in Formulae 1A to 1D, Ar₁ to Ar₃ may be identical, a1, a2 and a3 are 1, and R₁₁ and R₁₂ may be different from each other. In this case, for example, R₁₁ may be a hydrogen, and R₁₂ may not be a hydrogen.

In some embodiments, in Formulae 1A to 1D, Ar₁ to Ar₃ may be identical, a1 may be 0, a2 and a3 may not be 0, and R₁₂ and R₁₃ may not be a hydrogen.

In some embodiments, in Formulae 1A to 1D, Ar₁ to Ar₃ may be identical, a1 and a3 may be 0, a2 may not be 0, R₁₂ may not be a hydrogen.

In some embodiments, in Formulae 1A to 1D, Ar₁ and Ar₂ may be different from each other.

In some embodiments, in Formulae 1A to 1D, Ar₁ to Ar₃ may all be different.

In some embodiments, in Formulae 1A to 1D, each of Ar₁ to Ar₃ is a group derived from a benzene, and R₁₁ and R₁₂ may be different from each other. In this case, for example, R₁₁ may be a hydrogen, and R₁₂ may not be a hydrogen.

In some embodiments, in Formulae 1A to 1D, Ar₁ = Ar₂ = Ar₃, R₁₁ *≠* R₁₂, and R₁₂ may not be a hydrogen.

In some embodiments, the condensed cyclic compound may be represented by one of Formulae 1A(1) to 1D(1) below: 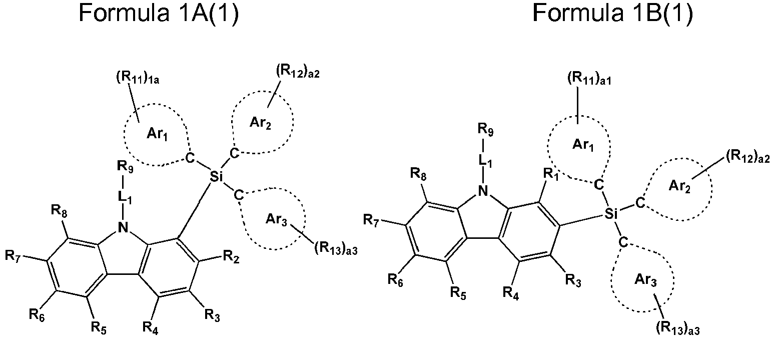 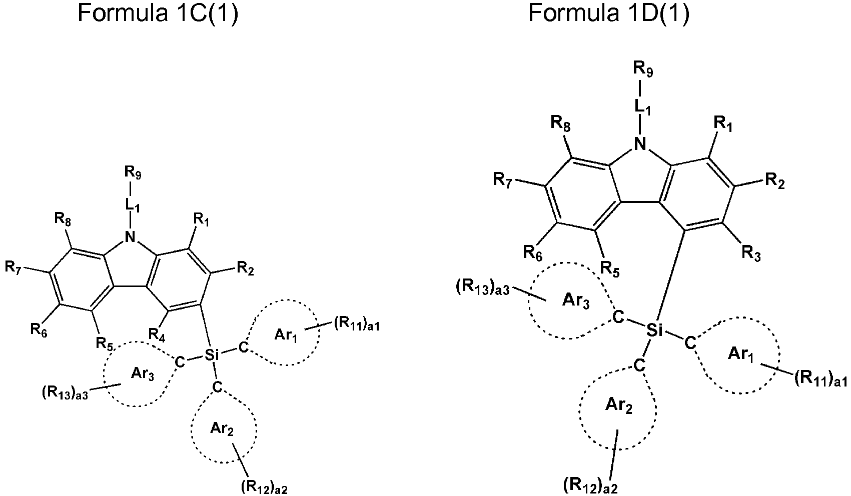 wherein L₁, Ar₁ to Ar₃, R₁ to R₉, R₁₁ to R₁₃, and a1 to a3 in Formulae 1A(1) to 1D(1) are the same as recited in the present specification. In Formulae 1A(1) to 1D(1), each of Ar₁ to Ar₃ is linked to Si via "carbon."

For example, in Formulae 1A(1) and 1D(1),
L₁ may be selected from
a phenylene group, a naphthylene group, a pyridinylene group, a pyrimidinylene group, and a triazinylene group; and
a phenylene group, a naphthylene group, a pyridinylene group, a pyrimidinylene group, and a triazinylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, and a naphthyl group,
Ar₁ to Ar₃ are each independently selected from groups represented by Formulae 2-5 to 2-7, 2-15, and 2-22 to 2-24,
a1 to a3 may be each independently 1, 2, or 3, but are not limited thereto.

In some embodiments, the condensed cyclic compound may be represented by any one selected from Formulae 1A(1)-1, 1A(1)-2, 1B(1)-1, 1B(1)-2, 1C(1)-1, 1C(1)-2, 1D(1)-1, and 1D(1)-2:

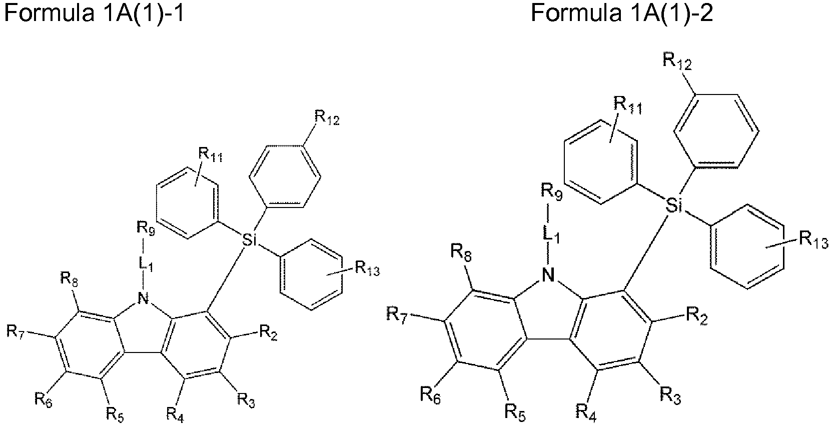

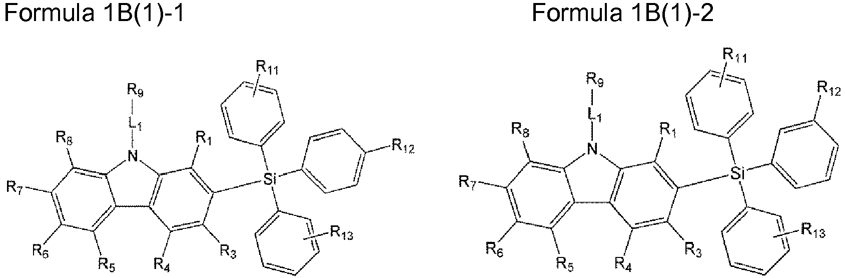

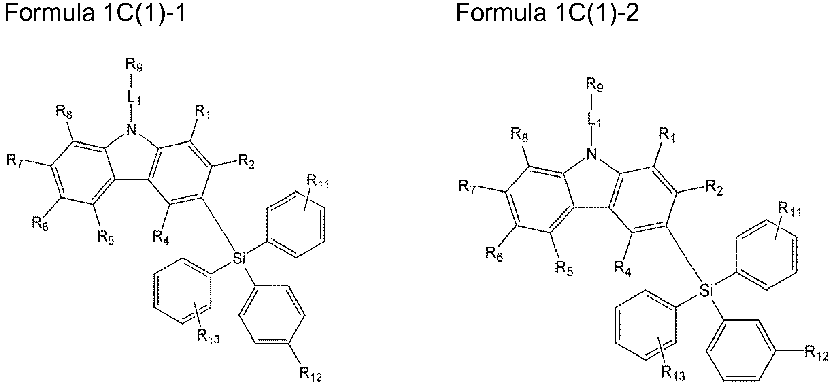

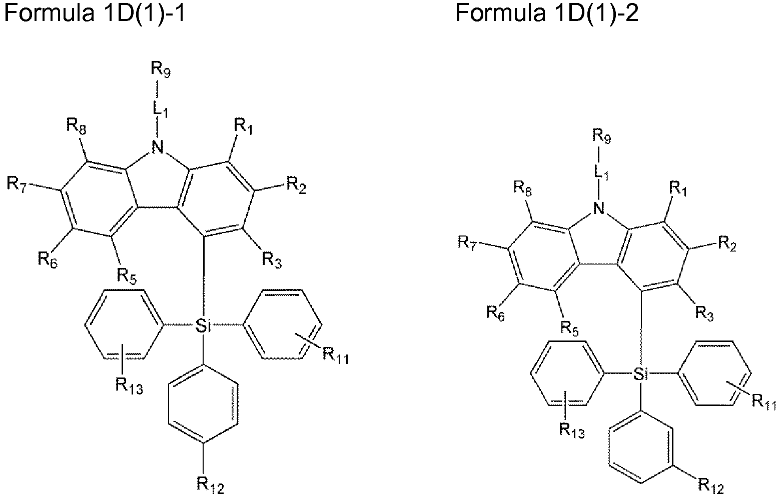

L₁, R₁ to R₉ and R₁₁ to R₁₃ in Formulae 1A(1)-1, 1A(1)-2, 1B(1)-1, 1B(1)-2, 1C(1)-1, 1C(1)-2, 1D(1)-1, and 1D(1)-2 are the same as defined herein, and R₁₁ and R₁₂ are different from each other.

For example, at least one of R₁₁ and R₁₃ in Formulae 1A(1)-1, 1A(1)-2, 1B(1)-1, 1B(1)-2, 1C(1)-1, 1C(1)-2, 1D(1)-1, and 1D(1)-2 is a hydrogen, and R₁₂ may not be a hydrogen.

In some embodiments, the condensed cyclic compound may be represented by any one selected from Formula 1A(1)-3 to 1A(1)-6, 1B(1)-3 to 1B(1)-6, 1C(1)-3 to 1C(1)-6, and 1D(1)-3 to 1D(1)-6:

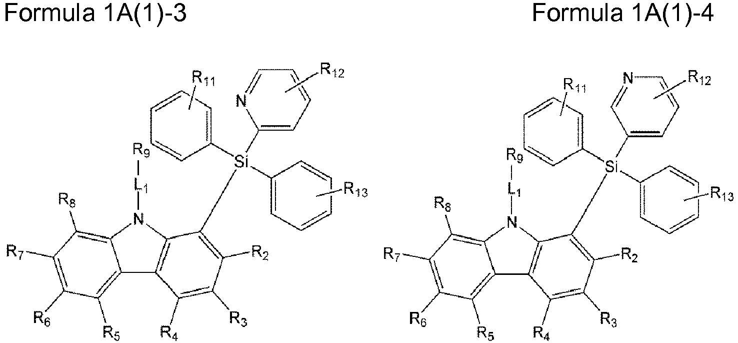

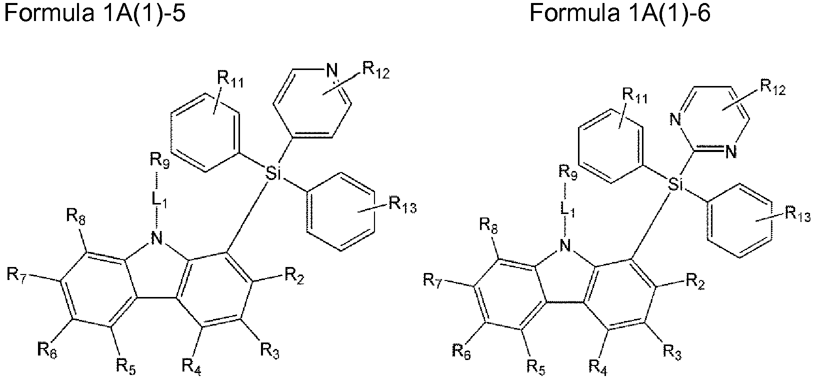

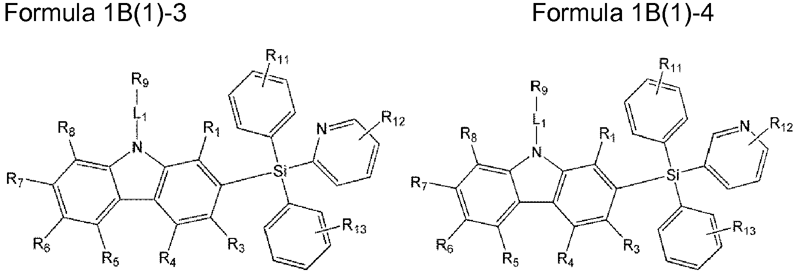

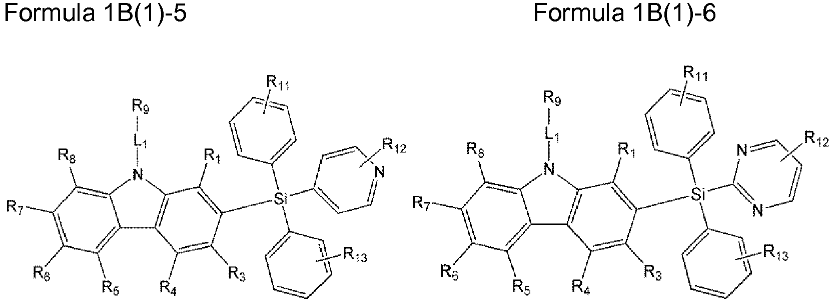

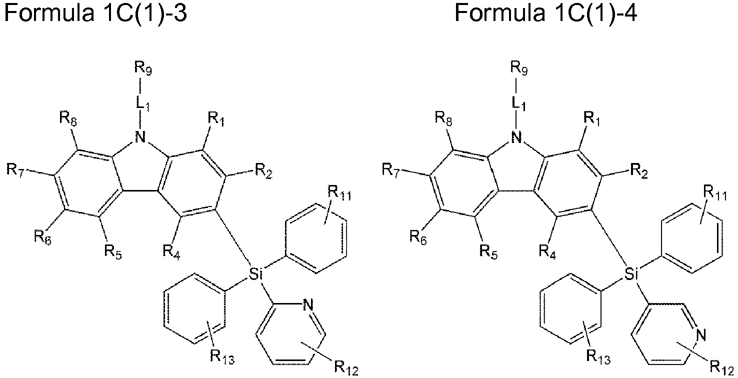

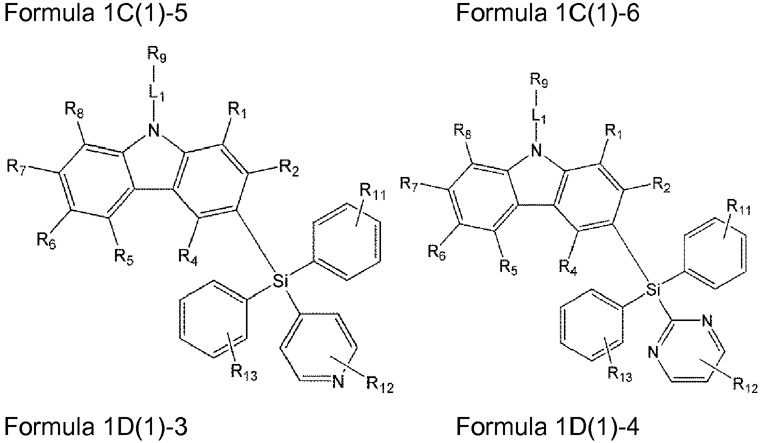

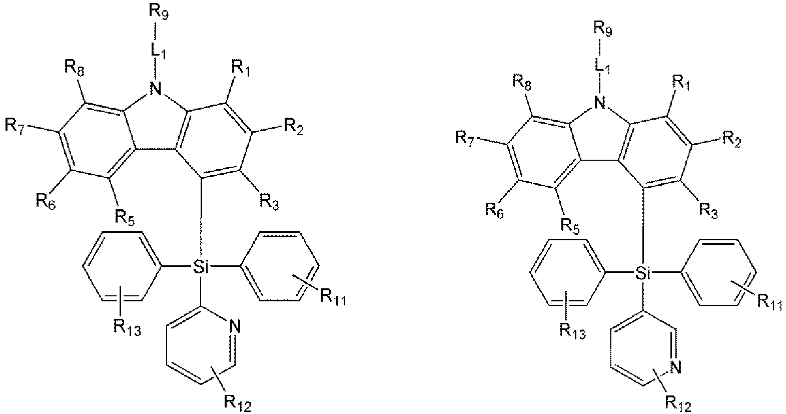

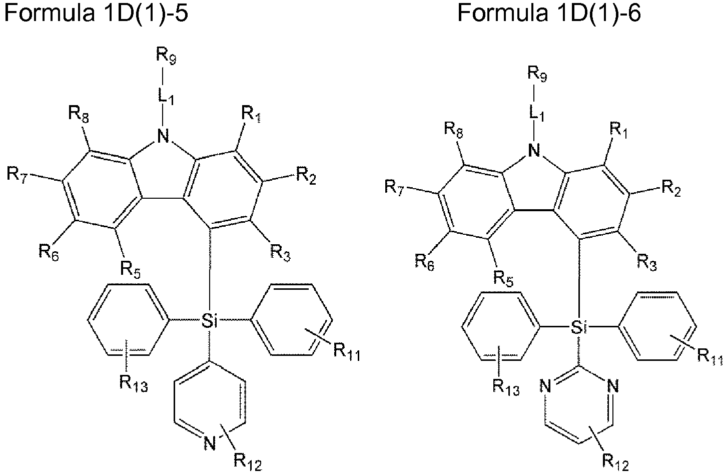

L₁, R₁ to R₉, and R₁₁ to R₁₃ in Formulae 1A(1)-3 to 1A(1)-6, 1B(1)-3 to 1B(1)-6, 1C(1)-3 to 1C(1)-6, and 1D(1)-3 to 1D(1)-6 are the same as described above.

In some embodiments, the condensed cyclic compound may be represented by Formulae 1B or 1C, but the inventive concept is not limited thereto.

In some embodiments, a condensed cyclic compound represented by one of Formulae 1A to 1D may have a molecular weight of 500 to 700. When the molecular weight of the condensed cyclic compound represented by one of Formulae 1A to 1D is within this range, a sublimation purification method may be used to easily purify the condensed cyclic compound.

For example, the condensed cyclic compound may be one of Compounds 1 to 100, 114 to 154, 156, 158, 159, 162 to 177, 184 to 195, 200 to 213, 220 to 231, 233, 233 to 235, 237 to 250, 257 to 268, 270, 271, 273 to 285, 289 to 305, 309 to 323, 325, 327, 328, 330 and 332 below, but is not limited thereto.

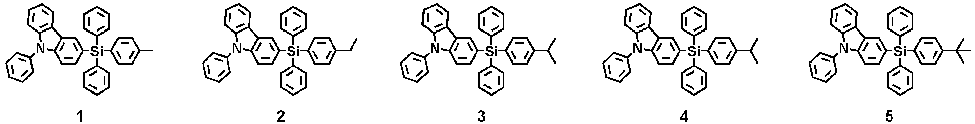

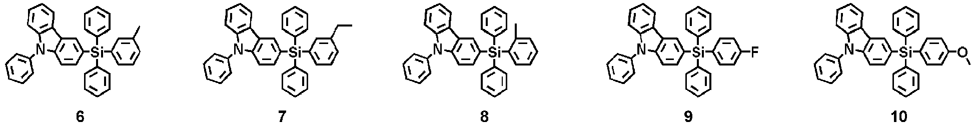

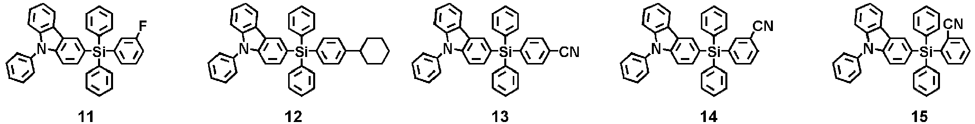

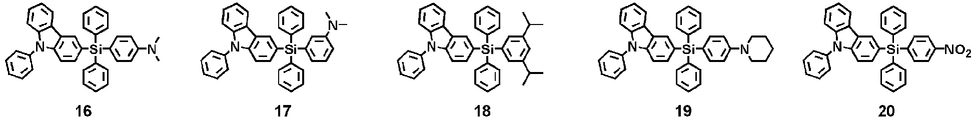

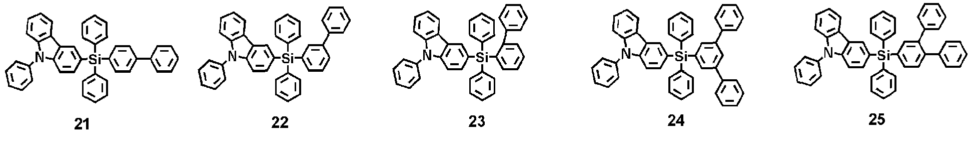

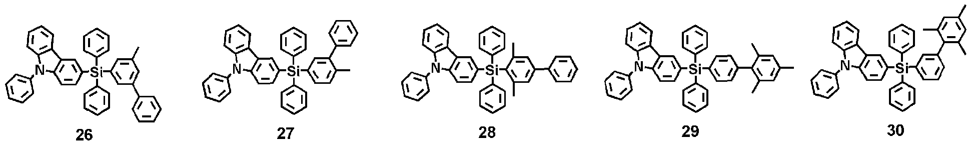

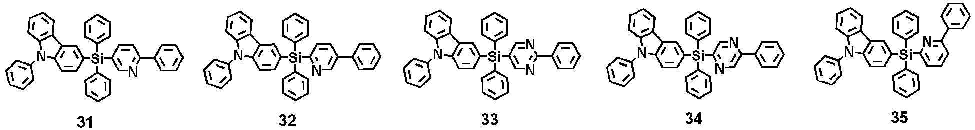

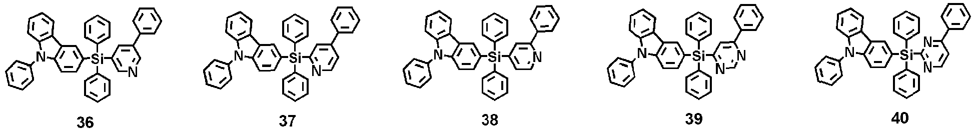

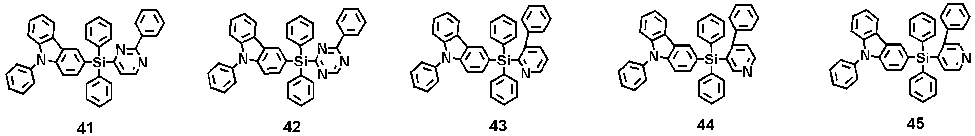

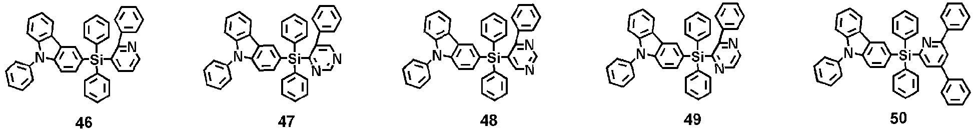

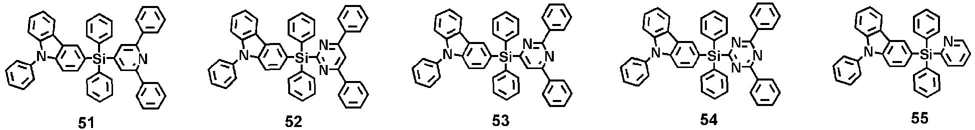

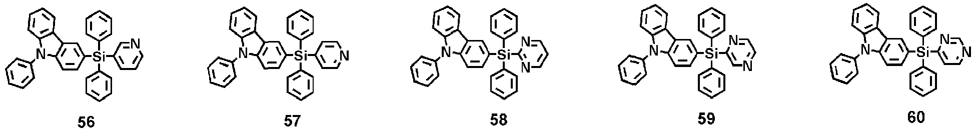

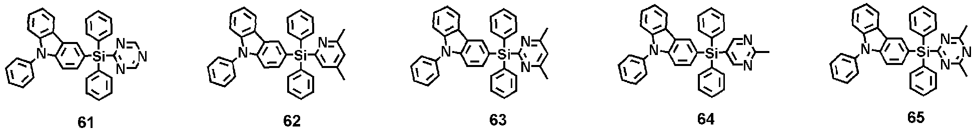

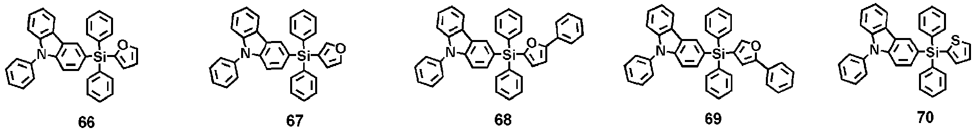

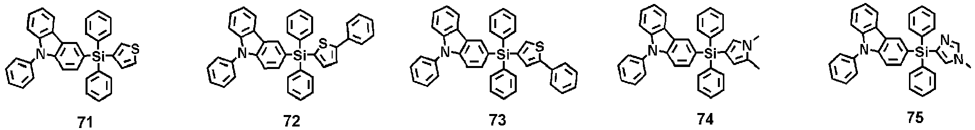

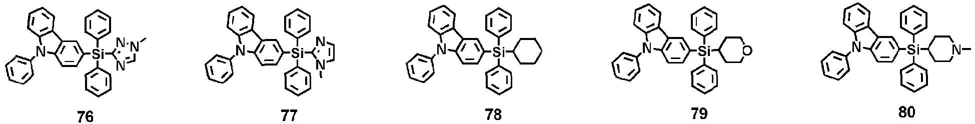

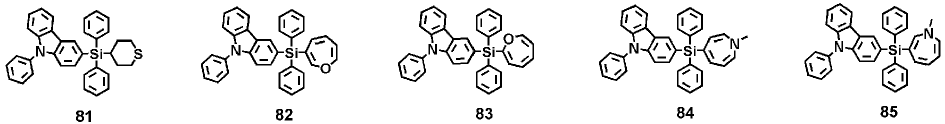

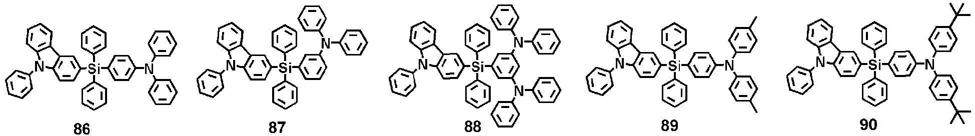

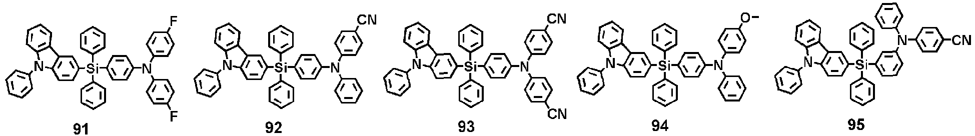

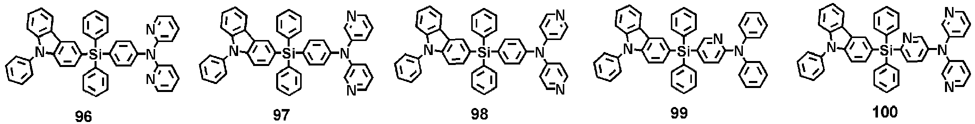

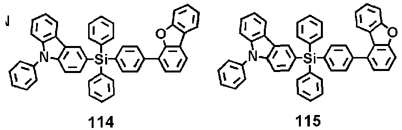

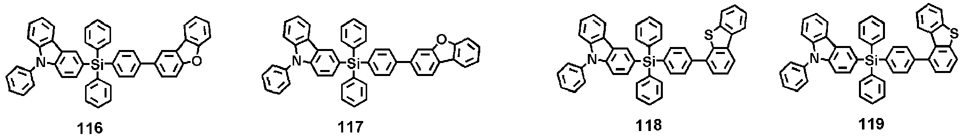

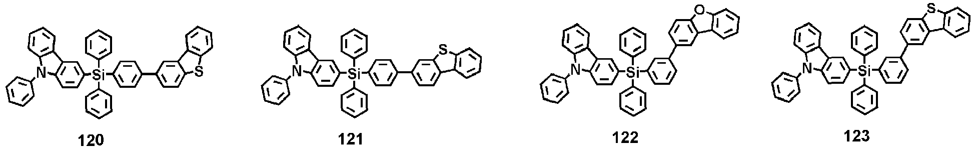

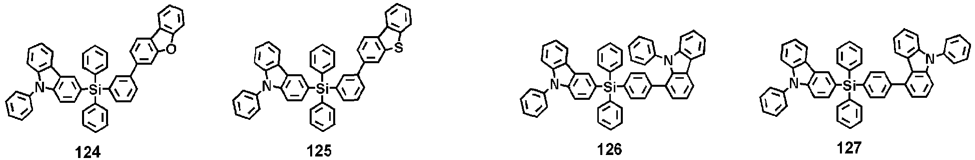

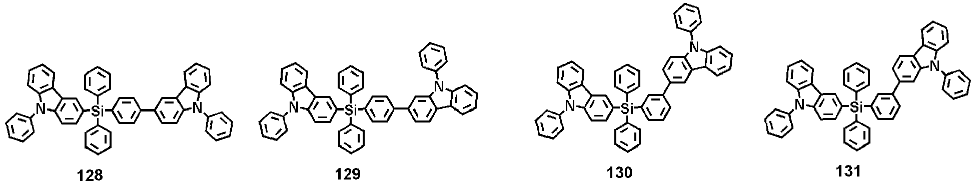

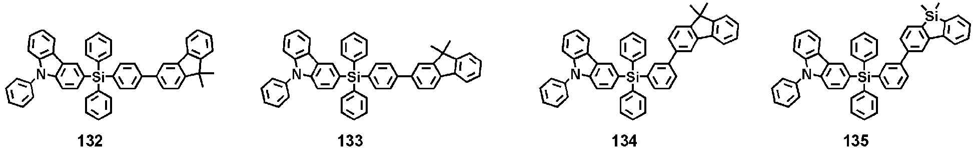

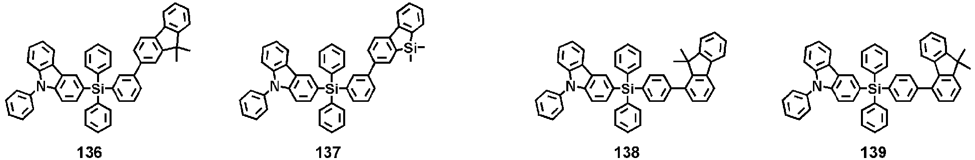

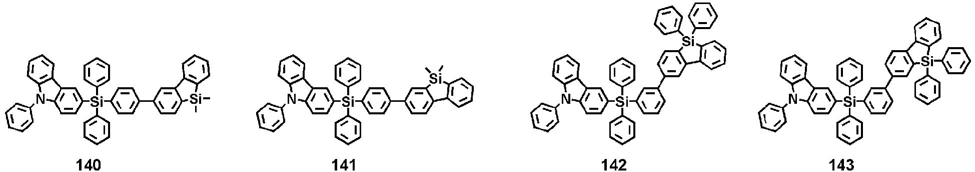

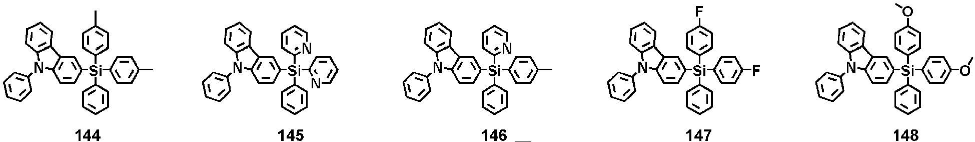

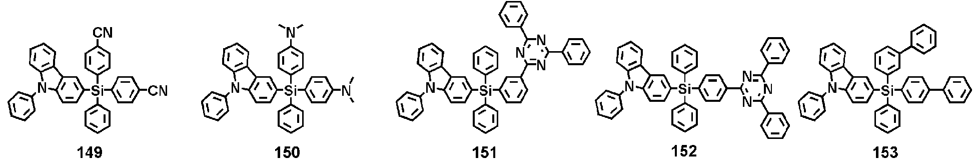

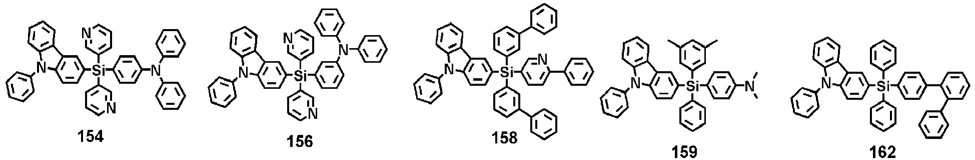

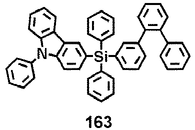

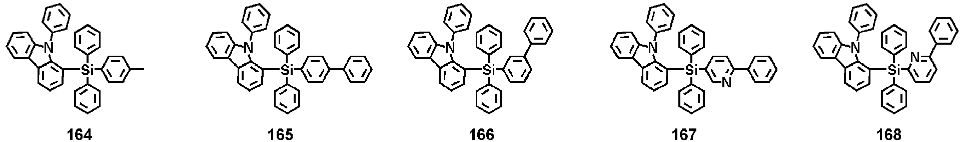

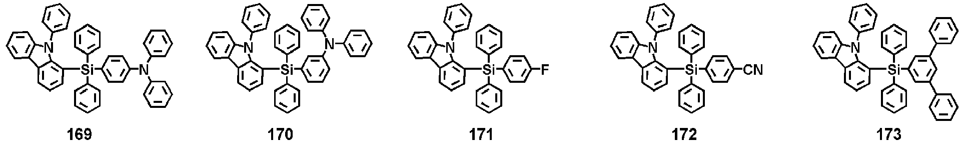

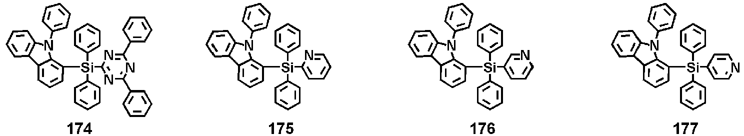

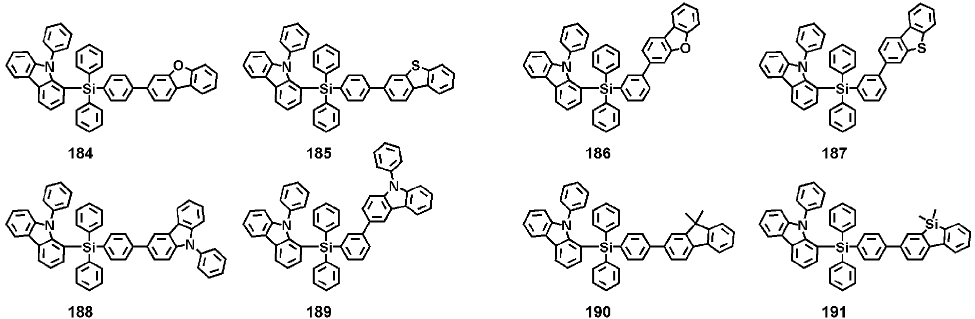

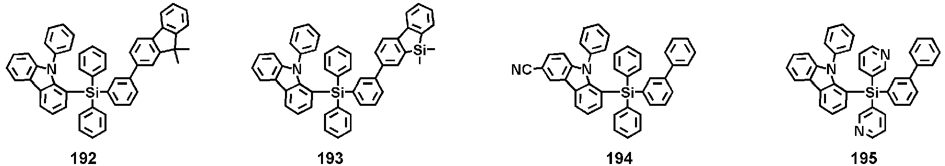

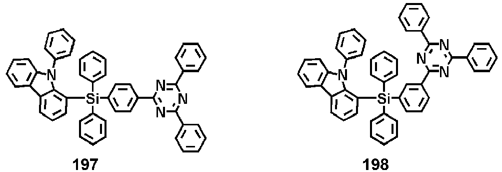

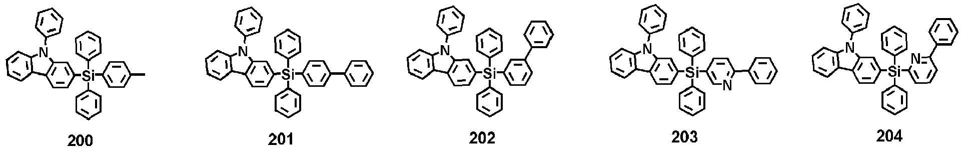

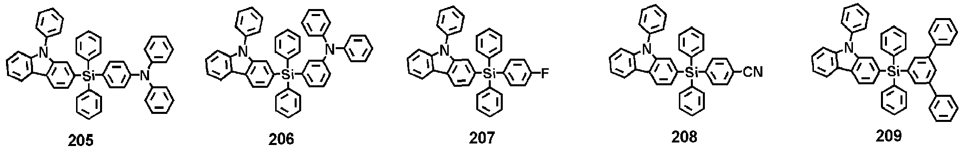

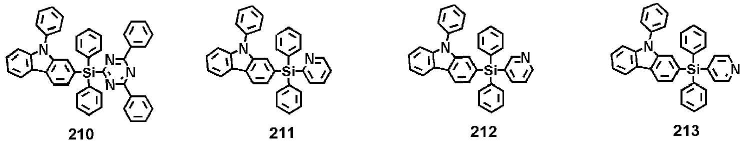

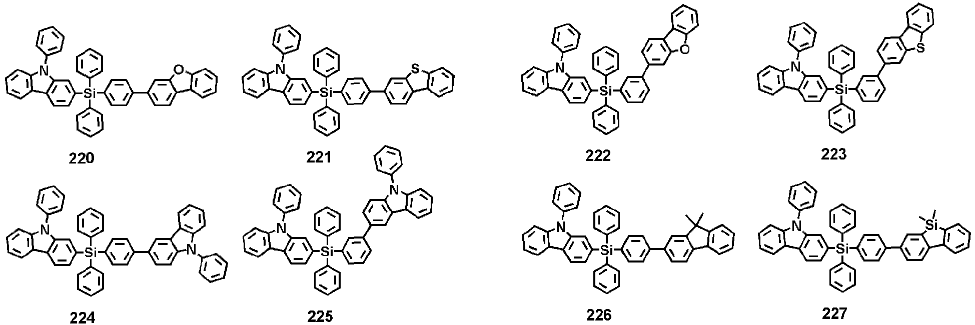

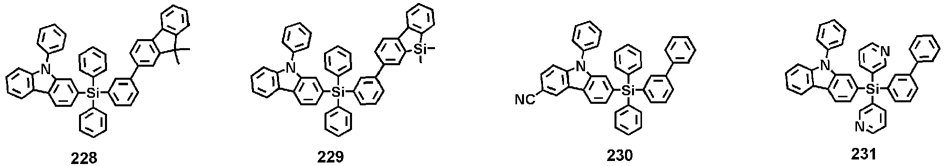

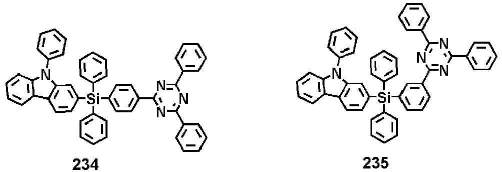

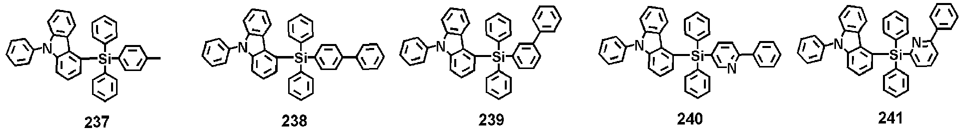

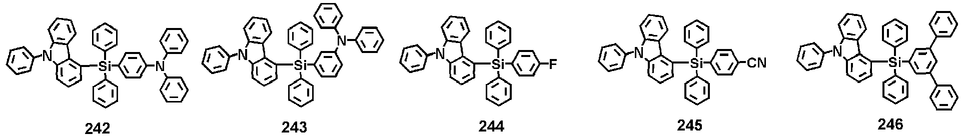

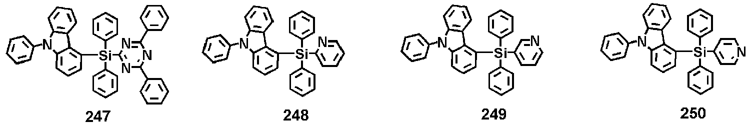

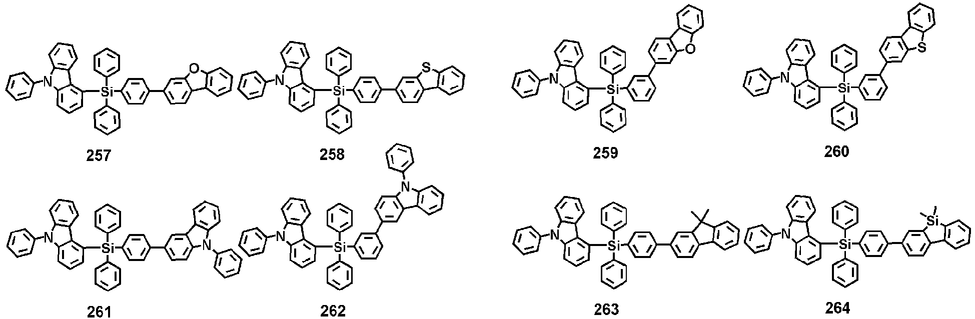

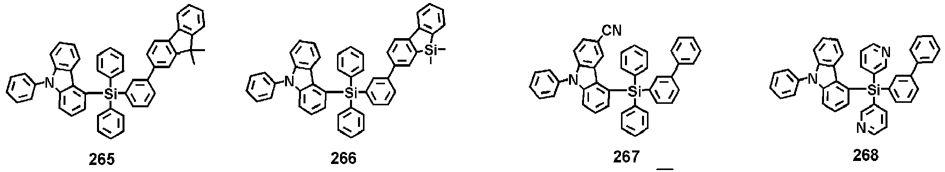

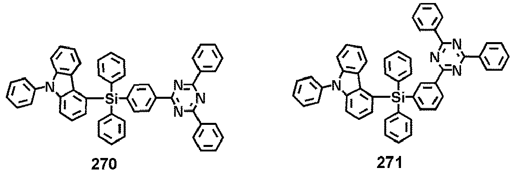

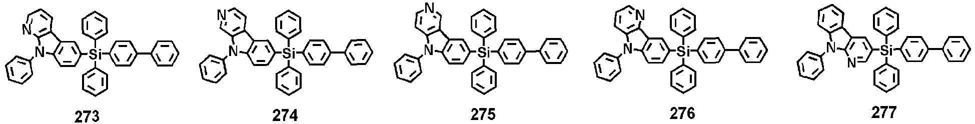

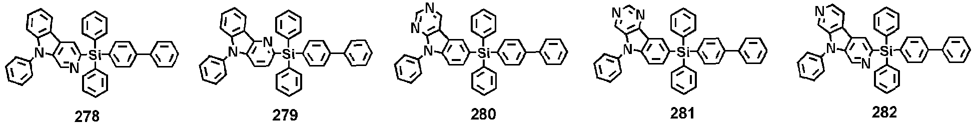

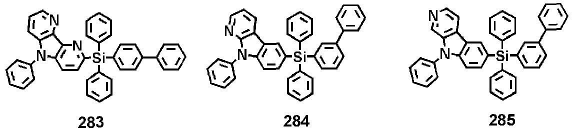

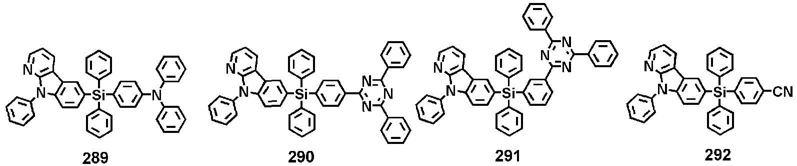

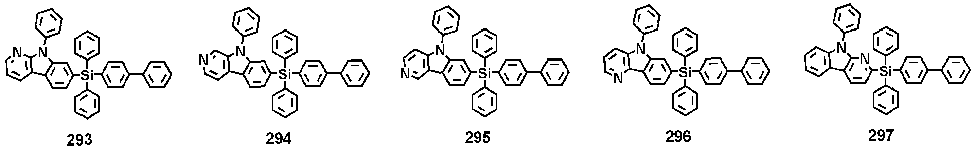

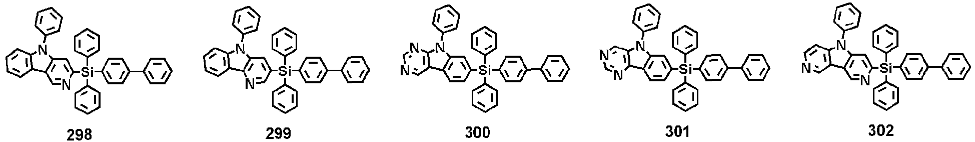

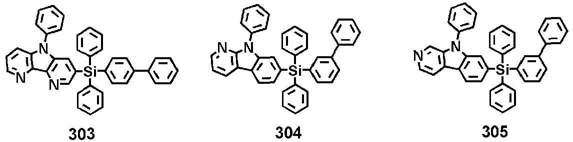

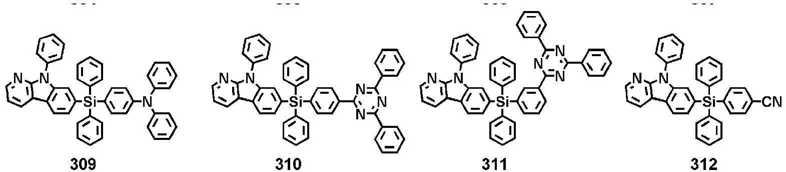

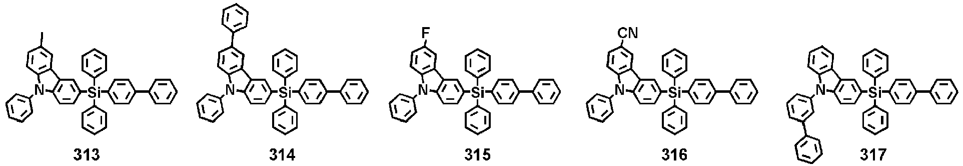

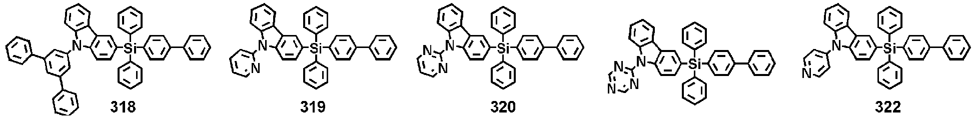

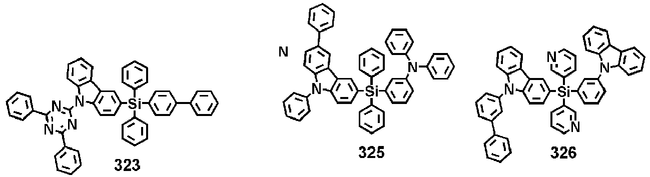

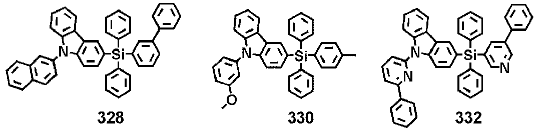

Ar₁ to Ar₃ in the condensed cyclic compound represented by one of Formulae 1A to 1D are be each independently a group derived from a C₅-C₉ non-condensed carbocyclic group or a C₁-C₇ non-condensed heterocyclic group. Accordingly, in comparison with an imaginary compound having the same structure as the instant condensed cyclic compound except that at least one selected from Ar₁ to Ar₃ is a condensed cyclic group, the instant condensed cyclic compound may have higher triplet energy. Thus, the condensed cyclic compound may be suitable for use as a material for an organic layer of an organic light-emitting device, for example, as a host material in an emission layer.

In the condensed cyclic compound represented by one of Formulae 1A to 1D, R₁ to R₉ and R₁₁ to R₁₃ do not include a "silyl group" as defined in the present specification. That is, each of Formulae 1A to 1D has only one " silyl group". Accordingly, in comparison with an imaginary compound having the same structure as the instant condensed cyclic compound except that two or more "silyl groups" are included in the imaginary compound, the instant condensed cyclic compound represented by one of Formulae 1A to 1D has higher charge mobility. Thus, the instant condensed cyclic compound may result in an organic light-emitting device having a low driving voltage.

Furthermore, in each of Formulae 1A to 1D, the case wherein *-Ar₁-(R₁₁)ₐ₁, *-Ar₂-(R₁₂)ₐ₂ and *-Ar₃-(R₁₃)ₐ₃ are all identical is excluded. As a result, the condensed cyclic compound represented by one of Formulae 1A to 1D has asymmetric structure around "silicon," and thus, resulting in low crystallinity of the compound. Accordingly, a thin film formed by using the condensed cyclic compound has excellent amorphous characteristics and thermal stability, and due to such characteristics, excellent surface morphology may be obtained. Thus, an organic light-emitting device including the condensed cyclic compound may have improved efficiency and lifespan characteristics.

A method of synthesizing the condensed cyclic compound represented by one of Formulae 1A to 1D may be understood by one of ordinary skill in the art by referring to Synthesis Examples used herein.

Since the condensed cyclic compound represented by one of Formulae 1A to 1D is appropriate for use in an organic layer of an organic light-emitting device, for example, for use as a host in an emission layer of the organic layer, another aspect provides an organic light-emitting device including:
a first electrode;
a second electrode; and
an organic layer that is disposed between a first electrode and a second electrode,
wherein the organic layer includes an emission layer and at least one condensed cyclic compound represented by one of Formulae 1A to 1D.

Due to the inclusion of the condensed cyclic compound represented by one of Formulae 1A to 1D, the organic light-emitting device may have low driving voltage, high efficiency, high brightness, and long lifespan.

The condensed cyclic compound represented by one of Formulae 1A to 1D may be used between a pair of electrode of an organic light-emitting device. For example, the condensed cyclic compound may be included in at least one selected from an emission layer, a hole transport region (including, for example, at least one of a hole injection layer, a hole transport layer, a buffer layer, and an electron blocking layer) that is disposed between the first electrode and the emission layer, and an electron transport region (including, for example, at least one selected from a hole blocking layer, an electron transport layer, and an electron injection layer) that is disposed between the emission layer and the second electrode. For example, the condensed cyclic compound represented by one of Formulae 1A to 1D may be included in the emission layer. In this regard, the emission layer further includes a dopant (for example, a phosphorescent dopant or a fluorescent dopant), and the condensed cyclic compound included in the emission layer acts as a host. The emission layer may be a green emission layer emitting green light or a blue emission layer emitting blue light, and the dopant may be a phosphorescent dopant.

The expression that "(an organic layer) includes at least one condensed cyclic compound" used herein may include a case in which "(an organic layer) includes identical compounds represented by Formulae 1A to 1D and a case in which (an organic layer) includes two or more different condensed cyclic compounds represented by Formulae 1A to 1D.

For example, the organic layer may include, as the condensed cyclic compound, only Compound 1. In this regard, Compound 1 may be situated in an emission layer of the organic light-emitting device. In another embodiment, the organic layer may include, as the condensed cyclic compound, Compound 1 and Compound 2. In this regard, Compound 1 and Compound 2 may be situated in either an identical layer (for example, Compound 1 and Compound 2 all may exist in an emission layer), or different layers.

The first electrode may be an anode, which is a hole injection electrode, and the second electrode may be a cathode, which is an electron injection electrode, or the first electrode may be a cathode, which is an electron injection electrode, or the second electrode may be an anode, which is a hole injection electrode.

For example, the first electrode is an anode, and the second electrode is a cathode, and the organic layer includes:
i) a hole transport region that is disposed between the first electrode and the emission layer, wherein the hole transport region includes at least one of a hole injection layer, a hole transport layer, and an electron blocking layer, and
ii) an electron transport region that is disposed between the emission layer and the second electrode, wherein the electron transport region includes at least one selected from a hole blocking layer, an electron transport layer, and an electron injection layer.

The term "organic layer" used herein refers to a single layer and/or a plurality of layers disposed between the first electrode and the second electrode of an organic light-emitting device. The "organic layer" may include, in addition to an organic compound, an organometallic complex including metal.

FIG. 1 is a schematic view of an organic light-emitting device 10 according to an embodiment. Hereinafter, the structure of an organic light-emitting device according to an embodiment and a method of manufacturing an organic light-emitting device according to an embodiment will be described in connection with FIG. 1. The organic light-emitting device 10 includes a first electrode 11, an organic layer 15, and a second electrode 19, which are sequentially stacked.

In FIG. 1, a substrate may be additionally disposed under the first electrode 11 or above the second electrode 19. For use as the substrate, any suitable substrate that is used in general organic light-emitting devices may be used, and the substrate may be a glass substrate or transparent plastic substrate, each with excellent mechanical strength, thermal stability, transparency, surface smoothness, ease of handling, and water-proofness.

The first electrode 11 may be formed by depositing or sputtering a material for forming the first electrode on the substrate. The first electrode 11 may be an anode. The material for the first electrode 11 may be selected from materials with a high work function to allow holes be easily provided. The first electrode 11 may be a reflective electrode or a transmissive electrode. The material for the first electrode 11 may be an indium tin oxide (ITO), indium zinc oxide (IZO), tin oxide (SnO₂), or zinc oxide (ZnO). In some embodiments, the material for the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layer structure or a multi-layer structure including two or more layers. For example, the first electrode 11 may have a three-layered structure of ITO/Ag/ITO, but the structure of the first electrode 11 is not limited thereto.

An organic layer 15 is disposed on the first electrode 11.

The organic layer 15 may include a hole transport region, an emission layer, and an electron transport region.

The hole transport region may be disposed between the first electrode 11 and the emission layer.

The hole transport region may include at least one of a hole injection layer, a hole transport layer, an electron blocking layer, and a buffer layer.

The hole transport region may include only either a hole injection layer or a hole transport layer. According to another embodiment, the hole transport region may have a structure of hole injection layer/hole transport layer or hole injection layer/hole transport layer/electron blocking layer, which are sequentially stacked in this stated order from the first electrode 11.

When the hole transport region includes a hole injection layer (HIL), the hole injection layer may be formed on the first electrode 11 by using any one of various methods, for example, vacuum deposition, spin coating, casting, or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 to about 500°C, a vacuum pressure of about 1.33x10⁻⁶ to about 0.133 Pa (10⁻⁸ to about 10⁻³ torr), and a deposition rate of about 1-10,000 picometer (pm) per second (i.e. about 0.01 to about 100 Angstroms per second (Å/sec)). However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 revolutions per minute (rpm) to about 5,000 rpm, and a temperature at which a heat treatment is performed to remove a solvent after coating may be from about 80°C to about 200°C. However, the coating conditions are not limited thereto.

Conditions for a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), (polyaniline)/poly(4-styrenesulfonate) (PANI/PSS), a compound represented by Formula 201 below, and a compound represented by Formula 202 below:

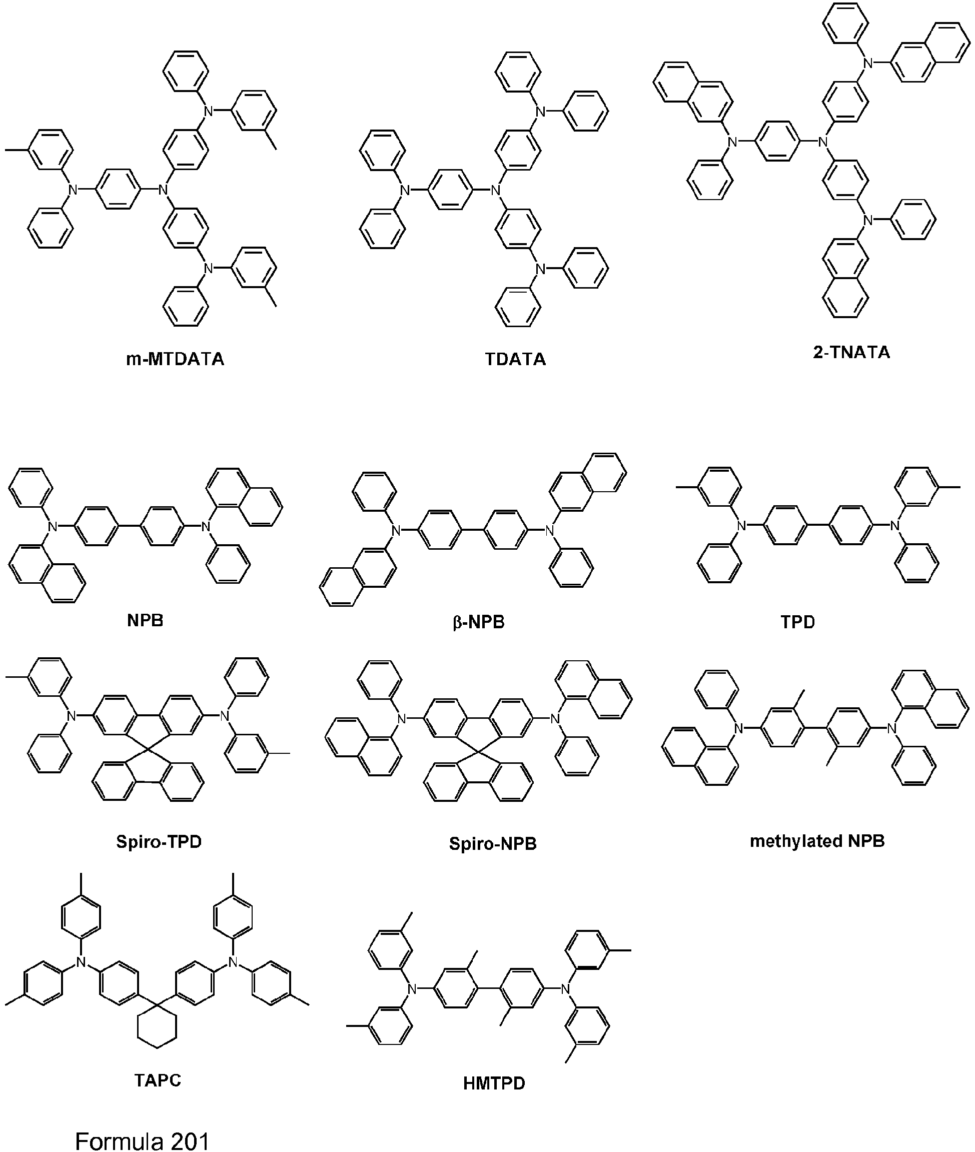

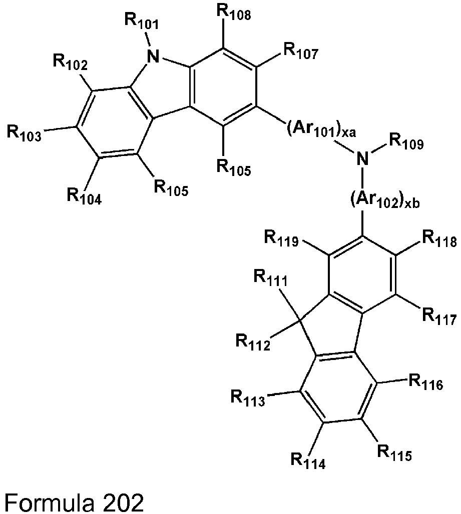

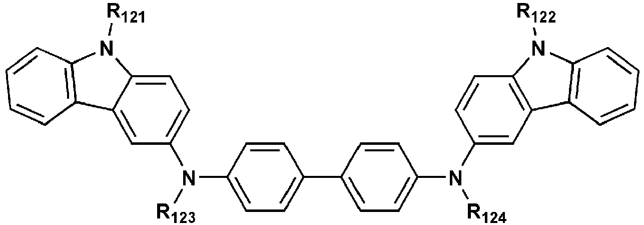

Ar₁₀₁ and Ar₁₀₂ in Formula 201 may be each independently selected from
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group, each substituted with at least one of a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.
xa and xb in Formula 201 may be each independently an integer of 0 to 5, or 0, 1, or 2. For example, xa may be 1 and xb may be 0, but xa and xb are not limited thereto.
R₁₀₁ to R₁₀₈, R₁₁₁ to R₁₁₉, and R₁₂₁ to R₁₂₄ in Formulae 201 and 202 may be each independently selected from
a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, a hexyl group, and so on), or a C₁-C₁₀ alkoxy group (for example, a methoxy group, an ethoxy group, a propoxy group, a butoxy group, a pentoxy group, and so on);
a C₁-C₁₀ alkyl group or a C₁-C₁₀ alkoxy group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof;
a phenyl group, a naphthyl group, an anthracenyl group, a fluorenyl group, or a pyrenyl group; or
a phenyl group, a naphthyl group, an anthracenyl group, a fluorenyl group, and a pyrenyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group and a C₁-C₁₀ alkoxy group, but they are not limited thereto.
R₁₀₉ in Formula 201 may be
a phenyl group, a naphthyl group, an anthracenyl group, and a pyridinyl group; and
a phenyl group, a naphthyl group, an anthracenyl group, or a pyridinyl group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group.

According to an embodiment, the compound represented by Formula 201 may be represented by Formula 201A below, but is not limited thereto:

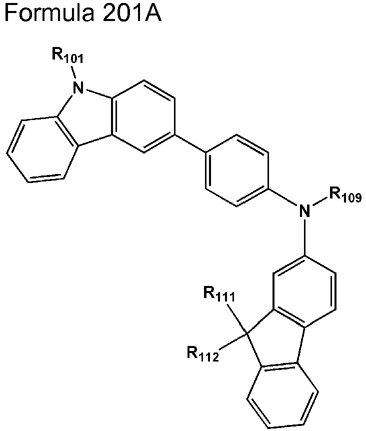

R₁₀₁, R₁₁₁, R₁₁₂, and R₁₀₉ in Formula 201A may be understood by referring to the description provided herein.

For example, the compound represented by Formula 201, and the compound represented by Formula 202 may include compounds HT1 to HT20 illustrated below, but are not limited thereto.

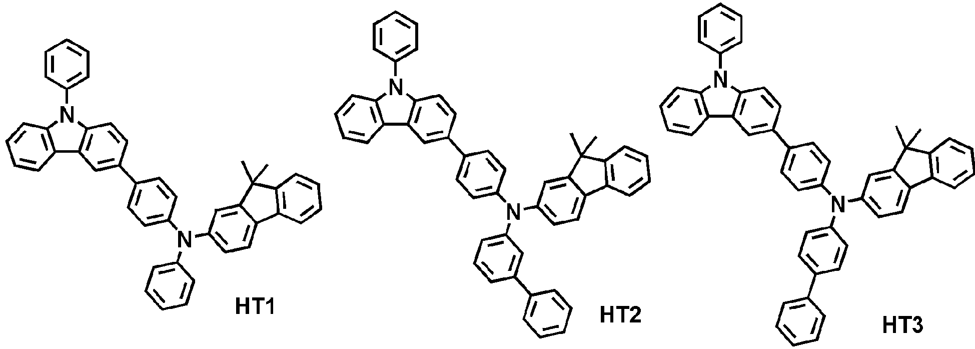

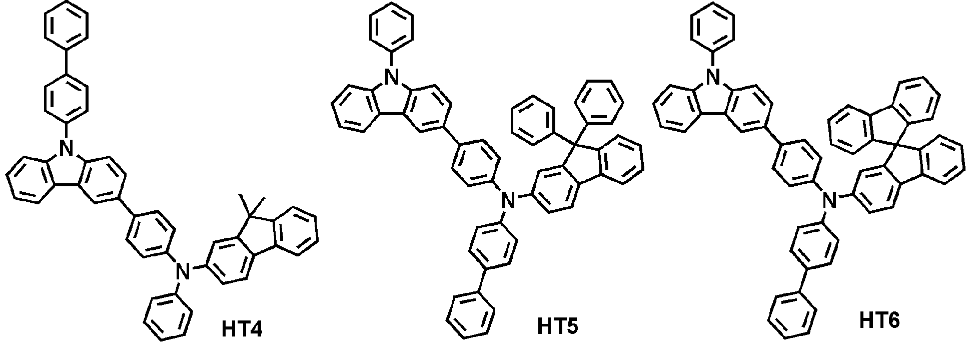

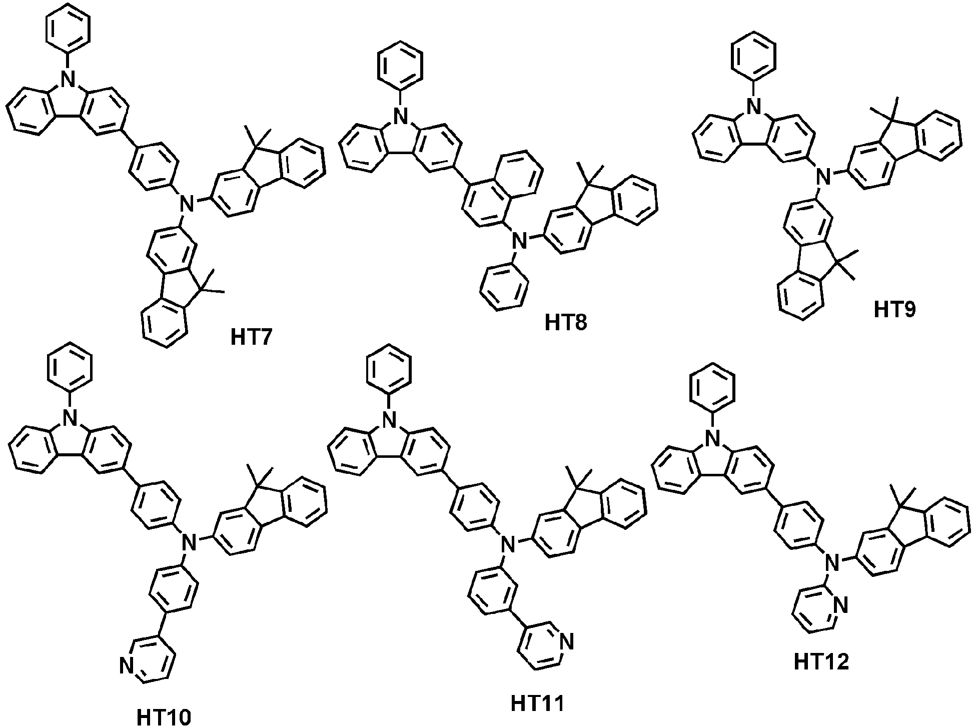

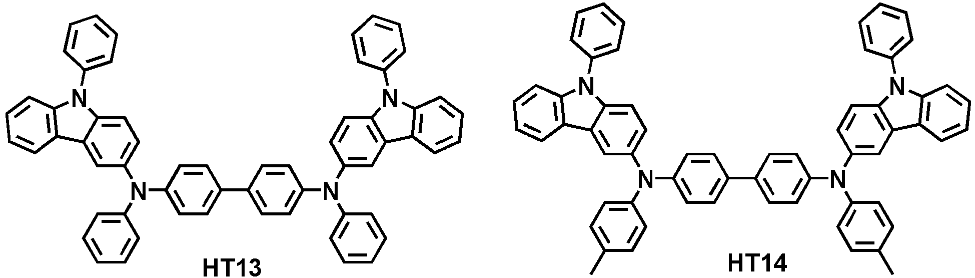

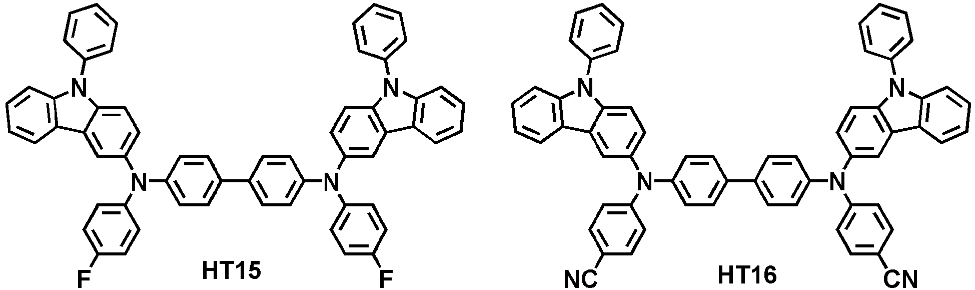

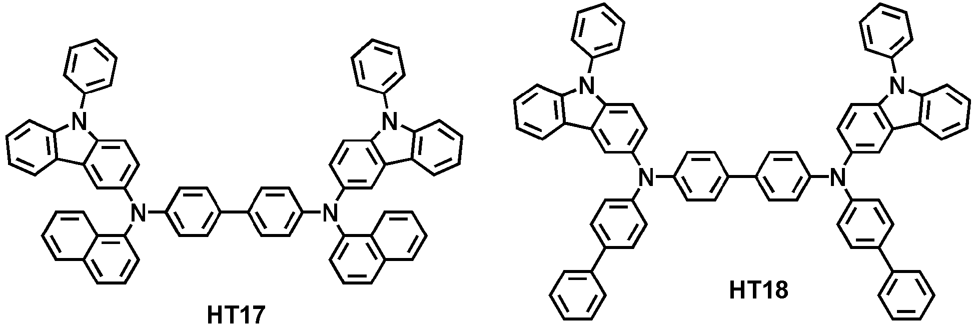

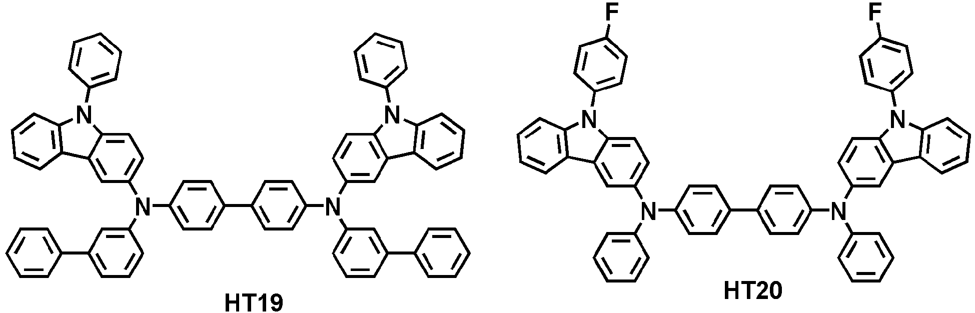

The hole transport region may include a buffer layer.

Also, the buffer layer may compensate for an optical resonance distance according to a wavelength of light emitted from the emission layer, and thus, efficiency of a formed organic light-emitting device may be improved.

Then, an emission layer (EML) may be formed on the hole transport region by vacuum deposition, spin coating, casting, LB deposition, or the like. When the emission layer is formed by vacuum deposition or spin coating, the deposition or coating conditions may be similar to those applied to form the hole injection layer, although the deposition or coating conditions may vary according to the material that is used to form the emission layer.

The hole transport region may further include an electron blocking layer to make the balance of charge mobility. A material for the electron blocking layer may be any known material, and for example, the electron blocking layer may include mCP, but the inventive concept is not limited thereto.

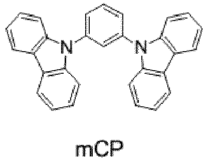

A thickness of the hole transport region may be in a range of about 10 nm (100 Å) to about 1,000 nm (10,000 Å), for example, about 10 nm (100 Å) to about 100 nm (1,000 Å). When the hole transport region includes both a hole injection layer and a hole transport layer, a thickness of the hole injection layer may be in a range of about 10 nm (100 Å) to about 1,000 nm (10,000 Å), for example, about 10 nm (100 Å) to about 100 nm (1,000 Å), and a thickness of the hole transport layer may be in a range of about 5 nm (50 Å) to about 200 nm (2,000 Å), for example about 10 nm (100 Å) to about 150 nm (1,500 Å). When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one of a quinone derivative, a metal oxide, and a cyano group-containing compound, but is not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenium oxide; and a cyano group-containing compound, such as Compound HT-D1 below, but are not limited thereto.

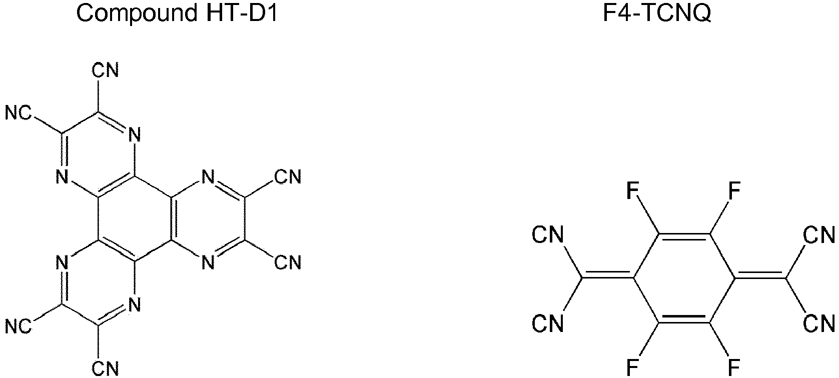

When the organic light-emitting device is a full color organic light-emitting device, the emission layer may be patterned into a red emission layer, a green emission layer, and a blue emission layer. According to another embodiment, due to a stack structure including a red emission layer, a green emission layer, and/or a blue emission layer, the emission layer may emit white light.

The emission layer may include the condensed cyclic compound represented by one of Formulae 1A to 1D. The emission layer may include a dopant. The dopant may include at least one selected from a phosphorescent dopant and a fluorescent dopant.

For example, a host in the emission layer may include the condensed cyclic compound represented by one of Formulae 1A to 1D.

A dopant in the emission layer may be a fluorescent dopant that emits light according to a fluorescent emission mechanism or a phosphorescent dopant that emits light according to a phosphorescent emission mechanism.

According to an embodiment, the dopant in the emission layer may be a phosphorescent dopant, and the phosphorescent dopant may include an organometallic compound represented by Formula 81 below: 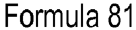 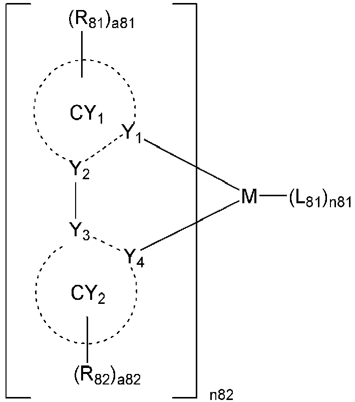 wherein in Formula 81,
M may be selected from iridium (Ir), platinum (Pt), osmium (Os), titanium (Ti), zirconium (Zr), hafnium (Hf), europium (Eu), terbium (Tb), and thulium (Tm);
Y₁ to Y₄ are each independently carbon (C) or nitrogen (N);
Y₁ and Y₂ are linked via a single bond or a double bond, and Y₃ and Y₄ are linked via a single bond or a double bond;
CY₁ and CY₂ are each independently selected from a benzene, a naphthalene, a fluorene, a spiro-fluorene, an indene, a pyrrole, a thiophene, a furan, an imidazole, a pyrazole, a thiazole, an isothiazole, an oxazole, an isooxazole, a pyridine, a pyrazine, a pyrimidine, a pyridazine, a quinoline, an isoquinoline, a benzoquinoline, a quinoxaline, a quinazoline, a carbazole, a benzoimidazole, a benzofuran, a benzothiophene, an isobenzothiophene, a benzooxazole, an isobenzooxazole, a triazole, a tetrazole, an oxadiazole, a triazine, a dibenzofuran, and a dibenzothiophene, provided that CY₁ and CY₂ are optionally linked to each other through a single bond or an organic linking group;
R₈₁ to R₈₂ may be each independently selected from a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), and -B(Q₆)(Q₇);
a81 and a82 are each independently an integer of 1 to 5;
n81 is an integer of 0 to 4;
n82 is 1, 2, or 3; and
L₈₁ is a monovalent organic ligand, a divalent organic ligand, or a trivalent organic ligand.

Descriptions of R₈₁ and R₈₂ are the same as defined in connection with R₄₁, and descriptions of Q₂ to Q₇ are the same as defined in connection with Q₁.

The phosphorescent dopant may include at least one of Compounds PD1 to PD78 below, but is not limited thereto:

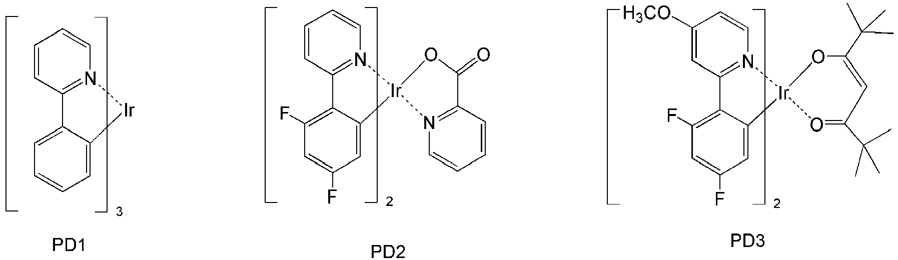

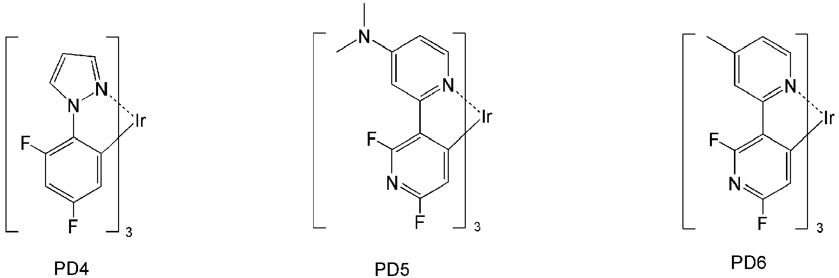

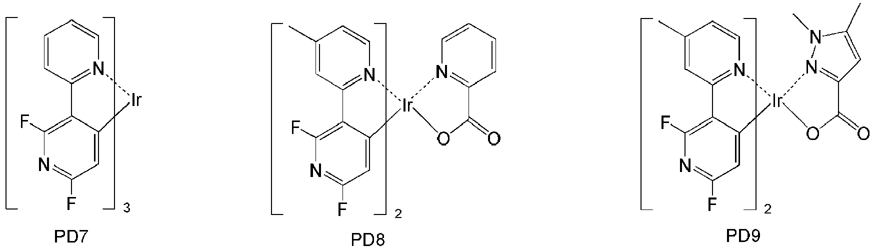

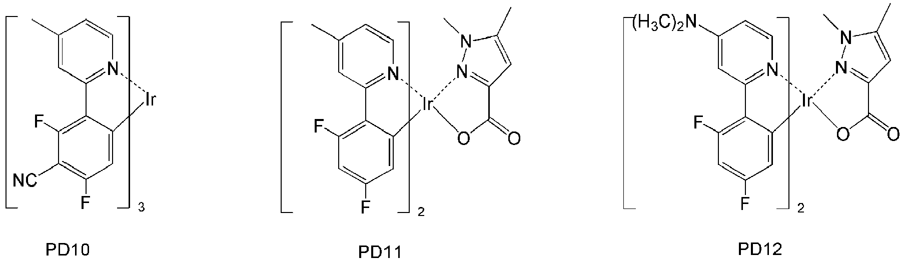

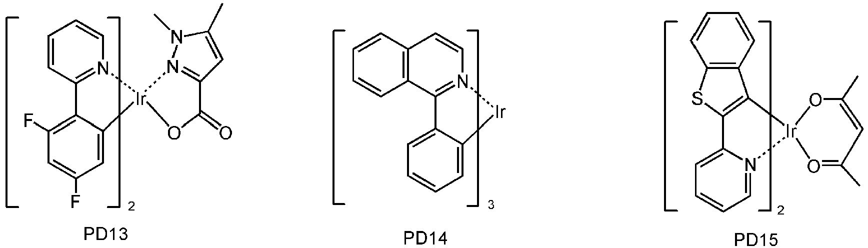

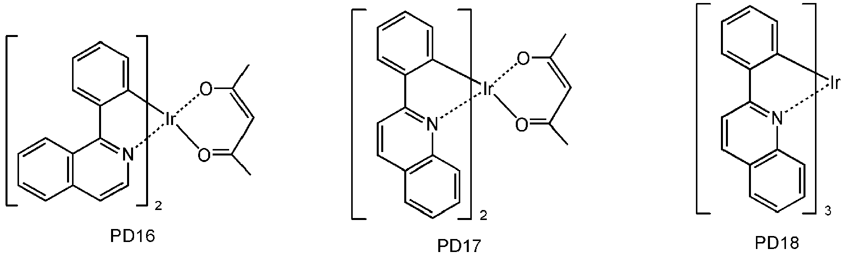

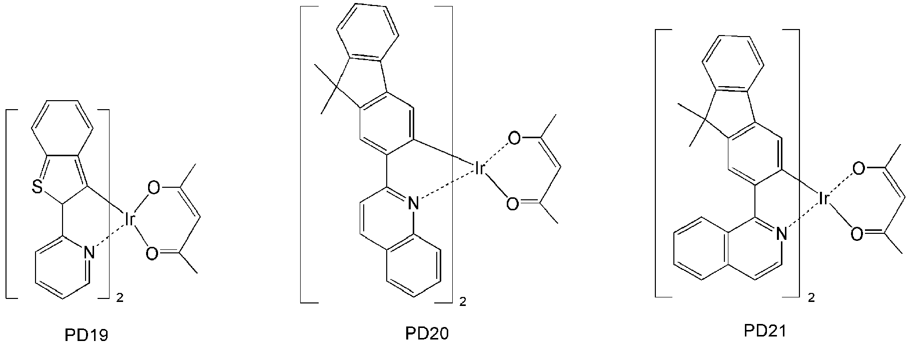

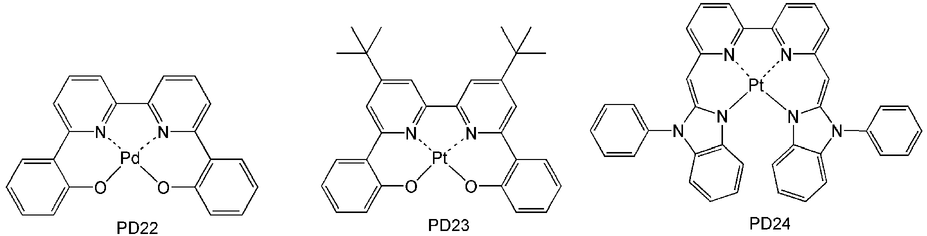

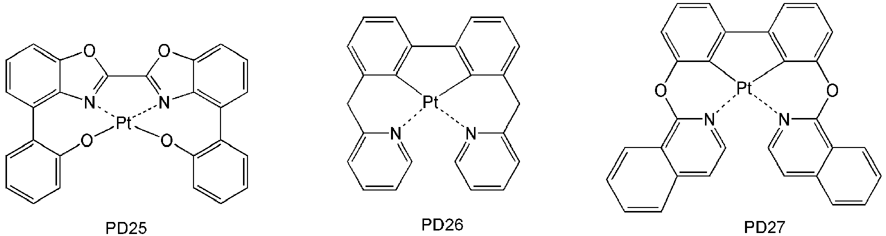

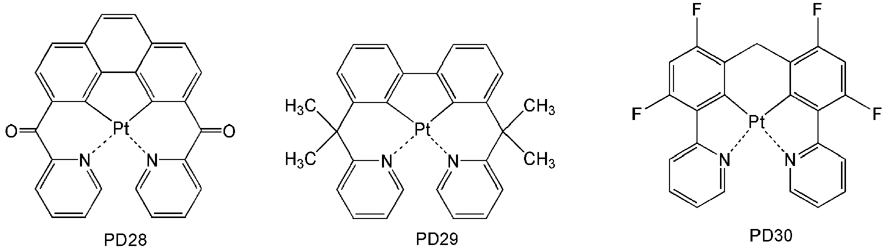

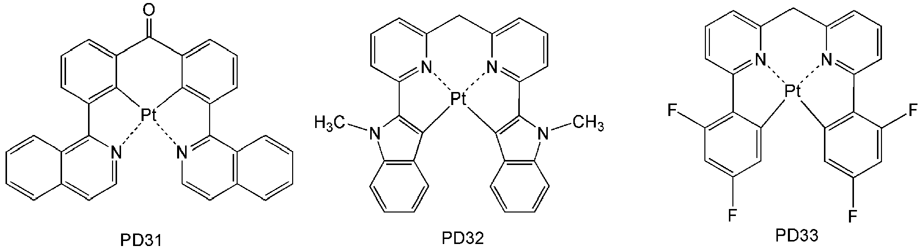

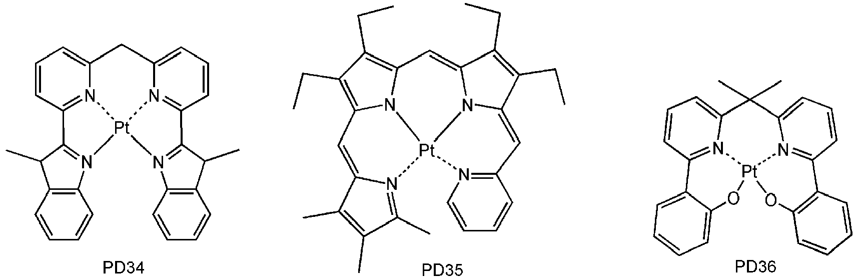

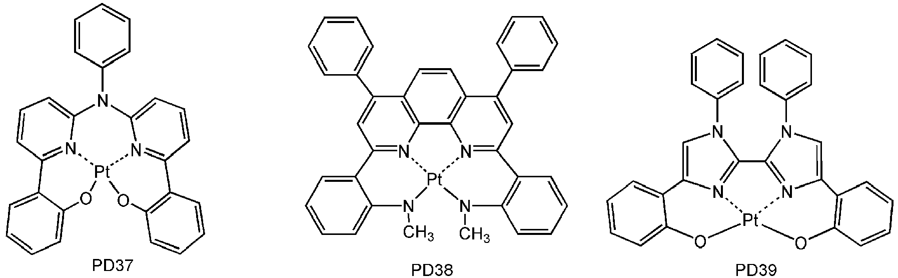

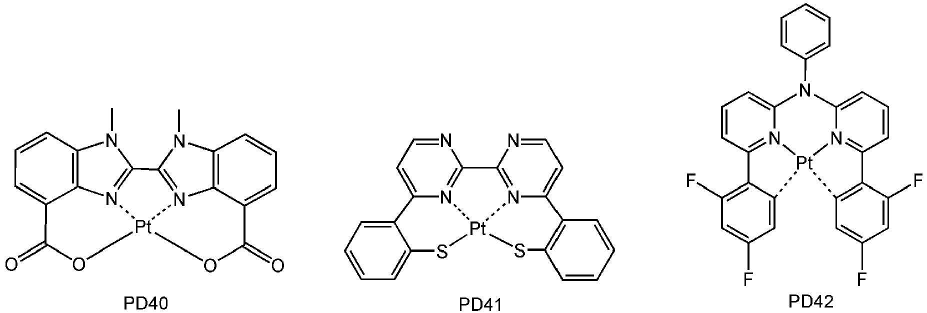

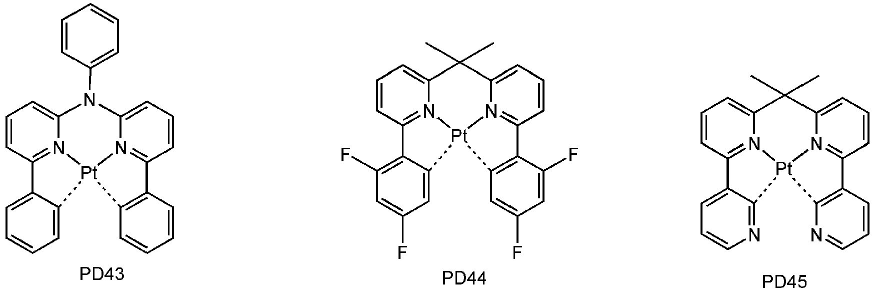

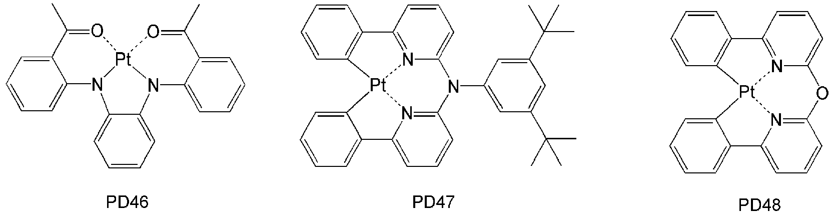

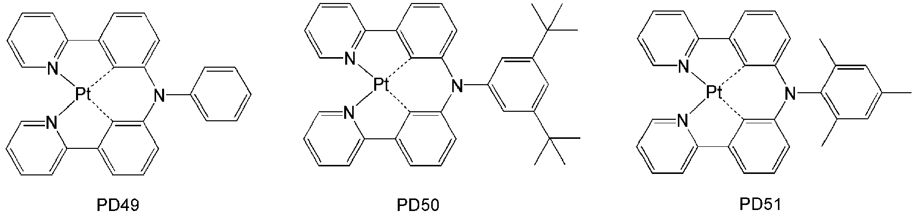

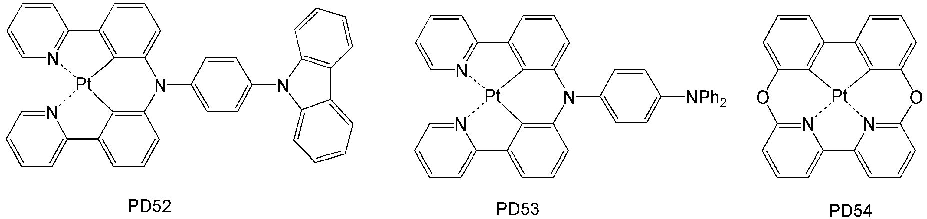

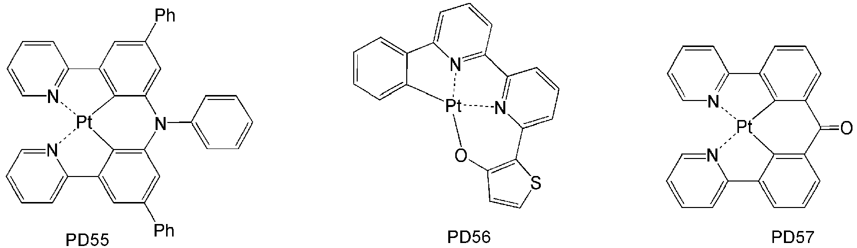

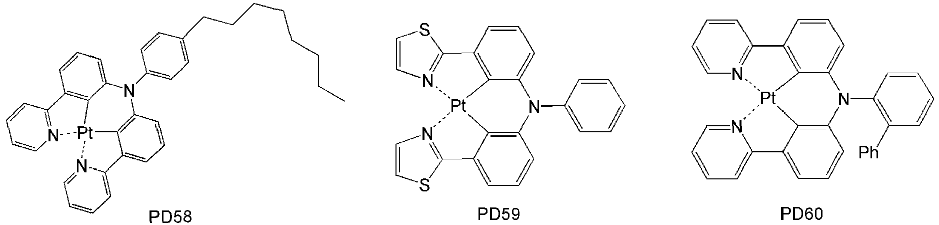

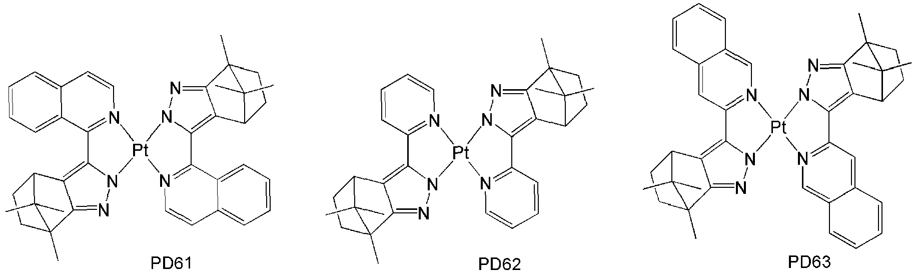

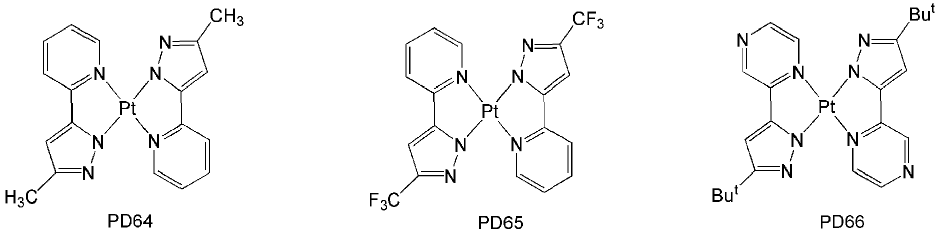

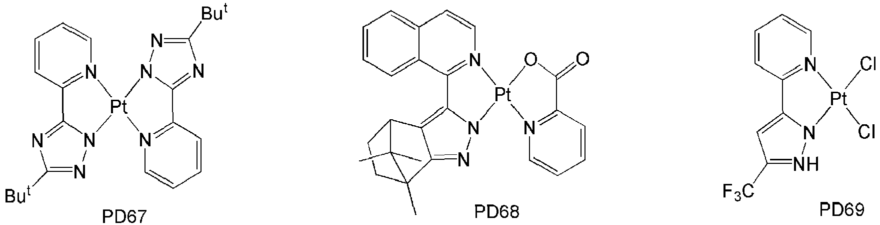

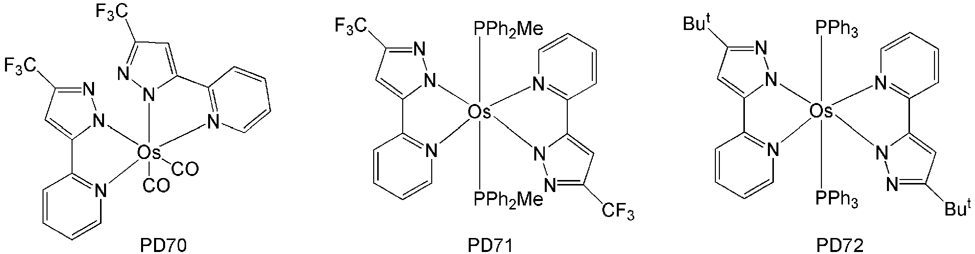

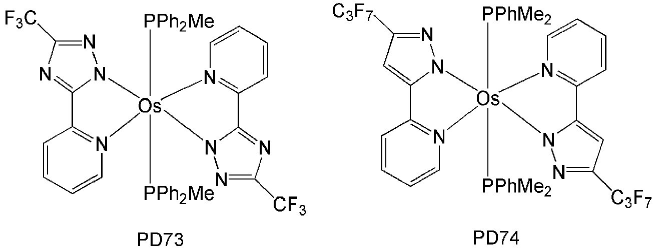

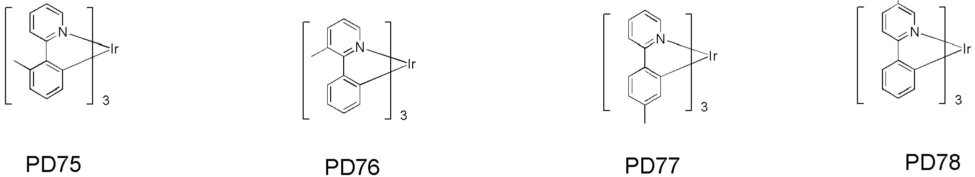

In some embodiments, the phosphorescent dopant may include PtOEP or FIr₆:

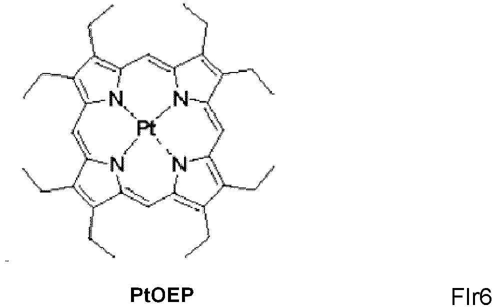

The fluorescent dopant may include at least one selected from DPAVBi, BDAVBi, TBPe, DCM, DCJTB, Coumarin 6, and C545T.

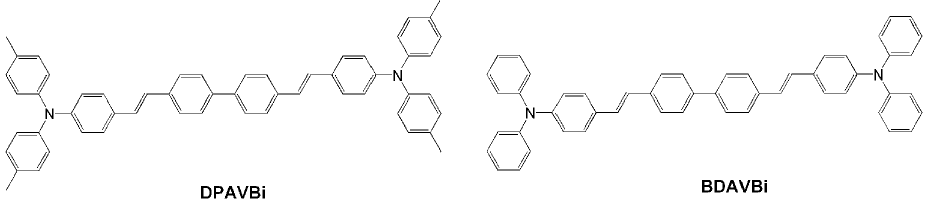

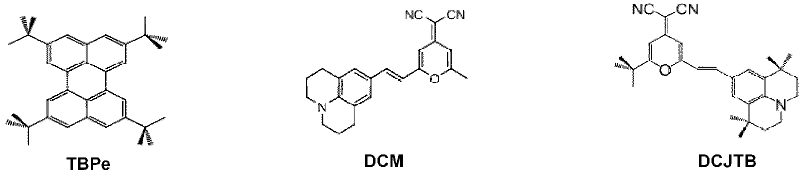

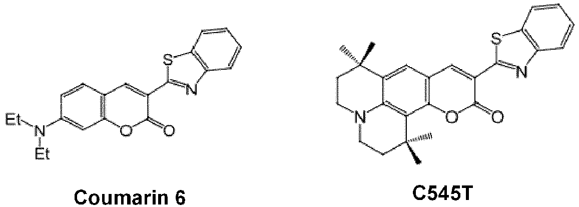

When the emission layer includes a host and a dopant, an amount of the dopant may be in a range of about 0.01 to about 20 parts by weight based on 100 parts by weight of the host, but is not limited thereto.

A thickness of the emission layer may be in a range of about 10 nm (100 Å) to about 100 nm (1,000 Å), for example, about 20 nm (200 Å) to about 60 nm (600 Å). When the thickness of the emission layer is within this range, excellent light-emission characteristics may be obtained without a substantial increase in driving voltage.

Then, an electron transport region may be disposed on the emission layer.

The electron transport region may include at least one selected from a hole blocking layer, an electron transport layer, and an electron injection layer.

For example, the electron transport region may have a structure of hole blocking layer/electron transport layer/electron injection layer or a structure of electron transport layer/electron injection layer, but the structure of the electron transport region is not limited thereto. The electron transport layer may have a single-layered structure or a multi-layer structure including two or more different materials.

Conditions for forming the hole blocking layer, the electron transport layer, and the electron injection layer which constitute the electron transport region may be understood by referring to the conditions for forming the hole injection layer.

When the electron transport layer includes a hole blocking layer, the hole blocking layer may include, for example, at least one of BCP, Bphen, and TmPyPB, but the inventive concept is not limited thereto.

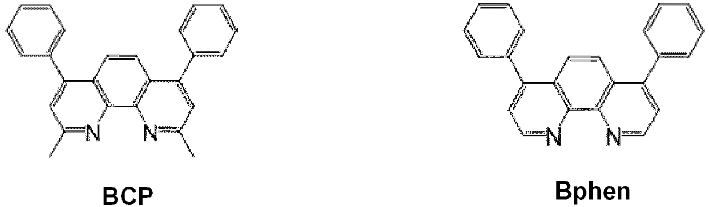

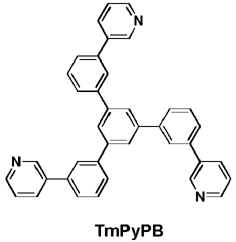

A thickness of the hole blocking layer may be in a range of about 2 nm (20 Å) to about 100 nm (1,000 Å), for example, about 3 nm (30 Å) to about 30 nm (300 Å). When the thickness of the hole blocking layer is within these ranges, the hole blocking layer may have excellent hole blocking characteristics without a substantial increase in driving voltage.

The electron transport layer may further include at least one selected from BCP, Bphen, Alq₃, Balq, TAZ, and NTAZ.

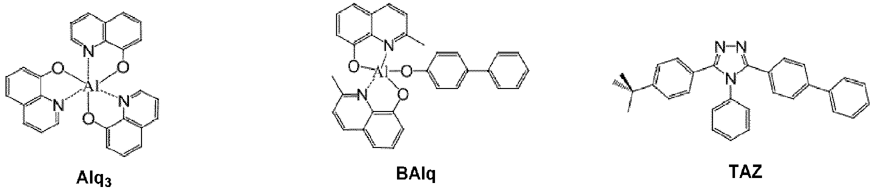

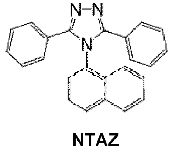

According to another embodiment, the electron transport layer may include at least one of ET1 and ET2, but are not limited thereto:

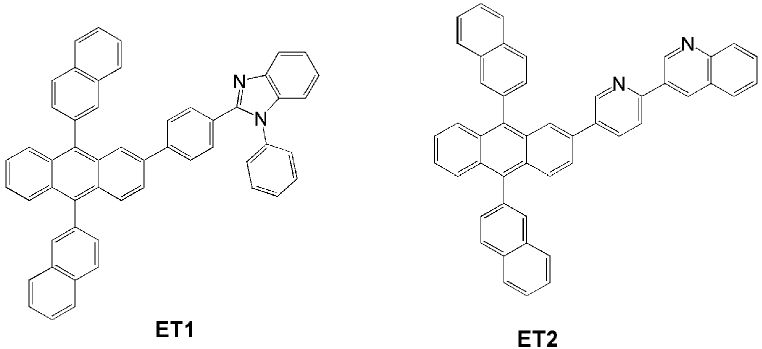

A thickness of the electron transport layer may be in a range of about 10 nm (100 Å) to about 100 nm (1,000 Å), for example, about 15 nm (150 Å) to about 50 nm (500 Å). When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium quinolate, LiQ) or ET-D2.

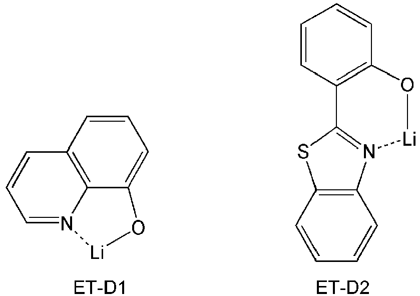

The electron transport region may include an electron injection layer (EIL) that allows electrons to be easily provided from a second electrode 19.

The electron injection layer may include at least one selected from, LiF, NaCl, CsF, Li₂O, BaO, and LiQ.

A thickness of the electron injection layer may be in a range of about 0.1 nm (1 Å) to about 10 nm (100 Å), for example, about 0.3 nm (3 Å) to about 9 nm (90 Å). When the thickness of the electron injection layer is within the range described above, the electron injection layer may have satisfactory electron injection characteristics without a substantial increase in driving voltage.

The second electrode 19 is disposed on the organic layer 15. The second electrode 19 may be a cathode. A material for forming the second electrode 19 may be metal, an alloy, an electrically conductive compound, and a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be formed as the material for forming the second electrode 19. To manufacture a top emission type light-emitting device, a transmissive electrode formed using ITO or IZO may be used as the second electrode 19.

Hereinbefore, the organic light-emitting device has been described with reference to FIG. 1, but is not limited thereto.

A C₁-C₆₀ alkyl group used herein refers to a linear or branched aliphatic hydrocarbon monovalent group having 1 to 60 carbon atoms. Detailed examples thereof are a methyl group, an ethyl group, a propyl group, an isobutyl group, a sec-butyl group, a tert-butyl group, a pentyl group, an iso-amyl group, and a hexyl group. A C₁-C₆₀ alkylene group used herein refers to a divalent group having the same structure as the C₁-C₆₀ alkyl group.

A C₁-C₆₀ alkoxy group used herein refers to a monovalent group represented by -OA₁₀₁ (wherein A₁₀₁ is the C₁-C₆₀ alkyl group). Detailed examples thereof are a methoxy group, an ethoxy group, and an isopropyloxy group.

A C₂-C₆₀ alkenyl group used herein refers to a hydrocarbon group formed by substituting at least one carbon double bond in the middle or at the terminal of the C₂-C₆₀ alkyl group. Detailed examples thereof are an ethenyl group, a propenyl group, and a butenyl group. A C₂-C₆₀ alkenylene group used herein refers to a divalent group having the same structure as the C₂-C₆₀ alkenyl group.

A C₂-C₆₀ alkynyl group used herein refers to a hydrocarbon group formed by substituting at least one carbon triple bond in the middle or at the terminal of the C₂-C₆₀ alkyl group. Detailed examples thereof are an ethynyl group, and a propynyl group. A C₂-C₆₀ alkynylene group used herein refers to a divalent group having the same structure as the C2-C60 alkynyl group.

A C₃-C₁₀ cycloalkyl group used herein refers to a monovalent hydrocarbon monocyclic group having 3 to 10 carbon atoms. Detailed examples thereof are a cyclopropyl group, a cyclobutyl group, a cyclopentyl group, a cyclohexyl group, and a cycloheptyl group. A C₃-C₁₀ cycloalkylene group used herein refers to a divalent group having the same structure as the C₃-C₁₀ cycloalkyl group.

A C₁-C₁₀ heterocycloalkyl group used herein refers to a monovalent monocyclic group having at least one hetero atom selected from N, O, P, and S as a ring-forming atom and 1 to 10 carbon atoms. Detailed examples thereof are a tetrahydrofuranyl group, and a tetrahydrothiophenyl group. A C₁-C₁₀ heterocycloalkylene group used herein refers to a divalent group having the same structure as the C₁-C₁₀ heterocycloalkyl group.

A C₃-C₁₀ cycloalkenyl group used herein refers to a monovalent monocyclic group that has 3 to 10 carbon atoms and at least one double bond in the ring thereof, and which is not aromatic. Detailed examples thereof are a cyclopentenyl group, a cyclohexenyl group, and a cycloheptenyl group. A C₃-C₁₀ cycloalkenylene group used herein refers to a divalent group having the same structure as the C₃-C₁₀ cycloalkenyl group.

A C₁-C₁₀ heterocycloalkenyl group used herein refers to a monovalent monocyclic group that has at least one hetero atom selected from N, O, P, and S as a ring-forming atom, 1 to 10 carbon atoms, and at least one double bond in its ring. Detailed examples of the C₁-C₁₀ heterocycloalkenyl group are a 2,3-dihydrofuranyl group and a 2,3-dihydrothiophenyl group. A C₁-C₁₀ heterocycloalkenylene group used herein refers to a divalent group having the same structure as the C₁-C₁₀ heterocycloalkenyl group.

A C₆-C₆₀ aryl group used herein refers to a monovalent group having a carbocyclic aromatic system having 6 to 60 carbon atoms, and a C₆-C₆₀ arylene group used herein refers to a divalent group having a carbocyclic aromatic system having 6 to 60 carbon atoms. Detailed examples of the C₆-C₆₀ aryl group are a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

A C₁-C₆₀ heteroaryl group used herein refers to a monovalent group having a carbocyclic aromatic system that has at least one hetero atom selected from N, O, P, and S as a ring-forming atom, and 1 to 60 carbon atoms. A C₁-C₆₀ heteroarylene group used herein refers to a divalent group having a carbocyclic aromatic system that has at least one hetero atom selected from N, O, P, and S as a ring-forming atom, and 1 to 60 carbon atoms. Examples of the C₁-C₆₀ heteroaryl group are a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

A C₆-C₆₀ aryloxy group used herein indicates -OA₁₀₂ (wherein A₁₀₂ is the C₆-C₆₀ aryl group), and a C₆-C₆₀ arylthio group indicates -SA₁₀₃ (wherein A₁₀₃ is the C₆-C₆₀ aryl group).

A monovalent non-aromatic condensed polycyclic group used herein refers to a monovalent group that has two or more rings condensed to each other, only carbon atoms (for example, the number of carbon atoms may be in a range of 8 to 60) as a ring forming atom, and which is non-aromatic. An example of the monovalent non-aromatic condensed polycyclic group is a fluorenyl group. A divalent non-aromatic condensed polycyclic group used herein refers to a divalent group having the same structure as the monovalent non-aromatic condensed polycyclic group.

A monovalent non-aromatic condensed heteropolycyclic group used herein refers to a monovalent group that has two or more rings condensed to each other, has a heteroatom selected from N, O, P, and S, other than carbon atoms (for example, the number of carbon atoms may be in a range of 1 to 60), as a ring forming atom, and which is non-aromatic. An example of the monovalent non-aromatic condensed heteropolycyclic group is a carbazolyl group. A divalent non-aromatic condensed heteropolycyclic group used herein refers to a divalent group having the same structure as the monovalent non-aromatic condensed heteropolycyclic group.

In the present specification, at least one substituent of the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from
a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₂-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -N(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -N(Q₂₁)(Q₂₂); and
-N(Q₃₁)(Q₃₂);
wherein Q₁, Q₂, Q₁₁, Q₁₂, Q₂₁, Q₂₂, Q₃₁ and Q₃₂ are each independently selected from
a hydrogen, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group; and
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

The "biphenyl group" used therein refers to "a phenyl group substituted with a phenyl group."

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that a molar equivalent of A was identical to a molar equivalent of B.

### Example

### Synthesis Example 1: Synthesis of Compound 1

Compound 1 was synthesized according to the Reaction Scheme below:

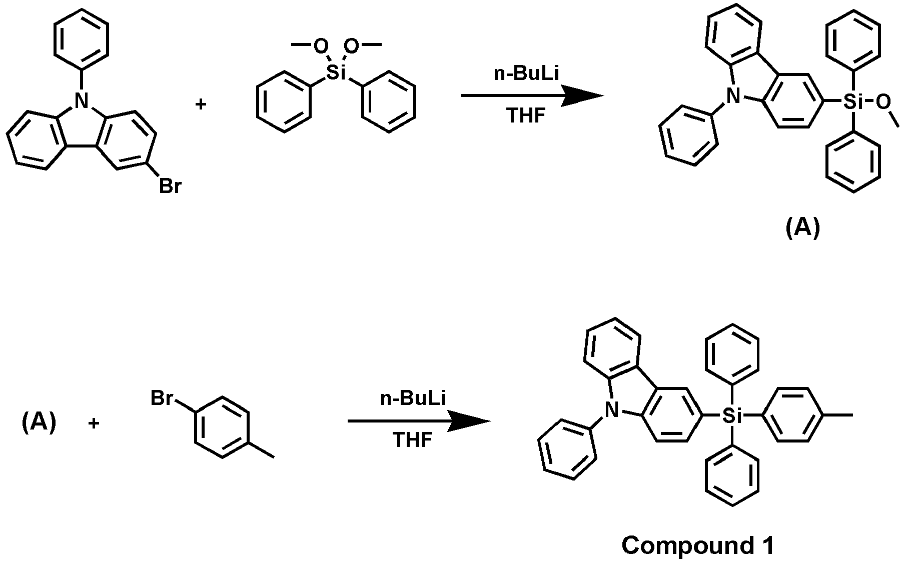

### Synthesis of Intermediate (A)

50.0 g (155 mmol) of 3-bromo-9-phenyl-9H-carbazole was dissolved in 500 mL of THF. The resulting solution was cooled to a temperature of -78°C. 97.0 mL (155.0 mmol, 1.6 molar (M) solution in n-hexane) of n-butyllithium was slowly added thereto for 30 minutes, and the reaction mixture was stirred at a temperature of -78°C for 1 hour. 45.5 g (186 mmol) of dimethoxydiphenylsilane dissolved in 250 mL of THF was slowly added to the resulting mixture for 30 minutes. The reaction temperature was raised slowly to room temperature for 1 hour, and the reaction mixture was additionally stirred at room temperature for 15 hours. When the reaction stopped, saturated ammonium chloride (NH₄Cl) aqueous solution was added thereto. Following the extraction, the organic layer was separated, and water was removed therefrom by using anhydrous magnesium sulfate (MgSO₄). The resulting dry solution was filtered and concentrated. The obtained product was purified by silica gel column chromatography (dichloromethane (DCM) : n-hexane = 1 : 10 volume to volume (v/v)) to obtain 42.4 g (yield of 60%) of Intermediate (A), which is the target compound.
LC-Mass (calculated: 455.17 g/mol, found: M+1 = 456 g/mol)

### Synthesis of Compound 1

5.13 g (30.0 mmol) of 1-bromo-4-methylbenzene was dissolved in 100 mL of THF, and the resulting solution was cooled to a temperature of -78°C. 18.8 mL (11.0 mmol, 1.6 M solution in n-hexane) of n-butyllithium was slowly added thereto for 30 minutes, and the reaction mixture was stirred at a temperature of -78°C for 1 hour. 13.6 g (30.0 mmol) of Intermediate (A) dissolved in 100 mL of THF was slowly added to the resulting mixture for 30 minutes. The reaction temperature was raised slowly to room temperature for 1 hour, and additionally stirred at room temperature for 15 hours. When the reaction stopped, saturated ammonium chloride (NH₄Cl) aqueous solution was added thereto. Following the extraction, the organic layer was extracted and separated, and water was removed therefrom by using anhydrous magnesium sulfate (MgSO₄). The resulting dry solution was filtered and concentrated. The obtained crude product was purified by silica gel column chromatography (dichloromethane (DCM) : n-hexane = 1 : 10 v/v), and the purified product was re-crystallized in ethanol to obtain 10.0 g (yield of 65%) of Compound 1, which is the target compound.
LC-Mass (calculated: 515.21 g/mol, found: M+1 = 516 g/mol)

### Synthesis Example 2: Synthesis of Compound 21

Compound 21 was synthesized according to a reaction scheme illustrated below:

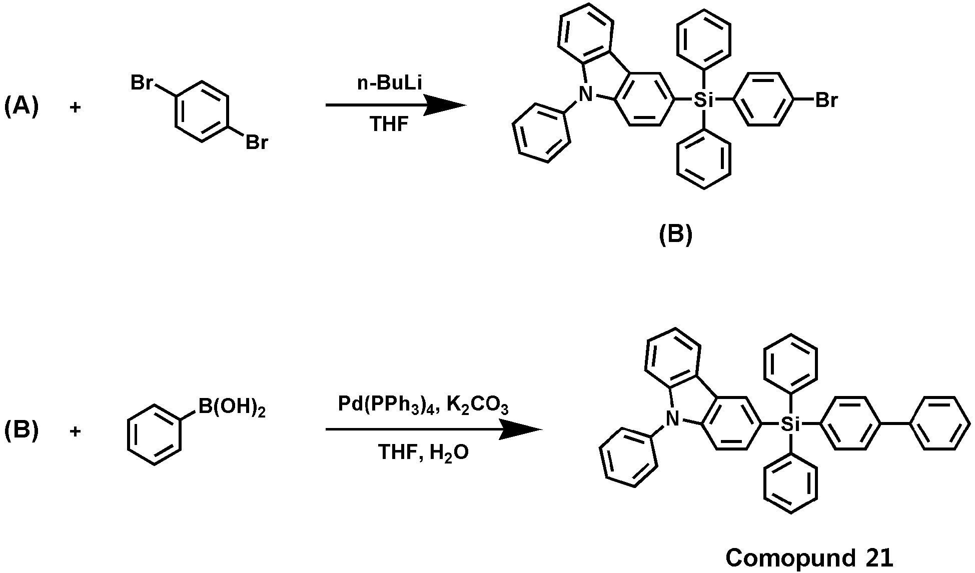

### Synthesis of Intermediate (B)

7.06 g (30.0 mmol) of 1,4-dibromobenzene was dissolved in 100 mL of THF, and the resulting solution was cooled to a temperature of -78°C. 18.8 mL (30.0 mmol, 1.6 M solution in n-hexane) of n-butyllithium was slowly added thereto for 30 minutes, and the resulting mixture was stirred at a temperature of -78°C for 1 hour. 13.6 g (30.0 mmol) of Intermediate (A) dissolved in 30 mL of THF was slowly added to the resulting mixture for 30 minutes. The reaction temperature was raised slowly to room temperature for 1 hour, and the reaction mixture was additionally stirred at room temperature for 15 hours. When the reaction stopped, saturated ammonium chloride (NH₄Cl) aqueous solution was added thereto. Following the extraction, the organic layer was separated, and water was removed therefrom by using anhydrous magnesium sulfate (MgSO₄). The dry solution was filtered and concentrated. The obtained product was purified by silica gel column chromatography (dichloromethane (DCM) : n-hexane = 1 : 10 v/v) to obtain 12.4 g (yield of 71%) of Intermediate (B), which is the target compound.
LC-Mass (calculated: 579.10 g/mol, found: M+1 = 580 g/mol)

### Synthesis of Compound 21

17.4 g (30.0 mmol) of Intermediate (B), 4.39 g (36.0 mmol) of phenylboronic acid, 3.47 g (3.00 mmol) of tetrakis(triphenylphosphine)palladium (0) (Pd(PPh₃)₄), and 12.4 g (90.0 mmol) of potassium carbonate were added to a mixed solution including 60 ml of tetrahydrofuran (THF) and 30 mL of water. The resulting mixture was stirred while refluxing. When the reaction stopped, the reaction solution was cooled to room temperature. The aqueous layer was removed by extraction, and the residual product was filtered through a plug of silica gel using vacuum suction. The obtained crude product was purified by silica gel column chromatography (dichloromethane (DCM) : n-hexane = 1 : 3 v/v), and the resulting purified product was re-crystallized in ethanol to obtain 13.3 g (yield of 77%) of Compound 21, which is the target compound.
LC-Mass (calculated: 577.22 g/mol, found: M+1 = 578 g/mol)

### Synthesis Example 3: Synthesis of Compound 22

Compound 22 was synthesized according to the Reaction Scheme below:

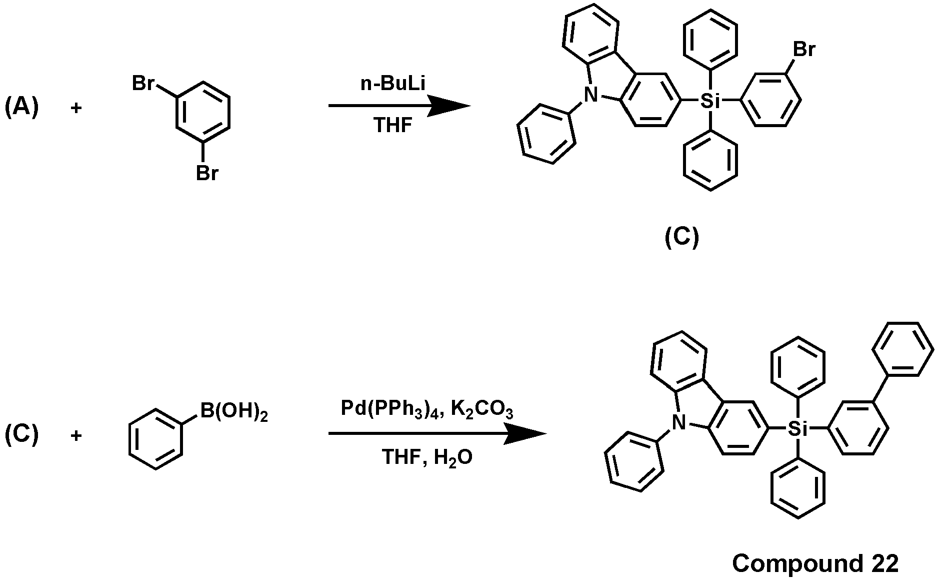

### Synthesis of Intermediate (C)

12.1 g (yield of 69%) of Intermediate (C), which is the target compound, was obtained in the same manner as Intermediate (B) in Synthesis Example 2, except that 7.06 g (30.0 mmol) of 1,3-dibromobenzene was used instead of 1,4-dibromobenzene.
LC-Mass (calculated: 579.10 g/mol, found: M+1 = 580 g/mol)

### Synthesis of Compound 22

7.45 g of Compound 22 (yield of 75%) was synthesized in the same manner as Compound 21 of Synthesis Example 2, except that 7.06 g (30.0 mmol) of Intermediate (C) was used instead of Intermediate (B).
LC-Mass (calculated: 577.22 g/mol, found: M+1 = 578 g/mol)

### Synthesis Example 4: Compound 35

Compound 35 was synthesized according to the Reaction Scheme below:

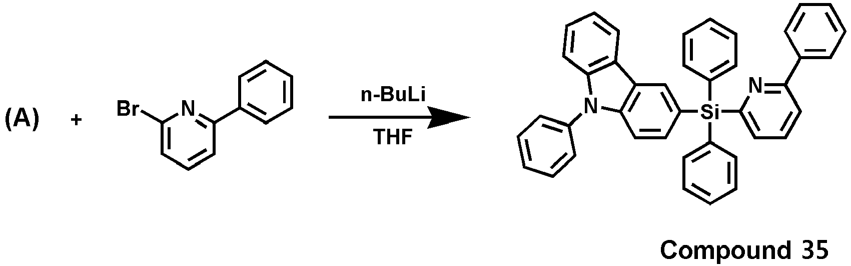

9.55 g (yield of 55%) of Compound 35, which is the target compound, was obtained in the same manner as Compound 1 in Synthesis Example 1, except that 7.02 g (30.0 mmol) of 2-bromo-6-phenylpyridine was used instead of 1-bromo-4-methylbenzene.
LC-Mass (calculated: 578.22 g/mol, found: M+1 = 579 g/mol)

### Synthesis Example 5: Compound 36

Compound 36 was synthesized according to the Reaction Scheme below:

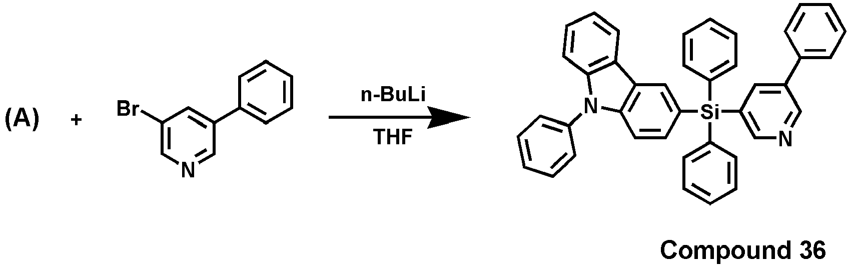

9.90 g (yield of 57%) of Compound 36, which is the target compound, was obtained in the same manner as Compound 1 in Synthesis Example 1, except that 7.02 g (30.0 mmol) of 3-bromo-5-phenylpyridine was used instead of 1-bromo-4-methylbenzene.
LC-Mass (calculated: 578.22 g/mol, found: M+1 = 579 g/mol)

### Synthesis Example 6: Synthesis of Compound 56

Compound 56 was synthesized according to the Reaction Scheme below:

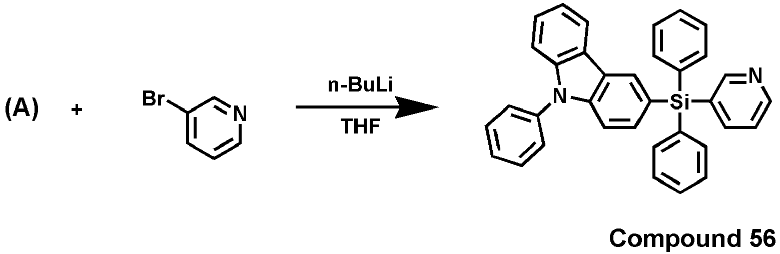

5.73 g (yield of 38%) of Compound 56, which is the target compound, was obtained in the same manner as Compound 1 in Synthesis Example 1, except that 4.74 g (30.0 mmol) of 3-bromopyridine was used instead of 1-bromo-4-methylbenzene.
LC-Mass (calculated: 502.19 g/mol, found: M+1 = 503 g/mol)

### Synthesis Example 7: Compound 68

Compound 68 was synthesized according to the Reaction Scheme below:

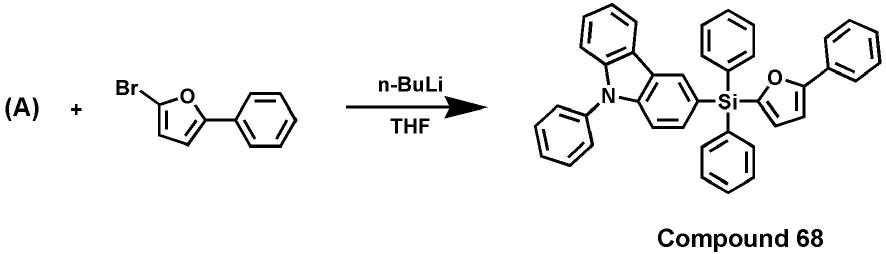

6.98 g (yield of 41%) of Compound 68, which is the target compound, was obtained in the same manner as Compound 1 in Synthesis Example 1, except that 6.69 g (30.0 mmol) of 2-bromo-5-phenylfuran was used instead of 1-bromo-4-methylbenzene.
LC-Mass (calculated: 567.20 g/mol, found: M+1 = 568 g/mol)

### Synthesis Example 8: Compound 86

Compound 86 was synthesized according to the Reaction Scheme below:

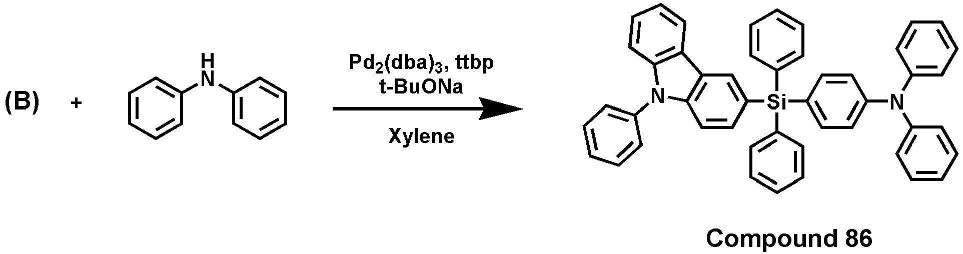

17.4 g (30.0 mmol) of Intermediate (B), 6.09 g (36.0 mmol) of diphenylamine, 2.75 g (3.00 mmol) of Pd₂(dba)₃, 3.00 mL (50% in toluene, 6.00 mmol) of tri-tert-butylphosphine (ttbp), and 5.76 g (60.0 mmol) of sodium tert-butoxide were added to 300 mL of xylene, and the mixture was heated at a temperature of 145°C while stirring. When the reaction stopped, the reaction product was cooled to room temperature and filtered through a plug of silica gel using suction filtration. The filtered solution was concentrated in vacuum. The obtained crude product was purified by recrystallization in ethanol to obtain 13.8 g (yield of 69%) of Compound 86 as the target compound.
LC-Mass (calculated: 668.26 g/mol, found: M+1 = 669 g/mol)

### Synthesis Example 9: Synthesis of Compound 101

Compound 101 was synthesized according to the Reaction Scheme below:

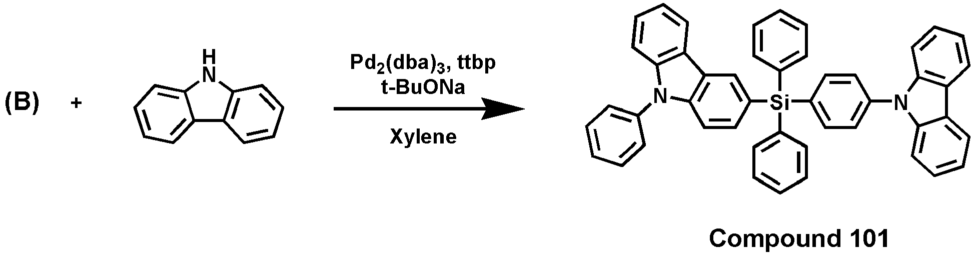

14.8 g of Compound 101 (yield of 74%) was synthesized in the same manner as in Synthesis Example 8, except that 6.02 g (36.0 mmol) of 9H-carbazole was used instead of diphenylamine.
LC-Mass (calculated: 666.25 g/mol, found: M+1 = 667 g/mol)

### Synthesis Example 10: Compound 102

Compound 102 was synthesized according to the Reaction Scheme below:

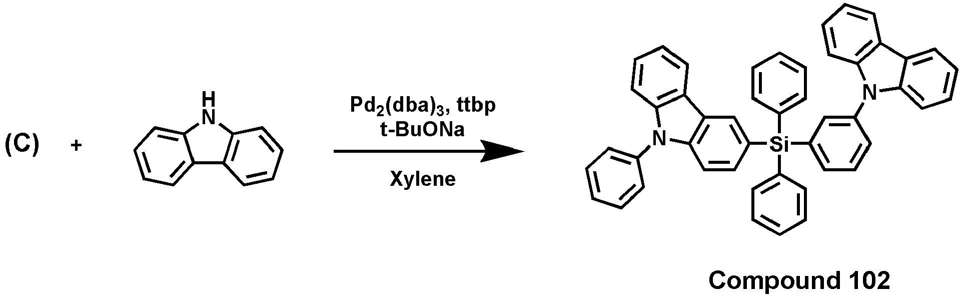

13.4 g of Compound 102 (yield of 67%) was synthesized in the same manner as in Synthesis Example 9, except that 17.4 g (30.0 mmol) of Intermediate (C) was used instead of Intermediate (B).
LC-Mass (calculated: 666.25 g/mol, found: M+1 = 667 g/mol)

### Synthesis Example 11: Compound 107

Compound 107 was synthesized according to the Reaction Scheme below:

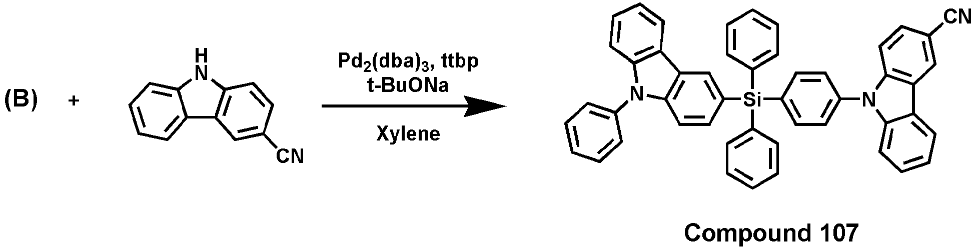

12.0 g of Compound 107 (yield of 58%) was synthesized in the same manner as in Synthesis Example 8, except that 6.92 g (36.0 mmol) of 9H-carbazole-3-carbonitrile was used instead of diphenylamine.
LC-Mass (calculated: 691.24 g/mol, found: M+1 = 692 g/mol)

### Synthesis Example 12: Compound 108

Compound 108 was synthesized according to the Reaction Scheme below:

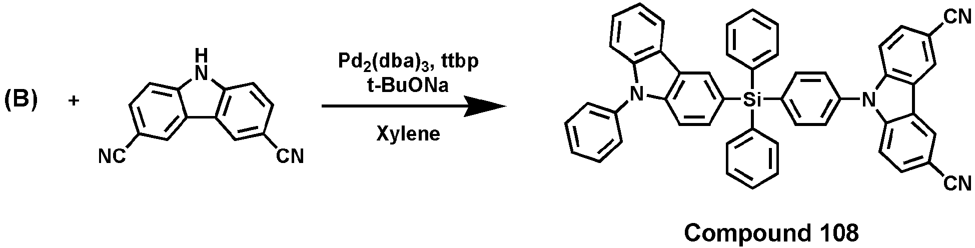

11.2 g of Compound 108 (yield of 52%) was synthesized in the same manner as in Synthesis Example 8, except that 7.82 g (36.0 mmol) of 9H-carbazole-3,6-dicarbonitrile was used instead of diphenylamine.
LC-Mass (calculated: 716.24 g/mol, found: M+1 = 717 g/mol)

### Synthesis Example 13: Compound 116

Compound 116 was synthesized according to the Reaction Scheme below:

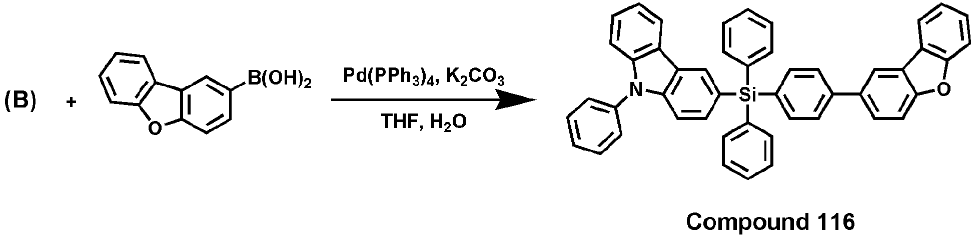

13.6 g (yield of 68%) of Compound 116, which is the target compound, was obtained in the same manner as Compound 21 in Synthesis Example 2, except that 7.63 g (36.0 mmol) of dibenzo[b,d]furan-2-ylboronic acid was used instead of phenylboronic acid.
LC-Mass (calculated: 667.23 g/mol, found: M+1 = 668 g/mol)

### Synthesis Example 14: Synthesis of Compound 120

Compound 120 was synthesized according to the Reaction Scheme below:

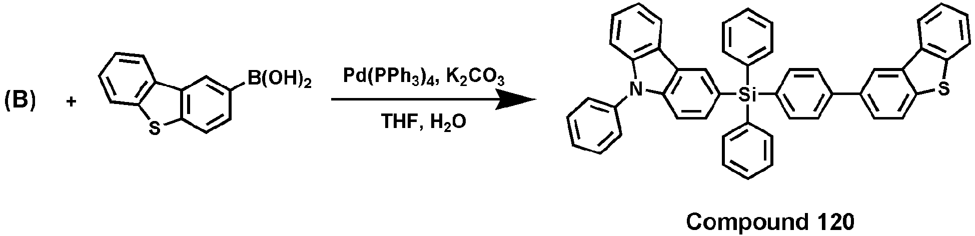

15.2 g (yield of 74%) of Compound 120, which is the target compound, was obtained in the same manner as Compound 21 in Synthesis Example 2, except that 8.21 g (36.0 mmol) of dibenzo[b,d]thiophen-2-ylboronic acid was used instead of phenylboronic acid.
LC-Mass (calculated: 683.21 g/mol, found: M+1 = 684 g/mol)

### Synthesis Example 15: Compound 128

Compound 128 was synthesized according to the Reaction Scheme below:

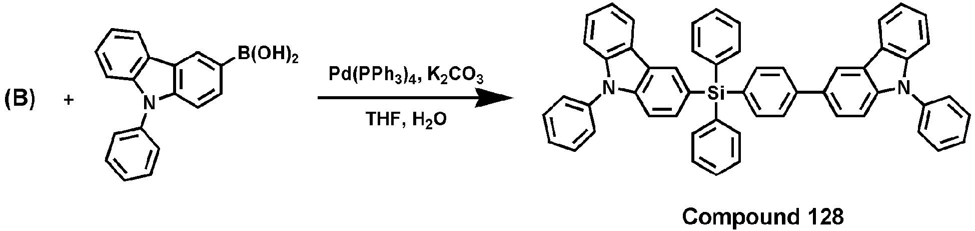

14.7 g (yield of 66%) of Compound 128, which is the target compound, was obtained in the same manner as Compound 21 in Synthesis Example 2, except that 10.3 g (36.0 mmol) of (9-phenyl-9H-carbazol-3-yl)boronic acid was used instead of phenylboronic acid.
LC-Mass (calculated: 742.28 g/mol, found: M+1 = 743 g/mol)

### Synthesis Example 16: Synthesis of Compound 130

Compound 130 was synthesized according to the Reaction Scheme below:

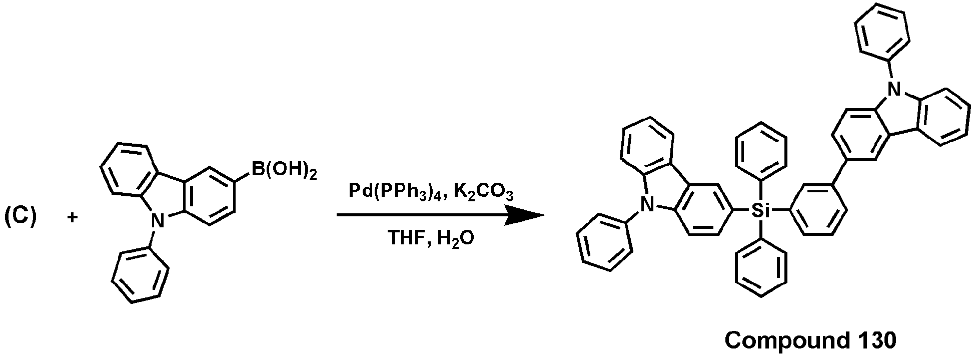

12.0 g of Compound 130 (yield of 54%) was synthesized in the same manner as in Synthesis Example 15, except that 17.4 g (30.0 mmol) of Intermediate (C) was used instead of Intermediate (B).
LC-Mass (calculated: 742.28 g/mol, found: M+1 = 743 g/mol)

### Synthesis Example 17: Synthesis of Compound 151

Compound 151 was synthesized according to the Reaction Scheme below:

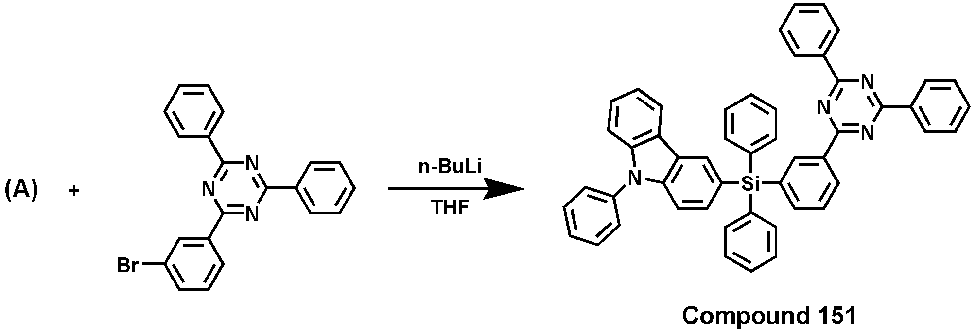

5.28 g (yield of 24%) of Compound 151, which is the target compound, was obtained in the same manner as Compound 1 in Synthesis Example 1, except that 11.6 g (30.0 mmol) of 2-(3-bromophenyl)-4,6-diphenyl-1,3,5-triazine was used instead of 1-bromo-4-methylbenzene.
LC-Mass (calculated: 732.27 g/mol, found: M+1 = 733 g/mol)

### Synthesis Example 18: Synthesis of Compound 152

Compound 152 was synthesized according to the Reaction Scheme below:

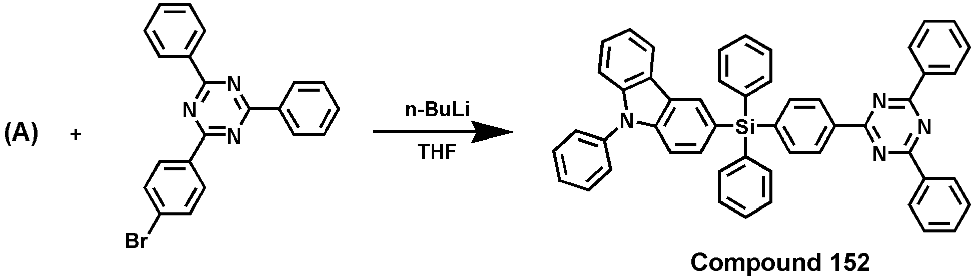

6.43 g (yield of 28%) of Compound 151, which is the target compound, was obtained in the same manner as Compound 1 in Synthesis Example 1, except that 11.6 g (30.0 mmol) of 2-(4-bromophenyl)-4,6-diphenyl-1,3,5-triazine was used instead of 1-bromo-4-methylbenzene.
LC-Mass (calculated: 732.27 g/mol, found: M+1 = 733 g/mol)

### Synthesis Example 19: Compound 201

Compound 201 was synthesized according to the Reaction Scheme below:

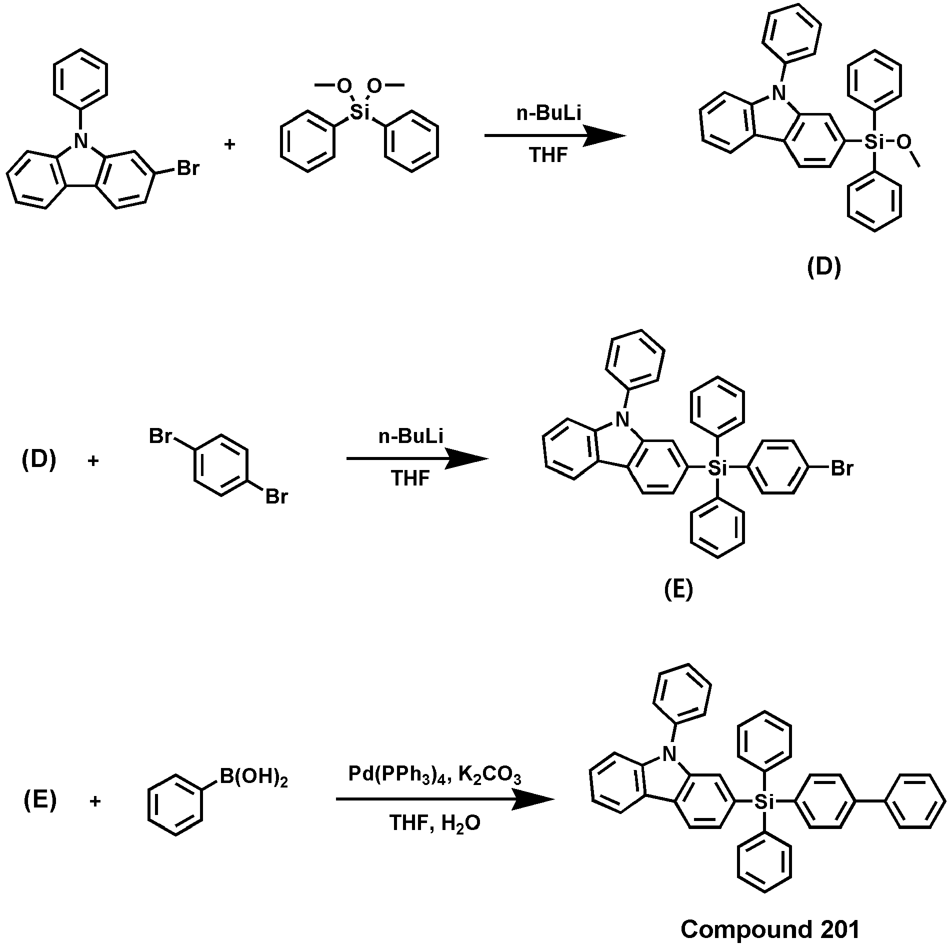

### Synthesis of Intermediate (D)

38.2 g (yield of 54%) of Intermediate (D), which is the target compound, was obtained in the same manner as Intermediate (A) in Synthesis Example 1, except that 50.0 g (155 mmol) of 2-bromo-9-phenyl-9H-carbazole was used instead of 3-bromo-9-phenyl-9H-carbazole.
LC-Mass (calculated: 455.17 g/mol, found: M+1 = 456 g/mol)

### Synthesis of Intermediate (E)

12.9 g of Intermediate (E) (yield of 74%) was synthesized in the same manner as Intermediate (B) of Synthesis Example 2, except that 13.7 g (30 mmol) of Intermediate (D) was used instead of Intermediate (A).
LC-Mass (calculated: 579.10 g/mol, found: M+1 = 580 g/mol)

### Compound 201

7.35 g of Compound 201 (yield of 74%) was synthesized in the same manner as Compound 21 of Synthesis Example 2, except that 17.4 g (30.0 mmol) of Intermediate (E) was used instead of Intermediate (B).
LC-Mass (calculated: 577.22 g/mol, found: M+1 = 578 g/mol)

### Synthesis Example 20: Compound 214

Compound 214 was synthesized according to the Reaction Scheme below:

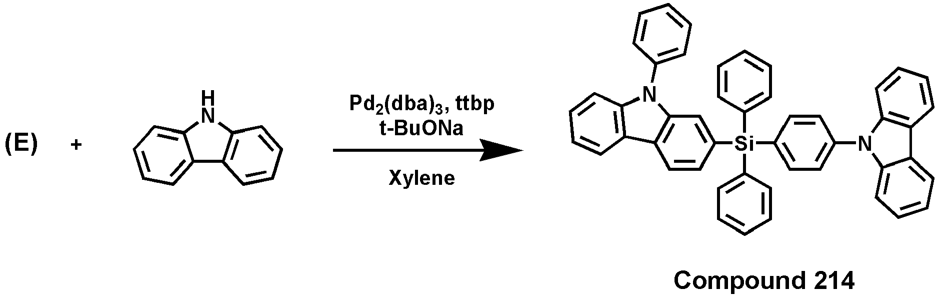

13.0 g of Compound 214 (yield of 65%) was synthesized in the same manner as Compound 101 of Synthesis Example 9, except that 17.4 g (30.0 mmol) of Intermediate (E) was used instead of Intermediate (B).
LC-Mass (calculated: 666.25 g/mol, found: M+1 = 667 g/mol)

### Synthesis Example 21: Compound 273

Compound 273 was synthesized according to the Reaction Scheme below:

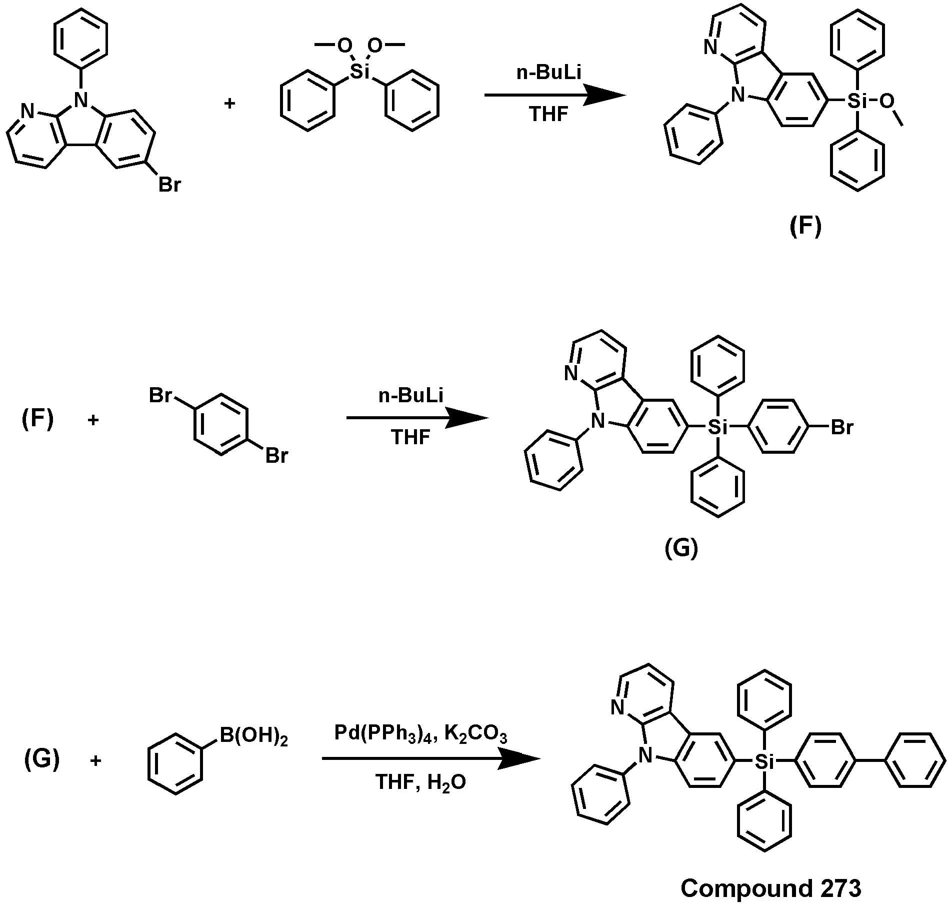

### Synthesis of Intermediate (F)

35.4 g (yield of 50%) of Intermediate (F), which is the target compound, was obtained in the same manner as Intermediate (A) in Synthesis Example 1, except that 50.1 g (155 mmol) of 6-bromo-9-phenyl-9H-pyrido[2,3-b]indole was used instead of 3-bromo-9-phenyl-9H-carbazole.
LC-Mass (calculated: 456.17 g/mol, found: M+1 = 457 g/mol)

### Synthesis of Intermediate (G)

11.3 g of Intermediate (G) (yield of 65%) was synthesized in the same manner as Intermediate (B) of Synthesis Example 2, except that 13.7 g (30 mmol) of Intermediate (F) was used instead of Intermediate (A).
LC-Mass (calculated: 580.10 g/mol, found: M+1 = 581 g/mol)

### Compound 273

6.26 g of Compound 273 (yield of 63%) was synthesized in the same manner as Compound 21 of Synthesis Example 2, except that 17.4 g (30.0 mmol) of Intermediate (G) was used instead of Intermediate (B).
LC-Mass (calculated: 578.22 g/mol, found: M+1 = 579 g/mol)

### Synthesis Example 22: Compound 286

Compound 286 was synthesized according to the Reaction Scheme below:

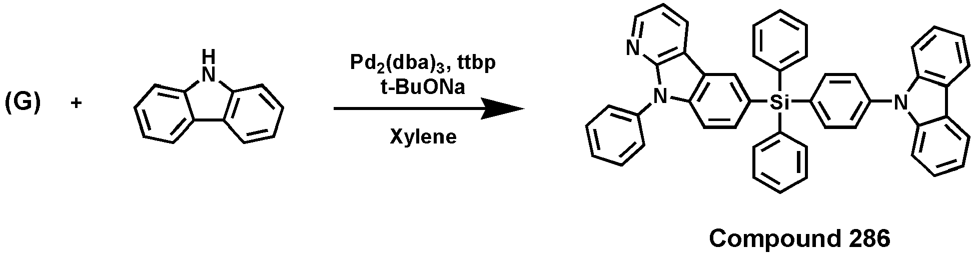

12.4 g of Compound 286 (yield of 62%) was synthesized in the same manner as Compound 101 of Synthesis Example 9, except that 17.4 g (36.0 mmol) of Intermediate (G) was used instead of Intermediate (B).
LC-Mass (calculated: 667.24 g/mol, found: M+1 = 668 g/mol)

### Evaluation Example 1 : Evaluation on HOMO, LUMO, and triplets (T1) energy levels

HOMO, LUMO and T1 energy levels of compounds synthesized in Synthesis Examples were evaluated according to the method indicated in Table 1, and results thereof are shown in Table 2.

**Table 1**

| | |
|---|---|
| HOMO energy level evaluation method | A potential (V) - current (A) graph of each compound was obtained by using cyclic voltammetry (CV) (electrolyte: 0.1 M Bu₄NClO₄ / solvent: MeCN / electrode: 3 electrode system (working electrode: C, reference electrode: Ag/AgCl, auxiliary electrode: Pt)), and from reduction onset of the graph, a HOMO energy level of the compound was calculated. |
| LUMO energy level evaluation method | Each compound was diluted in 2-methyltetrahydrofuran (2-Me-THF) to a concentration of 1x10⁻⁵ M, and then, UV absorption spectrum thereof was measured at room temperature by using Varian Cary 5000 UV-Vis-NIR spectrophotometer. A LUMO energy level of the compound was calculated by using an optical band gap (Eg) measured using the edge of the absorption spectrum. |
| T1 energy level evaluation method | A mixture (each compound was dissolved in an amount of 1 mg in 3 cubic centimeters (cc) of 2-Me-THF) of 2-Me-THF, and each compound was loaded into a quartz cell. The resulting quartz cell was loaded into liquid nitrogen (77K), and a photoluminescence spectrum thereof was measured by using a device for measuring photoluminescence. The obtained spectrum was compared with a photoluminescence spectrum measured at room temperature. The peaks observed only at low temperature were analyzed to calculate |
| | T1 energy levels. |

**Table 2**

| Compound No. | HOMO (eV) (absolute value) | LUMO (eV) (absolute value) | T1 energy level (eV) |
|---|---|---|---|
| Compound 1 | 5.67 | 2.03 | 2.95 |
| Compound 21 | 5.73 | 2.18 | 2.93 |
| Compound 22 | 5.72 | 2.18 | 2.92 |
| Compound 35 | 5.65 | 2.30 | 2.96 |
| Compound 36 | 5.78 | 2.27 | 2.94 |
| Compound 86 | 5.41 | 1.87 | 2.95 |
| Reference Compound 101 | 5.60 | 2.16 | 3.00 |
| Reference Compound 102 | 5.57 | 2.16 | 3.01 |
| Reference Compound 107 | 5.85 | 2.43 | 3.02 |
| Reference Compound 108 | 5.94 | 2.87 | 3.02 |
| Compound 116 | 5.70 | 2.27 | 2.92 |
| Compound 120 | 5.71 | 2.28 | 2.94 |
| Compound 128 | 5.55 | 2.08 | 2.87 |
| Compound 130 | 5.51 | 2.02 | 2.90 |
| Compound 151 | 5.72 | 3.05 | 2.87 |
| Compound 152 | 5.72 | 3.14 | 2.85 |
| Reference Compound 214 | 5.63 | 2.28 | 2.94 |
| Compound 273 | 5.91 | 2.41 | 2.93 |
| Reference Compound 286 | 5.63 | 2.47 | 2.98 |
| Compound A | 5.68 | 2.07 | 2.85 |

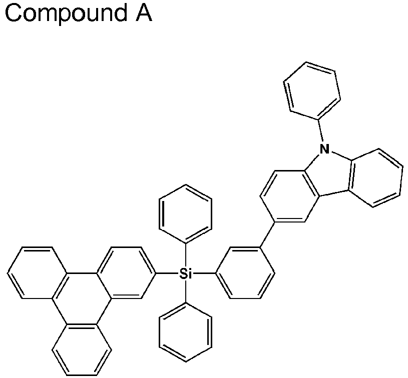

From Table 2, it is confirmed that the compounds produced in the synthesis examples above have electric characteristics that make the compounds suitable for use as materials for forming an organic light-emitting device. Without wishing to be bound by a theory, it is hypothesized that, since Compound A further includes, in addition to a phenyl-carbazole ring, a condensed cycle that is directly linked to Si (that is, a triphenylene ring), the conjugation length of Compound A increases. Due to the increase in the conjugation length, Compound A has smaller triplet energy than the compounds synthesized according to the Synthesis Examples. However, the inventive concept is not limited thereto.

### Evaluation Example 2: Thermal characteristics evaluation

Thermal analysis (N₂ atmosphere, temperature range: from room temperature to 600°C (10°C/min)-TGA, from room temperature to 400°C -DSC, Pan Type: Pt Pan in disposable Al Pan(TGA), and disposable Al pan(DSC)) was performed on Compounds 21, 22, 86, and 107 by using thermogravimetric analysis (TGA) and differential scanning calorimetry (DSC), and results thereof are shown in Table 3. As shown in Table 3, it was confirmed that the synthesized compounds had excellent thermal stability.

**Table 3**

| Compound No. | Tg (°C) | Td (1%,°C) |
|---|---|---|
| 21 | 84 | 349 |
| 22 | 90 | 392 |
| 86 | 94 | 376 |
| 107 | 128 | 419 |

### Example 1

ITO glass substrate (ITO layer acts as an anode) having a surface resistance of 15 Ohms per square centimeter (Ω/cm²) was cut to a size of 50 millimeters (mm) × 50 mm × 0.7 mm, sonicated in acetone, isopropyl alcohol, and pure water, for 15 minutes in each solvent, and cleaned with UV ozone for 30 minutes.

On the ITO anode, NPB was deposited at a vacuum degree of 650×10⁻⁷ pascals (Pa) at a deposition speed of 0.1 to 0.3 nanometers per second (nm/s) to form a hole transport layer having a thickness of 70 nm (700 Å). Then, mCP was deposited on the hole transport layer to form an electron blocking layer having a thickness of 5 nm (50 Å), thereby completely forming a hole transport region.

On the hole transport region, Compound 1 (host) and compound Flr₆ (dopant, 10 percent by weight (wt%)) were co-deposited to form an emission layer having a thickness of 30 nm (300 Å).

TmPyPB was vacuum-deposited on the emission layer to form a hole blocking layer having a thickness of 30 nm (300 Å), Alq₃ was vacuum-deposited on the hole blocking layer to form an electron transport layer having a thickness of 10 nm (100 Å). Then, LiF was deposited on the electron transport layer to form an electron injection layer having a thickness of 0.5 nm (5 Å), and an Al second electrode (cathode) having a thickness of 120 nm (1,200 Å) is formed on the electron injection layer to complete manufacturing of an organic light-emitting device.

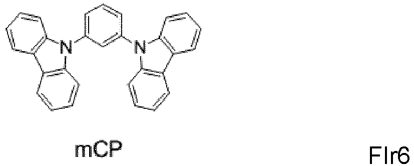

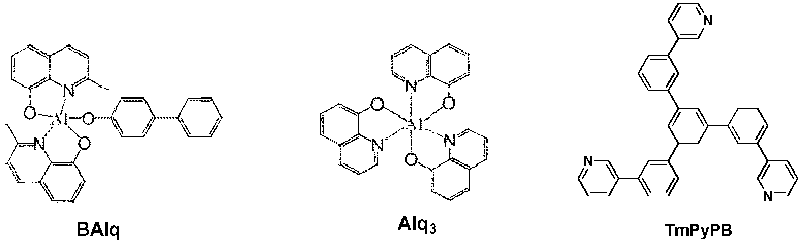

### Example 2

An organic light-emitting device was manufactured in the same manner as in Example 1, except that in forming an emission layer as a host, Compound 21 was used instead of Compound 1.

### Example 3

An organic light-emitting device was manufactured in the same manner as in Example 1, except that in forming an emission layer as a host, Compound 22 was used instead of Compound 1.

### Example 4

An organic light-emitting device was manufactured in the same manner as in Example 1, except that in forming an emission layer as a host, Compound 35 was used instead of Compound 1.

### Example 5

An organic light-emitting device was manufactured in the same manner as in Example 1, except that in forming an emission layer as a host, Compound 36 was used instead of Compound 1.

### Example 6

An organic light-emitting device was manufactured in the same manner as in Example 1, except that in forming an emission layer as a host, Compound 86 was used instead of Compound 1.

### Reference Example 7

An organic light-emitting device was manufactured in the same manner as in Example 1, except that in forming an emission layer as a host, Compound 101 was used instead of Compound 1.

### Reference Example 8

An organic light-emitting device was manufactured in the same manner as in Example 1, except that in forming an emission layer as a host, Compound 102 was used instead of Compound 1.

### Reference Example 9

An organic light-emitting device was manufactured in the same manner as in Example 1, except that in forming an emission layer as a host, Compound 107 was used instead of Compound 1.

### Reference Example 10

An organic light-emitting device was manufactured in the same manner as in Example 1, except that in forming an emission layer as a host, Compound 108 was used instead of Compound 1.

### Example 11

An organic light-emitting device was manufactured in the same manner as in Example 1, except that in forming an emission layer as a host, Compound 116 was used instead of Compound 1.

### Example 12

An organic light-emitting device was manufactured in the same manner as in Example 1, except that in forming an emission layer as a host, Compound 120 was used instead of Compound 1.

### Example 13

An organic light-emitting device was manufactured in the same manner as in Example 1, except that in forming an emission layer as a host, Compound 128 was used instead of Compound 1.

### Example 14

An organic light-emitting device was manufactured in the same manner as in Example 1, except that in forming an emission layer as a host, Compound 130 was used instead of Compound 1.

### Example 15

An organic light-emitting device was manufactured in the same manner as in Example 1, except that in forming an emission layer as a host, Compound 151 was used instead of Compound 1.

### Example 16

An organic light-emitting device was manufactured in the same manner as in Example 1, except that in forming an emission layer as a host, Compound 152 was used instead of Compound 1.

### Reference Example 17

An organic light-emitting device was manufactured in the same manner as in Example 1, except that in forming an emission layer as a host, Compound 214 was used instead of Compound 1.

### Example 18

An organic light-emitting device was manufactured in the same manner as in Example 1, except that in forming an emission layer as a host, Compound 273 was used instead of Compound 1.

### Reference Example 19

An organic light-emitting device was manufactured in the same manner as in Example 1, except that in forming an emission layer as a host, Compound 286 was used instead of Compound 1.

### Comparative Example 1

An organic light-emitting device was manufactured in the same manner as in Example 1, except that in forming an emission layer as a host, Compound AA was used instead of Compound 1.

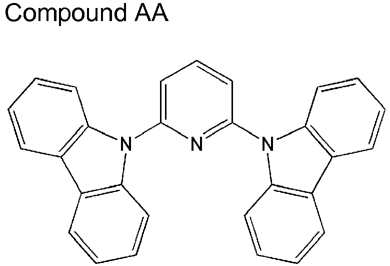

### Comparative Example 2

An organic light-emitting device was manufactured in the same manner as in Example 1, except that in forming an emission layer as a host, Compound A was used instead of Compound 1.

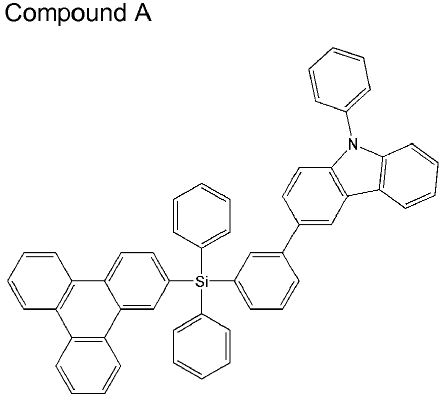

### Comparative Example 3

An organic light-emitting device was manufactured in the same manner as in Example 1, except that in forming an emission layer as a host, Compound B was used instead of Compound 1.

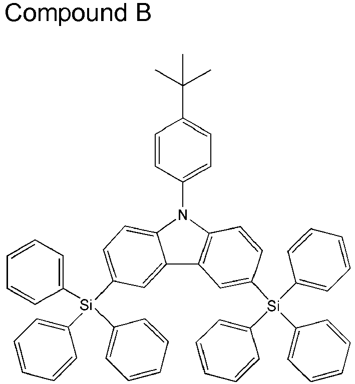

### Evaluation Example 3: Evaluation on characteristics of organic light-emitting devices.

The driving voltage, current efficiency, and brightness of the organic light-emitting devices manufactured according to Examples 1 to 19, and Comparative Examples 1 to 3 were measured by using Kethley SMU 236 and a brightness photometer PR650. The results are shown in Table 4. The driving voltage and current efficiency of Examples 2 to 19, and Comparative Examples 1 to 3 are shown as values relative to the driving voltage and current efficiency of Example 1 which are regarded as "100". These results are shown in Table 4. Lifespan (T₉₅) in Table 4 indicates a time (hour (hr)) taken when 100% of the initial brightness at 500 nit is reduced to 95%. In Table 4, lifespan (T₉₅) of Examples 2 to 19, and Comparative Examples 1 to 3 are shown as values relative to lifespan (T₉₅) of the organic light-emitting device of Example 1 which is regarded as "100."

**Table 4**

| | Host | Driving voltage (relative value) | Current efficiency (relative value) | lifespan (T95) (relative value) | Color |
|---|---|---|---|---|---|
| Example 1 | Compound 1 | 100 | 100 | 100 | Blue |
| Example 2 | Compound 21 | 95 | 115 | 121 | Blue |
| Example 3 | Compound 22 | 93 | 118 | 118 | Blue |
| Example 4 | Compound 35 | 85 | 124 | 106 | Blue |
| Example 5 | Compound 36 | 84 | 120 | 105 | Blue |
| Example 6 | Compound 86 | 71 | 143 | 80 | Blue |
| Reference Example 7 | Compound 101 | 75 | 149 | 166 | Blue |
| Reference Example 8 | Compound 102 | 72 | 143 | 160 | Blue |
| Reference | Compound | 62 | 149 | 164 | Blue |
| Example 9 | 107 | | | | |
| Reference Example 10 | Compound 108 | 60 | 163 | 178 | Blue |
| Example 11 | Compound 116 | 72 | 141 | 124 | Blue |
| Example 12 | Compound 120 | 76 | 144 | 121 | Blue |
| Example 13 | Compound 128 | 78 | 125 | 118 | Blue |
| Example 14 | Compound 130 | 76 | 138 | 124 | Blue |
| Example 15 | Compound 151 | 61 | 116 | 149 | Blue |
| Example 16 | Compound 152 | 64 | 123 | 153 | Blue |
| Reference Example 17 | Compound 214 | 78 | 131 | 131 | Blue |
| Example 18 | Compound 273 | 79 | 133 | 102 | Blue |
| Reference | Compound | 82 | 145 | 106 | Blue |
| Example 19 | 286 | | | | |
| Comparati ve Example 1 | Compound AA | 93 | 103 | 106 | Blue |
| Comparati ve Example 2 | Compound A | 94 | 95 | 105 | Blue |
| Comparati ve Example 3 | Compound B | 125 | 107 | 108 | Blue |

From Table 4, it was confirmed that the organic light-emitting devices according to Examples 1 to 19 have lower driving voltage, higher efficiency, and/or higher durability than the organic light-emitting devices of Comparative Examples 1 to 3.

The condensed cyclic compounds according to embodiments have excellent electric characteristics and thermal stability. Accordingly, organic light-emitting devices including the condensed cyclic compounds may have a low driving voltage, high efficiency, high brightness, and a long lifespan.

The subject of the inventions is defined by the following claims.

## Claims

1. A condensed cyclic compound represented by one of Formulae 1A to 1D: 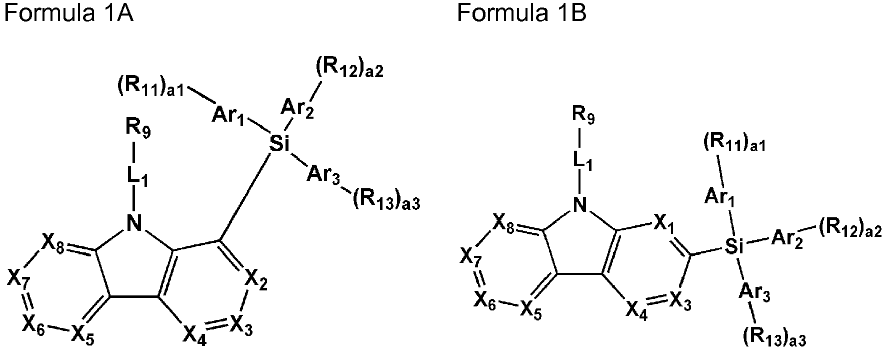 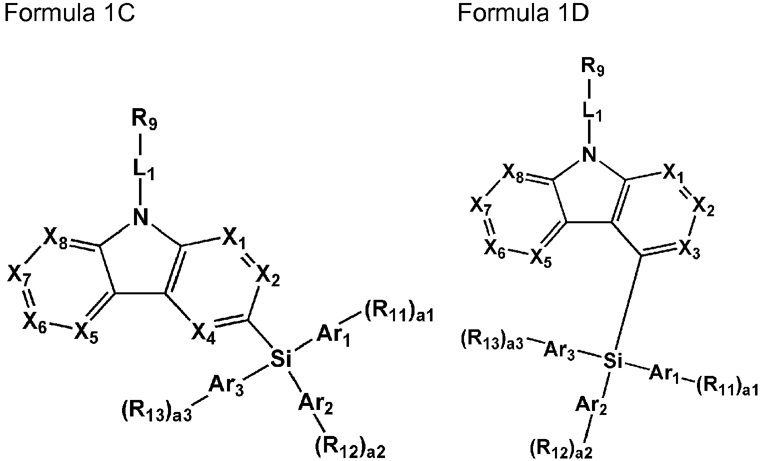 wherein in Formulae 1A to 1D,
X₁ is N or C(R₁),
X₂ is N or C(R₂),
X₃ is N or C(R₃),
X₄ is N or C(R₄),
X₅ is N or C(R₅),
X₆ is N or C(R₆),
X₇ is N or C(R₇), and
X₈ is N or C(R₈);
L₁ is each independently selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
Ar₁ to Ar₃ are each independently a group derived from a C₅-C₉ non-condensed carbocyclic group or a C₁-C₇ non-condensed heterocyclic group,
R₁ to R₈ are each independently selected from a hydrogen, a deuterium, -F, -Cl, -Br, -I, a cyano group, and a substituted or unsubstituted group selected from: a methyl group, an ethyl group, a propyl group, an isobutyl group, a sec-butyl group, a tert-butyl group, a pentyl group, an iso-amyl group, a hexyl group, a phenyl group, a naphthyl group, an anthracenyl group, a phenanthrenyl group, a pyrenyl group, and a chrysenyl group;
R₉ is selected from a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted a monovalent non-aromatic condensed polycyclic group and a substituted or unsubstituted monovalent non-aromatic condensed heteropolycyclic group;
R₁₁ to R₁₃ are each independently a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, -N(Q₁)(Q₂), wherein Q₁ and Q₂ are each independently selected from a C₁-C₁₀ alkyl group, and a group represented by Formulae 3-1 to 3-29 and 3-31 to 3-37: 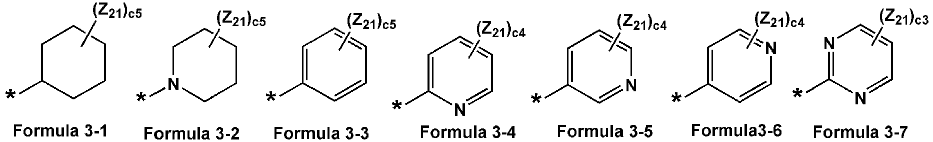 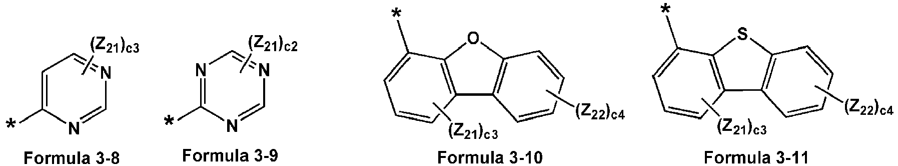 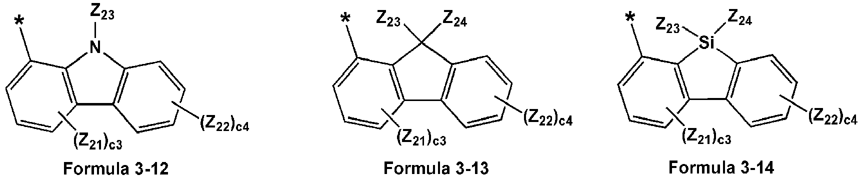 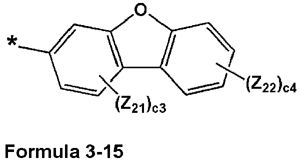 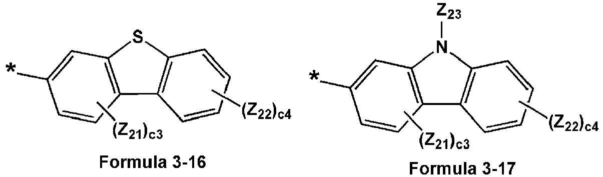 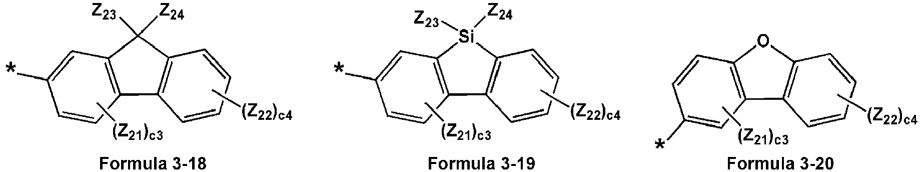 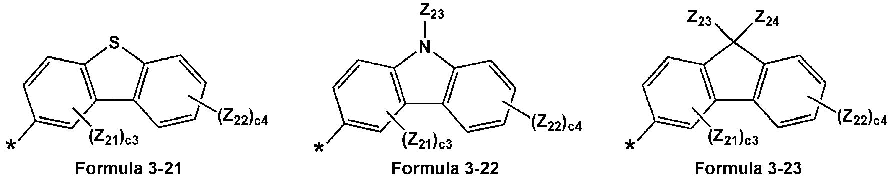 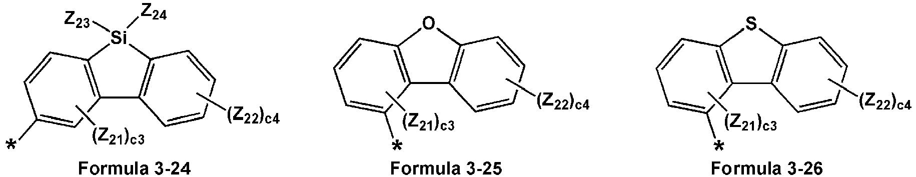 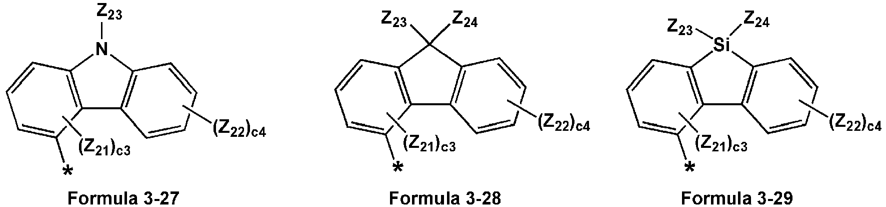 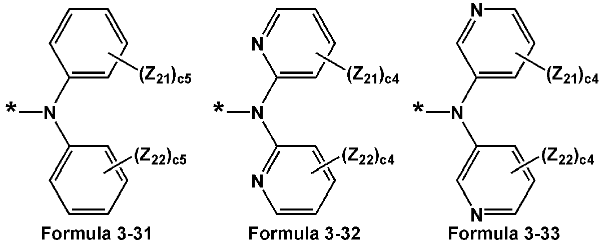 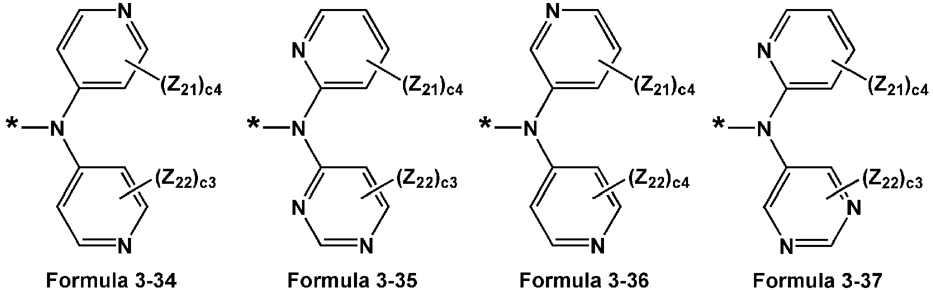 wherein in Formulae 3-1 to 3-29 and 3-31 to 3-37,
Z₂₁ to Z₂₄ are each independently selected from a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a pyridinyl group, a pyrimidinyl group, and a triazinyl group,
c5 is an integer selected from 1, 2, 3, 4, and 5,
c4 is an integer selected from 1, 2, 3, and 4,
c3 is an integer selected from 1, 2, and 3,
c2 is an integer selected from 1 to 2, and
* indicates a binding site to each of Ar₁ to Ar₃ in Formulae 1A to 1D,
a1 to a3 are each independently an integer selected from 1, 2, 3, 4, 5, and 6, provided that when a1 is 2 or more, two or more groups R₁₁ are identical to or different from each other, when a2 is 2 or more, two or more groups R₁₂ are identical to or different from each other, and when a3 is 2 or more, two or more groups R₁₃ are identical to or different from each other;
wherein in each of Formulae 1A to 1D, groups *-Ar₁-(R₁₁)ₐ₁, *-Ar₂-(R₁₂)ₐ₂ and *-Ar₃-(R₁₃)ₐ₃ are not identical to each other;
at least one of substituents of the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is selected from
a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -N(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₂-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, and -N(Q₂₁)(Q₂₂); and
-N(Q₃₁)(Q₃₂);
wherein Q₁₁, Q₁₂, Q₂₁, Q₂₂, Q₃₁ and Q₃₂ are each independently selected from
a hydrogen, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group; and
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

2. The condensed cyclic compound of claim 1, wherein
X₁ is C(R₁),
X₂ is C(R₂),
X₃ is C(R₃),
X₄ is C(R₄),
X₅ is C(R₅),
X₆ is C(R₆),
X₇ is C(R₇), and
X₈ is N or C(R₈).

3. The condensed cyclic compound of claim 1 or 2, wherein
L₁ is selected from
a phenylene group, a naphthylene group, a fluorenylene group, a phenanthrenylene group, an anthracenylene group, a triphenylenylene group, a pyrenylene group, a chrysenylene group, a pyridinylene group, a pyrazinylene group, a pyrimidinylene group, a pyridazinylene group, and a triazinylene group; and
a phenylene group, a naphthylene group, a fluorenylene group, a phenanthrenylene group, an anthracenylene group, a triphenylenylene group, a pyrenylene group, a chrysenylene group, a pyridinylene group, a pyrazinylene group, a pyrimidinylene group, a pyridazinylene group, and a triazinylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, a fluorenyl group, a phenanthrenyl group, an anthracenyl group, a triphenylenyl group, a pyrenyl group, a chrysenyl group, a pyridinyl group, a pyrazinyl group, a pyrimidinyl group, a pyridazinyl group, and a triazinyl group, and wherein
L₁ is preferably selected from
a phenylene group, a naphthylene group, a pyridinylene group, a pyrimidinylene group, and a triazinylene group; and
a phenylene group, a naphthylene group, a pyridinylene group, a pyrimidinylene group, and a triazinylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, and a naphthyl group.

4. The condensed cyclic compound of any of claims 1-3, wherein
Ar₁ to Ar₃ are each independently selected from the groups represented by Formulae 2-1 to 2-40: 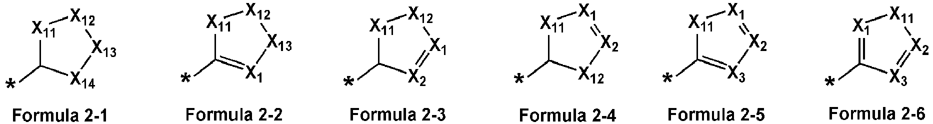 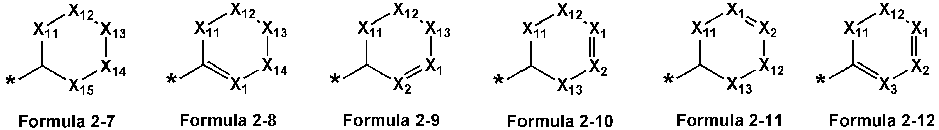 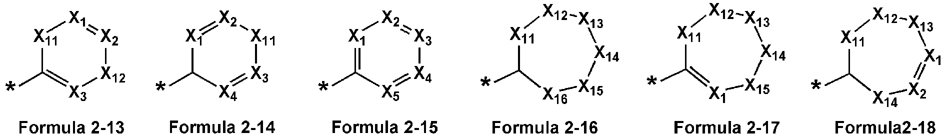 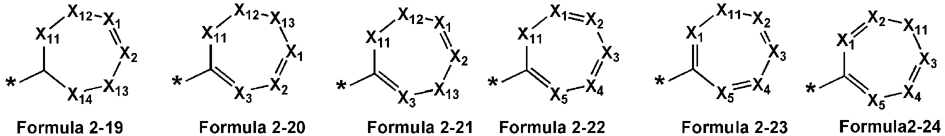 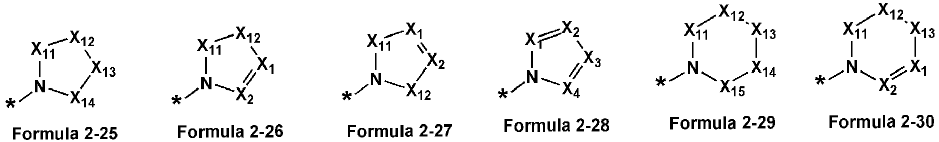 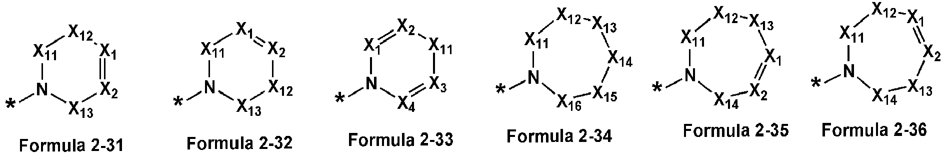 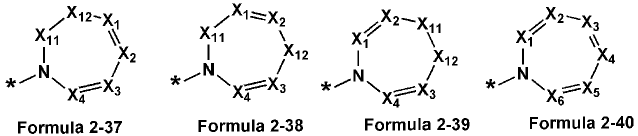 wherein in Formulae 2-1 to 2-40,
X₁₁ is O, S, N(Z₁₁) or C(Z₁₁ₐ)(Z_{11b}),
X₁₂ is O, S, N(Z₁₂) or C(Z₁₂ₐ)(Z_{12b}),
X₁₃ is O, S, N(Z₁₃) or C(Z₁₃ₐ)(Z_{13b}),
X₁₄ is O, S, N(Z₁₄) or C(Z₁₄ₐ)(Z_{14b}),
X₁₅ is O, S, N(Z₁₅) or C(Z₁₅ₐ)(Z_{15b}),
X₁₆ is O, S, N(Z₁₆) or C(Z₁₆ₐ)(Z_{16b});
X₁ is N or C(Z₁),
X₂ is N or C(Z₂),
X₃ is N or C(Z₃),
X₄ is N or C(Z₄),
X₅ is N or C(Z₅), and
X₆ is N or C(Z₆);
Z₁₁ to Z₁₆, Z₁₁ₐ to Z₁₆ₐ, Z_{11b} to Z_{16b}, and Z₁ to Z₆ are each independently a hydrogen, or a binding site to each of R₁₁, R₁₂, and R₁₃ in Formulae 1A to 1D; and
* is a binding site to Si in Formulae 1A to 1D, and, wherein
Ar₁ to Ar₃ preferably are each independently a group derived from a compound selected from a benzene, a pyridine, a pyrazine, a pyrimidine, a pyridazine, a triazine, a furan, a thiophene, a pyrrole, an imidazole, a triazole, a cyclohexane, a tetrahydro-2H-pyran, a piperidine, a tetrahydro-2H-thiopyran, a (2Z,4Z,6Z)-oxepine, a (2Z,4Z,6Z)-1H-azepine, and a (2Z,4Z,6Z)-thiepine.

5. The condensed cyclic compound of any of claims 1-4, wherein
R₁ to R₈ are each independently selected from a hydrogen, a deuterium, -F, -CI, -Br, -I, a cyano group and a substituted or unsubstituted group selected from: a methyl group, an ethyl group, a propyl group, an isobutyl group, a sec-butyl group, a tert-butyl group, a pentyl group, an iso-amyl group, a hexyl group and a phenyl group;
R₉ is selected from
a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₂₀ alkoxy group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a pyridinyl group, a pyrimidinyl group, and a triazinyl group;
a cyclopentyl group, a cyclohexyl group, a cyclopentenyl group, a cyclohexenyl group, a cycloheptenyl group, a piperidinyl group, a tetrahydro-2H-pyranyl group, a tetrahydro-2H-thiopyranyl group, a phenyl group, a fluorenyl group, a dibenzosilolyl group, a pyrrolyl group, an imidazolyl group, a pyrazolyl group, a pyridinyl group, a pyrazinyl group, a pyrimidinyl group, a pyridazinyl group, an isoindolyl group, an indolyl group, a furanyl group, a thiophenyl group, a thiazolyl group, an isothiazolyl group, an isoxazolyl group, an oxazolyl group, a triazolyl group, a tetrazolyl group, an oxadiazolyl group, a triazinyl group, a carbazolyl group, a dibenzofuranyl group, and a dibenzothiophenyl group; and
a cyclopentyl group, a cyclohexyl group, a cyclopentenyl group, a cyclohexenyl group, a cycloheptenyl group, a piperidinyl group, a tetrahydro-2H-pyranyl group, a tetrahydro-2H-thiopyranyl group, a phenyl group, a fluorenyl group, a dibenzosilolyl group, a pyrrolyl group, an imidazolyl group, a pyrazolyl group, a pyridinyl group, a pyrazinyl group, a pyrimidinyl group, a pyridazinyl group, an isoindolyl group, an indolyl group, a furanyl group, a thiophenyl group, a thiazolyl group, an isothiazolyl group, an isoxazolyl group, an oxazolyl group, a triazolyl group, a tetrazolyl group, an oxadiazolyl group, a triazinyl group, a carbazolyl group, a dibenzofuranyl group, and a dibenzothiophenyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cyclopentenyl group, a cyclohexenyl group, a cycloheptenyl group, a piperidinyl group, a tetrahydro-2H-pyranyl group, a tetrahydro-2H-thiopyranyl group, a phenyl group, a fluorenyl group, a dibenzosilolyl group, a pyrrolyl group, an imidazolyl group, a pyrazolyl group, a pyridinyl group, a pyrazinyl group, a pyrimidinyl group, a pyridazinyl group, an isoindolyl group, an indolyl group, a furanyl group, a thiophenyl group, a thiazolyl group, an isothiazolyl group, an isoxazolyl group, an oxazolyl group, a triazolyl group, a tetrazolyl group, an oxadiazolyl group, a triazinyl group, a carbazolyl group, a dibenzofuranyl group, a dibenzothiophenyl group, and -N(Q₃₁)(Q₃₂);
wherein Q₁, Q₂, Q₃₁, and Q₃₂ are each independently selected from
a hydrogen, a C₁-C₂₀ alkyl group, a C₁-C₂₀ alkoxy group, a cyclopentyl group, a cyclohexyl group, a cyclopentenyl group, a cyclohexenyl group, a cycloheptenyl group, a piperidinyl group, a tetrahydro-2H-pyranyl group, a tetrahydro-2H-thiopyranyl group, a phenyl group, a fluorenyl group, a dibenzosilolyl group, a pyrrolyl group, an imidazolyl group, a pyrazolyl group, a pyridinyl group, a pyrazinyl group, a pyrimidinyl group, a pyridazinyl group, an isoindolyl group, an indolyl group, a furanyl group, a thiophenyl group, a thiazolyl group, an isothiazolyl group, an isoxazolyl group, an oxazolyl group, a triazolyl group, a tetrazolyl group, an oxadiazolyl group, a triazinyl group, a carbazolyl group, a dibenzofuranyl group, and a dibenzothiophenyl group; and
a cyclopentyl group, a cyclohexyl group, a cyclopentenyl group, a cyclohexenyl group, a cycloheptenyl group, a piperidinyl group, a tetrahydro-2H-pyranyl group, a tetrahydro-2H-thiopyranyl group, a phenyl group, a fluorenyl group, a dibenzosilolyl group, a pyrrolyl group, an imidazolyl group, a pyrazolyl group, a pyridinyl group, a pyrazinyl group, a pyrimidinyl group, a pyridazinyl group, an isoindolyl group, an indolyl group, a furanyl group, a thiophenyl group, a thiazolyl group, an isothiazolyl group, an isoxazolyl group, an oxazolyl group, a triazolyl group, a tetrazolyl group, an oxadiazolyl group, a triazinyl group, a carbazolyl group, a dibenzofuranyl group, and a dibenzothiophenyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cyclopentenyl group, a cyclohexenyl group, a cycloheptenyl group, a piperidinyl group, a tetrahydro-2H-pyranyl group, a tetrahydro-2H-thiopyranyl group, a phenyl group, a fluorenyl group, a dibenzosilolyl group, a pyrrolyl group, an imidazolyl group, a pyrazolyl group, a pyridinyl group, a pyrazinyl group, a pyrimidinyl group, a pyridazinyl group, an isoindolyl group, an indolyl group, a furanyl group, a thiophenyl group, a thiazolyl group, an isothiazolyl group, an isoxazolyl group, an oxazolyl group, a triazolyl group, a tetrazolyl group, an oxadiazolyl group, a triazinyl group, a carbazolyl group, a dibenzofuranyl group, and a dibenzothiophenyl group.

6. The condensed cyclic compound of claim 5, wherein
R₁ to R₈ are each independently selected from a hydrogen, a deuterium, -F, -CI, -Br, -I, a cyano group and a substituted or unsubstituted group selected from: a methyl group, an ethyl group, a propyl group, an isobutyl group, a sec-butyl group, a tert-butyl group, a pentyl group, an iso-amyl group, a hexyl group and a phenyl group;
R₉ is selected from a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a pyridinyl group, and a pyrimidinyl group.

7. The condensed cyclic compound of any of claims 1-6, wherein
R₁₁ to R₁₃ are each independently a hydrogen, a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, -N(Q₁)(Q₂), wherein Q₁ and Q₂ are each independently selected from a C₁-C₅ alkyl group, and a group represented by Formulae 4-1 to 4-49 and 4-61 to 4-74: 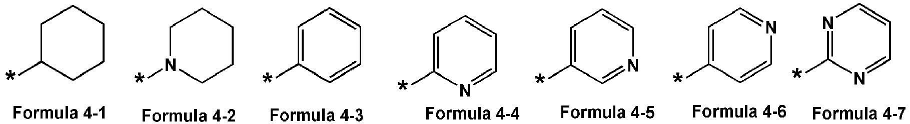 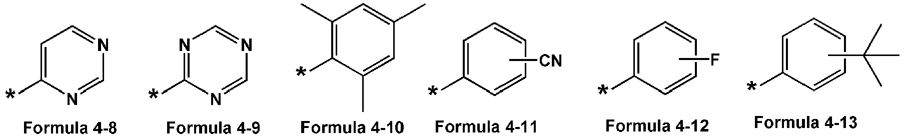 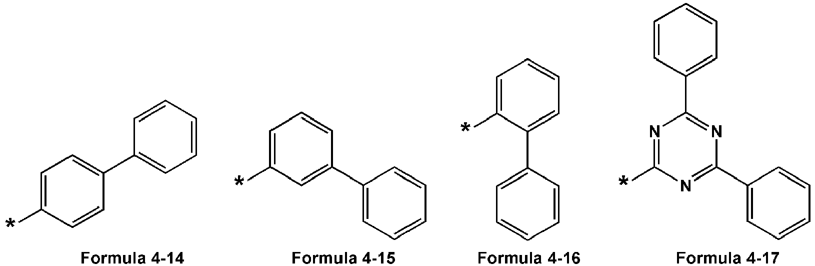 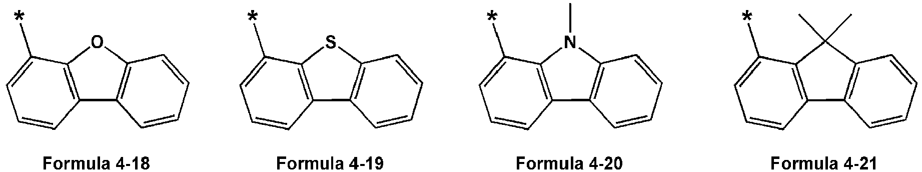 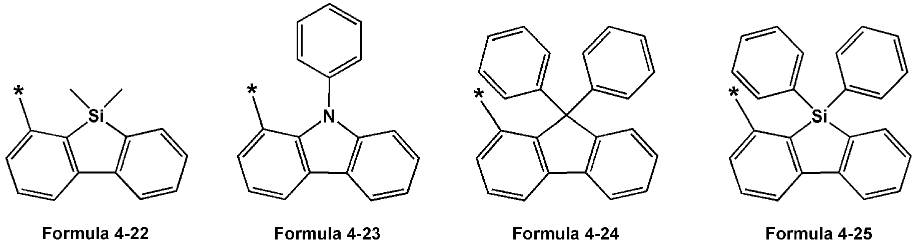 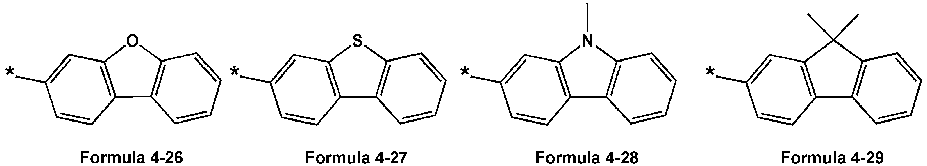 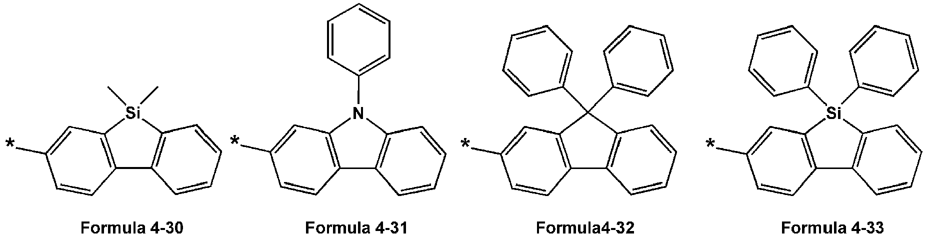 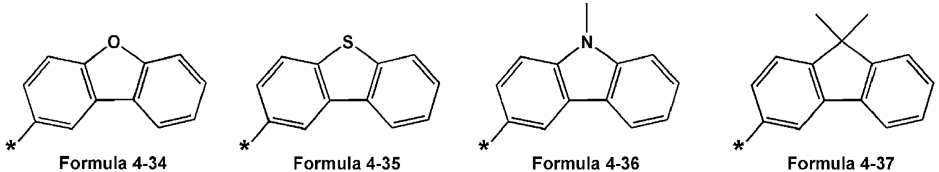 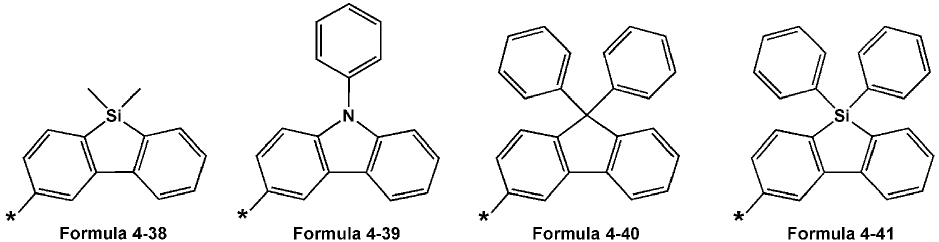 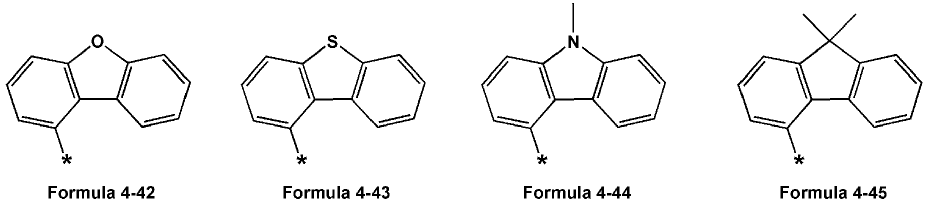 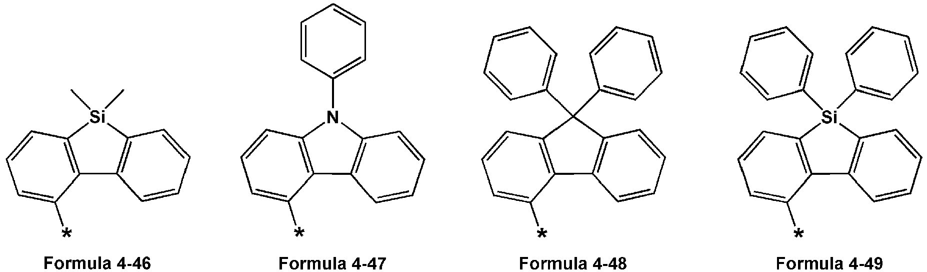 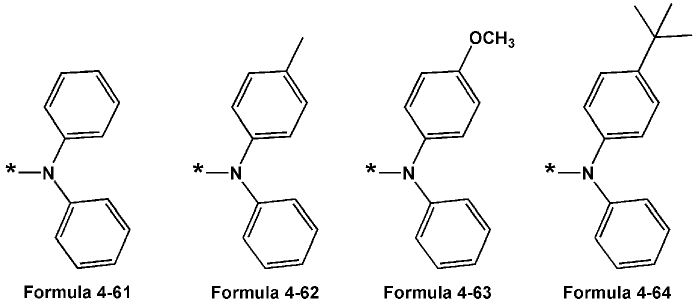 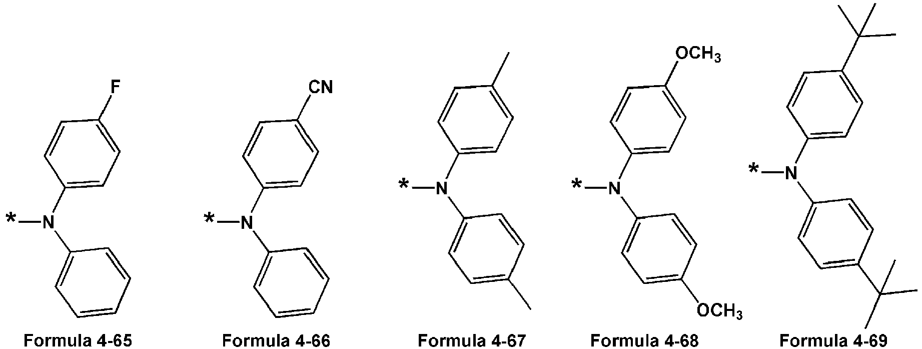 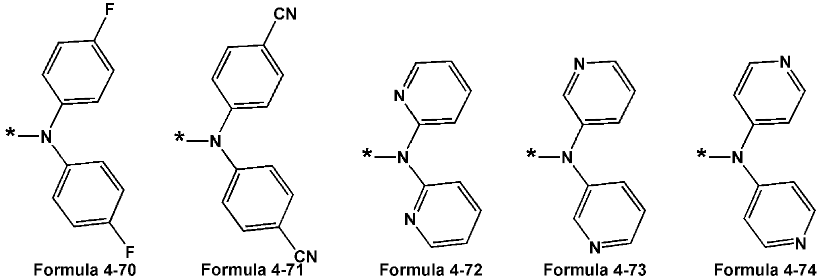 wherein in Formulae 4-1 to 4-49 and 4-61 to 4-74, * indicates a binding site to each of Ar₁ to Ar₃ in Formulae 1A to 1D.

8. The condensed cyclic compound of any of claims 1-7, wherein
*-Ar₁-(R₁₁)ₐ₁ in Formulae 1A to 1D is a group represented by any one selected from Formulae 11-1 to 11-47,
*-Ar₂-(R₁₂)ₐ₂ in Formulae 1A to 1D is a group represented by any one selected from Formulae 12-1 to 12-47, and
*-Ar₃-(R₁₃)ₐ₃ in Formulae 1A to 1D is a group represented by any one selected from Formulae 13-1 to 13-47: 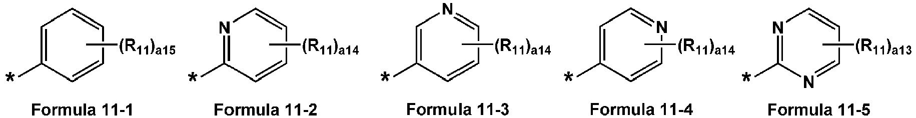 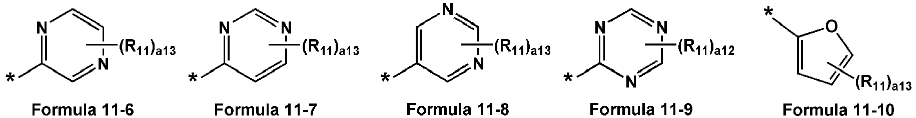 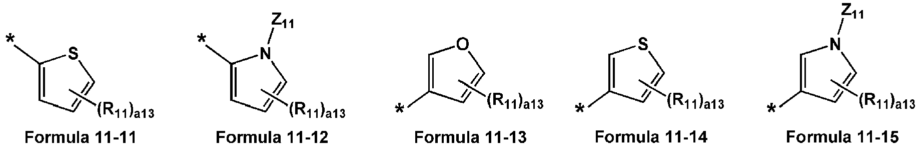 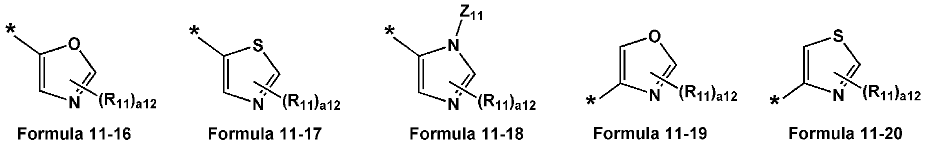 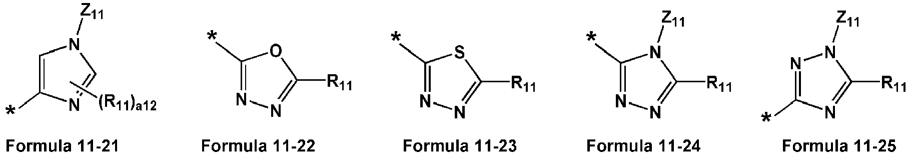 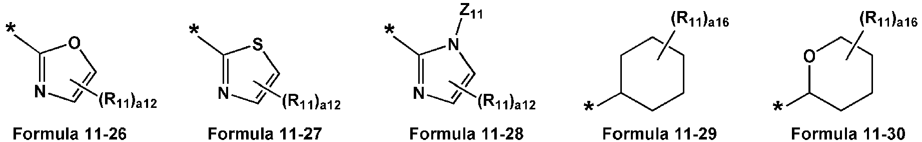 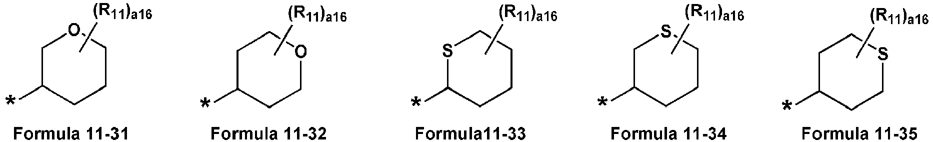 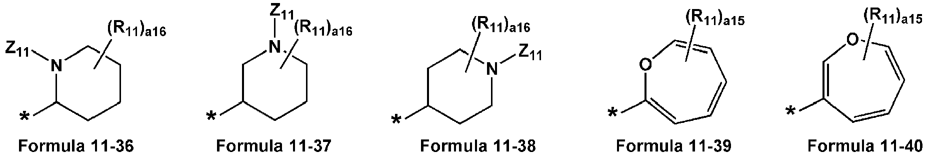 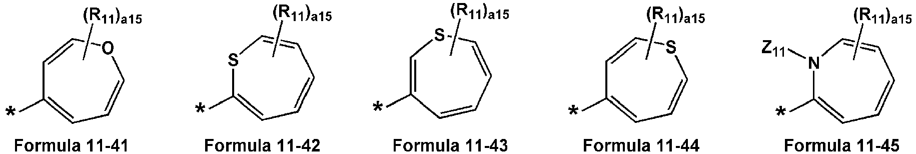 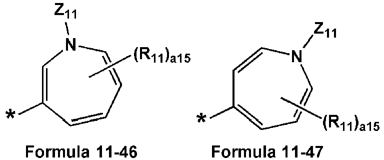 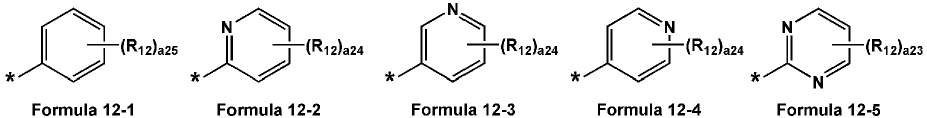 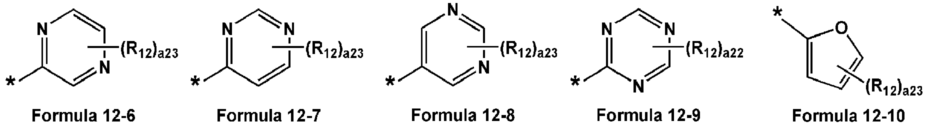 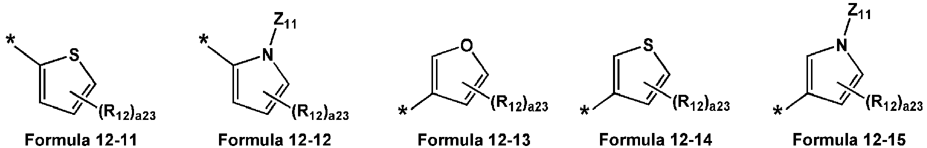 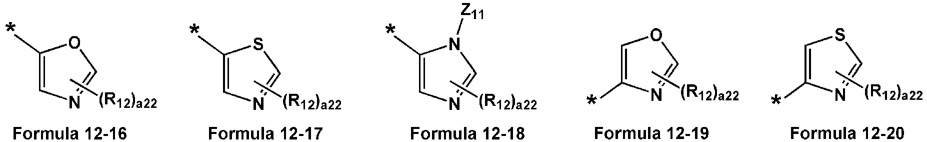 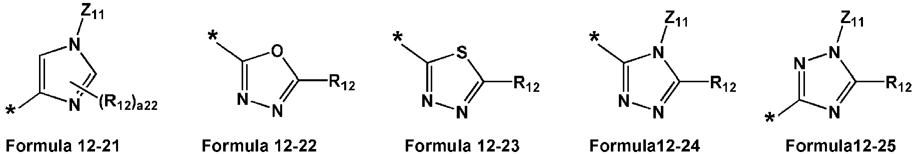 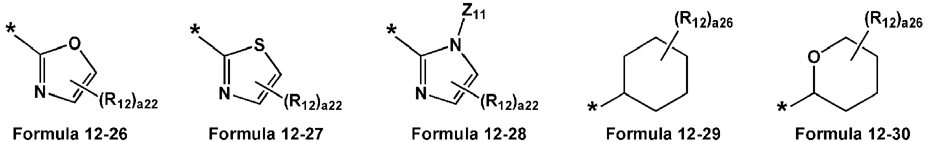 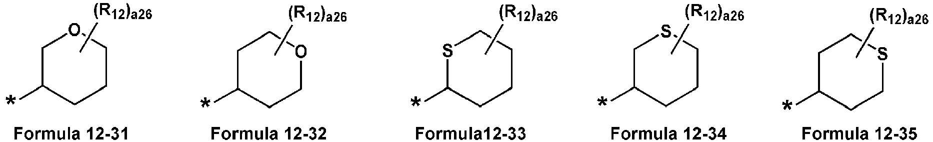 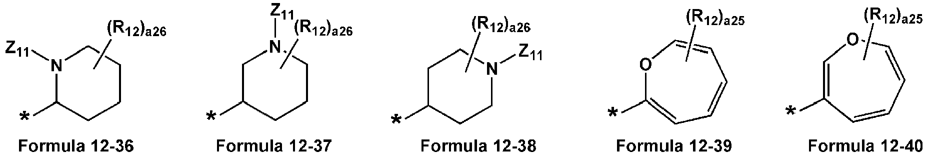 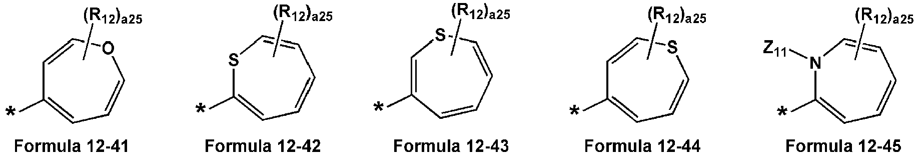 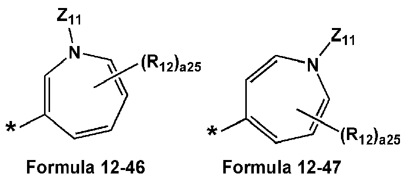 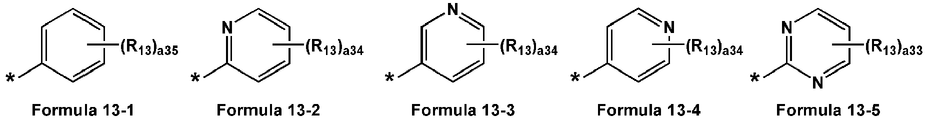 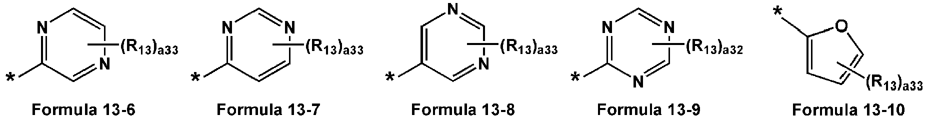 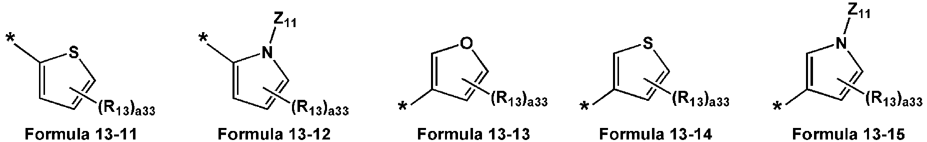 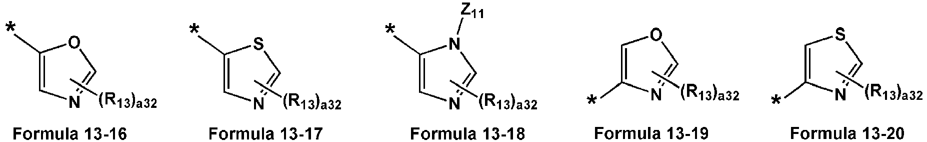 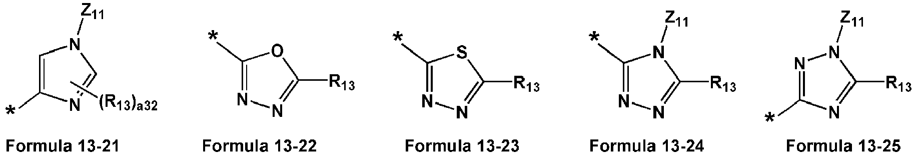 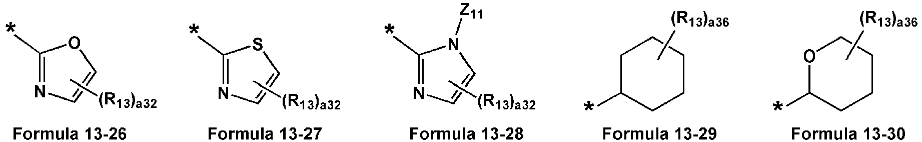 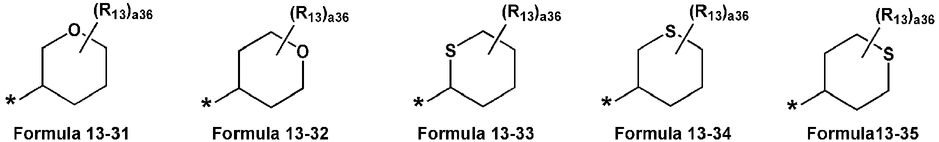 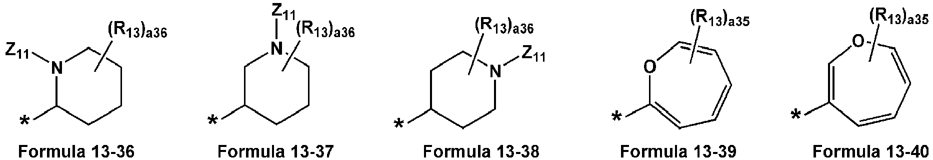 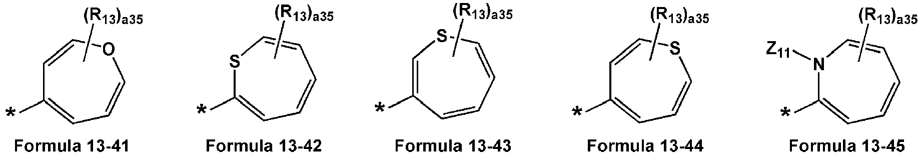 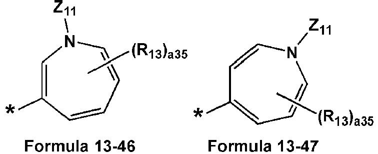 wherein in Formulae 11-1 to 11-47, 12-1 to 12-47, and 13-1 to 13-47,
R₁₁ to R₁₃ are the same as in claim 1,
a16, a26, and a36 are each independently an integer selected from 1, 2, 3, 4, 5, and 6,
a15, a25, and a35 are each independently an integer selected from 1, 2, 3, 4, and 5,
a14, a24, and a34 are each independently an integer selected from 1, 2, 3, and 4,
a13, a23, and a33 are each independently an integer selected from 1, 2, and 3,
a12, a22, and a32 are each independently an integer selected from 1 and 2, and
* is a binding site to Si in Formulae 1A to 1D.

9. The condensed cyclic compound of claim 8, wherein
Ar₁ in Formulae 1A to 1D is a group represented by any one selected from Formulae 11-1 to 11-9 above, and
Ar₃ is a group represented by any one selected from Formulae 13-1 to 13-9 above.

10. The condensed cyclic compound of any of claims 1-9, wherein
in Formulae 1A to 1D, Ar₁ = Ar₂ = Ar₃, R₁₁ ≠ R₁₂, and R₁₂ is not a hydrogen.

11. The condensed cyclic compound of any of claims 1-10, wherein
the condensed cyclic compound is represented by one selected from Formulae 1A(1) to 1D(8): 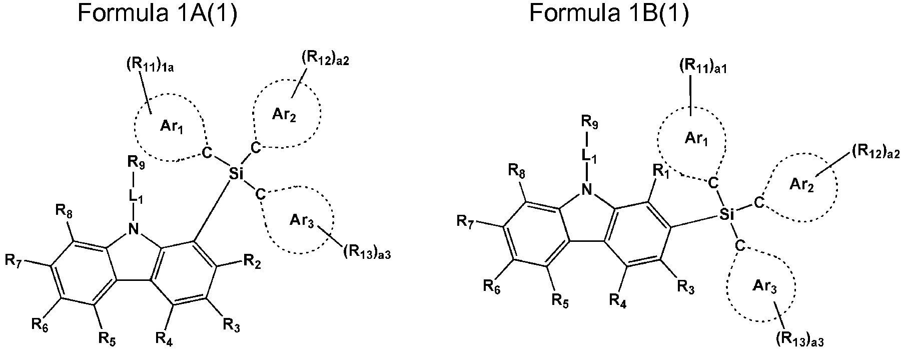 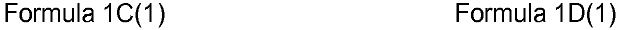 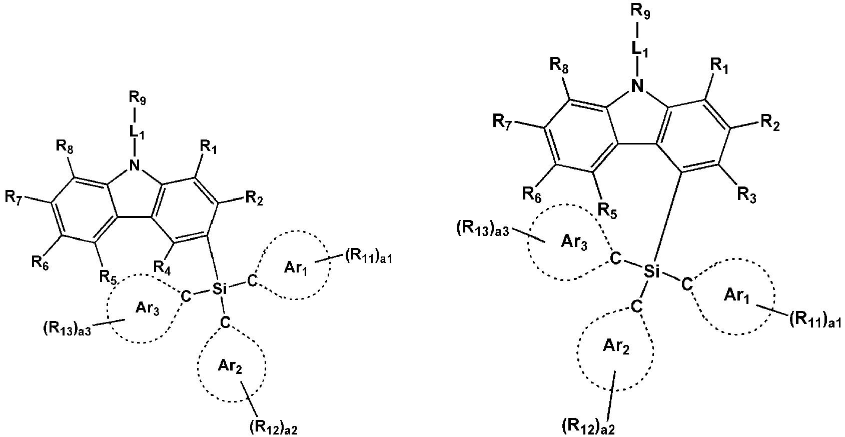 wherein L₁, Ar₁ to Ar₃, R₁ to R₉, R₁₁ to R₁₃, and a1 to a3 in Formulae 1A(1) to 1D(1) are the same as in claim 1.

12. The condensed cyclic compound of claim 1, wherein
the condensed cyclic compound is one of Compounds 1 to 100, 114 to 154, 156, 158, 159, 162 to 177, 184 to 195, 200 to 213, 220 to 231, 233, 233 to 235, 237 to 250, 257 to 268, 270, 271, 273 to 285, 289 to 305, 309 to 323, 325, 327, 328, 330 and 332:

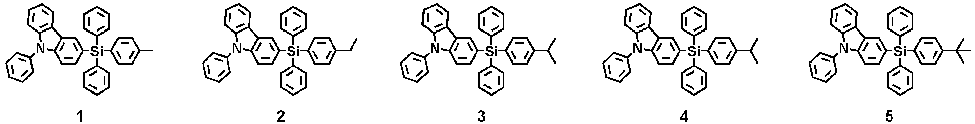

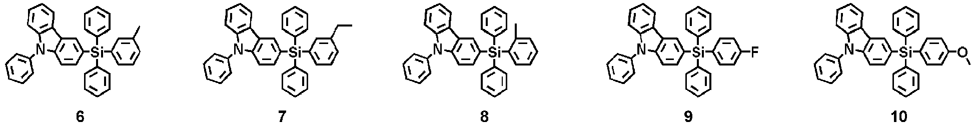

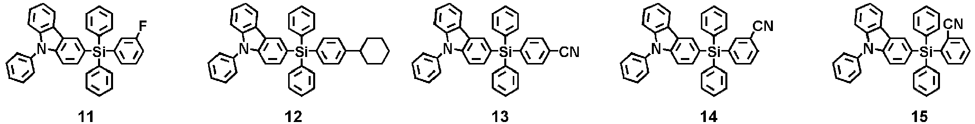

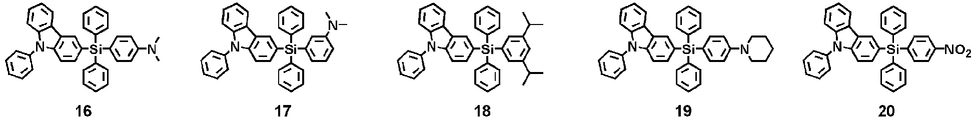

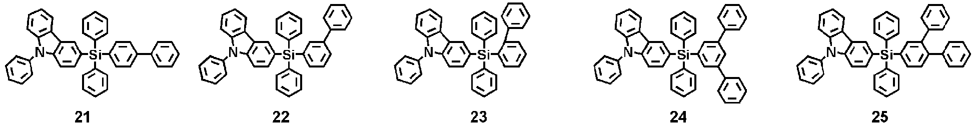

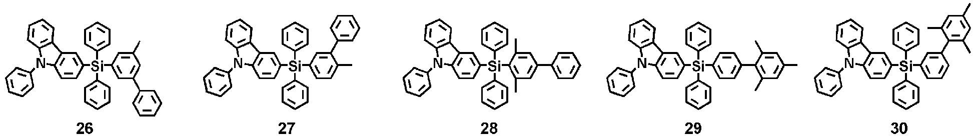

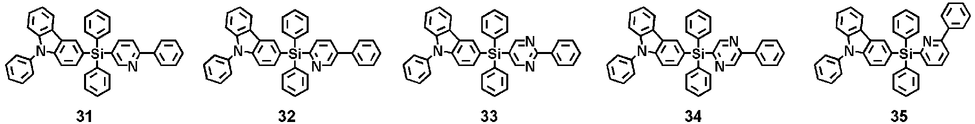

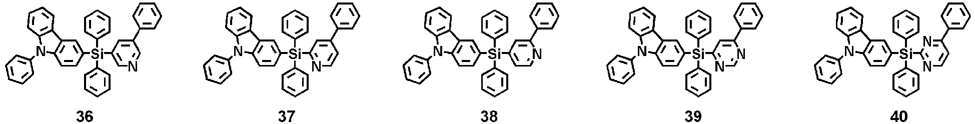

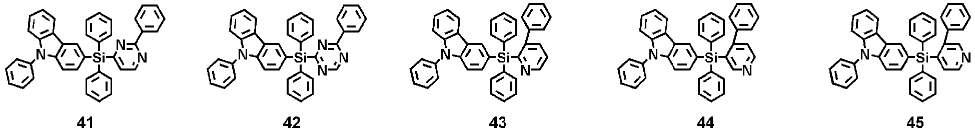

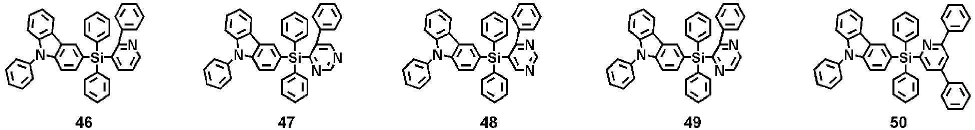

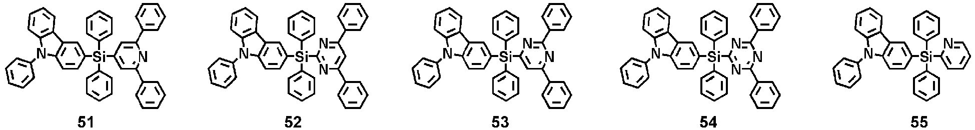

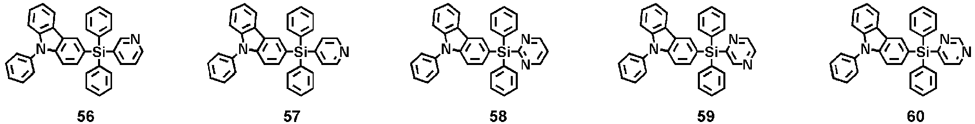

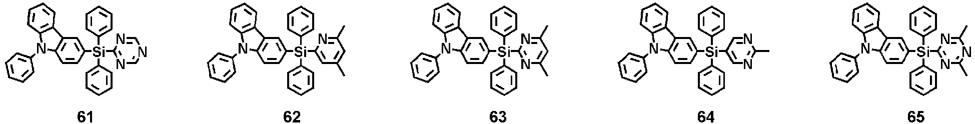

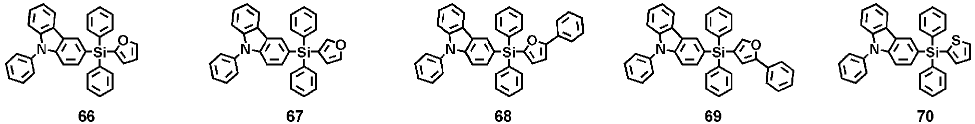

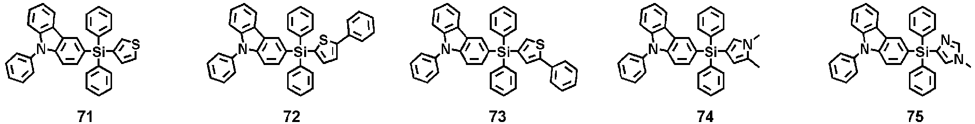

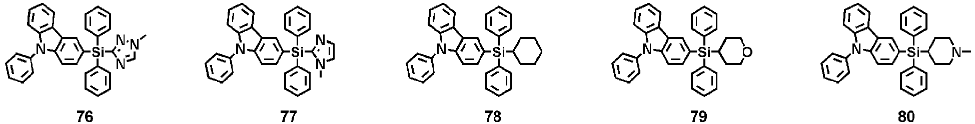

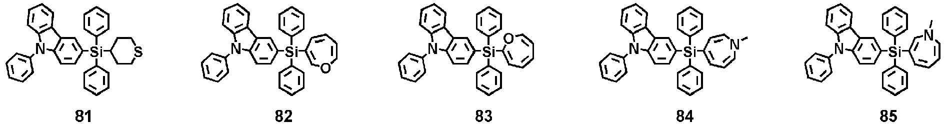

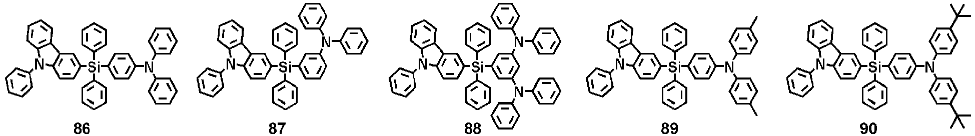

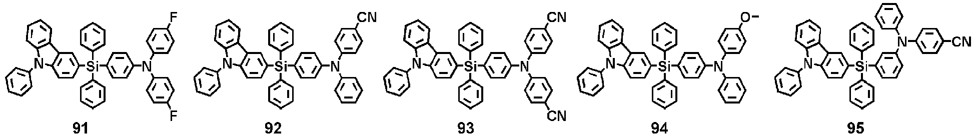

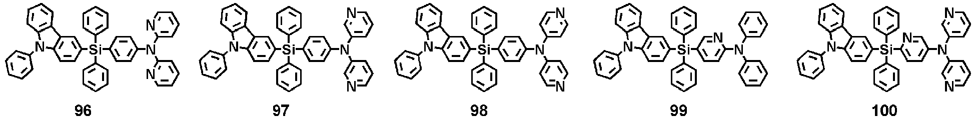

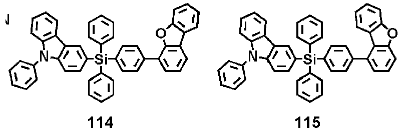

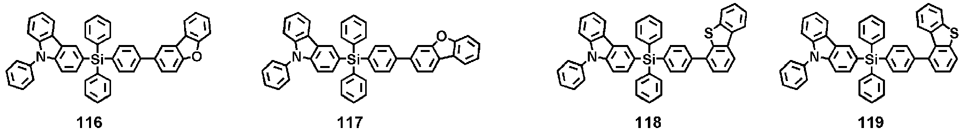

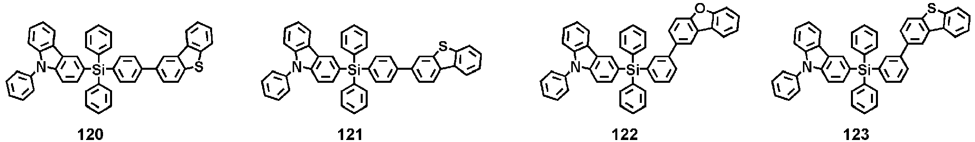

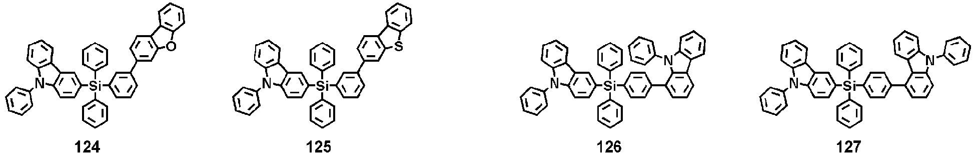

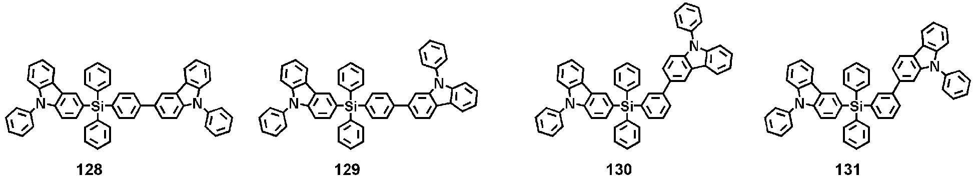

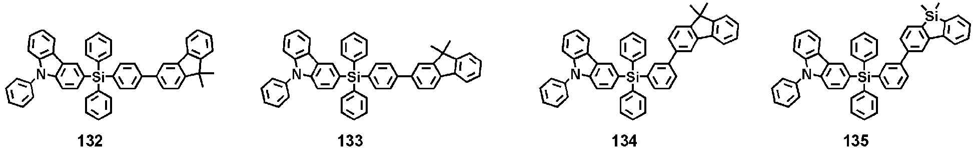

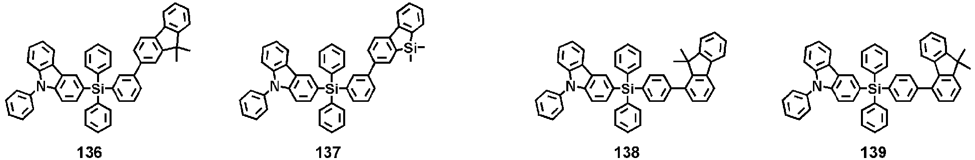

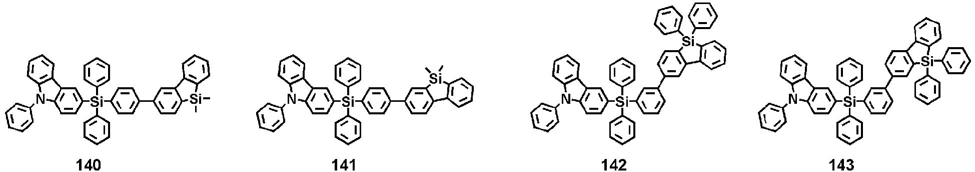

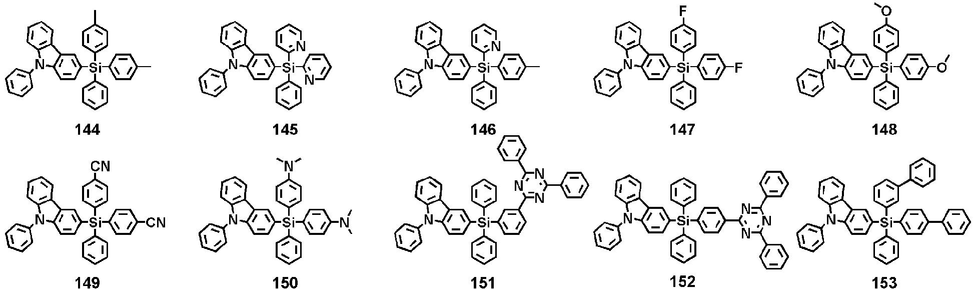

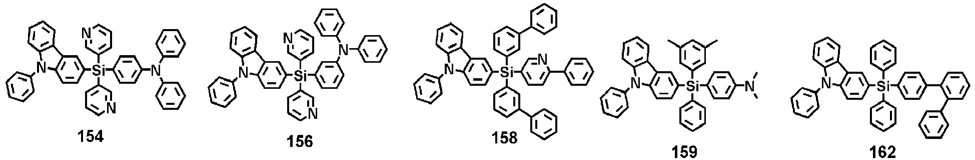

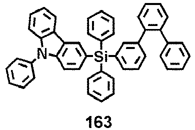

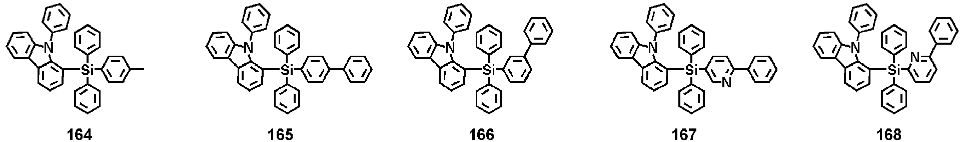

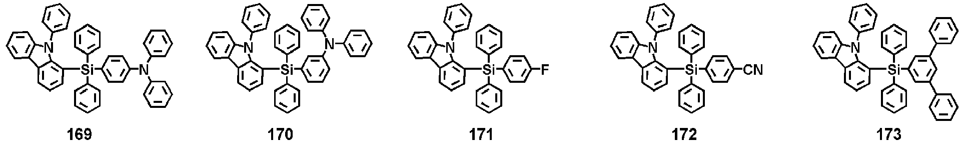

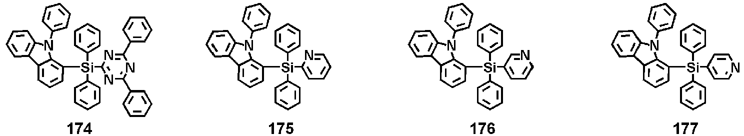

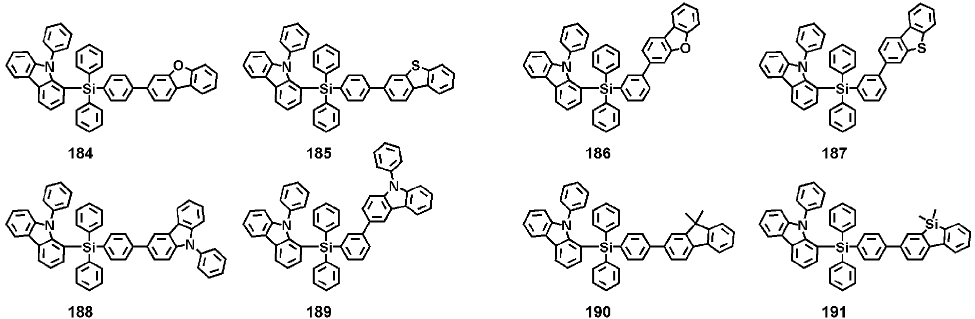

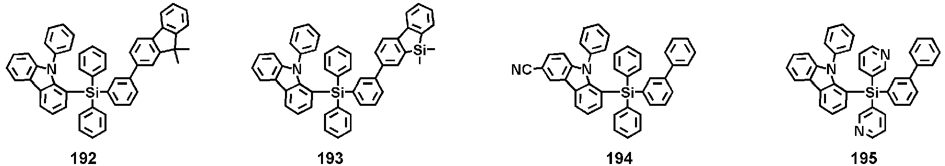

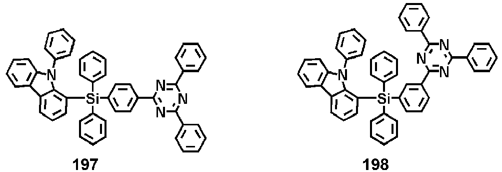

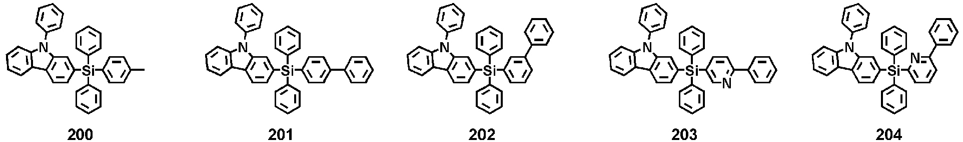

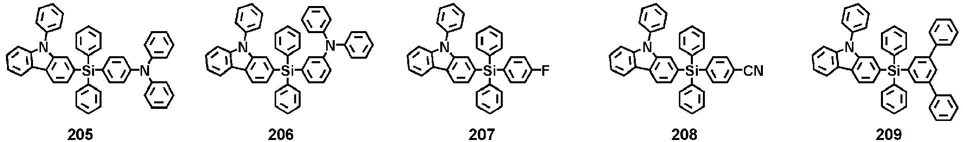

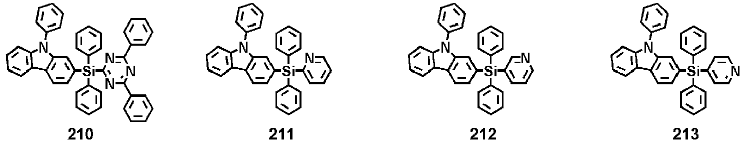

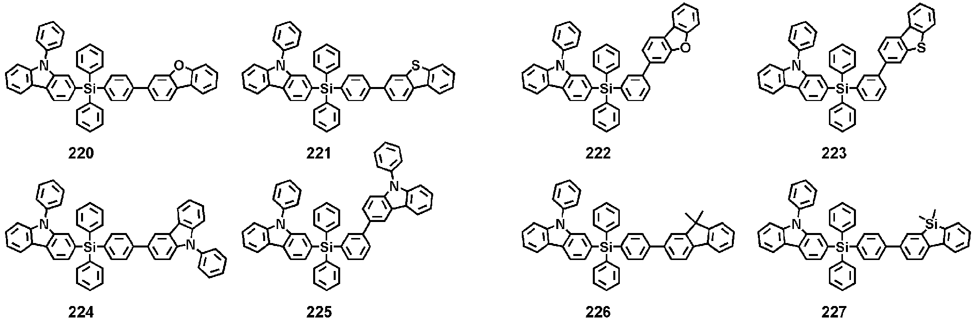

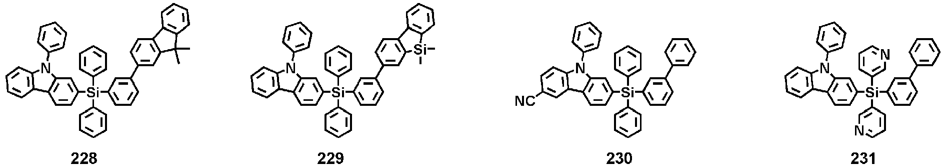

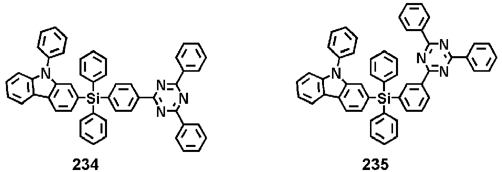

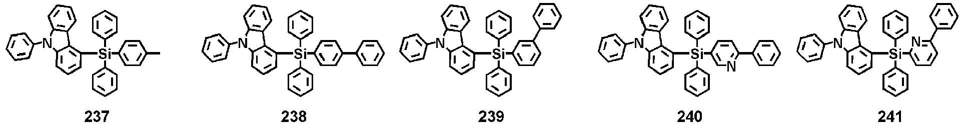

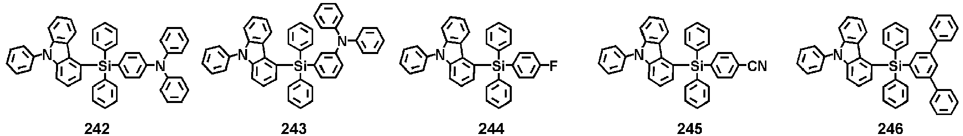

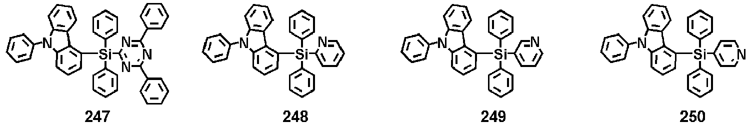

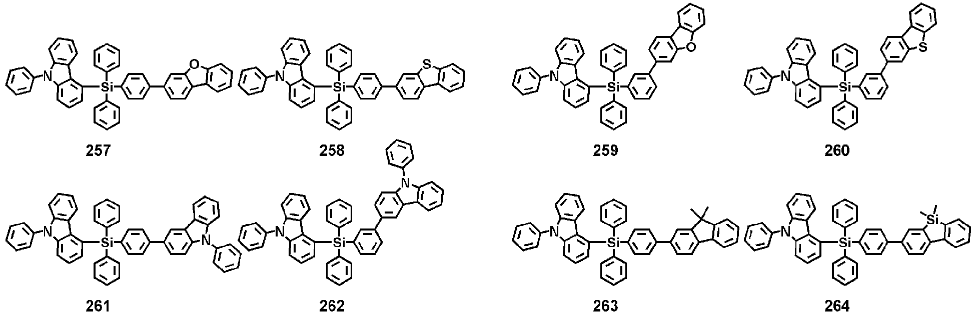

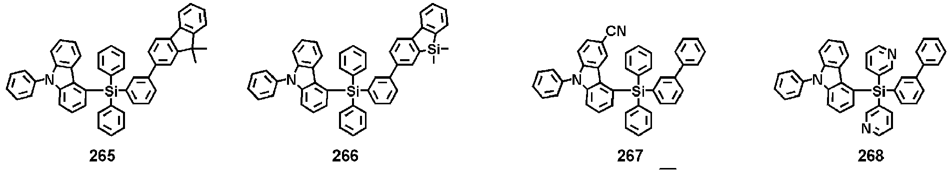

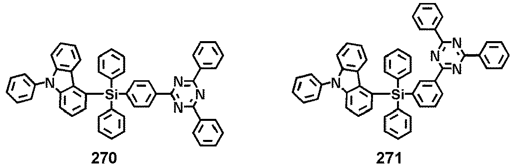

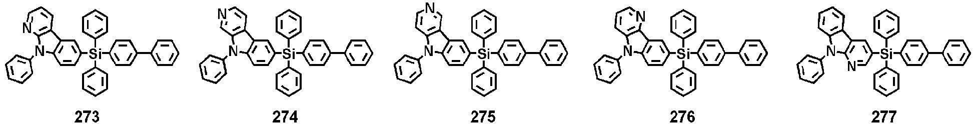

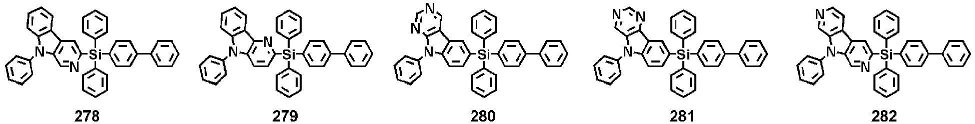

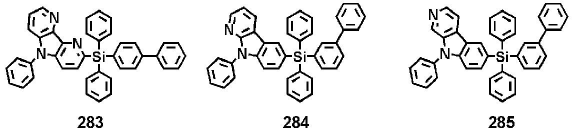

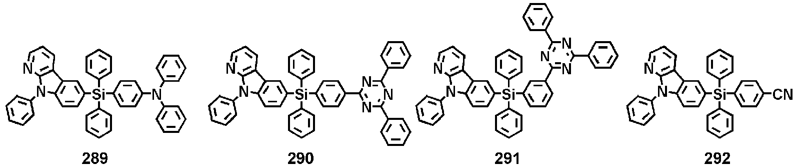

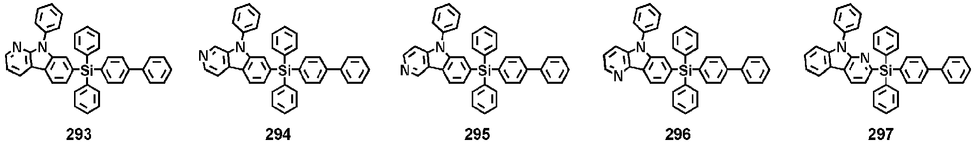

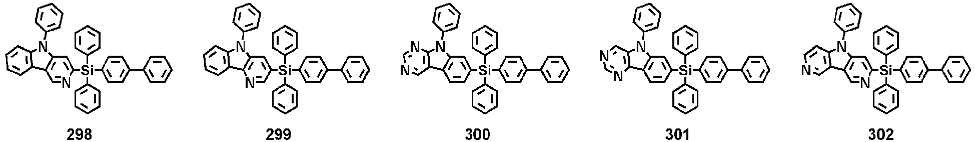

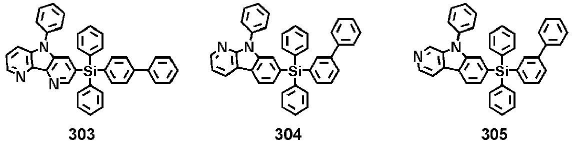

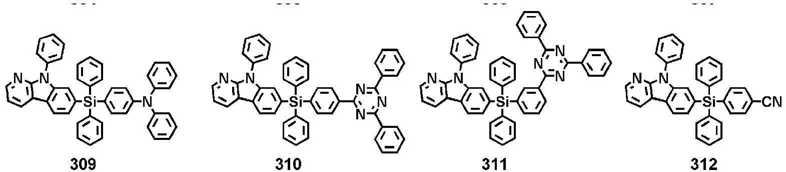

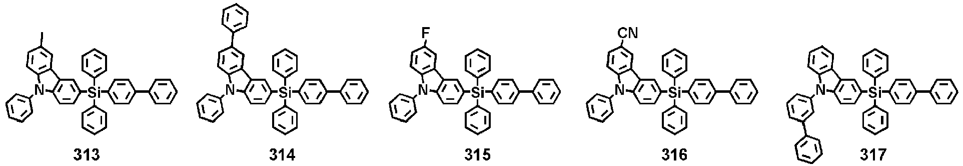

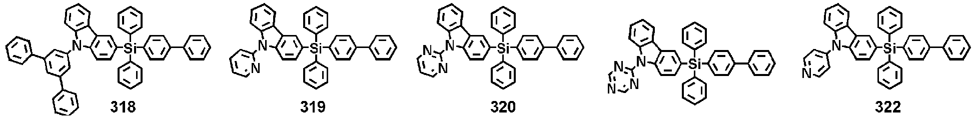

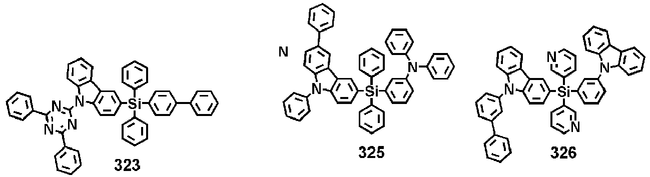

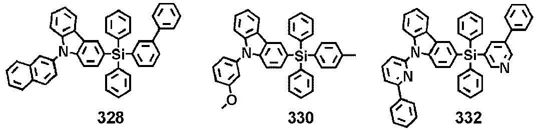

13. An organic light-emitting device comprising:
a first electrode;
a second electrode; and
an organic layer disposed between the first electrode and the second electrode,
wherein the organic layer comprises an emission layer, and further comprises at least one condensed cyclic compound represented by one of Formulae 1A to 1D of any of claims 1-12.

14. The organic light-emitting device of claim 13, wherein
the emission layer comprises the at least one condensed cyclic compound, wherein
the emission layer optionally further comprises a phosphorescent dopant, and the at least one condensed cyclic compound in the emission layer acts as a host.

## Patentansprüche

1. Kondensierte cyclische Verbindung, dargestellt durch eine der Formeln 1A bis 1D: 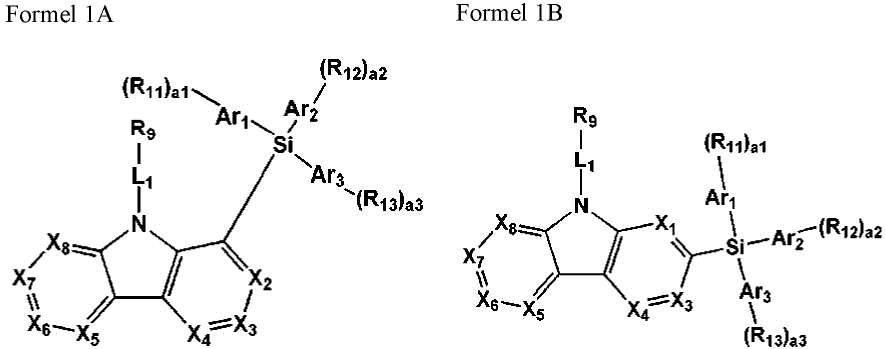 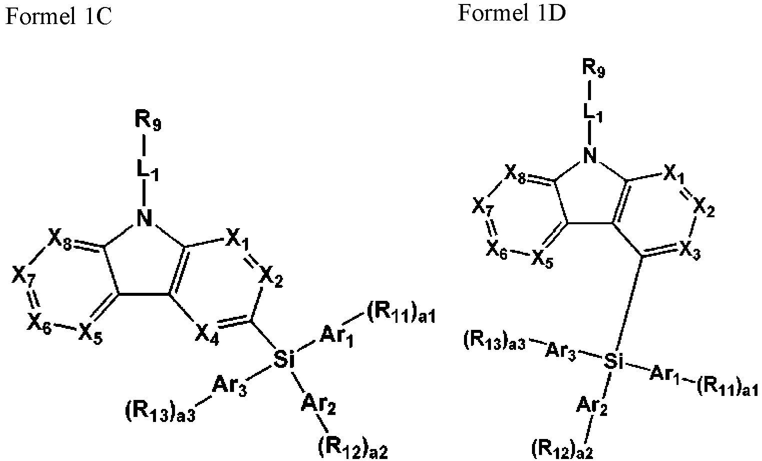 wobei in den Formeln 1A bis 1D
X₁ N oder C(R₁) ist,
X₂ N oder C(R₂) ist,
X₃ N oder C(R₃) ist,
X₄ N oder C(R₄) ist,
X₅ N oder C(R₅) ist,
X₆ N oder C(R₆) ist,
X₇ N oder C(R₇) ist, und
X₈ N oder C(R₈) ist;
L₁ jeweils unabhängig ausgewählt ist aus einer substituierten oder unsubstituierten C₃-C₁₀-Cycloalkylengruppe, einer substituierten oder unsubstituierten C₁-C₁₀-Heterocycloalkylengruppe, einer substituierten oder unsubstituierten C₃-C₁₀-Cycloalkenylengruppe, einer substituierten oder unsubstituierten C₁-C₁₀-Heterocycloalkenylengruppe, einer substituierten oder unsubstituierten C₆-C₆₀-Arylengruppe, einer substituierten oder unsubstituierten C₁-C₆₀-Heteroarylengruppe, einer substituierten oder unsubstituierten zweiwertigen nicht-aromatischen kondensierten polycyclischen Gruppe und einer substituierten oder unsubstituierten zweiwertigen nicht-aromatischen kondensierten heteropolycyclischen Gruppe;
Ar₁ bis Ar₃ jeweils unabhängig eine Gruppe sind, die von einer nicht-kondensierten carbocyclischen C₅-C₉-Gruppe oder einer nicht-kondensierten heterocyclischen C₁-C₇-Gruppe ist,
R₁ bis R₈ jeweils unabhängig ausgewählt sind aus einem Wasserstoff, einer Deuterium-,-F, -Cl, -Br, -I, einer Cyanogruppe und einer substituierten oder unsubstituierten Gruppe, ausgewählt aus: einer Methylgruppe, einer Ethylgruppe, einer Propylgruppe, einer Isobutylgruppe, einer sec-Butylgruppe, einer tert-Butylgruppe, einer Pentylgruppe, einer iso-Amylgruppe, einer Hexylgruppe, einer Phenylgruppe, einer Naphthylgruppe, einer Anthracenylgruppe, einer Phenanthrenylgruppe, einer Pyrenylgruppe und einer Chrysenylgruppe;
R₉ ausgewählt ist aus einem Wasserstoff, einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer substituierten oder unsubstituierten C₁-C₆₀-Alkylgruppe, einer substituierten oder unsubstituierten C₂-C₆₀-Alkenylgruppe, einer substituierten oder unsubstituierten C₂-C₆₀-Alkinylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, einer substituierten oder unsubstituierten C₃-C₁₀-Cycloalkenylgruppe, einer substituierten oder unsubstituierten C₁-C₁₀-Heterocycloalkenylgruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₁-C₆₀-Heteroarylgruppe, einer substituierten oder unsubstituierten einer einwertigen nicht-aromatischen kondensierten polycyclischen Gruppe und einer substituierten oder unsubstituierten einwertigen nicht-aromatischen kondensierten heteropolycyclischen Gruppe;
R₁₁ bis R₁₃ jeweils unabhängig ein Wasserstoff, ein Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, C₁-C₁₀-Alkylgruppe, eine C₁-C₁₀-Alkoxygruppe, -N(Q₁)(Q₂) ist, wobei Q₁ und Q₂ jeweils unabhängig ausgewählt sind aus einer C₁-C₁₀-Alkylgruppe und einer Gruppe, dargestellt durch die Formeln 3-1 bis 3-29 und 3-31 bis 3-37: 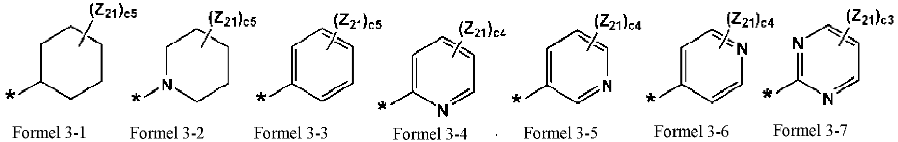 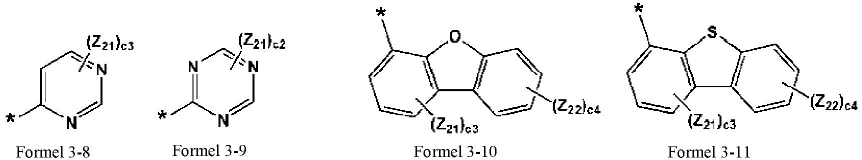 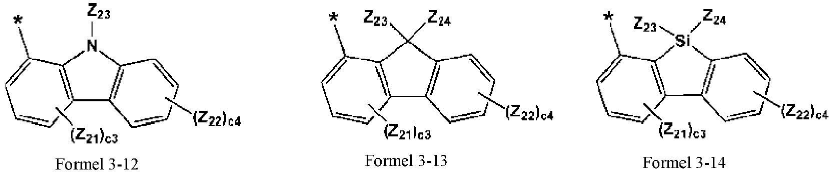 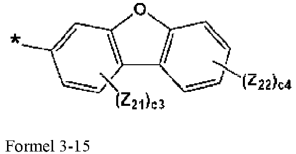 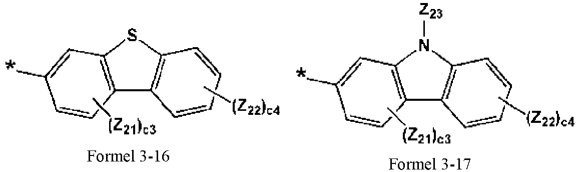 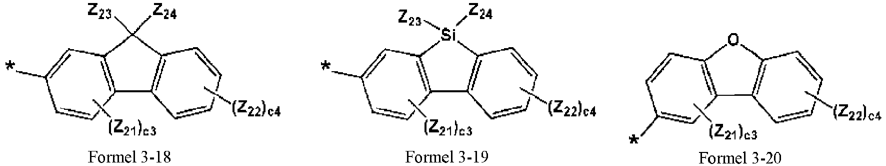 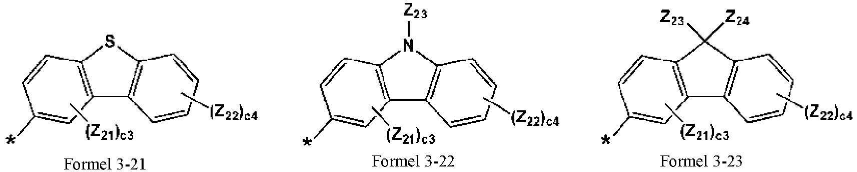 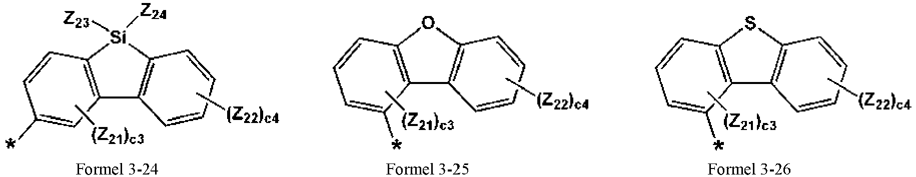 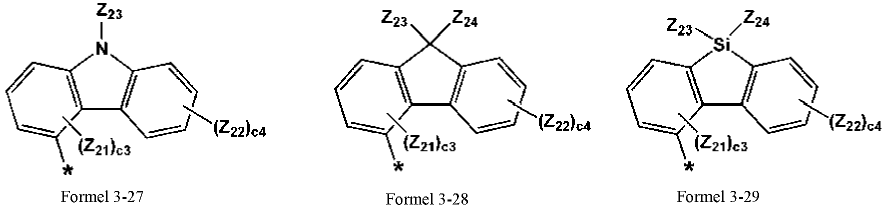 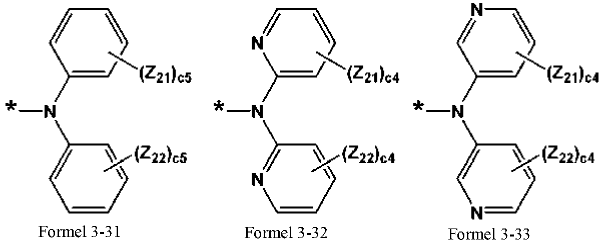 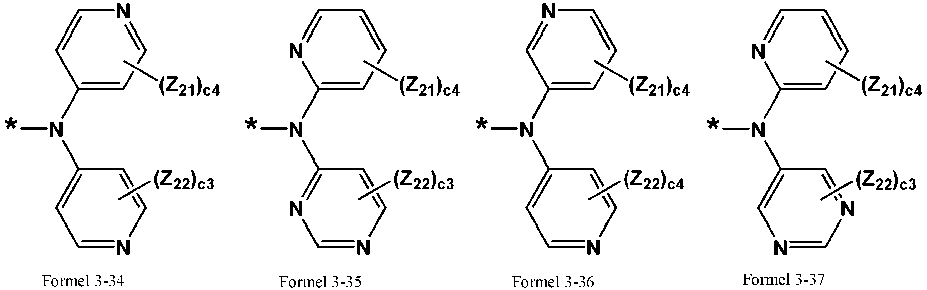 wobei in den Formeln 3-1 bis 3-29 und 3-31 bis 3-37,
Z₂₁ bis Z₂₄ jeweils unabhängig ausgewählt sind aus einem Wasserstoff, einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₁₀-Alkylgruppe, einer C₁-C₁₀-Alkoxygruppe, einer Phenylgruppe, einer Pyridinylgruppe, einer Pyrimidinylgruppe und einer Triazinylgruppe,
c₅ eine Ganzzahl ist, ausgewählt aus 1, 2, 3, 4 und 5,
c₄ eine Ganzzahl ist, ausgewählt aus 1, 2, 3 und 4,
c₃ eine Ganzzahl ist, ausgewählt aus 1, 2 und 3,
c₂ eine Ganzzahl ist, ausgewählt aus 1 bis 2,
* eine Bindungsstelle mit jedem von Ar₁ bis Ar₃ in den Formeln 1A bis 1D angibt,
a1 bis a3 jeweils unabhängig eine Ganzzahl sind, ausgewählt aus 1, 2, 3, 4, 5 und 6, mit der Maßgabe, dass, wenn a1 2 oder mehr ist, zwei oder mehrere Gruppen R₁₁ gleich oder verschieden voneinander sind, wenn a2 2 oder mehr ist, zwei oder mehrere Gruppen R₁₂ gleich oder verschieden voneinander sind, und wenn a3 2 oder mehr ist, zwei oder mehrere Gruppen R₁₃ gleich oder verschieden voneinander sind;
wobei in jeder der Formeln 1A bis 1D die Gruppen *-Ar₁-(R₁₁)ₐ₁, *-Ar₂-(R₁₂)ₐ₂ und *-Ar₃-(R₁₃)a₃ nicht miteinander identisch sind;
mindestens einer der Substituenten der substituierten C₃-C₁₀ Cycloalkylengruppe, der substituierten C₁-C₁₀-Heterocycloalkylengruppe, der substituierten C₃-C₁₀-Cycloalkenylengruppe, der substituierten C₁-C₁₀-Heterocycloalkenylengruppe, der substituierten C₆-C₆₀-Arylengruppe, der substituierten C₁-C₆₀-Heteroarylengruppe, der substituierten zweiwertigen nicht-aromatischen kondensierten polycyclischen Gruppe, der substituierten zweiwertigen nicht-aromatischen kondensierten heteropolycyclischen Gruppe, der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₁-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten monovalenten nicht-aromatischen kondensierten polycyclischen Gruppe und der substituierten monovalenten nicht-aromatischen kondensierten heteropolycyclischen Gruppe ausgewählt ist aus
einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkinylgruppe und einer C₁-C₆₀-Alkoxygruppe;
einer C₁-C₆₀-Alkylgruppe, einer C₂-C₆₀-Alkenylgruppe, einer C₂-C₆₀-Alkinylgruppe und einer C₁-C₆₀-Alkoxygruppe, jeweils substituiert mit mindestens einem, ausgewählt aus Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe, einer monovalenten nicht-aromatischen kondensierten heteropolycyclischen Gruppe, und -N(Q₁₁)(Q₁₂);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe;
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe, jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkinylgruppe, einer C₂-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe, und -N(Q₂₁)(Q₂₂); und
-N(Q₃₁)(Q₃₂);
wobei Q₁₁, Q₁₂, Q₂₁, Q₂₂, Q₃₁ und Q₃₂ jeweils unabhängig ausgewählt sind aus
einem Wasserstoff, einer C₁-C₆₀-Alkylgruppe, einer C₂-C₆₀-Alkenylgruppe, einer C₂-C₆₀ Alkinylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe; und
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe, jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten nicht-aromatischen kondensierten polycyclischen Gruppe und einer monovalenten nicht-aromatischen kondensierten heteropolycyclischen Gruppe.

2. Kondensierte zyklische Verbindung nach Anspruch 1, wobei
X₁ C(R₁) ist,
X₂ C(R₂) ist,
X₃ C(R₃) ist,
X₄ C(R₄) ist,
X₅ C(R₅) ist,
X₆ C(R₆) ist,
X₇ C(R₇) ist, und
X₈ N oder C(R₈) ist.

3. Kondensierte zyklische Verbindung nach Anspruch 1 oder 2, wobei
L₁ ausgewählt ist aus
einer Phenylengruppe, einer Naphthylengruppe, einer Fluorenylengruppe, einer Phenanthrenylengruppe, einer Anthracenylengruppe, einer Triphenylenylengruppe, einer Pyrenylengruppe, einer Chrysenylengruppe, einer Pyridinylengruppe, einer Pyrazinylengruppe, einer Pyrimidinylengruppe, einer Pyridazinylengruppe und einer Triazinylengruppe; und
einer Phenylengruppe, einer Naphthylengruppe, einer Fluorenylengruppe, einer Phenanthrenylengruppe, einer Anthracenylengruppe, einer Triphenylenylengruppe, einer Pyrenylengruppe, einer Chrysenylengruppe, einer Pyridinylengruppe, einer Pyrazinylengruppe, einer Pyrimidinylengruppe, einer Pyridazinylengruppe und einer Triazinylengruppe, jeweils substituiert mit mindestens einem ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Phenylgruppe, einer Naphthylgruppe, einer Fluorenylgruppe, einer Phenanthrenylgruppe, einer Anthracenylgruppe, einer Triphenylenylgruppe, einer Pyrenylgruppe, einer Chrysenylgruppe, einer Pyridinylgruppe, einer Pyrazinylgruppe, einer Pyrimidinylgruppe, einer Pyridazinylgruppe und einer Triazinylgruppe, und wobei
L₁ vorzugsweise ausgewählt ist aus
einer Phenylengruppe, einer Naphthylengruppe, einer Pyridinylengruppe, einer Pyrimidinylengruppe und einer Triazinylengruppe; und
einer Phenylengruppe, einer Naphthylengruppe, einer Pyridinylengruppe, einer Pyrimidinylengruppe und einer Triazinylengruppe, jeweils substituiert mit mindestens einem ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₁₀-Alkylgruppe, einer C₁-C₁₀-Alkoxygruppe, einer Phenylgruppe und einer Naphthylgruppe.

4. Kondensierte cyclische Verbindung nach einem der Ansprüche 1 bis 3, wobei Ar₁ bis Ar₃ jeweils unabhängig ausgewählt sind aus den Gruppen, dargestellt durch die Formeln 2-1 bis 2-40: 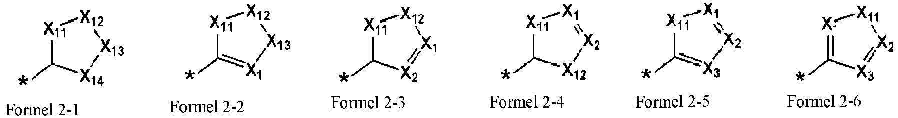 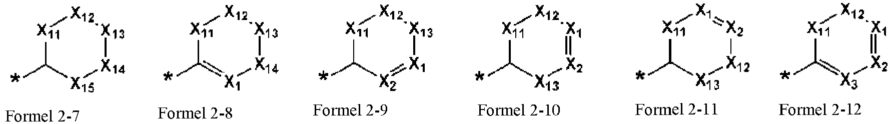 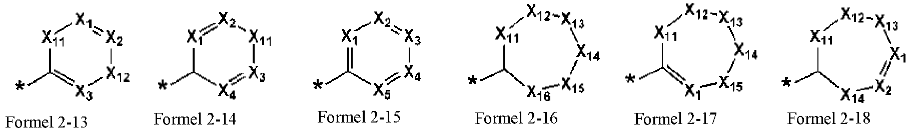 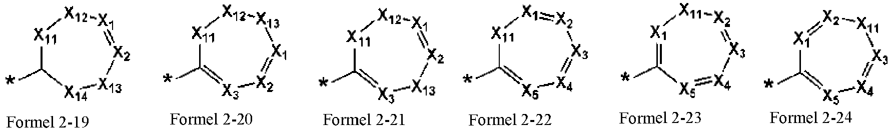 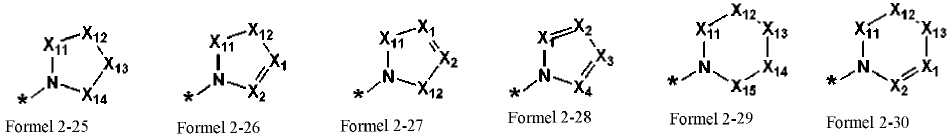 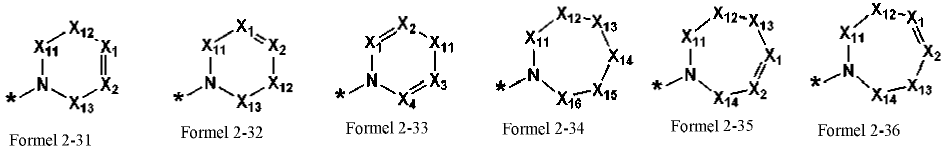 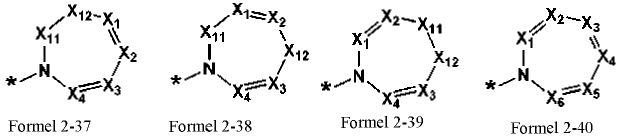 wobei in Formeln 2-1 bis 2-40,
X₁₁ O, S, N(Z₁₁) oder C(Z₁₁ₐ)(Z_{11b}) ist,
X₁₂ O, S, N(Z₁₂) oder C(Z₁₂ₐ)(Z_{12b}) ist,
X₁₃ O, S, N(Z₁₃) oder C(Z₁₃ₐ)(Z_{13b}) ist,
X₁₄ O, S, N(Z₁₄) oder C(Z₁₄ₐ)(Z_{14b}) ist,
X₁₅ O, S, N(Z₁₅) oder C(Z₁₅ₐ)(Z_{15b}) ist,
X₁₆ O, S, N(Z₁₆) oder C(Z₁₆ₐ)(Z_{16b}) ist;
X₁ N oder C(Z₁) ist,
X₂ N oder C(Z₂) ist,
X₃ N oder C(Z₃) ist,
X₄ N oder C(Z₄) ist,
X₅ N oder C(Z₅) ist, und
X₆ N oder C(Z₆) ist;
Z₁₁ bis Z₁₆, Z₁₁ₐ bis Z₁₆ₐ, Z_{11b} bis Z_{16b} und Z₁ bis Z₆ jeweils unabhängig ein Wasserstoff oder eine Bindungsstelle für jedes von R₁₁, R₁₂ und R₁₃ in den Formeln 1A bis 1D sind; und
* eine Bindungsstelle für Si in den Formeln 1A bis 1D ist, und wobei
Ar₁ bis Ar₃ vorzugsweise jeweils unabhängig eine Gruppe sind, die abgeleitet ist von einer Verbindung, ausgewählt aus einem Benzol, einem Pyridin, einem Pyrazin, einem Pyrimidin, einem Pyridazin, einem Triazin, einem Furan, einem Thiophen, einem Pyrrol, einem Imidazol, einem Triazol, einem Cyclohexan, einem Tetrahydro-2H-pyran, einem Piperidin, einem Tetrahydro-2H-thiopyran, einem (2Z,4Z,6Z)-Oxepin, einem (2Z,4Z,6Z)-1H-Azepin und einem (2Z,4Z,6Z)-Thiapin.

5. Kondensierte cyclische Verbindung nach einem der Ansprüche 1 bis 4, wobei R₁ bis R₈ jeweils unabhängig ausgewählt sind aus einem Wasserstoff, einem Deuterium, -F, -Cl, -Br, -I, einer Cyanogruppe und einer substituierten oder unsubstituierten Gruppe, ausgewählt aus: einer Methylgruppe, einer Ethylgruppe, einer Propylgruppe, einer Isobutylgruppe, einer sec-Butylgruppe, einer tert-Butylgruppe, einer Pentylgruppe, einer iso-Amylgruppe, einer Hexylgruppe und einer Phenylgruppe;
R₉ ausgewählt ist aus
einem Wasserstoff, einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder ein Salz davon, einer Sulfonsäuregruppe oder einem Salz davon, einer Phosphorsäuregruppe oder einem Salz davon, einer C₁-C₂₀-Alkylgruppe und einer C₁-C₂₀-Alkoxygruppe;
einer C₁-C₂₀-Alkylgruppe und einer C₁-C₂₀-Alkoxygruppe, jeweils substituiert mit mindestens einer Gruppe, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer Phenylgruppe, einer Pyridinylgruppe, einer Pyrimidinylgruppe und einer Triazinylgruppe;
einer Cyclopentylgruppe, einer Cyclohexylgruppe, einer Cyclopentenylgruppe, einer Cyclohexenylgruppe, einer Cycloheptenylgruppe, einer Piperidinylgruppe, einer Tetrahydro-2H-pyranylgruppe, einer Tetrahydro-2H-thiopyranylgruppe, einer Phenylgruppe, einer Fluorenylgruppe, einer Dibenzosilolylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Furanylgruppe, einer Thiophenylgruppe, einer Thiazolylgruppe, einer Isothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe und einer Dibenzothiophenylgruppe; und
einer Cyclopentylgruppe, einer Cyclohexylgruppe, einer Cyclopentenylgruppe, einer Cyclohexenylgruppe, einer Cycloheptenylgruppe, einer Piperidinylgruppe, einer Tetrahydro-2H-pyranylgruppe, einer Tetrahydro-2H-thiopyranylgruppe, einer Phenylgruppe, einer Fluorenylgruppe, einer Dibenzosilolylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Furanylgruppe, einer Thiophenylgruppe, einer Thiazolylgruppe, einer Isothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe und einer Dibenzothiophenylgruppe jeweils substituiert mit mindestens einem ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Cyclopentylgruppe, einer Cyclohexylgruppe, einer Cyclopentenylgruppe, einer Cyclohexenylgruppe, einer Cycloheptenylgruppe, einer Piperidinylgruppe, einer Tetrahydro-2H-pyranylgruppe, einer Tetrahydro-2H-thiopyranylgruppe, einer Phenylgruppe, einer Fluorenylgruppe, einer Dibenzosilolylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Furanylgruppe, einer Thiophenylgruppe, einer Thiazolylgruppe, einer Isothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe, einer Dibenzothiophenylgruppe, und -N(Q₃₁)(Q₃₂);
wobei Q₁, Q₂, Q₃₁ und Q₃₂ jeweils unabhängig ausgewählt sind aus einem Wasserstoff, einer C₁-C₂₀-Alkylgruppe, einer C₁-C₂₀-Alkoxygruppe, einer Cyclopentylgruppe, einer Cyclohexylgruppe, einer Cyclopentenylgruppe, einer Cyclohexenylgruppe, einer Cycloheptenylgruppe, einer Piperidinylgruppe, einer Tetrahydro-2H-pyranylgruppe, einer Tetrahydro-2H-thiopyranylgruppe, einer Phenylgruppe, einer Fluorenylgruppe, einer Dibenzosilolylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Furanylgruppe, einer Thiophenylgruppe, einer Thiazolylgruppe, einer Isothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe und einer Dibenzothiophenylgruppe; und
einer Cyclopentylgruppe, einer Cyclohexylgruppe, einer Cyclopentenylgruppe, einer Cyclohexenylgruppe, einer Cycloheptenylgruppe, einer Piperidinylgruppe, einer Tetrahydro-2H-pyranylgruppe, einer Tetrahydro-2H-thiopyranylgruppe, einer Phenylgruppe, einer Fluorenylgruppe, einer Dibenzosilolylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Furanylgruppe, einer Thiophenylgruppe, einer Thiazolylgruppe, einer Isothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe und einer Dibenzothiophenylgruppe jeweils substituiert mit mindestens einem ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Cyclopentylgruppe, einer Cyclohexylgruppe, einer Cyclopentenylgruppe, einer Cyclohexenylgruppe, einer Cycloheptenylgruppe, einer Piperidinylgruppe, einer Tetrahydro-2H-pyranylgruppe, einer Tetrahydro-2H-thiopyranylgruppe, einer Phenylgruppe, einer Fluorenylgruppe, einer Dibenzosilolylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Furanylgruppe, einer Thiophenylgruppe, einer Thiazolylgruppe, einer Isothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Carbazolylgruppe, einer Dibenzofuranylgruppe und einer Dibenzothiophenylgruppe.

6. Kondensierte cyclische Verbindung nach Anspruch 5, wobei
R₁ bis R₈ jeweils unabhängig ausgewählt sind aus einem Wasserstoff, einem Deuterium, - F, -Cl, -Br, -I, einer Cyanogruppe und einer substituierten oder unsubstituierten Gruppe, ausgewählt aus: einer Methylgruppe, einer Ethylgruppe, einer Propylgruppe, einer Isobutylgruppe, einer sec-Butylgruppe, einer tert-Butylgruppe, einer Pentylgruppe, einer iso-Amylgruppe, einer Hexylgruppe und einer Phenylgruppe;
R₉ ausgewählt ist aus einem Wasserstoff, einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Phenylgruppe, eine Pyridinylgruppe und einer Pyrimidinylgruppe.

7. Kondensierte zyklische Verbindung nach einem der Ansprüche 1-6, wobei
R₁₁ bis R₁₃ jeweils unabhängig ein Wasserstoff, ein Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, C₁-C₁₀-Alkylgruppe, eine C₁-C₁₀-Alkoxygruppe, -N(Q₁)(Q₂) sind, wobei Q₁ und Q₂ jeweils unabhängig ausgewählt sind aus einer C₁-C₅-Alkylgruppe und einer Gruppe, dargestellt durch die Formeln 4-1 bis 4-49 und 4-61 bis 4-74: 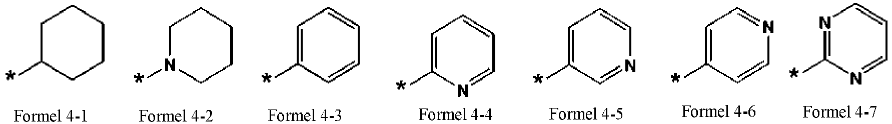 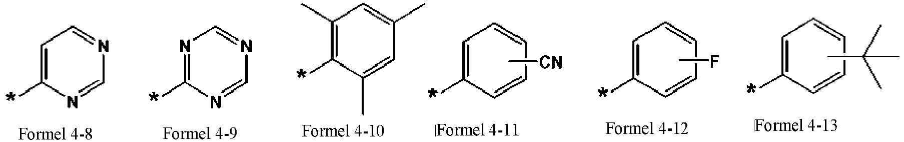 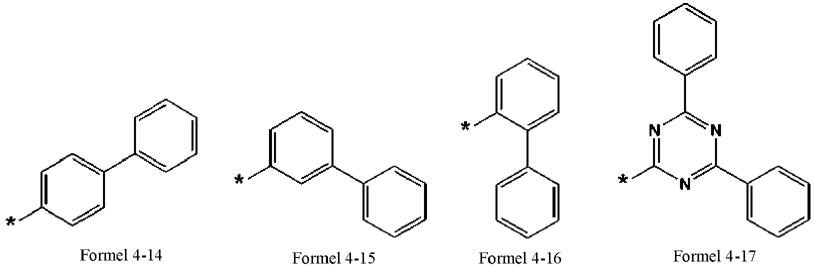 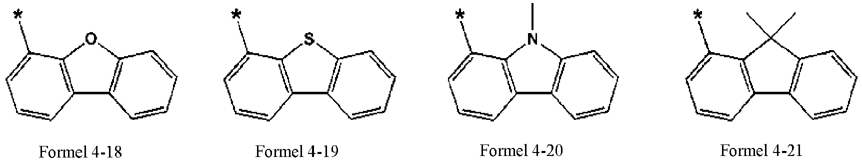 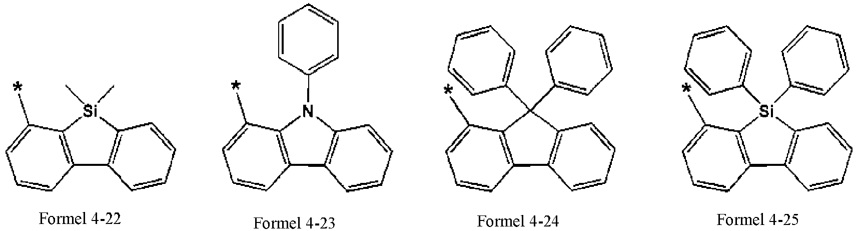 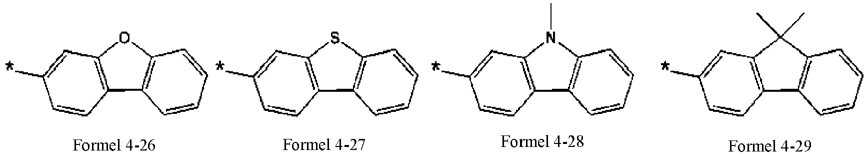 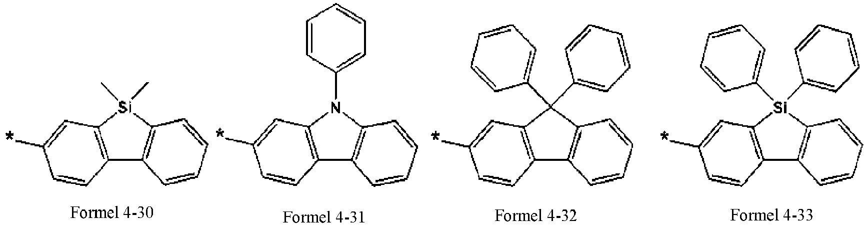 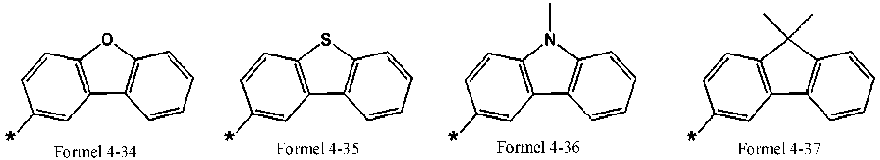 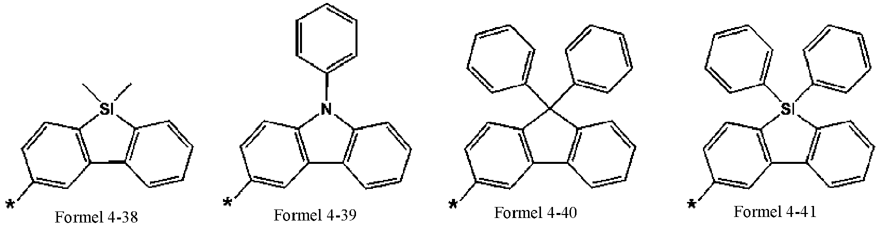 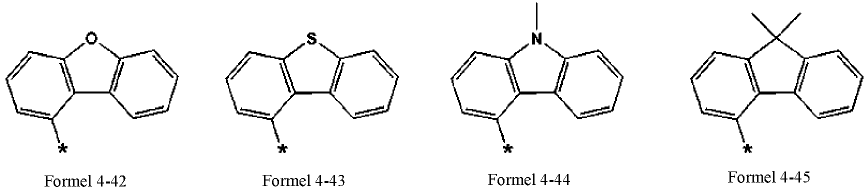 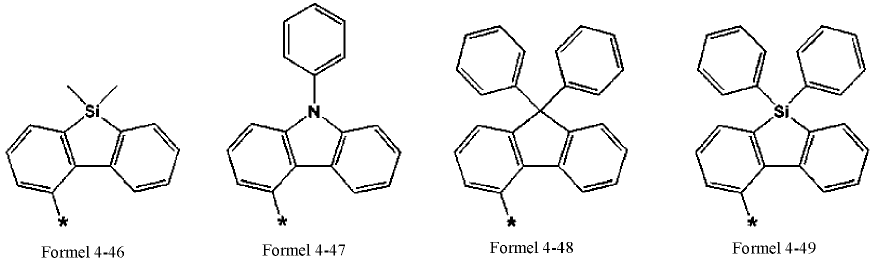 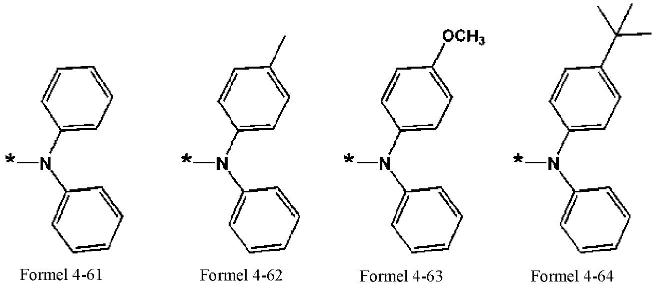 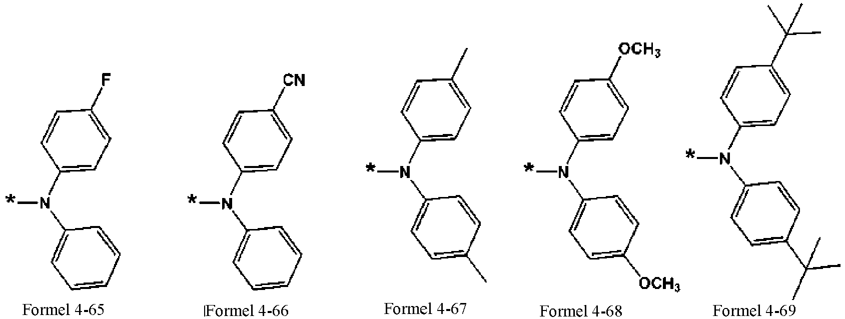 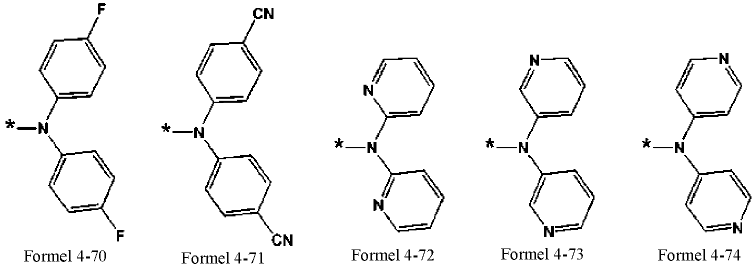 wobei in den Formeln 4-1 bis 4-49 und 4-61 bis 4-74 * eine Bindungsstelle mit jedem von Ar₁ bis Ar₃ in den Formeln 1A bis 1D angibt.

8. Kondensierte zyklische Verbindung nach einem der Ansprüche 1-7, wobei
*-Ar₁-(R₁₁)a₁ in den Formeln 1A bis 1D eine Gruppe ist, die durch eine aus den Formeln 11-1 bis 11-47 ausgewählte Gruppe dargestellt ist,
*-Ar₂-(R₁₂)a₂ in den Formeln 1A bis 1D eine Gruppe ist, die durch eine aus den Formeln 12-1 bis 12-47 ausgewählte Gruppe dargestellt ist, und
*-Ar₃-(R₁₃)a₃ in den Formeln 1A bis 1D eine Gruppe ist, die durch eine aus den Formeln 13-1 bis 13-47 ausgewählte Gruppe dargestellt ist: 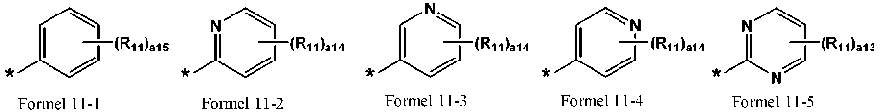 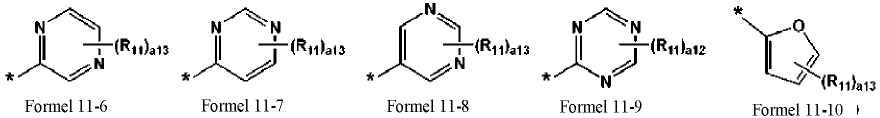 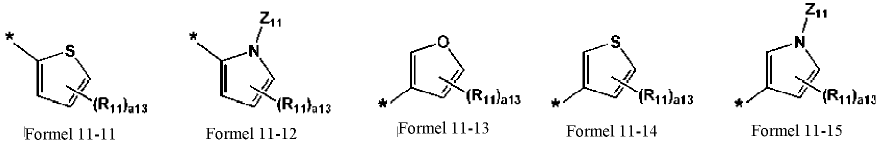 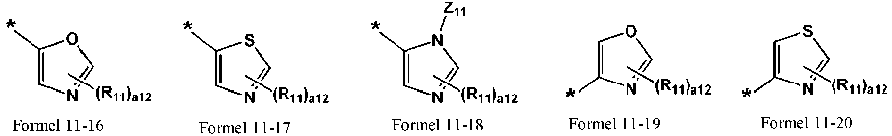 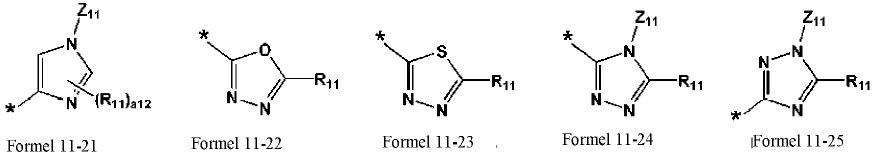 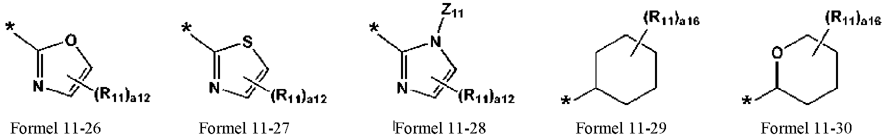 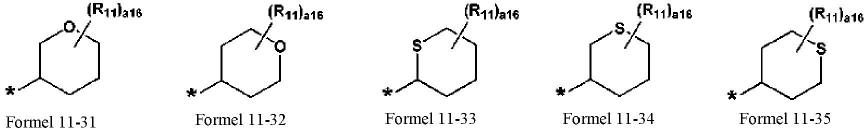 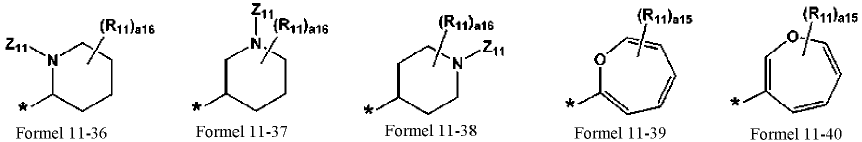 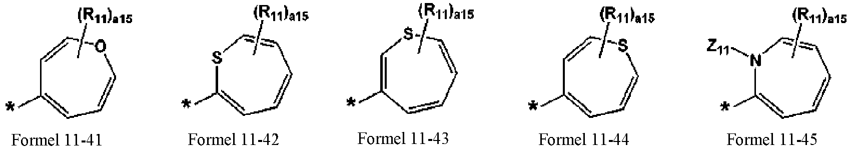 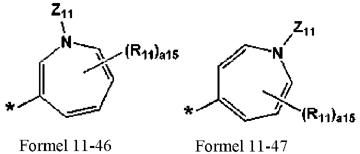 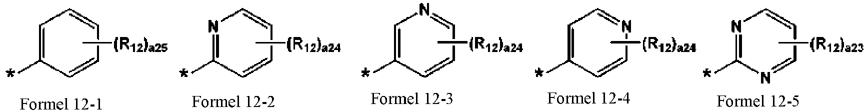 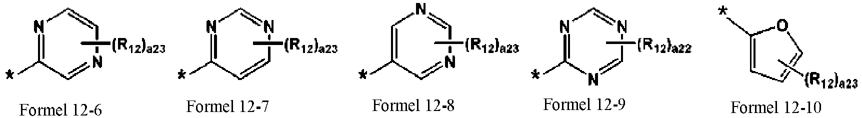 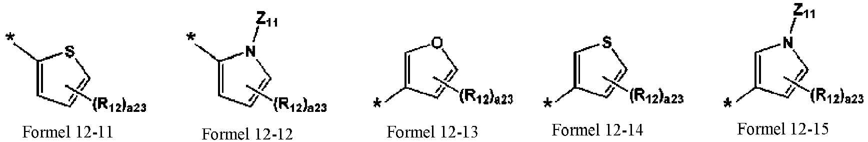 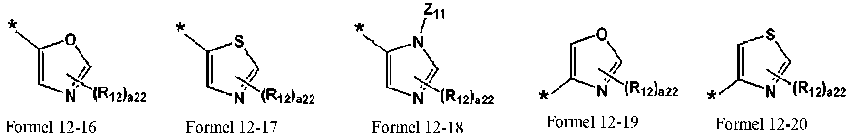 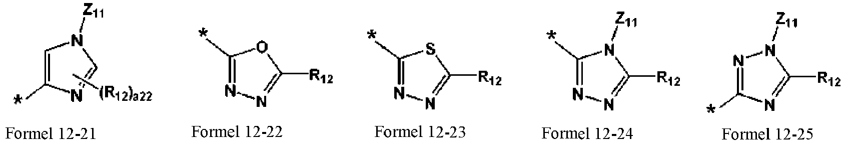 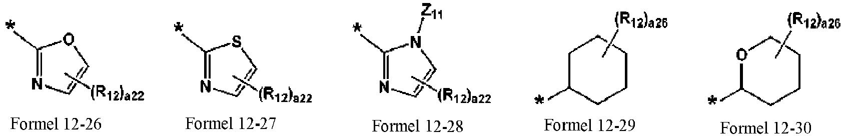 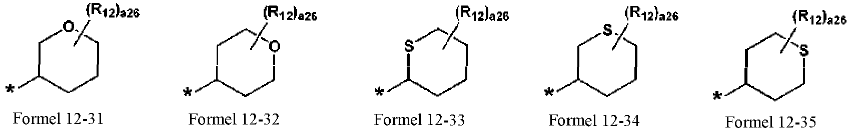 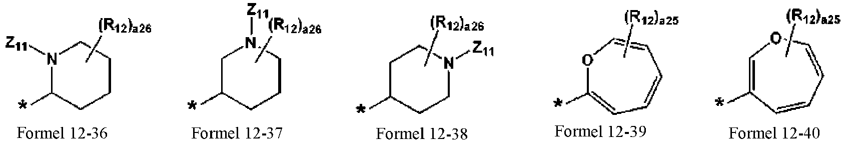 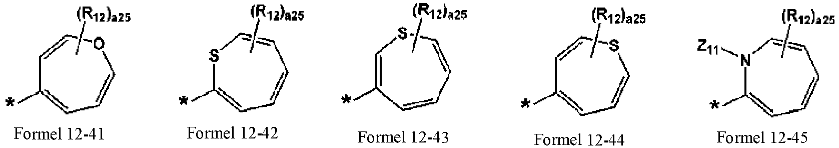 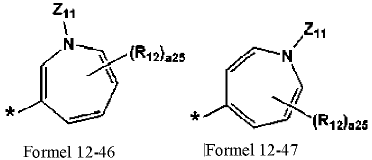 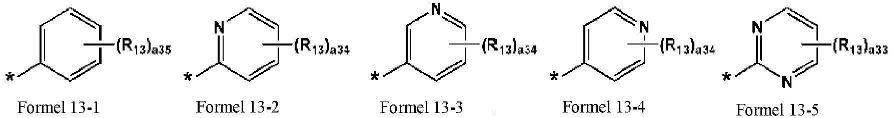 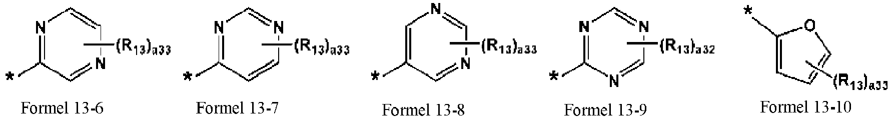 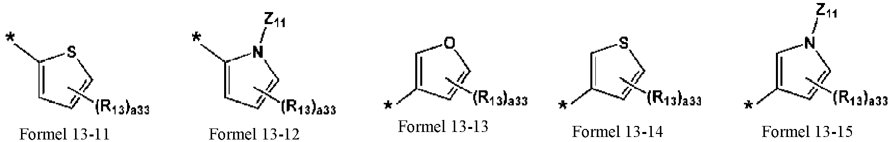 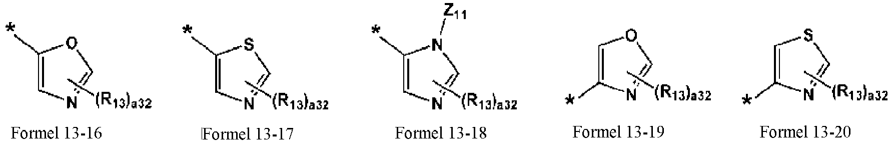 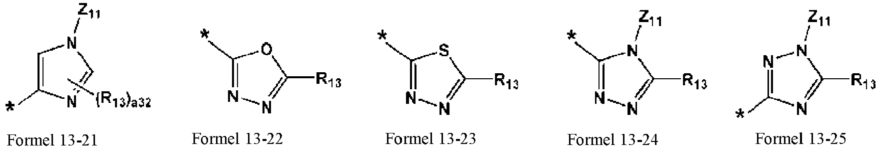 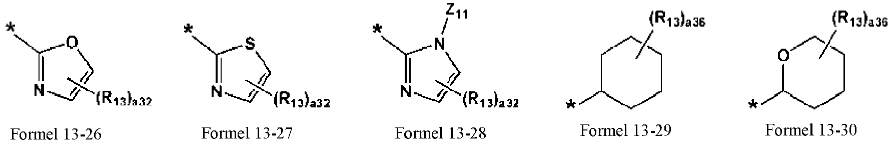 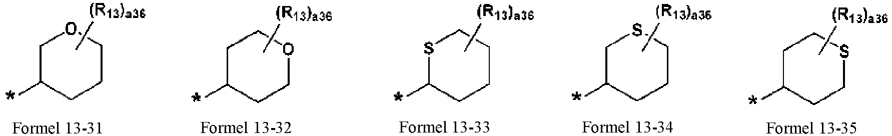 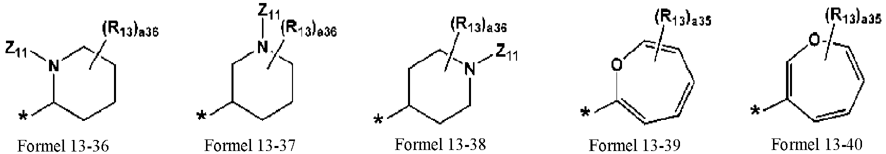 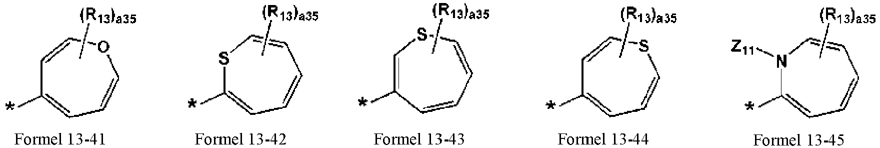 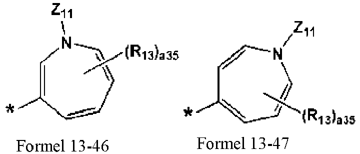 wobei in den Formeln 11-1 bis 11-47, 12-1 bis 12-47 und 13-1 bis 13-47,
R₁₁ bis R₁₃ gleich sind wie in Anspruch 1,
a16, a26 und a36 jeweils unabhängig eine Ganzzahl sind, ausgewählt aus 1, 2, 3, 4, 5 und 6,
a15, a25 und a35 jeweils unabhängig eine Ganzzahl sind, ausgewählt aus 1, 2, 3, 4 und 5,
a14, a24 und a34 jeweils unabhängig eine Ganzzahl sind, ausgewählt aus 1, 2, 3 und 4,
a13, a23 und a33 jeweils unabhängig eine Ganzzahl sind, ausgewählt aus 1, 2 und 3,
a12, a22 und a32 jeweils unabhängig eine Ganzzahl sind, ausgewählt aus 1 und 2, und
* eine Bindungsstelle für Si in den Formeln 1A bis 1D ist.

9. Kondensierte cyclische Verbindung nach Anspruch 8, wobei
Ar₁ in den Formeln 1A bis 1D eine Gruppe ist, die durch eine aus den obigen Formeln 11-1 bis 11-9 ausgewählte Gruppe dargestellt ist, und
Ar₃ eine Gruppe ist, die durch eine aus den obigen Formeln 13-1 bis 13-9 ausgewählte Gruppe dargestellt ist.

10. Kondensierte zyklische Verbindung nach einem der Ansprüche 1-9, wobei
in den Formeln 1A bis 1D, Ar₁ = Ar₂ = Ar₃, R₁₁ ≠ R₁₂ und R₁₂ kein Wasserstoff ist.

11. Kondensierte cyclische Verbindung nach einem der Ansprüche 1-10, wobei
die kondensierte zyklische Verbindung durch eine aus den Formeln 1A(1) bis 1 D(8) ausgewählte Verbindung dargestellt ist: 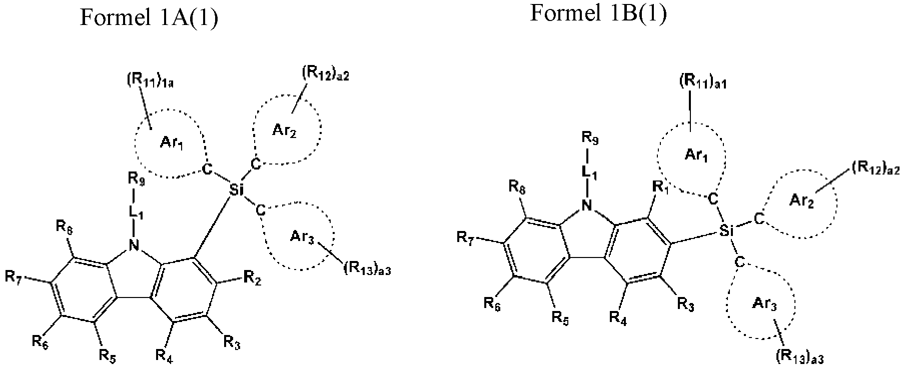 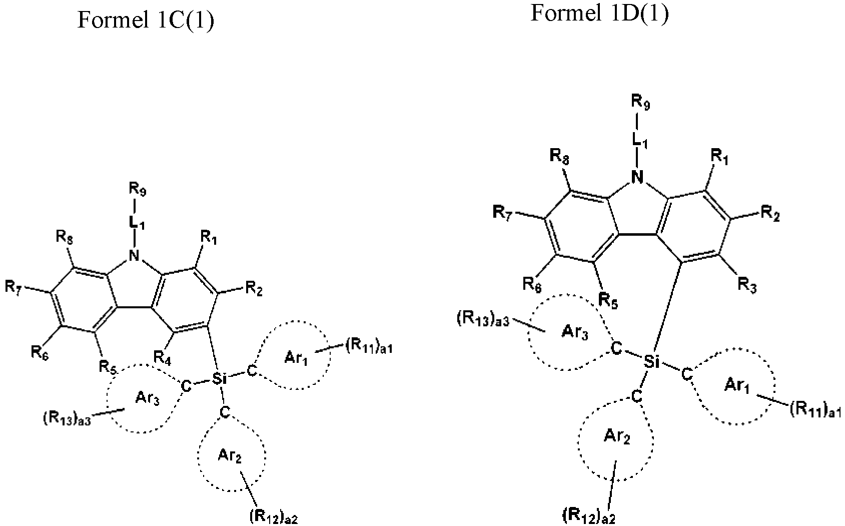 wobei L₁, Ar₁ bis Ar₃, R₁ bis R₉, R₁₁ bis R₁₃, und a1 bis a3 in den Formeln 1A(1) bis 1D(1) gleich sind wie in Anspruch 1.

12. Kondensierte zyklische Verbindung nach Anspruch 1, wobei die kondensierte zyklische Verbindung eine der Verbindungen 1 bis 100, 114 bis 154, 156, 158, 159, 162 bis 177, 184 bis 195, 200 bis 213, 220 bis 231, 233, 233 bis 235, 237 bis 250, 257 bis 268, 270, 271, 273 bis 285, 289 bis 305, 309 bis 323, 325, 327, 328, 330 und 332 ist:

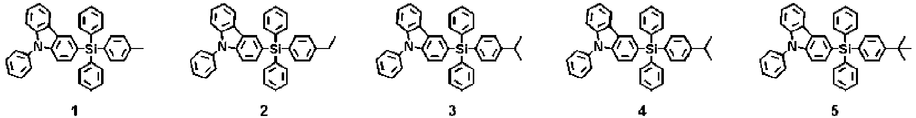

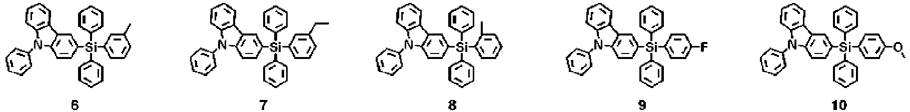

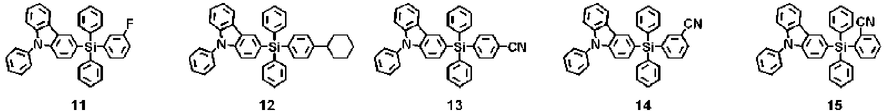

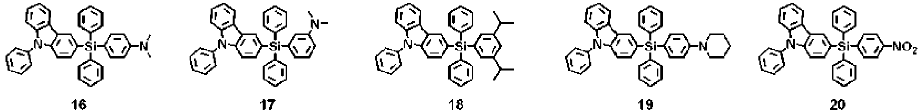

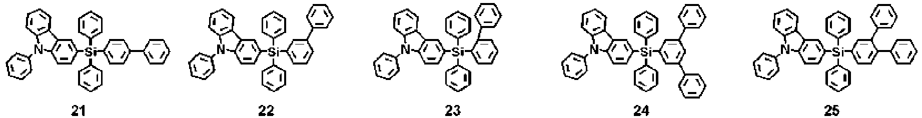

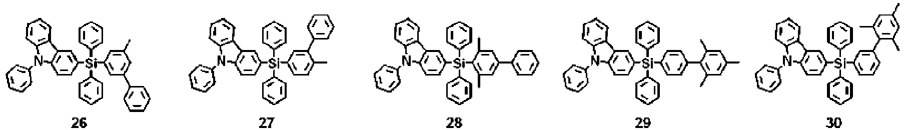

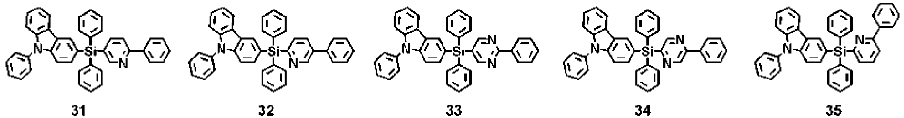

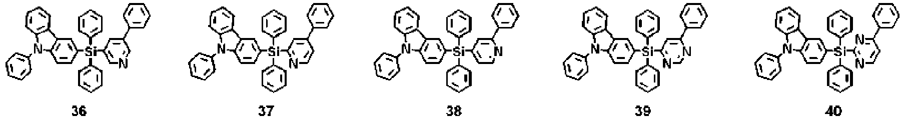

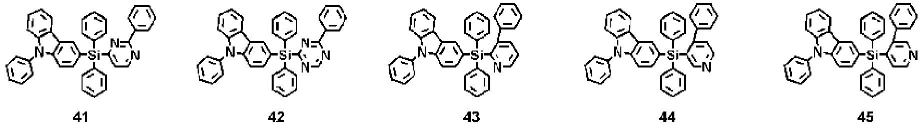

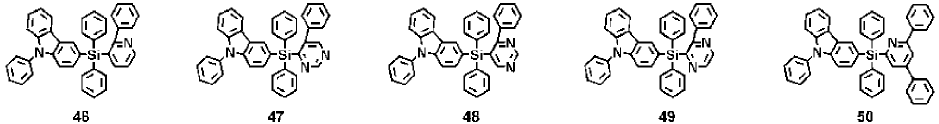

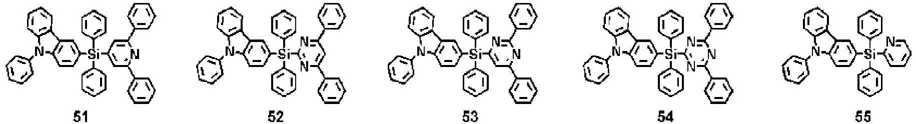

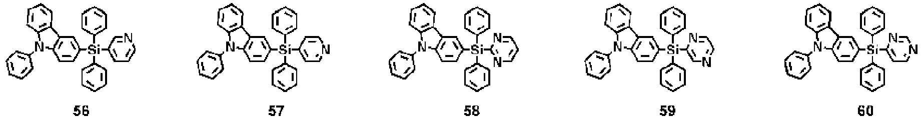

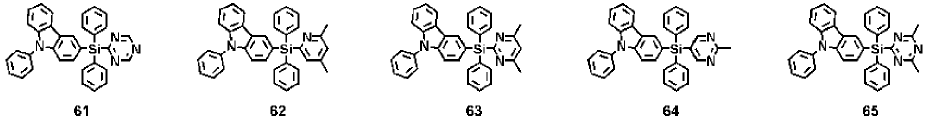

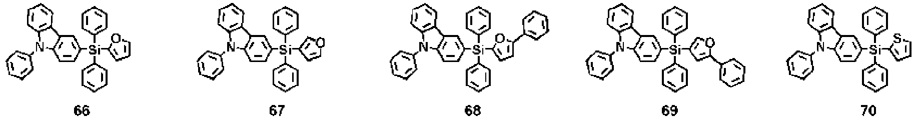

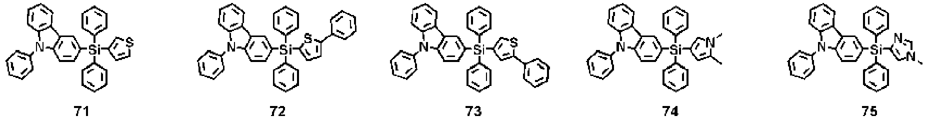

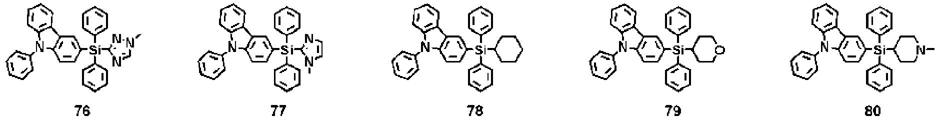

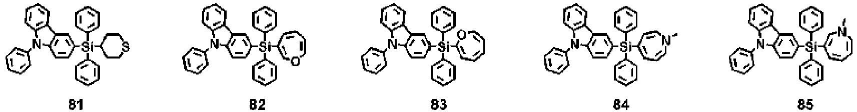

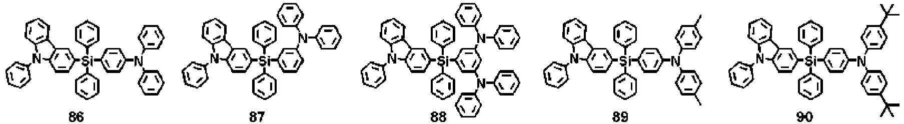

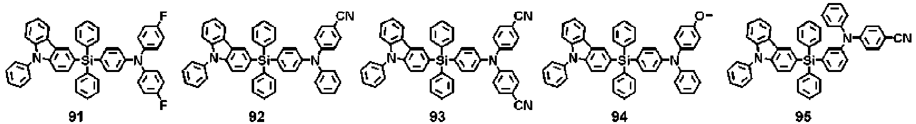

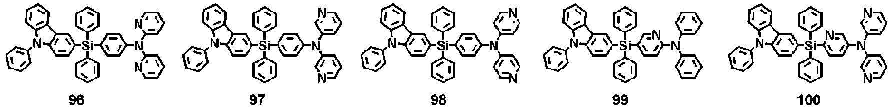

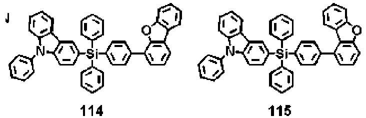

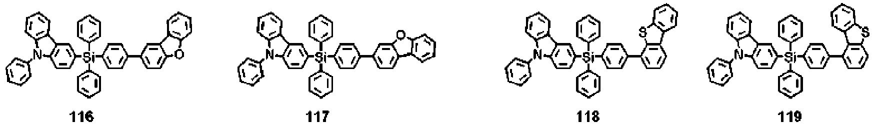

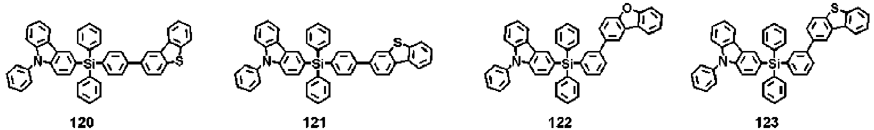

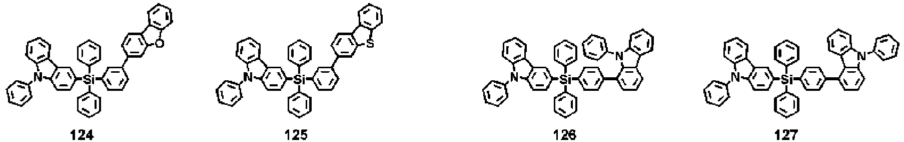

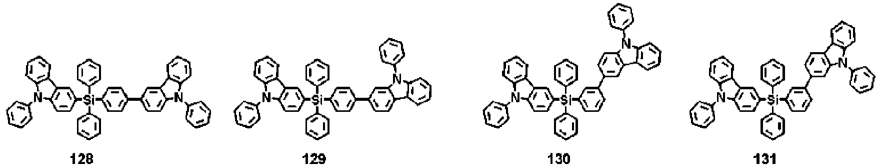

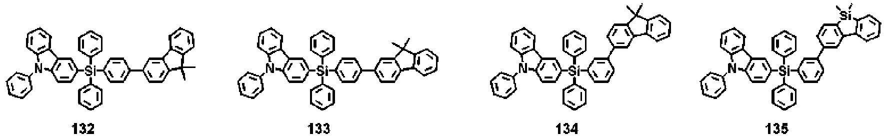

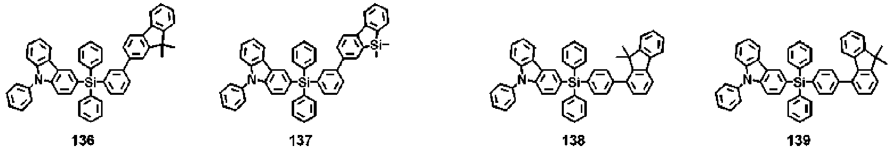

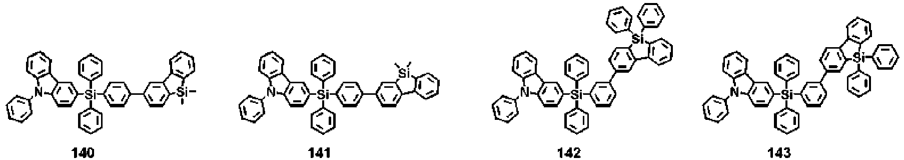

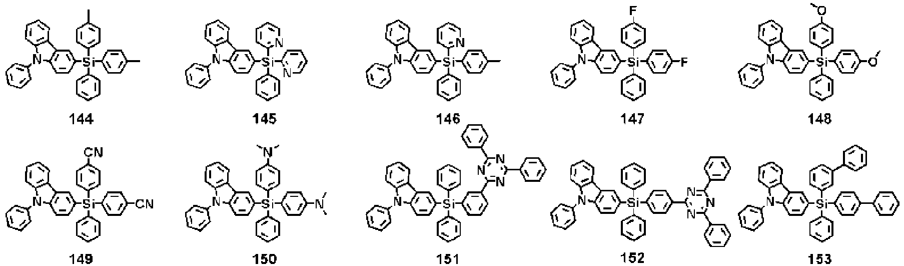

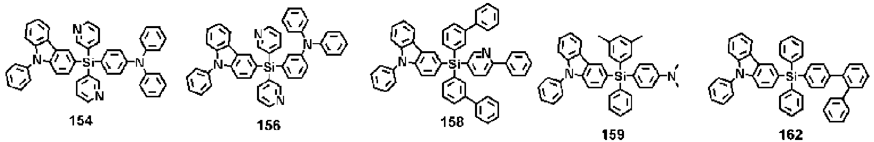

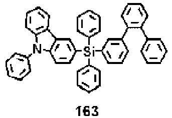

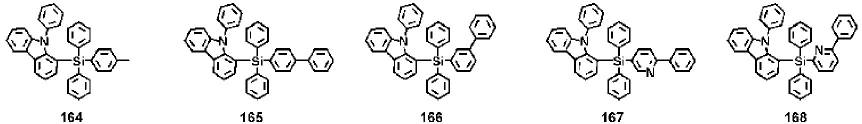

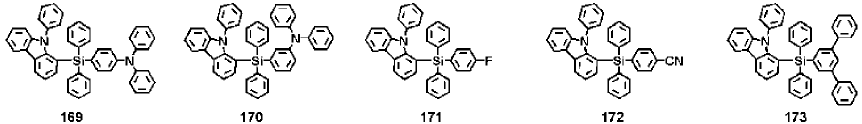

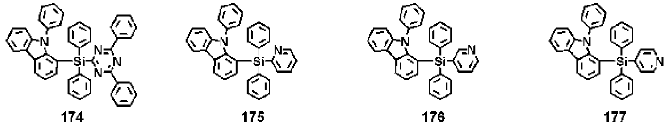

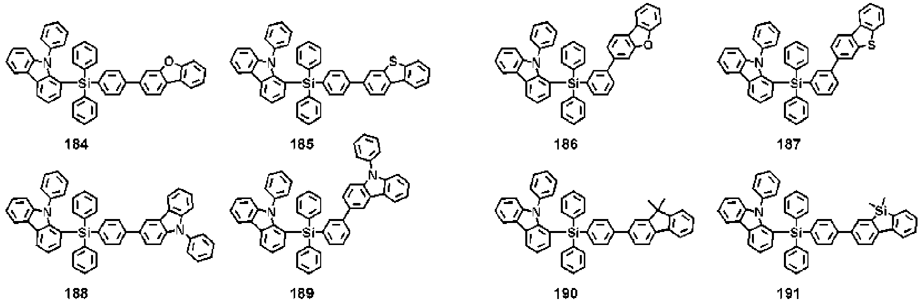

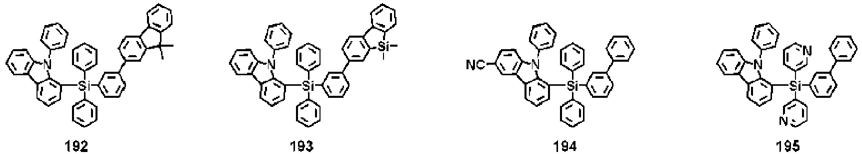

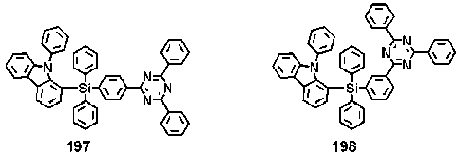

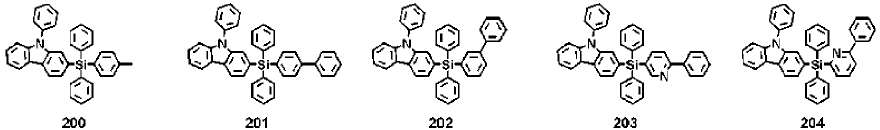

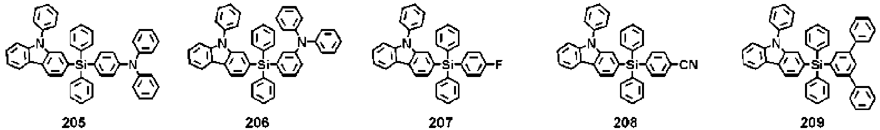

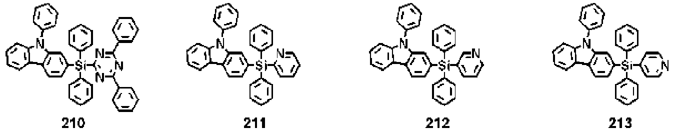

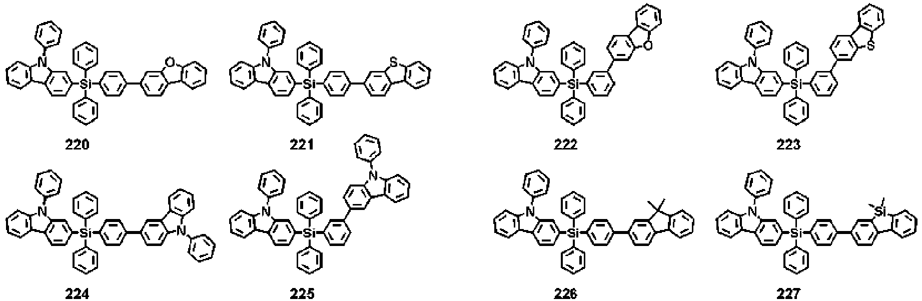

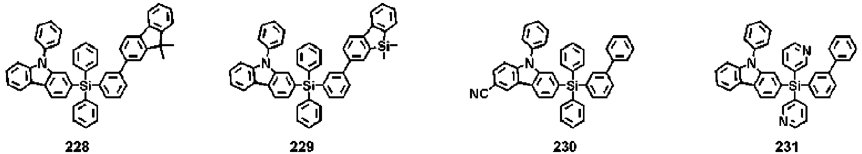

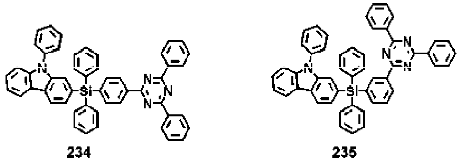

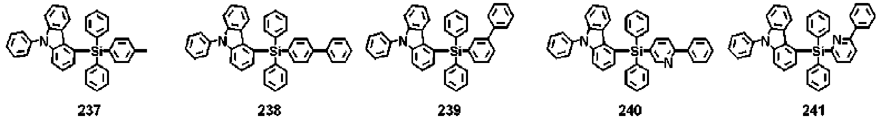

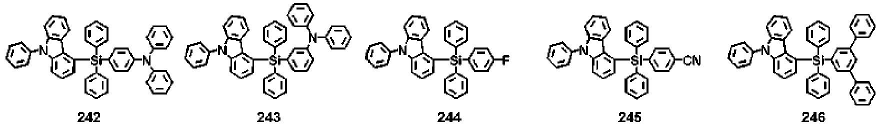

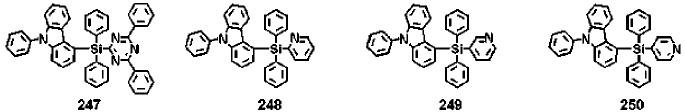

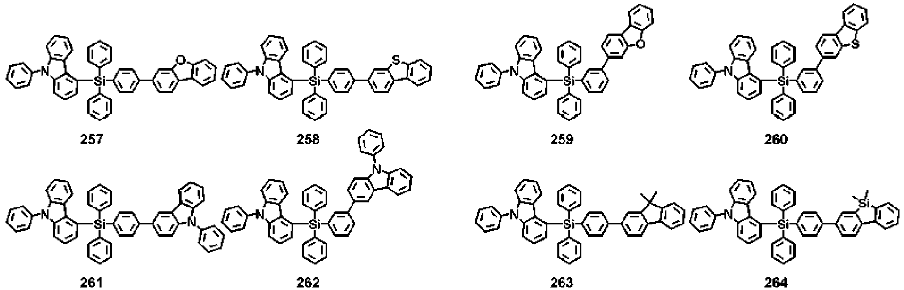

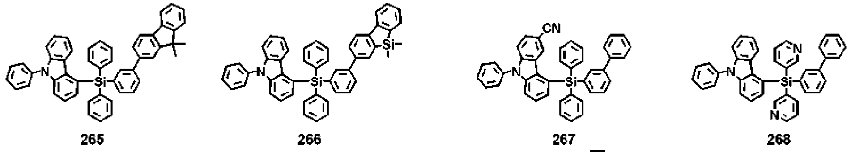

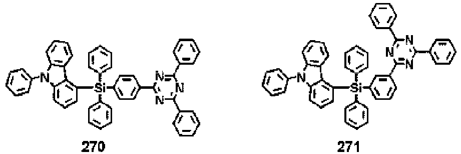

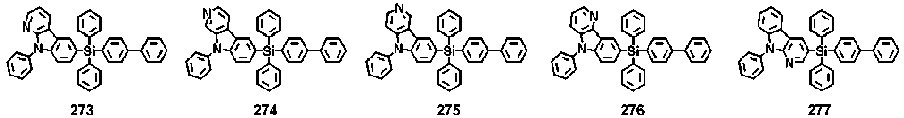

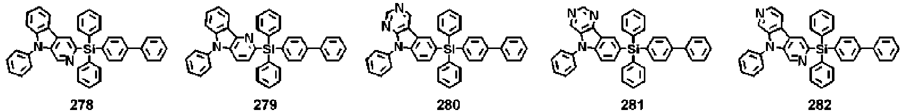

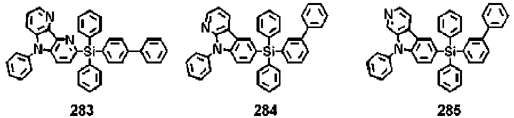

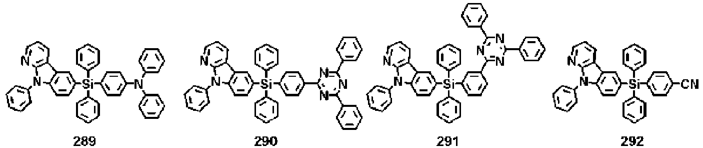

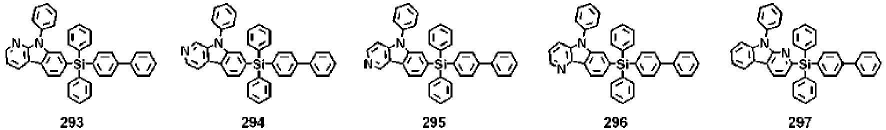

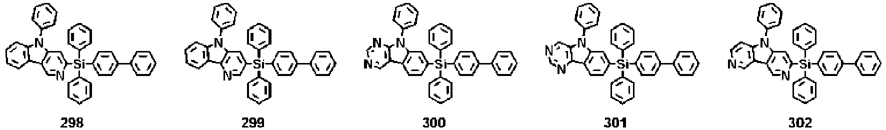

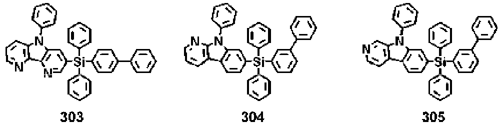

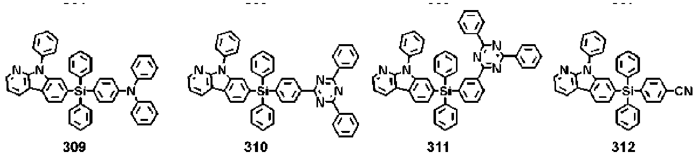

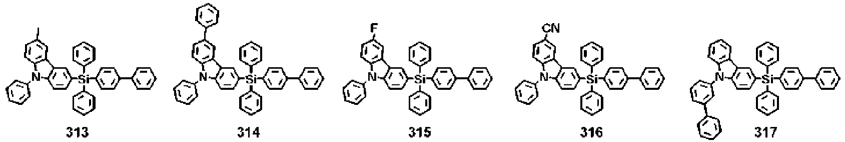

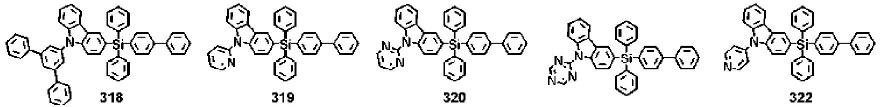

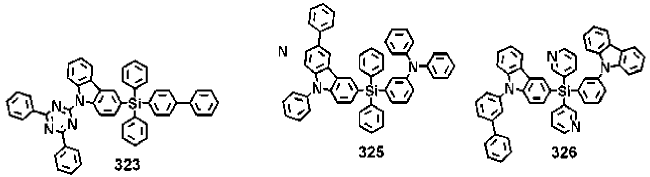

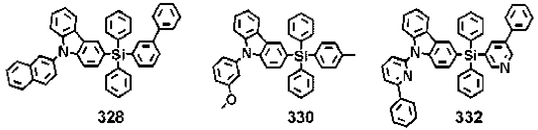

13. Organische lichtemittierende Vorrichtung, umfassend:
eine erste Elektrode;
eine zweite Elektrode; und
eine organische Schicht, die zwischen der ersten Elektrode und der zweiten Elektrode angeordnet ist,
wobei die organische Schicht eine Emissionsschicht umfasst und ferner mindestens eine kondensierte zyklische Verbindung umfasst, die durch eine der Formeln 1A bis 1D nach einem der Ansprüche 1-12 dargestellt ist.

14. Organische lichtemittierende Vorrichtung nach Anspruch 13, wobei
die Emissionsschicht die mindestens eine kondensierte zyklische Verbindung umfasst, wobei
die Emissionsschicht optional ferner einen phosphoreszierenden Dotierstoff umfasst und die mindestens eine kondensierte zyklische Verbindung in der Emissionsschicht als Wirt wirkt.

## Revendications

1. Composé cyclique condensé représenté par une des Formules 1A à 1D : 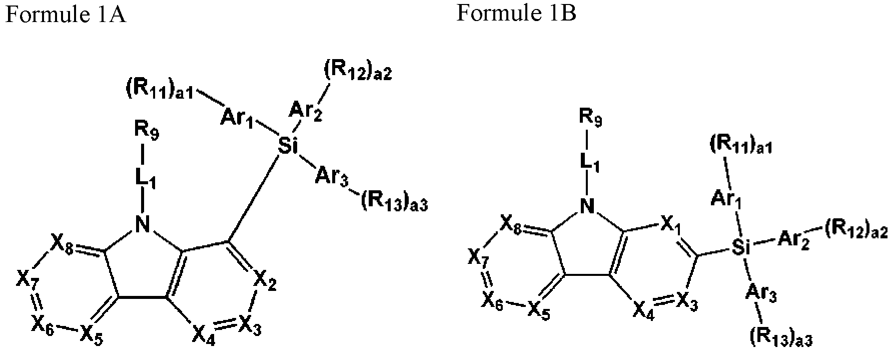 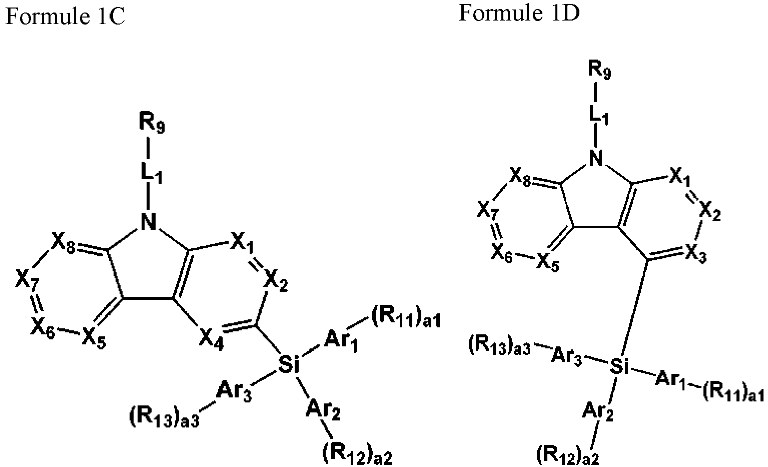 dans lequel dans les Formules 1A à 1D,
X₁ est N ou C(R₁),
X₂ est N ou C(R₂),
X₃ est N ou C(R₃),
X₄ est N ou C(R₄),
X₅ est N ou C(R₅),
X₆ est N ou C(R₆),
X₇ est N ou C(R₇), et
X₈ est N ou C(R₈) ;
L₁ est chacun indépendamment choisi parmi groupe cycloalkylène C₃-C₁₀ substitué ou non substitué, groupe hétérocycloalkylène C₁-C₁₀ substitué ou non substitué, groupe cycloalkénylène C₃-C₁₀ substitué ou non substitué, groupe hétérocycloalkénylène C₁-C₁₀ substitué ou non substitué, groupe arylène C₆-C₆₀ substitué ou non substitué, groupe hétéroarylène C₁-C₆₀ substitué ou non substitué, groupe polycyclique condensé non aromatique divalent substitué ou non substitué, et groupe hétéropolycyclique condensé non aromatique divalent substitué ou non substitué ;
Ar₁ to Ar₃ sont chacun indépendamment un groupe dérivé d'un groupe carbocyclique non condensé C₅-C₉ ou un groupe hétérocyclique non condensé C₁-C₇,
R₁ à R₈ sont chacun indépendamment choisis parmi hydrogène, deutérium, -F, -Cl, -Br, - I, groupe cyano, et groupe substitué ou non substitué choisi parmi : groupe méthyle, groupe éthyle, groupe propyle, groupe isobutyle, groupe sec-butyle, groupe tert-butyle, groupe pentyle, groupe iso-amyle, groupe hexyle, groupe phényle, groupe naphthyle, groupe anthracényle, groupe phénanthrényle, groupe pyrényle, et groupe chrysényle ;
R₉ est choisi parmi hydrogène, deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₆₀ substitué ou non substitué, groupe alkényle C₂-C₆₀ substitué ou non substitué, groupe alkynyle C₂-C₆₀ substitué ou non substitué, groupe alkoxy C₁-C₆₀ substitué ou non substitué, groupe cycloalkyle C₃-C₁₀ substitué ou non substitué, groupe hétérocycloalkyle C₁-C₁₀ substitué ou non substitué, groupe cycloalkényle C₃-C₁₀ substitué ou non substitué, groupe hétérocycloalkényle C₁-C₁₀ substitué ou non substitué, groupe aryle C₆-C₆₀ substitué ou non substitué, groupe aryloxy C₆-C₆₀ substitué ou non substitué, groupe arylthio C₆-C₆₀ substitué ou non substitué, groupe hétéroaryle C₁-C₆₀ substitué ou non substitué, groupe polycyclique condensé non aromatique monovalent substitué ou non substitué et groupe hétéropolycyclique condensé non aromatique monovalent substitué ou non substitué ;
R₁₁ à R₁₃ sont chacun indépendamment choisis parmi hydrogène, deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₁₀, groupe alkoxy C₁-C₁₀, -N(Q₁)(Q₂), où Q₁ et Q₂ sont indépendamment choisis parmi un groupe alkyle C₁-C₁₀, et un groupe représenté par les Formules 3-1 à 3-29 et 3-31 à 3-37 : 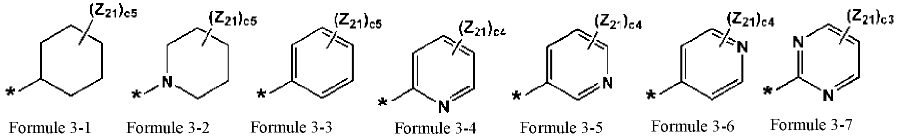 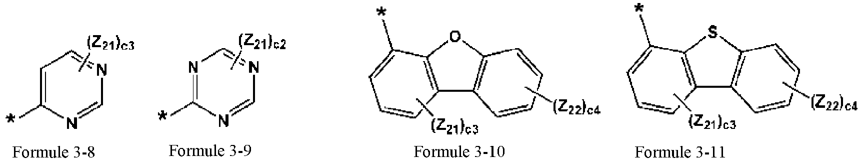 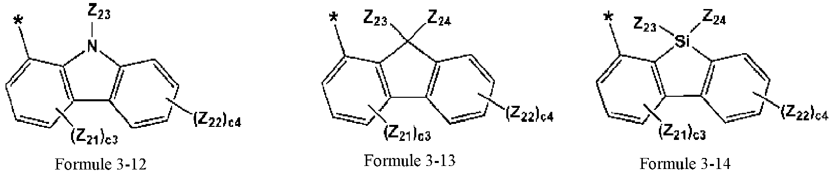 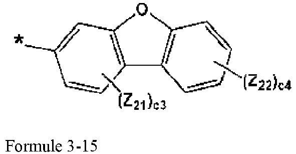 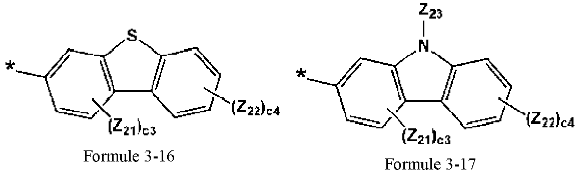 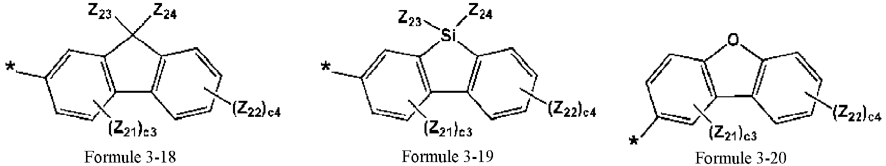 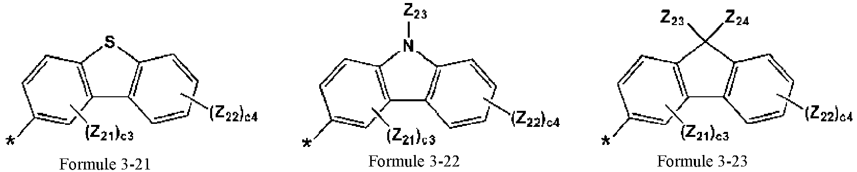 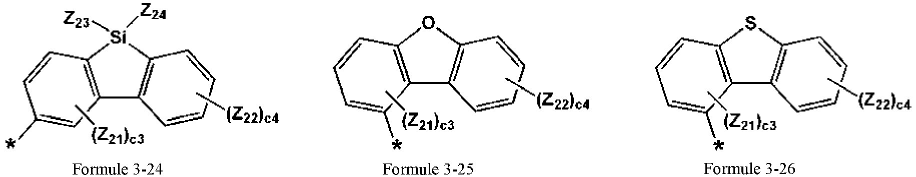 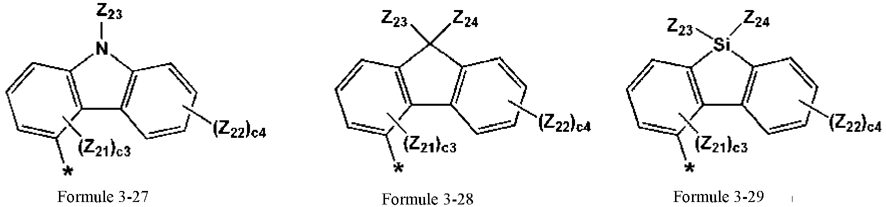 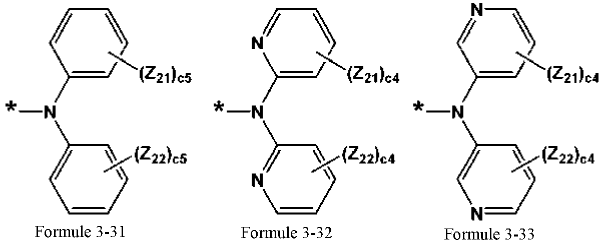 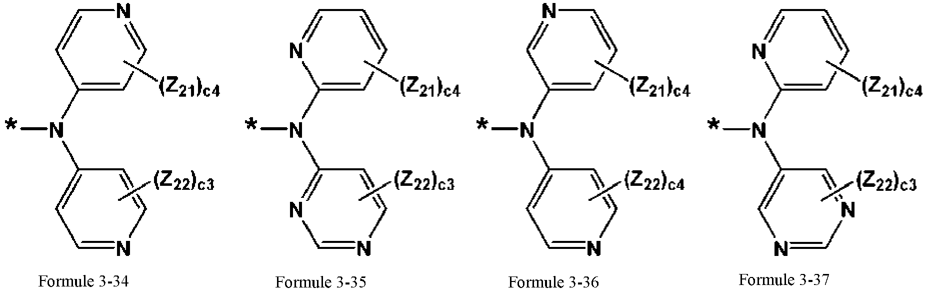 où dans les Formules 3-1 à 3-29 et 3-31 à 3-37,
Z₂₁ à Z₂₄ sont chacun indépendamment choisis parmi hydrogène, deutérium, -F, -Cl, - Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₁₀, groupe alkoxy C₁-C₁₀, groupe phényle, groupe pyridinyle, groupe pyrimidinyle, et groupe triazinyle,
c₅ est un nombre entier choisi parmi 1, 2, 3, 4, et 5,
c₄ est un nombre entier choisi parmi 1, 2, 3, et 4,
c₃ est un nombre entier choisi parmi 1, 2, et 3,
c₂ est un nombre entier choisi parmi 1 à 2, et
* indique un site de liaison à chacun de Ar₁ à Ar₃ dans les Formules 1A à 1D,
a1 à a3 sont chacun indépendamment un nombre entier choisi parmi 1, 2, 3, 4, 5, et 6, à condition que lorsque a1 est 2 ou plus, deux groupes R₁₁ ou plus sont identiques entre eux ou différents les uns des autres, lorsque a2 est 2 ou plus, deux groupes R₁₂ ou plus sont identiques entre eux ou différents les uns des autres, et lorsque a3 est 2 ou plus, deux groupes R₁₃ ou plus sont identiques entre eux ou différents les uns des autres ;
où dans chacune des Formules 1A à 1D, les groupes *-Ar₁-(R₁₁)ₐ₁, *-Ar₂-(R₁₂)ₐ₂ et *-Ar₃-(R₁₃)a₃ ne sont pas identiques entre eux ;
au moins un des substituants du groupe cycloalkylène C₃-C₁₀ substitué, groupe hétérocycloalkylène C₁-C₁₀ substitué, groupe cycloalkénylène C₃-C₁₀ substitué, groupe hétérocycloalkénylène C₁-C₁₀ substitué, groupe arylène C₆-C₆₀ substitué, groupe hétéroarylène C₁-C₆₀ substitué, groupe polycyclique condensé non aromatique divalent substitué, groupe hétéropolycyclique condensé non aromatique divalent substitué, groupe alkyle C₁-C₆₀ substitué, groupe alkényle C₂-C₆₀ substitué, groupe alkynyle C₂-C₆₀ substitué, groupe alkoxy C₁-C₆₀ substitué, groupe cycloalkyle C₃-C₁₀ substitué, groupe hétérocycloalkyle C₁-C₁₀ substitué, groupe cycloalkényle C₃-C₁₀ substitué, groupe hétérocycloalkényle C₁-C₁₀ substitué, groupe aryle C₆-C₆₀ substitué, groupe aryloxy C₆-C₆₀ substitué, groupe arylthio C₆-C₆₀ substitué, groupe hétéroaryle C₁-C₆₀ substitué, groupe polycyclique condensé non aromatique monovalent substitué, et groupe hétéropolycyclique condensé non aromatique monovalent substitué est choisi parmi
deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₆₀, groupe alkényle C₂-C₆₀, groupe alkynyle C₂-C₆₀, et groupe alkoxy C₁-C₆₀ ;
groupe alkyle C₁-C₆₀, groupe alkényle C₂-C₆₀, groupe alkynyle C₂-C₆₀, et groupe alkoxy C₁-C₆₀, chacun étant substitué par au moins un élément choisi parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe aryloxy C₆-C₆₀, groupe arylthio C₆-C₆₀, groupe hétéroaryle C₁-C₆₀, groupe polycyclique condensé non aromatique monovalent, groupe hétéropolycyclique condensé non aromatique monovalent, et -N(Q₁₁)(Q₁₂) ;
groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe aryloxy C₆-C₆₀, groupe arylthio C₆-C₆₀, groupe hétéroaryle C₁-C₆₀, groupe polycyclique condensé non aromatique monovalent, et groupe hétéropolycyclique condensé non aromatique monovalent ;
groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe aryloxy C₆-C₆₀, groupe arylthio C₆-C₆₀, groupe hétéroaryle C₁-C₆₀, groupe polycyclique condensé non aromatique monovalent, et groupe hétéropolycyclique condensé non aromatique monovalent, chacun étant substitué par au moins un élément choisi parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₆₀, groupe alkényle C₂-C₆₀, groupe alkynyle C₂-C₆₀, groupe alkoxy C₂-C₆₀, groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe aryloxy C₆-C₆₀, groupe arylthio C₆-C₆₀, groupe hétéoaryle C₁-C₆₀, groupe polycyclique condensé non aromatique monovalent, et groupe hétéropolycyclique condensé non aromatique monovalent, et -N(Q₂₁)(Q₂₂) ; et
-N(Q₃₁)(Q₃₂) ;
où Q₁₁, Q₁₂, Q₂₁, Q₂₂, Q₃₁ et Q₃₂ sont chacun indépendamment choisis parmi
hydrogène, groupe alkyle C₁-C₆₀, groupe alkényle C₂-C₆₀, groupe alkynyle C₂-C₆₀, groupe alkoxy C₁-C₆₀, groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe hétéroaryle C₁-C₆₀, groupe polycyclique condensé non aromatique monovalent, et groupe hétéropolycyclique condensé non aromatique monovalent ; et
groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe hétéroaryle C₁-C₆₀, groupe polycyclique condensé non aromatique monovalent, et groupe hétéropolycyclique condensé non aromatique monovalent, chacun étant substitué par au moins un des éléments choisis parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₆₀, groupe alkoxy C₁-C₆₀, groupe cycloalkyle C₃-C₁₀, groupe hétérocycloalkyle C₁-C₁₀, groupe cycloalkényle C₃-C₁₀, groupe hétérocycloalkényle C₁-C₁₀, groupe aryle C₆-C₆₀, groupe hétéroaryle C₁-C₆₀, groupe polycyclique condensé non aromatique monovalent, groupe hétéropolycyclique condensé non aromatique monovalent.

2. Composé cyclique condensé selon la revendication 1, dans lequel
X₁ est C(R₁),
X₂ est C(R₂),
X₃ est C(R₃),
X₄ est C(R₄),
X₅ est C(R₅),
X₆ est C(R₆),
X₇ est C(R₇), et
X₈ est N ou C(R₈).

3. Composé cyclique condensé selon la revendication 1 ou 2, dans lequel
L₁ est choisi parmi
groupe phénylène, groupe naphthylène, groupe fluorénylène, groupe phénanthrénylène, groupe anthracénylène, groupe triphénylénylène, groupe pyrénylène, groupe chrysénylène, groupe pyridinylène, groupe pyrazinylène, groupe pyrimidinylène, groupe pyridazinylène, et groupe triazinylène ; et
groupe phénylène, groupe naphthylène, groupe fluorénylène, groupe phénanthrénylène, groupe anthracénylène, groupe triphénylénylène, groupe pyrénylène, groupe chrysénylène, groupe pyridinylène, groupe pyrazinylène, groupe pyrimidinylène, groupe pyridazinylène, et groupe triazinylène, chacun étant substitué par au moins un des éléments choisis parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₂₀, groupe alkoxy C₁-C₂₀, groupe phényle, groupe naphthyle, groupe fluorényle, groupe phénanthrényle, groupe anthracényle, groupe triphénylényle, groupe pyrényle, groupe chrysényle, groupe pyridinyle, groupe pyrazinyle, groupe pyrimidinyle, groupe pyridazinyle, et groupe triazinyle, et dans lequel
L₁ est choisi de préférence parmi
groupe phénylène, groupe naphthylène, groupe pyridinylène, groupe pyrimidinylène, et groupe triazinylène ; et
groupe phenylène, groupe naphthylène, groupe pyridinylène, groupe pyrimidinylène, et groupe triazinylène, chacun étant substitué par au moins un élément choisi parmi deutérium, -F, - Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₁₀, groupe alkoxy C₁-C₁₀, groupe phényle, et groupe naphthyle.

4. Composé cyclique condensé selon les revendications 1 à 3, dans lequel
Ar₁ à Ar₃ sont chacun indépendamment choisis parmi les groupes représentés par les Formules 2-1 à 2-40 : 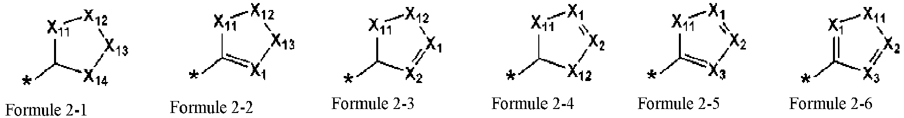 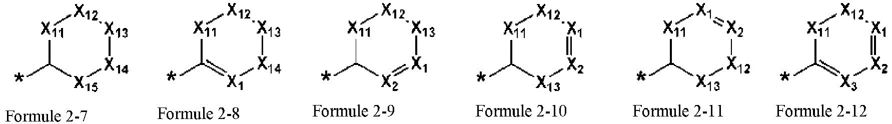 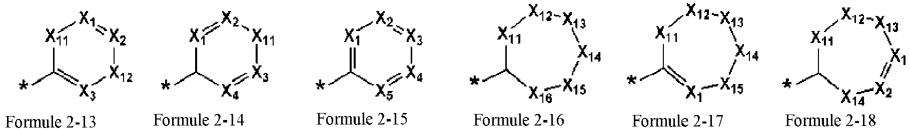 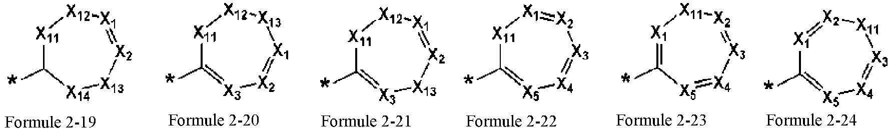 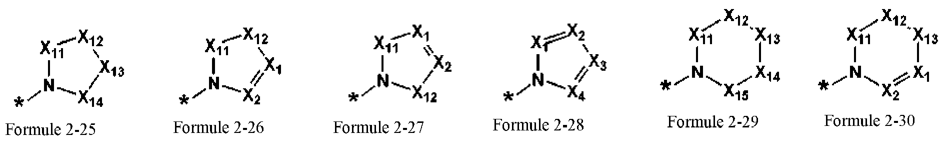 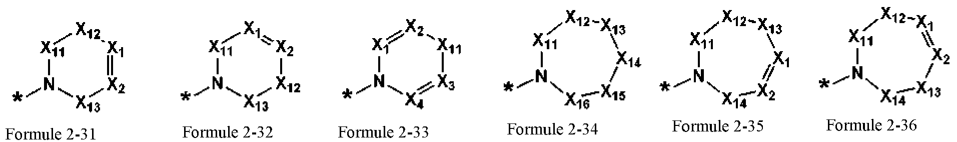 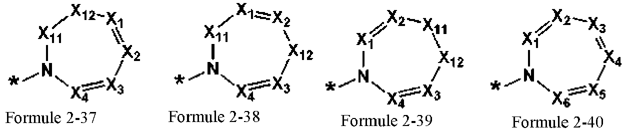 où dans les Formules 2-1 à 2-40,
X₁₁ est O, S, N(Z₁₁) ou C(Z₁₁ₐ)(Z_{11b}),
X₁₂ est O, S, N(Z₁₂) ou C(Z₁₂ₐ)(Z_{12b}),
X₁₃ est O, S, N(Z₁₃) ou C(Z₁₃ₐ)(Z_{13b}),
X₁₄ est O, S, N(Z₁₄) ou C(Z₁₄ₐ)(Z_{14b}),
X₁₅ est O, S, N(Z₁₅) ou C(Z₁₅ₐ)(Z_{15b}),
X₁₆ est O, S, N(Z₁₆) ou C(Z₁₆ₐ)(Z_{16b}) ;
X₁ est N ou C(Z₁),
X₂ est N ou C(Z₂),
X₃ est N ou C(Z₃),
X₄ est N ou C(Z₄),
X₅ est N ou C(Z₅), et
X₆ est N ou C(Z₆) ;
Z₁₁ à Z₁₆, Z₁₁ₐ à Z₁₆ₐ, Z_{11b} à Z₁₆b, et Z₁ à Z₆ sont chacun indépendamment un hydrogène, ou un site de liaison à chacun de R₁₁, R₁₂, et R₁₃ dans les Formules 1A à 1D ; et
* est un site de liaison à Si dans les Formules 1A à 1D, et, où
Ar₁ à Ar₃ sont de préférence chacun indépendamment un groupe dérivé d'un composé choisi parmi benzène, pyridine, pyrazine, pyrimidine, pyridazine, triazine, furane, thiophène, pyrrole, imidazole, triazole, cyclohexane, tétrahydro-2H-pyrane, pipéridine, tétrahydro-2H-thiopyrane, (2Z,4Z,6Z)-oxépine, (2Z,4Z,6Z)-1H-azépine, et (2Z,4Z,6Z)-thiépine.

5. Composé cyclique condensé selon l'une quelconque des revendications 1 à 4, dans lequel
R1 à R₈ sont chacun indépendamment choisis parmi hydrogène, deutérium, -F, -Cl, -Br, - I, groupe cyano, et un groupe substitué ou non substitué choisi parmi : groupe méthyle, groupe éthyle, groupe propyle, groupe isobutyle, groupe sec-butyle, groupe tert-butyle, groupe pentyle, groupe iso-amyle, groupe hexyle et groupe phényle ;
R₉ est choisi parmi
hydrogène, deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₂₀, et groupe alkoxy C₁-C₂₀ ;
groupe alkyle C₁-C₂₀ et groupe alkoxy C₁-C₂₀, chacun étant substitué par au moins un élément choisi parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe phényle, groupe pyridinyle, groupe pyrimidinyle, et groupe triazinyle ;
groupe cyclopentyle, groupe cyclohexyle, groupe cyclopentényle, groupe cyclohexényle, groupe cycloheptényle, groupe pipéridinyle, groupe tétrahydro-2H-pyranyle, groupe tétrahydro-2H-thiopyranyle, groupe phényle, groupe fluorényle, groupe dibenzosilolyle, groupe pyrrolyle, groupe imidazolyle, groupe pyrazolyle, groupe pyridinyle, groupe pyrazinyle, groupe pyrimidinyle, groupe pyridazinyle, groupe isoindolyle, groupe indolyle, groupe furanyle, groupe thiophényle, groupe thiazolyle, groupe isothiazolyle, groupe isoxazolyle, groupe oxazolyle, groupe triazolyle, groupe tétrazolyle, groupe oxadiazolyle, groupe triazinyle, groupe carbazolyle, groupe dibenzofuranyle, et groupe dibenzothiophényle ; et
groupe cyclopentyle, groupe cyclohexyle, groupe cyclopentényle, groupe cyclohexényle, groupe cycloheptényle, groupe pipéridinyle, groupe tétrahydro-2H-pyranyle, groupe tétrahydro-2H-thiopyranyle, groupe phényle, groupe fluorényle, groupe dibenzosilolyle, groupe pyrrolyle, groupe imidazolyle, groupe pyrazolyle, groupe pyridinyle, groupe pyrazinyle, groupe pyrimidinyle, groupe pyridazinyle, groupe isoindolyle, groupe indolyle, groupe furanyle, groupe thiophényle, groupe thiazolyle, groupe isothiazolyle, groupe isoxazolyle, groupe oxazolyle, groupe triazolyle, groupe tétrazolyle, groupe oxadiazolyle, groupe triazinyle, groupe carbazolyle, groupe dibenzofuranyle, et groupe dibenzothiophényle, chacun étant substitué par au moins un élément choisi parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₂₀, groupe alkoxy C₁-C₂₀, groupe cyclopentyle, groupe cyclohexyle, groupe cyclopentényle, groupe cyclohexényle, groupe cycloheptényle, groupe pipéridinyle, groupe tétrahydro-2H-pyranyle, groupe tétrahydro-2H-thiopyranyle, groupe phényle, groupe fluorényle, groupe dibenzosilolyle, groupe pyrrolyle, groupe imidazolyle, groupe pyrazolyle, groupe pyridinyle, groupe pyrazinyle, groupe pyrimidinyle, groupe pyridazinyle, groupe isoindolyle, groupe indolyle, groupe furanyle, groupe thiophényle, groupe thiazolyle, groupe isothiazolyle, groupe isoxazolyle, groupe oxazolyle, groupe triazolyle, groupe tétrazolyle, groupe oxadiazolyle, groupe triazinyle, groupe carbazolyle, groupe dibenzofuranyle, groupe dibenzothiophényle, et -N(Q₃₁)(Q₃₂) ;
où Q₁, Q₂, Q₃₁, et Q₃₂ sont chacun indépendamment choisis parmi
hydrogène, groupe alkyle C₁-C₂₀, groupe alkoxy C₁-C₂₀ , groupe cyclopentyle, groupe cyclohexyle, groupe cyclopentényle, groupe cyclohexényle, groupe cycloheptényle, groupe pipéridinyle, groupe tétrahydro-2H-pyranyle, groupe tétrahydro-2H-thiopyranyle, groupe phényle, groupe fluorényle, groupe dibenzosilolyle, groupe pyrrolyle, groupe imidazolyle, groupe pyrazolyle, groupe pyridinyle, groupe pyrazinyle, groupe pyrimidinyle, groupe pyridazinyle, groupe isoindolyle, groupe indolyle, groupe furanyle, groupe thiophényle, groupe thiazolyle, groupe isothiazolyle, groupe isoxazolyle, groupe oxazolyle, groupe triazolyle, groupe tétrazolyle, groupe oxadiazolyle, groupe triazinyle, groupe carbazolyle, groupe dibenzofuranyle, et groupe dibenzothiophényle ; et
groupe cyclopentyle, groupe cyclohexyle, groupe cyclopentényle, groupe cyclohexényle, groupe cycloheptényle, groupe pipéridinyle, groupe tétrahydro-2H-pyranyle, groupe tétrahydro-2H-thiopyranyle, groupe phényle, groupe fluorényle, groupe dibenzosilolyle, groupe pyrrolyle, groupe imidazolyle, groupe pyrazolyle, groupe pyridinyle, groupe pyrazinyle, groupe pyrimidinyle, groupe pyridazinyle, groupe isoindolyle, groupe indolyle, groupe furanyle, groupe thiophényle, groupe thiazolyle, groupe isothiazolyle, groupe isoxazolyle, groupe oxazolyle, groupe triazolyle, groupe tétrazolyle, groupe oxadiazolyle, groupe triazinyle, groupe carbazolyle, groupe dibenzofuranyle, et groupe dibenzothiophényle, chacun étant substitué par au moins un élément choisi parmi deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₂₀, groupe alkoxy C₁-C₂₀, groupe cyclopentyle, groupe cyclohexyle, groupe cyclopentényle, groupe cyclohexényle, groupe cycloheptényle, groupe pipéridinyle, groupe tétrahydro-2H-pyranyle, groupe tétrahydro-2H-thiopyranyle, groupe phényle, groupe fluorényle, groupe dibenzosilolyle, groupe pyrrolyle, groupe imidazolyle, groupe pyrazolyle, groupe pyridinyle, groupe pyrazinyle, groupe pyrimidinyle, groupe pyridazinyle, groupe isoindolyle, groupe indolyle, groupe furanyle, groupe thiophényle, groupe thiazolyle, groupe isothiazolyle, groupe isoxazolyle, groupe oxazolyle, groupe triazolyle, groupe tétrazolyle, groupe oxadiazolyle, groupe triazinyle, groupe carbazolyle, groupe dibenzofuranyle, groupe dibenzothiophényle.

6. Composé cyclique condensé selon la revendication 5, dans lequel
R₁ à R₈ sont chacun indépendamment choisis parmi hydrogène, deutérium, -F, -Cl, -Br, - I, groupe cyano, et un groupe substitué ou non substitué choisi parmi : groupe méthyle, groupe éthyle, groupe propyle, groupe isobutyle, groupe sec-butyle, groupe tert-butyle, groupe pentyle, groupe iso-amyle, groupe hexyle et groupe phényle ;
R₉ est choisi parmi hydrogène, deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₂₀, groupe alkoxy C₁-C₂₀, groupe phényle, groupe pyridinyle, et groupe pyrimidinyle.

7. Composé cyclique condensé selon l'une quelconque des revendications 1 à 6, dans lequel
R₁₁ à R₁₃ sont chacun indépendamment hydrogène, deutérium, -F, -Cl, -Br, -I, groupe hydroxyle, groupe cyano, groupe nitro, groupe amino, groupe amidino, groupe hydrazine, groupe hydrazone, groupe acide carboxylique ou un sel de celui-ci, groupe acide sulfonique ou un sel de celui-ci, groupe acide phosphorique ou un sel de celui-ci, groupe alkyle C₁-C₁₀, groupe alkoxy C₁-C₁₀, -N(Q₁)(Q₂), où Q₁ et Q₂ sont chacun indépendamment choisis parmi groupe alkyle C₁-C₅, et un groupe représenté par les Formules 4-1 à 4-49 et 4-61 à 4-74 : 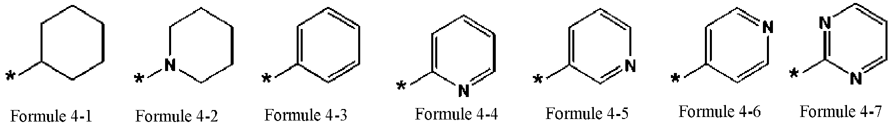 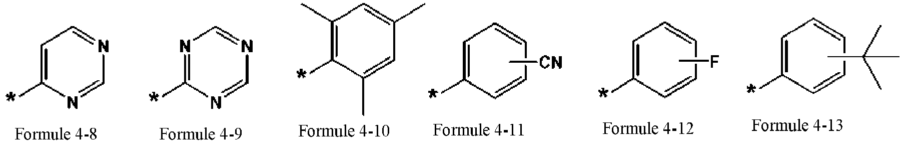 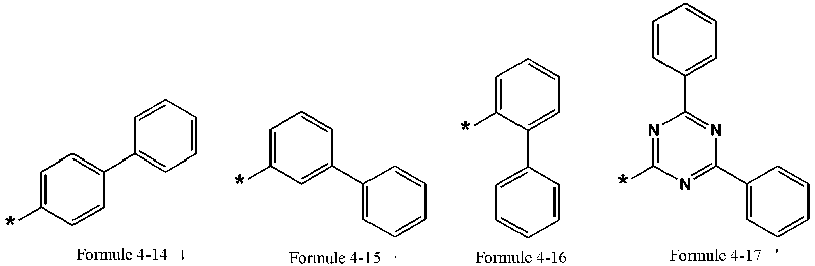 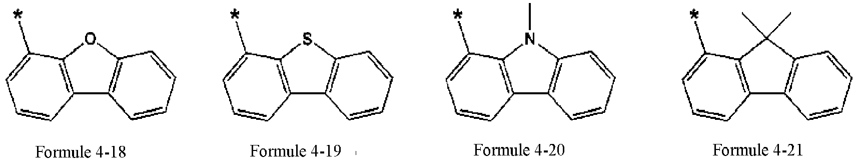 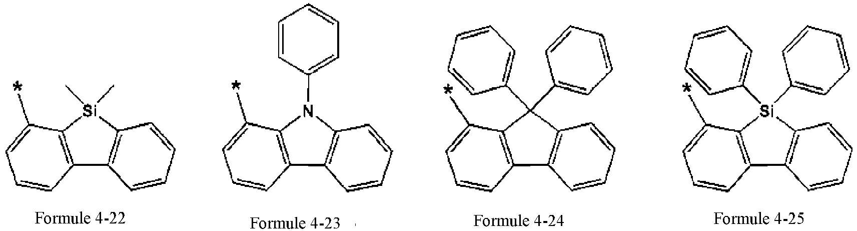 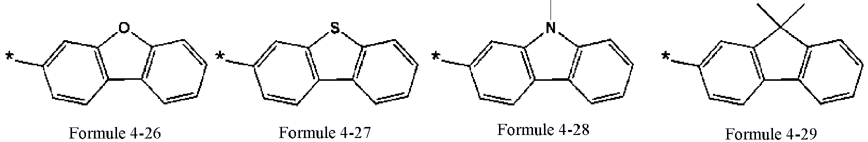 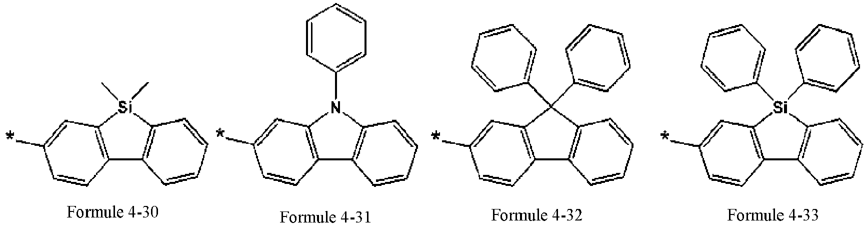 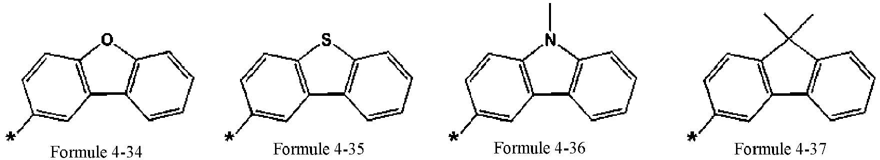 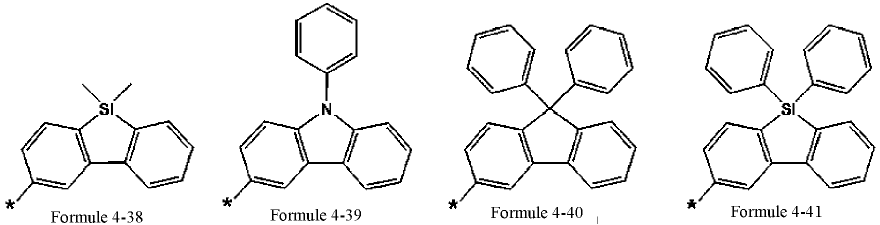 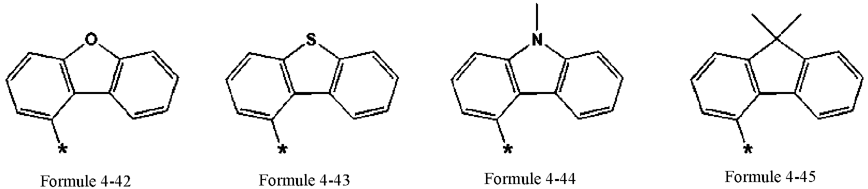 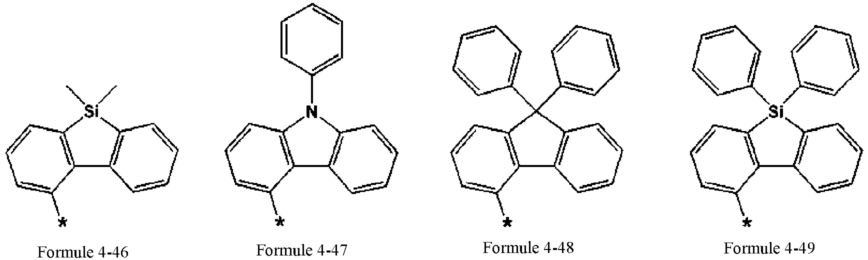 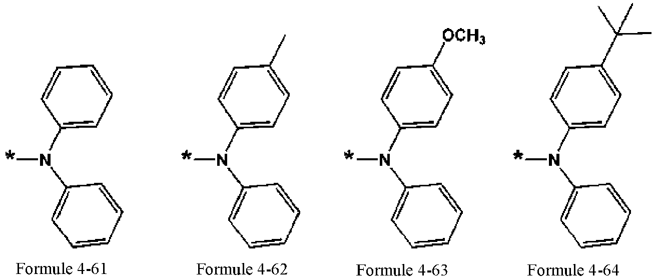 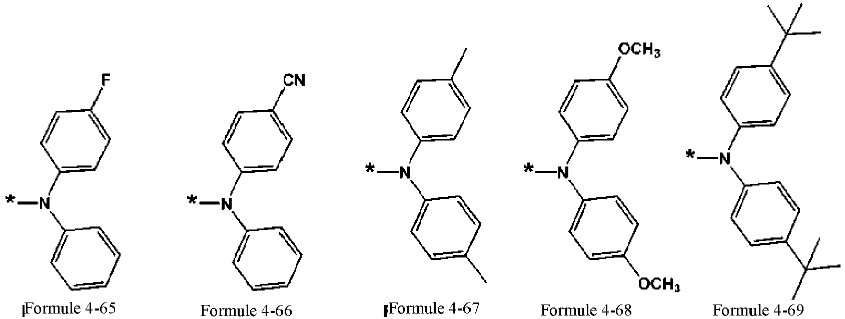 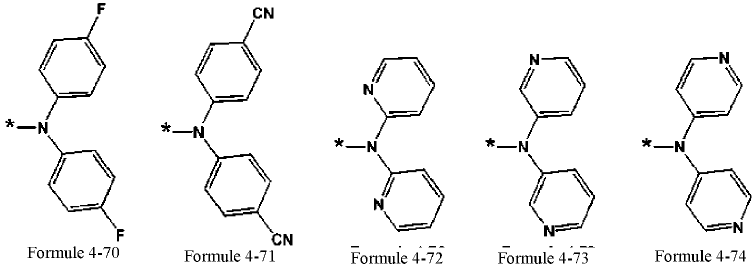 où dans les Formules 4-1 à 4-49 et 4-61 à 4-74, * indique un site de liaison à chacun de Ar₁ à Ar₃ dans les Formules 1A à 1D.

8. Composé cyclique condensé selon l'une quelconque des revendications 1 à 7, dans lequel
*-Ar1-(R₁₁)a₁ dans les Formules 1A à 1D est un groupe représenté par n'importe quelle Formule choisie parmi les Formules 11-1 à 11-47,
*-Ar₂-(R₁₂)ₐ₂ dans les Formules 1A à 1D est un groupe représenté par n'importe quelle Formule choisie parmi les Formules 12-1 à 12-47, et
*-Ar₃-(R₁₃)ₐ₃ dans les Formules 1A à 1D est un groupe représenté par n'importe quelle Formule choisie parmi les Formules 13-1 à 13-47 : 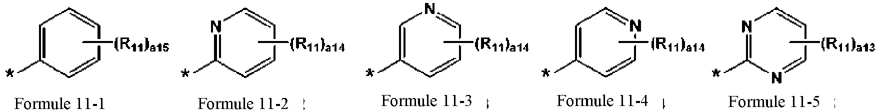 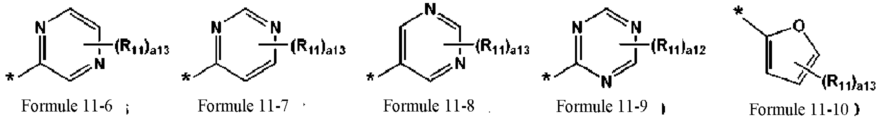 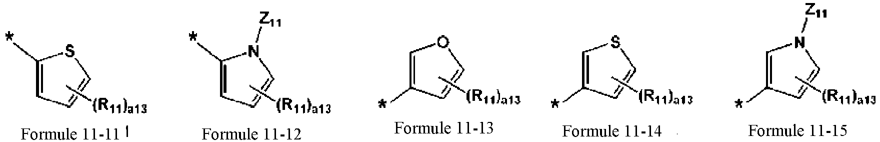 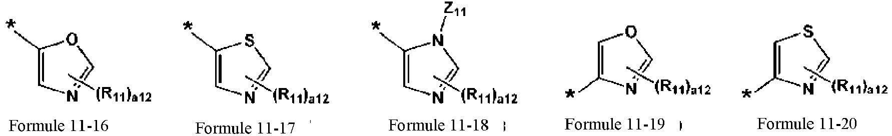 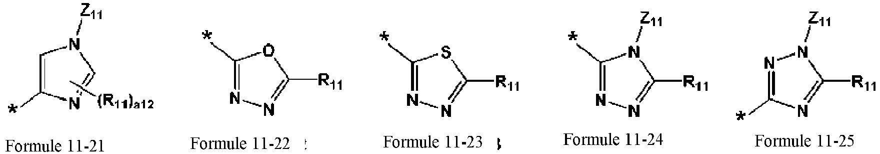 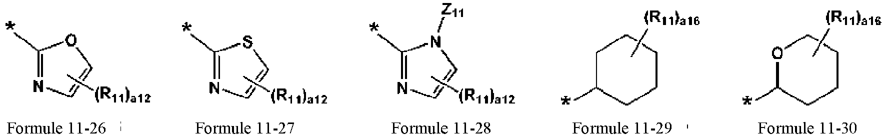 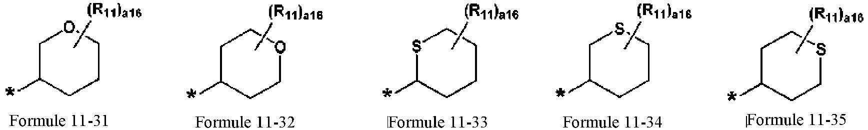 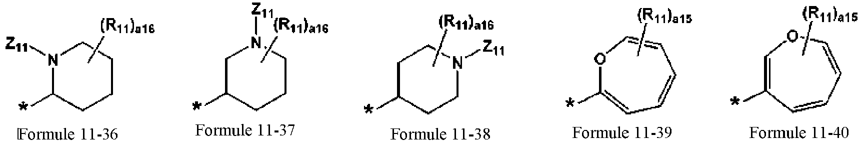 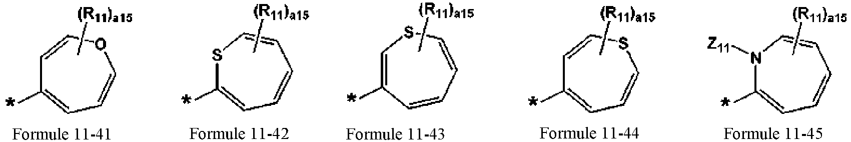 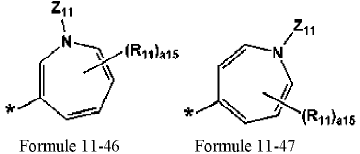 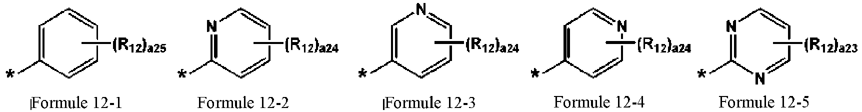 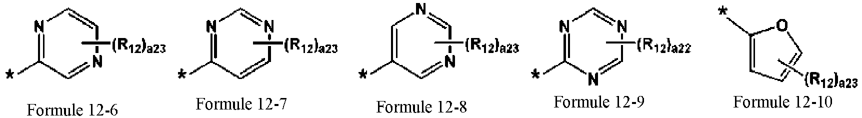 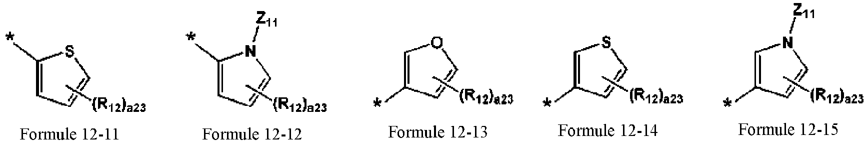 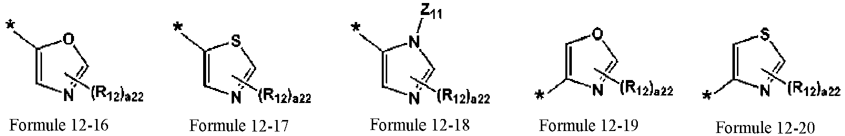 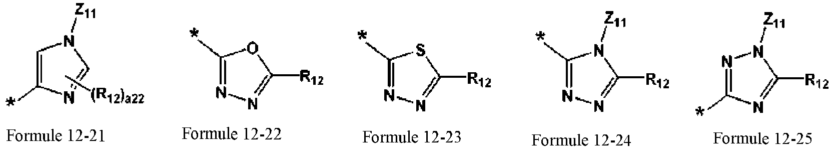 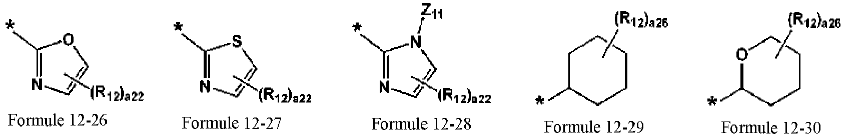 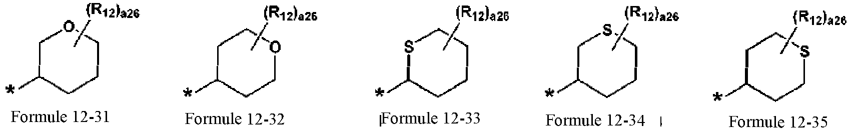 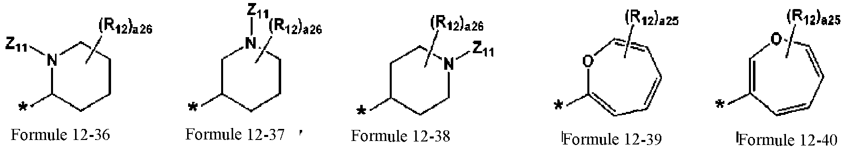 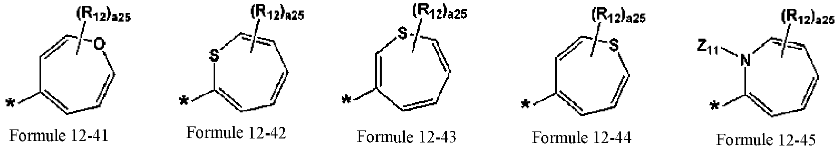 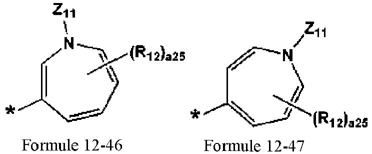 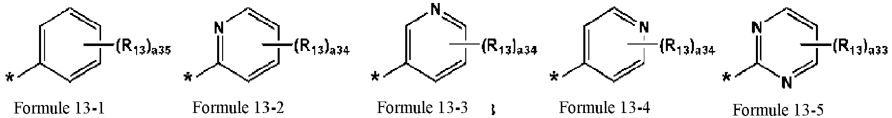 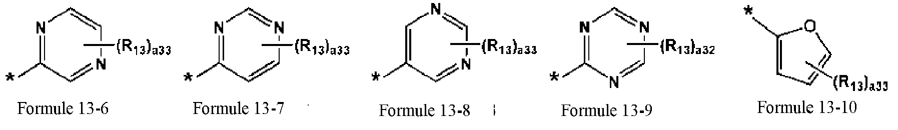 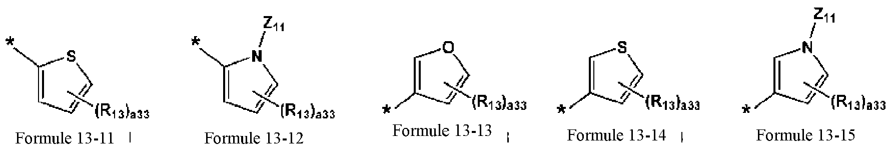 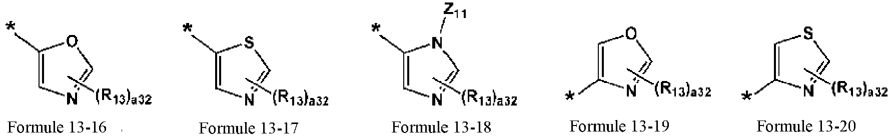 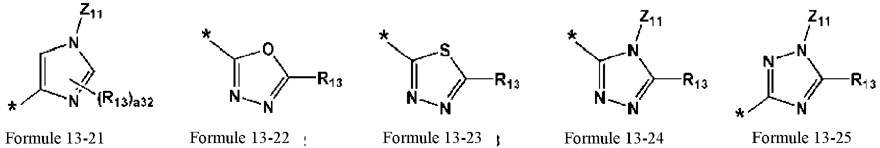 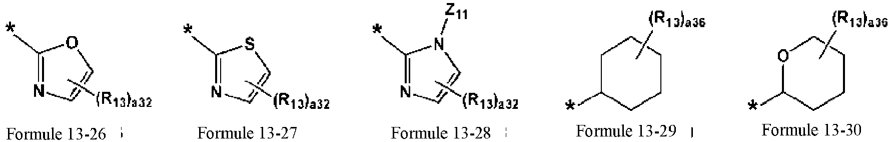 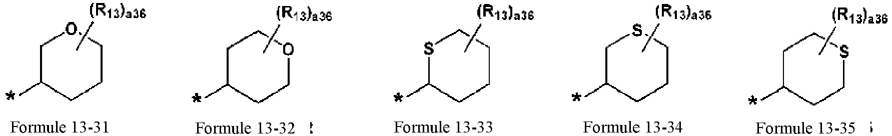 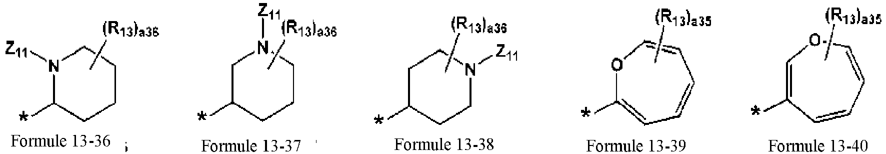 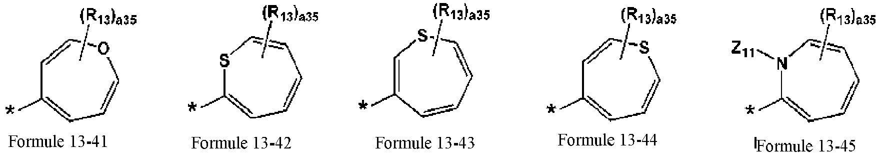 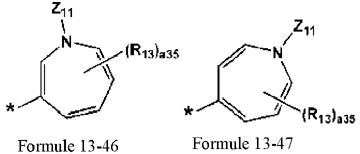 où dans les Formules 11-1 à 11-47, 12-1 à 12-47, et 13-1 à 13-47,
R₁₁ to R₁₃ sont les mêmes que décrits dans la revendication 1,
a16, a26, et a36 sont chacun indépendamment un nombre entier choisi parmi 1, 2, 3, 4, 5, et 6,
a15, a25, et a35 sont chacun indépendamment un nombre entier choisi parmi 1, 2, 3, 4, et 5,
a14, a24, et a34 sont chacun indépendamment un nombre entier choisi parmi 1, 2, 3, et 4,
a13, a23, et a33 sont chacun indépendamment un nombre entier choisi parmi 1, 2, et 3,
a12, a22, et a32 sont chacun indépendamment un nombre entier choisi entre 1 et 2, et
* est un site de liaison à Si dans les Formules 1A à 1D.

9. Composé cyclique condensé selon la revendication 8, dans lequel
Ar₁ dans les Formules 1A à 1D est un groupe représenté par n'importe quelle Formule choisie parmi les Formules 11-1 à 11-9 ci-dessus, et
Ar₃ est un groupe représenté par n'importe quelle Formule choisie parmi les Formules 13-1 à 13-9 ci-dessus.

10. Composé cyclique condensé selon l'une quelconque des revendications 1 à 9, dans lequel
dans les Formules 1A à 1D, Ar₁ = Ar₂ = Ar₃, R₁₁ ≠ R₁₂, et R₁₂ n'est pas hydrogène.

11. Composé cyclique condensé selon l'une quelconque des revendications 1 à 10, dans lequel
le composé cyclique condensé est représenté par une Formule choisie parmi les Formules 1A(1) à 1D(8) : 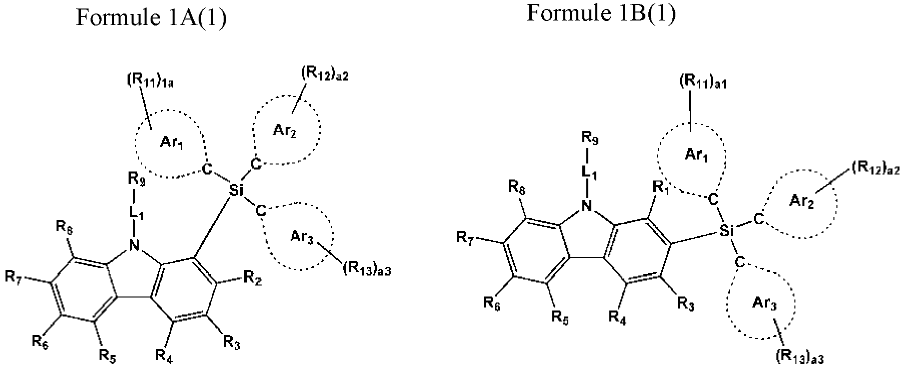 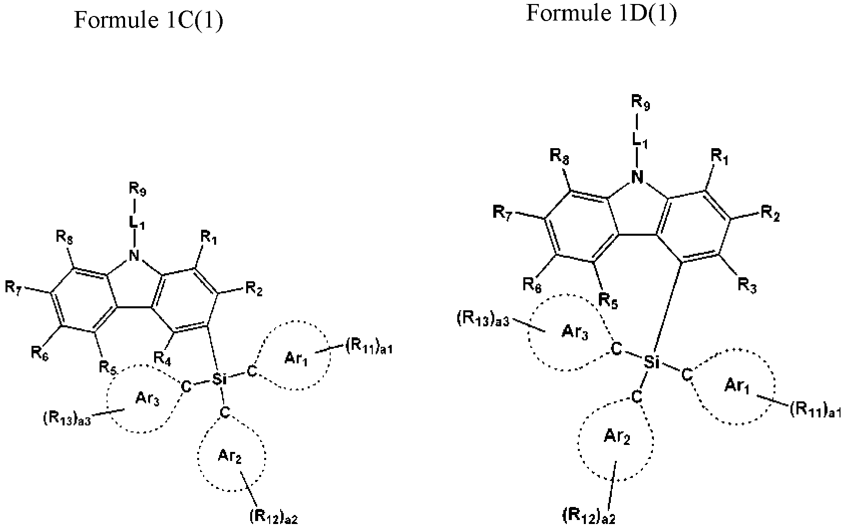 où L₁, Ar₁ à Ar₃, R₁ à R₉, R₁₁ à R₁₃, et a1 à a3 dans les Formules 1A(1) à 1D(1) sont les mêmes que décrits dans la revendication 1.

12. Composé cyclique condensé selon la revendication 1, dans lequel le composé cyclique condensé est l'un des Composés 1 à 100, 114 à 154, 156, 158, 159, 162 à 177, 184 à 195, 200 à 213, 220 à 231, 233, 233 à 235, 237 à 250, 257 à 268, 270, 271, 273 à 285, 289 à 305, 309 à 323, 325, 327, 328, 330 et 332 :

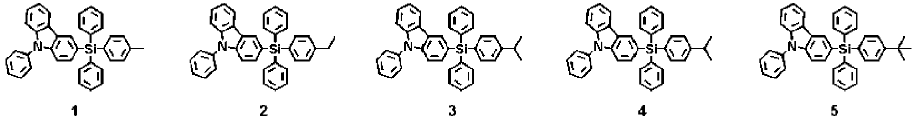

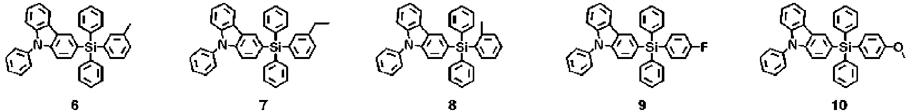

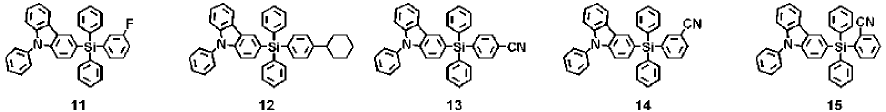

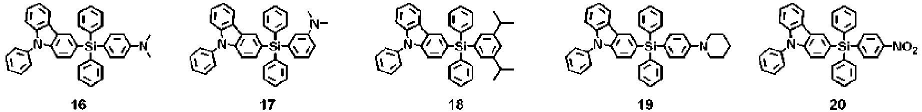

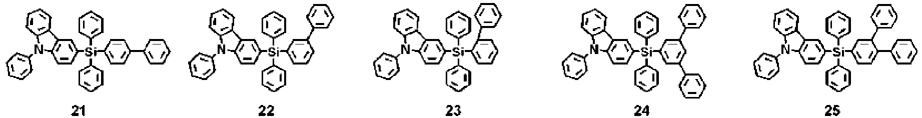

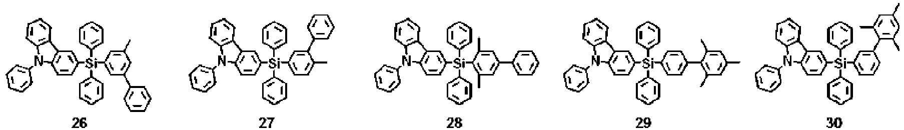

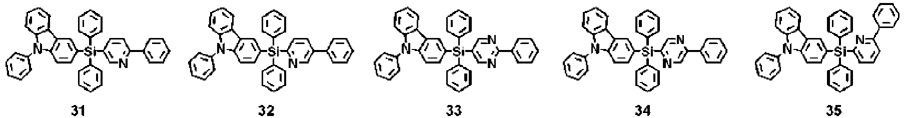

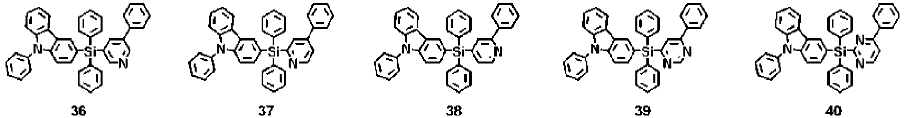

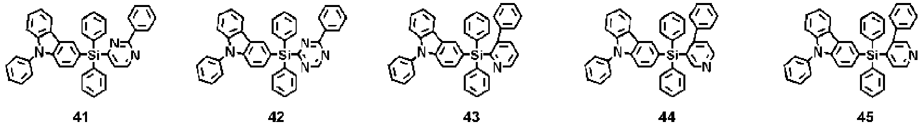

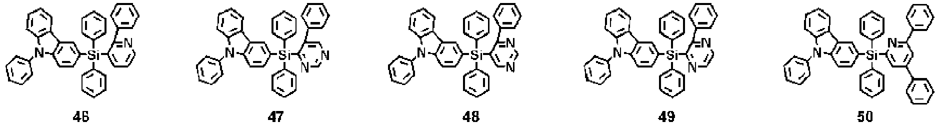

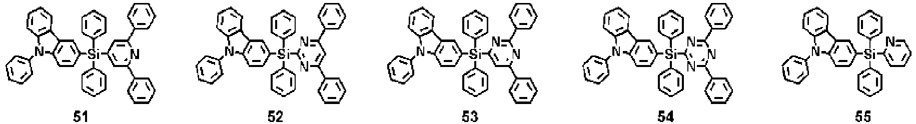

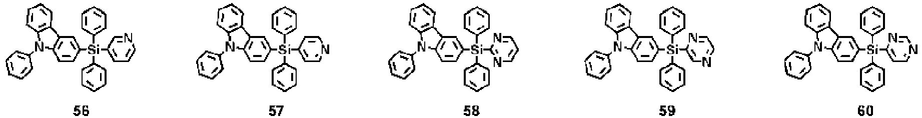

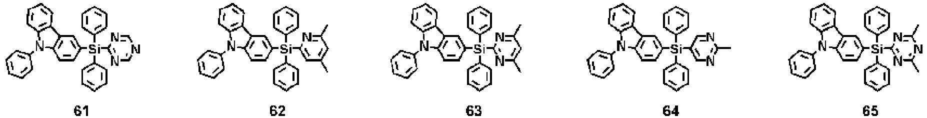

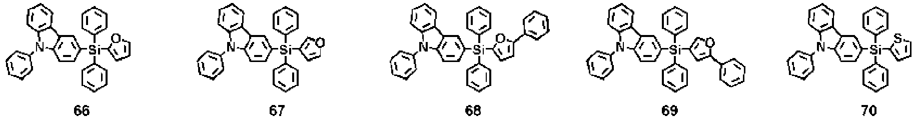

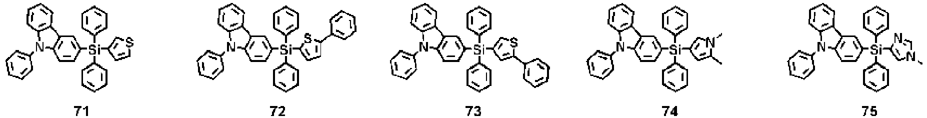

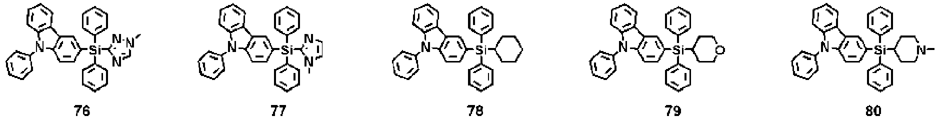

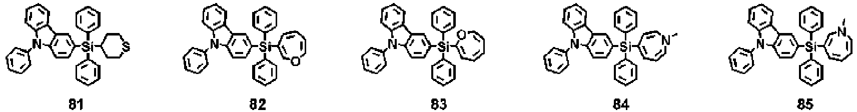

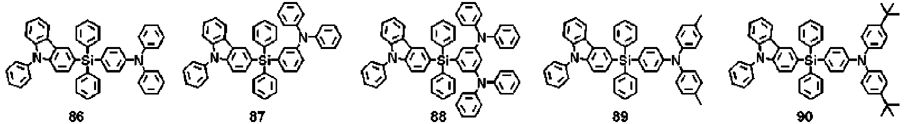

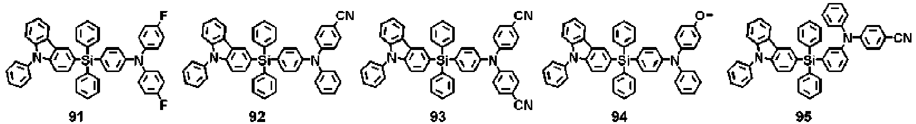

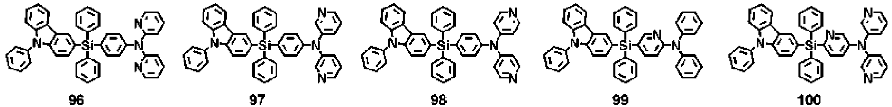

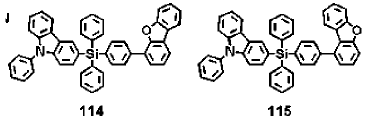

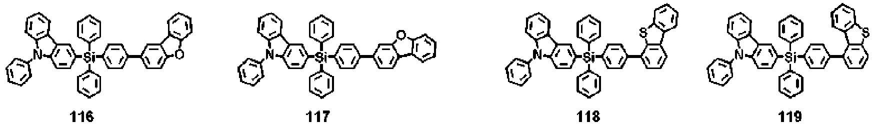

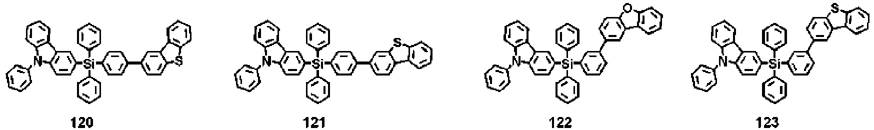

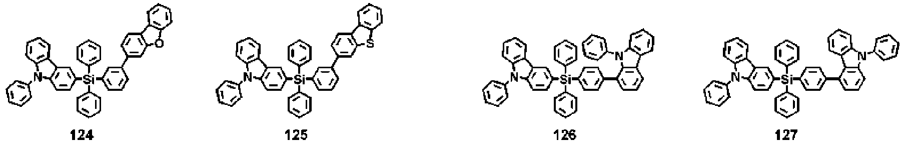

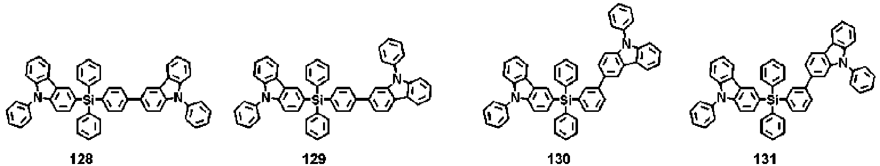

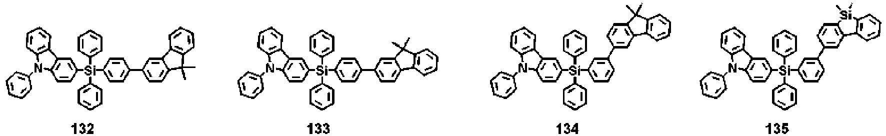

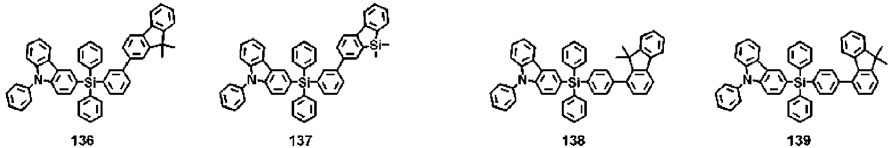

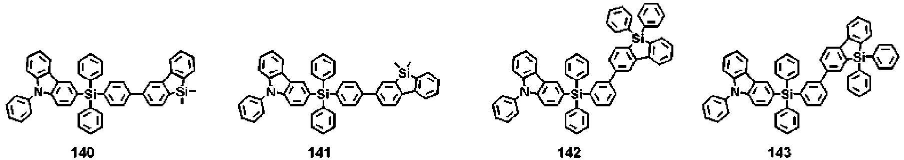

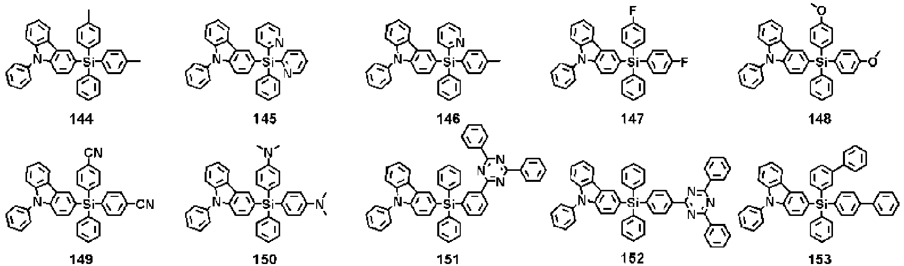

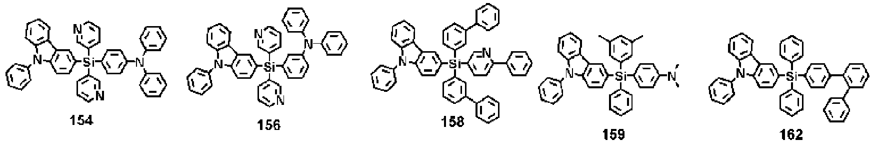

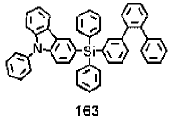

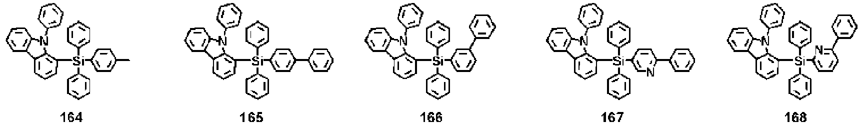

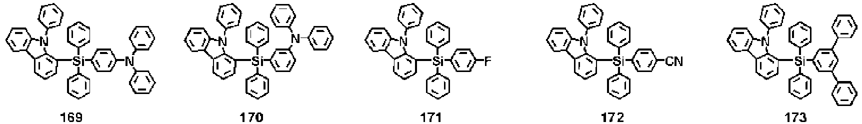

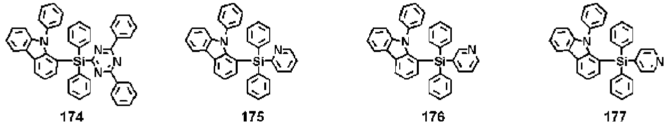

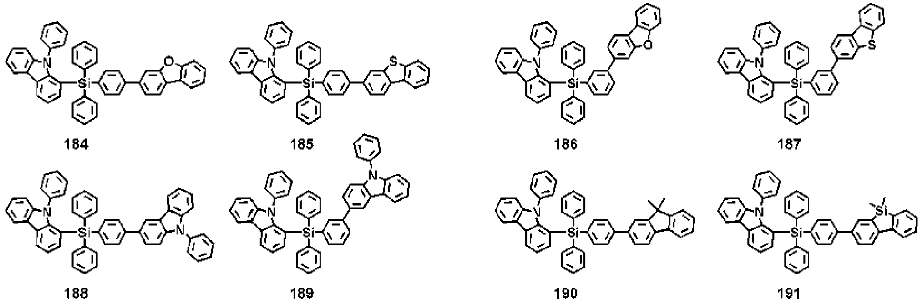

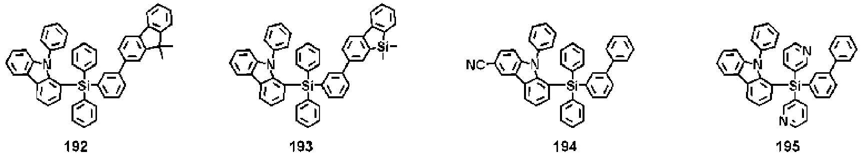

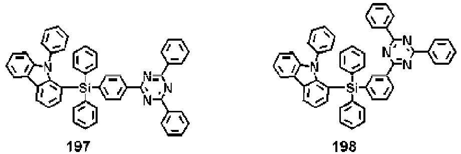

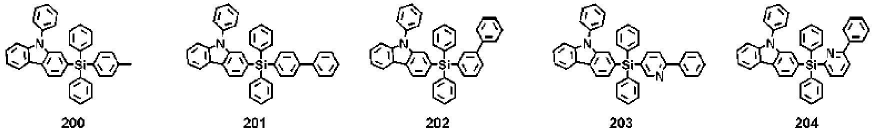

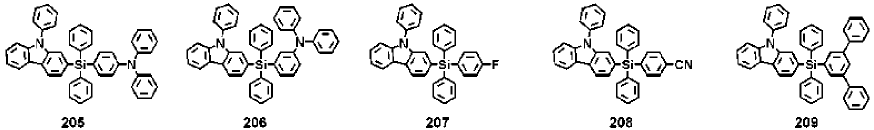

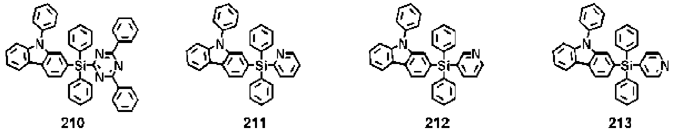

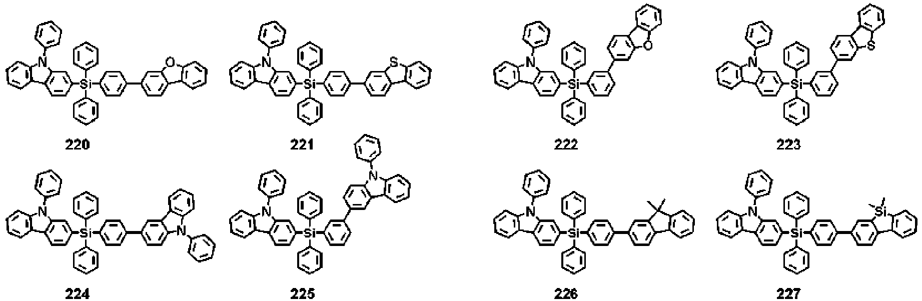

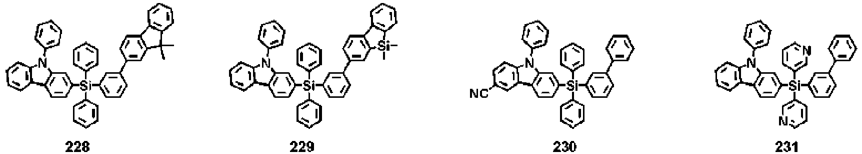

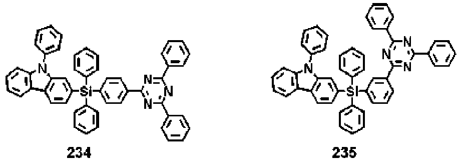

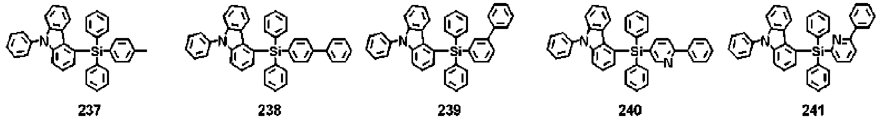

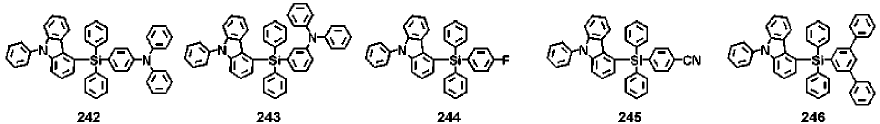

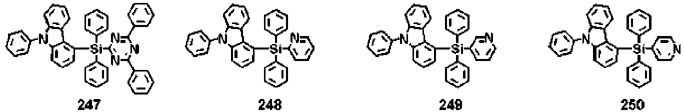

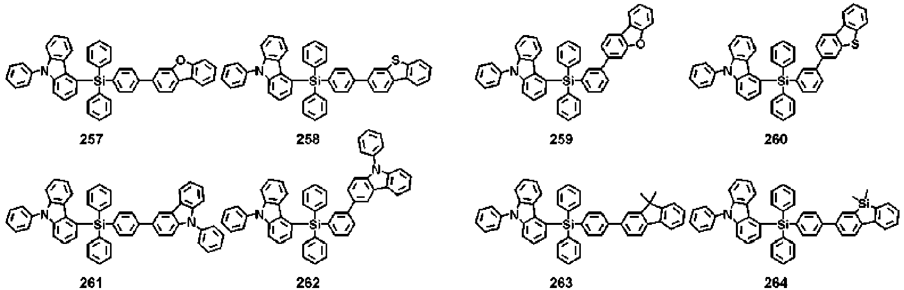

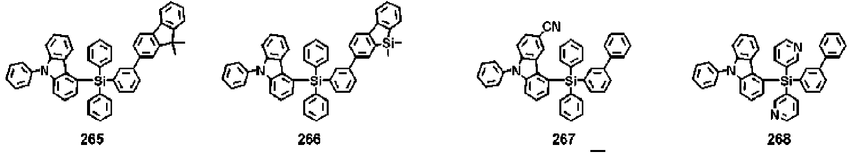

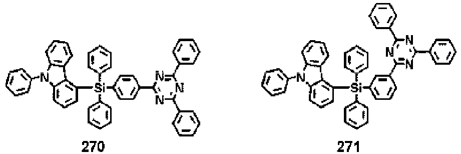

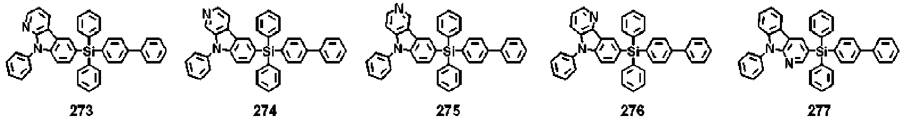

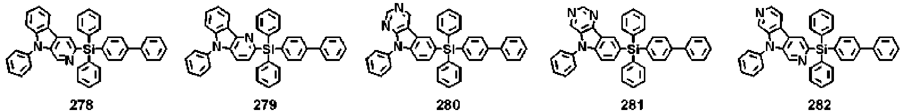

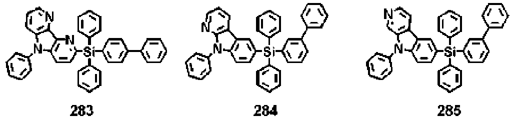

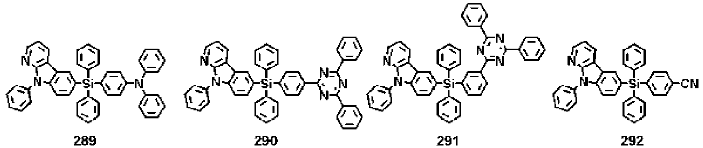

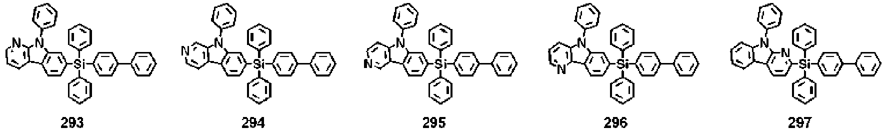

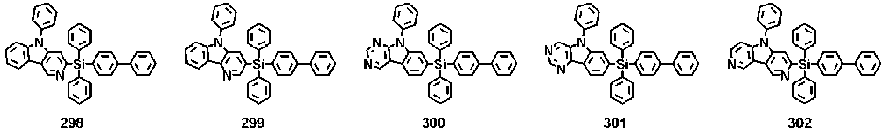

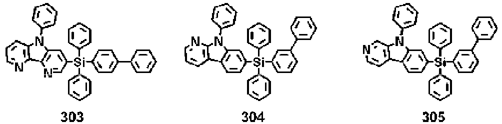

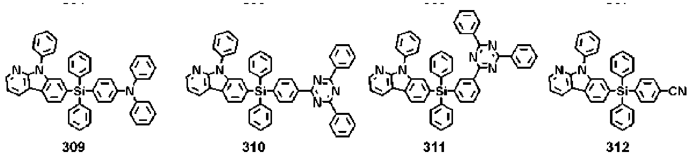

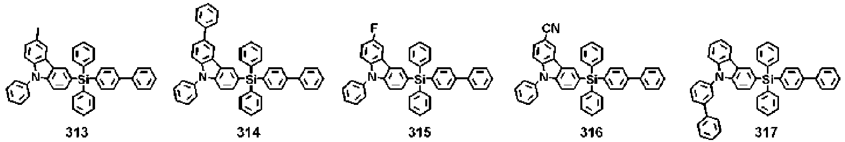

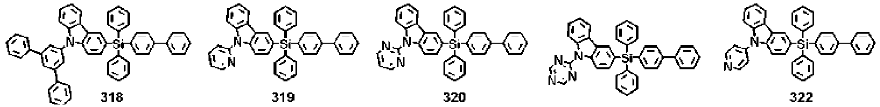

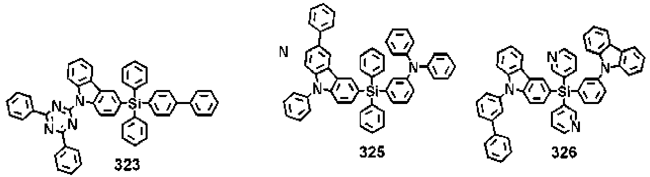

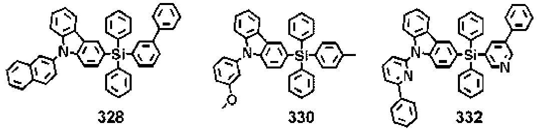

13. Dispositif électroluminescent organique comprenant :
une première électrode ;
une seconde électrode ; et
une couche organique interposée entre la première électrode et la seconde électrode, la couche organique comprenant une couche d'émission et comprenant en outre au moins un composé cyclique condensé représenté par l'une des Formules 1A à 1D selon l'une quelconque des revendications 1 à 12.

14. Dispositif électroluminescent organique selon la revendication 13, dans lequel
la couche d'émission comprend le ou les composés cycliques condensés, la couche d'émission comprenant en outre éventuellement un dopant phosphorescent, et le ou les composés cycliques condensés dans la couche d'émission agissent comme hôte.
